(19) Europäisches Patentamt / European Patent Office / Office européen des brevets

(11) EP 3 878 837 A1

(12) **EUROPEAN PATENT APPLICATION**

(43) Date of publication:
**15.09.2021 Bulletin 2021/37**

(21) Application number: **20162558.9**

(22) Date of filing: **11.03.2020**

(51) Int Cl.:
***C07C 235/64*** *(2006.01)* ***C07C 66/00*** *(2006.01)*
***C07C 229/38*** *(2006.01)* ***C07C 233/51*** *(2006.01)*
***C07C 275/42*** *(2006.01)* ***C07C 309/51*** *(2006.01)*
***C07C 317/46*** *(2006.01)* ***C07C 323/56*** *(2006.01)*
***C07D 213/79*** *(2006.01)* ***C07D 235/18*** *(2006.01)*
***C07D 257/04*** *(2006.01)* ***A61K 31/196*** *(2006.01)*
***A61P 3/00*** *(2006.01)* ***A61P 35/00*** *(2006.01)*
***A61P 37/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **OM Pharma SA**
**1217 Meyrin (CH)**

(72) Inventors:
- **BAUER, Jacques**
  **1162 St-Prex (CH)**
- **MARTIN, Olivier**
  **45160 Saint Hilaire Saint Mesmin (FR)**

(74) Representative: **Lecca, Patricia S.**
**Cabinet Lecca**
**21, rue de Fécamp**
**75012 Paris (FR)**

(54) **2,5- OR 2,6-DISUBSTITUTED HYDROQUINONE DERIVATIVES WITH AT LEAST ONE CARBOXY, SULFO OR AMIDO GROUP USEFUL AS MEDICAMENTS**

(57) The invention provides hydroquinone derivatives of formula (I), processes of preparation, as well as pharmaceutical compositions and methods of treating and/or preventing e.g. autoimmune, immunological, rheumatology, vascular, ophthalmologic, fibrotic, metabolic and gastro-intestinal disorders, neuroinflammatory and neurodegenerative diseases, neoplasms and cancer associated disorders, hormone related diseases and immunological disorders resulting from viral and bacterial infectious diseases and complications thereof.

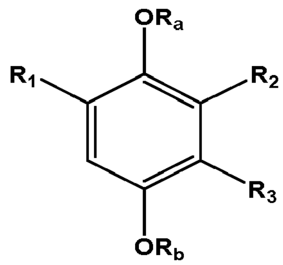

(I)

wherein: $R_a$, $R_b$ = H, acyl or aryl alkyl; $R_1$ = $COOR_4$, $(CH_2)_nCOOR_4$, $SO_3H$, $(CH_2)_nSO_3H$ or CONH-$R_{10}$; $R_2$ and $R_3$ = H or $R_5$, with the proviso that when one of $R_2$ and $R_3$ is $R_5$, the other is H; $R_4$ = H, alkyl or aryl alkyl; $R_5$ = $R_6$, $R_7$ or $R_8$; $R_6$ = CONH-$R_9$, $CONHCOR_9$, $CONH(CH_2)_n$- $R_9$, $CONHCH(COOR_4)(CH_2)_k$ $R_9$ or heteroaryl-$R_9$; k = 0-4; $R_7$ = optionally substituted benzoheteroaryl ; $R_8$ = $(CH_2)_mX(CH_2)_p$ $R_9$; X = O, S, $SO_2$, NH, NAc or $N(CH_2)_q$ $R_9$; $R_9$ = optionally substituted aryl, heteroaryl or cycloalkyl ; $R_{10}$ = optionally substituted aryl or heteroaryl ; n, m, p and q = independently 1-4.

EP 3 878 837 A1

## Description

### FIELD OF THE INVENTION

This invention relates generally to novel hydroquinone derivatives, their preparation and to methods of treating disorders by administration of such compounds to a warm-blooded animal in need thereof. The present invention also relates to compounds that are useful in the prevention and/or treatment of autoimmune, immunological, rheumatology, vascular disorders, ophthalmologic disorders, fibrotic disorders, metabolic and gastro-intestinal disorders, neuroinflammatory and neurodegenerative diseases, neoplasms and cancer associated disorders, hormone related diseases and immunological disorders resulting from viral and bacterial infectious diseases, and complications thereof.

### BACKGROUND OF THE INVENTION

Some hydroquinone-based derivatives like for example, 2,5-dihydroxybenzenesulfonic acid derivatives and specifically calcium dobesilate, ethamsylate and persilate are known in the art as active agents for the treatment of male sexual dysfunction and other vascular disorders of endothelial origin, both alone and in combination with other agents. For example, US Patent No. 6,147,112 describes a method for the use of 2,5-dihydroxybenzenesulfonic acid derivatives, preferably calcium dobesilate, ethamsylate and persilate. Calcium dobesilate or hydroquinone calcium sulfonate, with the chemical name 2,5-dihydroxybenzenesulfonic acid calcium salt, is being sold as Dexium (Delalande) and Doxium (Carrion), and a process for its preparation is described in US Patent No. 3,509,207.
There are many drugs that contain phenol or catechol groups which suffer from premature metabolism at the hydroxy group during absorption after oral administration. Previous efforts to protect the hydroxy groups have been generally unsuccessful as the protecting groups employed are either too labile (O=COR or O=CR) or too stable ($CH_3$). It is thus an object of the present invention to provide novel derivatives of hydroxy quinones, their pharmaceutically acceptable salts and formulations. These novel hydroxyquinones derivatives have particularly potent anti-fibrotic, anti-inflammatory and anti-angiogenic properties.
International publication WO2018160618A1 discloses certain substituted hydroquinones, 1,4-quinones, catechols, 1,2-quinones, anthraquinones, and anthrahydroquinones for use as redox mediators in emerging technologies, such as in mediated fuel cells or organic-mediator flow batteries.
Richter et al, in Synthesis 1976; 1976(3): 192-194 disclose Bis-N-arylcarbamates and 2,5-Dihydroxy-3,6-bis[N-arylcarboxamido]-1,4-benzoquinones and their preparation from 2,5-Dihydroxy-1,4-benzoquinone and Aryl Isocyanates.
Japanese publication JPS5126839 A discloses Benzanilides I (R = H, OH) as possessing antitubercular, analgesic, anti-inflammatory, and uric acid-excreting activities:

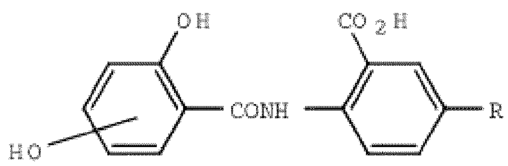

Benzanilides I

US granted patent US 3,973,038 also discloses benzoylaniline compounds having general formula:

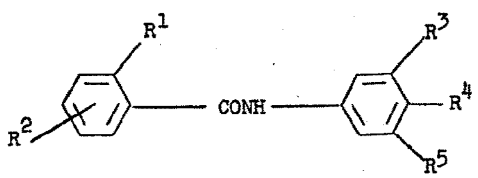

wherein (R1 = OH, acyloxy; R2 = H, OH, lower alkyl, halo, acyloxy; R3 = Cl, Br, lower alkyl; R4 = OH, NH2, lower alkoxy, acyloxy; R5 = H, halo, CO2H, lower alkyl, acyloxy) having in-vivo enzyme inhibition properties, in particular, histamine deacetylase and xanthine oxidase
None of the prior art documents however disclose the novel class of hydroquinone derivative compounds of the present invention, which are particularly useful for the prevention and/or treatment of a variety of diseases including autoimmune, immunological, rheumatology, vascular disorders, ophthalmologic disorders, fibrotic disorders, metabolic and gastro-intestinal disorders, neuroinflammatory and neurodegenerative diseases, neoplasms and cancer associated disorders, hormone related diseases and immunological disorders resulting from viral and bacterial infectious diseases, and complications thereof.

The invention also relates to the treatment, prevention, and reduction of metabolic disorders, such as diabetes and obesity. As the levels of blood glucose rise postprandially, insulin is secreted and stimulates cells of the peripheral tissues (skeletal muscles and fat) to actively take up glucose from the blood as a source of energy. Loss of glucose homeostasis as a result of faulty insulin secretion or action typically results in metabolic disorders such as diabetes, which may be co-triggered or further exacerbated by obesity. Because these conditions are often fatal, strategies to restore adequate glucose clearance from the bloodstream are required. Metabolic disorders, particularly glucose and lipid regulatory disorders, are becoming increasingly prevalent as the populations in industrialized nations age and sedentary lifestyles become more common. Such disorders are frequently interrelated and are often predictors or results of each other.

For example, diabetes is caused by a combination of insulin resistance and defective secretion of insulin by pancreatic-β cells. Individuals with insulin resistance often have abdominal obesity, dyslipidemia, hypertension, glucose intolerance and a prothrombitic state (Metabolic syndrome). Correspondingly, obese individuals as a whole are at higher risk for acquiring insulin resistance. The breakdown of a metabolic pathway thus can trigger myriad disorders such as hyperlipidemia, obesity, diabetes, insulin resistance, glucose intolerance, hyperglycemia, metabolic syndrome and hypertension which may in turn trigger further metabolic dysfunction resulting in systemic issues and putting individuals at risk for additional complications and premature morbidity. Glucose and lipid levels are regulated in part by the liver which plays a role in synthesizing, storing, secreting, transforming, and breaking down glucose, proteins and lipids. Disease or traumatic injury can greatly reduce the liver's ability to carry out these normal activities. Thus, most of the clinical manifestations of liver dysfunction stem from cell damage and impairment of the normal liver capacities. Liver dysfunction can result from genetic conditions, inflammatory disorders, toxins such as drugs and alcohol, immunological disorders, vascular disorders or metabolic conditions. Regardless of the cause, liver damage can have a systemic effect on the function of metabolic processes and the regulation of blood glucose and serum lipid levels, exacerbating chronic disease states and leading to increased risks for further disease and morbidity. Both elevated and reduced levels of blood glucose trigger hormonal responses designed to restore glucose homeostasis. Low blood glucose triggers the release of glucagon from pancreatic a-cells. High blood glucose triggers the release of insulin from pancreatic b-cells. ACTH and growth hormones released from the pituitary, act to increase blood glucose by inhibiting uptake by extrahepatic tissues. Glucocorticoids also act to increase blood glucose levels by inhibiting glucose uptake. Cortisol, the major glucocorticoid released from the adrenal cortex, is secreted in response to the increase in circulating ACTH. The adrenal medullary hormone, epinephrine, stimulates production of glucose by activating glycogenolysis in response to stressful stimuli. etabolic disorders that effect glucose and lipid metabolism such as hyperlipidemia, obesity, diabetes, insulin resistance, hyperglycemia, glucose intolerance, metabolic syndrome and hypertension have long term health consequences leading to chronic conditions including cardiovascular disease and premature morbidity. Such metabolic and cardiovascular disorders may be interrelated, aggravating or triggering each other and generating feedback mechanisms that are difficult to interrupt. Current pharmaceutical treatments for metabolic and cardiovascular disorders include combinations of lipid-lowering drugs, hypoglycemic drugs, anti-hypertensive agents, diet and exercise. However, complicated therapeutic regimens can cause polypharmacy problems of increased side effects, drug-drug interactions, failure of adherence, and increased medication errors. There is therefore a compelling, unmet need in the art to identify new compounds, formulations and methods to safely and effectively treat metabolic and cardiovascular disorders and conditions associated with metabolic disorders.

Examples of metabolic conditions include, but are not limited to, pain, wound healing, fever, neuroinflammatory and neurodegenerative conditions, inflammation, heat production, homeothermy, breakdown of triglycerides, glycolysis, Krebs cycle, fermentation, photosynthesis, metabolic rate, biotic and abiotic stress, secretions, oxidative stress, stress, neoplastic growth, skin condition, cardiovascular conditions, neuroinflammatory and neurodegenerative conditions, mental and behavioural disorders. Such processes or conditions can occur in a cell, group of cells, or an entire organism.

Recent progress in molecular medicine has led to certain improvements in diagnostics and treatment of neoplastic diseases. In spite of this partial success, these pathologies remain a considerable challenge. For certain types of cancers, the current therapy in some cases fails for several reasons. On the one hand, it is inherent resistance of tumour cells, their ability of constant mutation and therapy evasion, on the other hand it is also the heterogeneity of the tumour environment. It was shown that tumours of the same type highly differ for individual subjects from the viewpoint of their genomic profile which indicates the necessity of the so-called "personal" therapy. Even a bigger problem is the heterogeneity of mutations in the same tumour, as it has been recently shown for renal tumours, and this situation can be expected for other types of tumours as well. For this reason, it is necessary to search for new approaches and for an invariable intervention point(s) common for all or most malignant cells in the tumour and which preferably affects essential functions in cancer cells. It seems that such an intervention point could be at the metabolic level say for example mitochondria, i.e. organelles which are fundamental for the generation of energy necessary for all physiological as well as pathophysiological processes in cells. Although tumour cells use, from a major part, the so-called aerobic glycolysis

for energy generation, mitochondrial respiration (i.e. consumption of oxygen linked to ATP formation) is inherent to most (if not all) types of tumours. Hence there is an unmet and compelling need in the art to identify new compounds, formulations and methods to safely and effectively treat such neoplastic disorders.

## SUMMARY OF THE INVENTION

The present invention provides novel hydroquinone derivatives, novel process of preparation, and novel key intermediates.

The present invention also relates to pharmaceutical compositions comprising therapeutically effective amount of such hydroquinone derivatives and pharmaceutically acceptable excipient.

The invention further provides a method of treating and/or preventing angiogenic (vascular), inflammatory and/or fibrotic pathobiology, as they have anti-fibrotic, anti-inflammatory and anti-angiogenic effects. The present invention thus finally provides a method of treating and/or preventing a disease or disorder including immunological/rheumatology/vascular disorders, ophthalmologic disorders, fibrotic disorders, metabolic & gastro-intestinal disorders, neoplasms and cancer associated disorders comprising administering a subject in a need a therapeutically effective dose of the compounds and/or pharmaceutical compositions of the present invention as described above. In particular, the present invention provides a method of treating and/or preventing a disease or disorder autoimmune, immunological, rheumatology, vascular disorders, ophthalmologic disorders, fibrotic disorders, metabolic and gastro-intestinal disorders, neuroinflammatory and neurodegenerative diseases, neoplasms and cancer associated disorders, hormone related diseases and immunological disorders resulting from viral and bacterial infectious diseases and complications thereof.

## BRIEF DESCRIPTION OF THE FIGURES

**FIGURE 1:** (A) Confocal immunofluorescence images of vascular permeability assessed by Evans blue dye leakage in retinal whole mounts in a representative mouse from each experimental group. Arrows indicate extravasation location. Scale bars, 30 μm. (B) Quantification of vascular leakage by assessing the number of extravasations per field (0.44 mm2). The effect of Ia-007a eye drops was significant by reducing the vascular leakage to the same level that occur in the non-diabetic control group. *p < 0.01 in comparison with the other groups.

**FIGURE 2:** (A) Schmidt score is the evaluation of the efficacy of the compounds based on the four histopathology assessment criteria which describe pancreatic injury based on the follwing scores (number in parenthesis) for the four parameters. (a) Edema Interstitial oedema with scores (0) None (1) Interlobular (2) Lobule involved (3) Isolated island like acinar cells. (b) Inflammatory infiltration = leukocytes infiltration with scores (0) None, (1) < 20%, (2) 20%-50%, (3) > 50%. (c) Parenchymal necrosis = Acinar cell necrosis with scores (0) None, (1) < 5%, (2) 5%-20%, (3) > 20%. (d) Haemorrhage with scores (0) None, (1) 1-2 points, (2) 3-5 points, (3) > 5 points. Schmidt highest score = 10 is caerulein induced + vehicle treatment and lowest score = 0 is non-induced animals. Schmidt score is the total of a+b+c+d scores and **** means statistically significant difference with caerulein induced group as shown in Figure 2A. All products show improved Schmidts score compared to caerulein control animals.

(B) IL-33 dosage in terminal Pancreatitis as shown for all compounds in Figure 2B.
(C) TGF-B dosage in terminal Pancreatitis as shown for all compounds in Figure 2C.

**FIGURES 3A-D:** Figures 3A-B represent a disease list with main cytokines involved in selected diseases (Akdis M. et al, J Allergy Clin Immunol 2016;138:984-1010) and Figure 3C represents part of the search results with cytokines and genes ranked by scores and likely to be involved in the selected disease given as an example "diabetic retinopathy" after search in https://www.targetvalidation.org/. In Figure 3D, search was performed using VEGFA as the cytokine to find a list of diseases where VEGFA is mentioned. Only part of the diseases where VEGFA is involved are represented in figure 3D. ref: Open Targets Platform: new developments and updates two years on. Denise Carvalho et al (Nucleic Acids Research, Volume 47, Issue D1, 08 January 2019, D1056-D1065, https://doi.org/10.1093/nar/gky 1133).

**FIGURES 4 (A-D):** represent a table listing the conditions and yields of compounds of type Ia-Id as described in Examples 1.1 to 1.4. Overall yield calculated from starting material 2,5-dihydroxyterephthalic acid

**FIGURE 5 (A-C):** represent a table listing the conditions and yields of compounds of type IIa-IIc as described in Examples 1.5 to 1.7.

**FIGURE 6:** represent a table listing the conditions and yields of compounds of type IIIa and IIIb as described in Examples 1.8 and 1.9.

**FIGURE 7:** represent a table listing the conditions and yields of compounds of type IIIc as described in Example 1.10.

**FIGURE 8:** is a table listing deprotection procedures as described in Example 1.12 relative to compounds type V.

**FIGURE 9:** shows wound healing accelerating in the STZ-induced diabetes model in 3 animals per group sampled after 7 days dosing.

**FIGURE 10:** shows accelerating wound healing in the STZ-induced diabetes model in 3 animals per group sampled after 7 days dosing daily by intravenous or per os routes.

## DETAILED DESCRIPTION OF THE INVENTION

Disclosed herein are novel compounds useful for the treatment of subjects suffering from a disease, disorder, or dysfunction. Examples of such diseases, disorders, or dysfunctions include without limitation autoimmune, metabolic, inflammatory, degenerative and neoplastic disorders, in particular inflammatory pathologies, angiogenic diseases and/or fibrotic disorders such as diabetic retinopathy, diabetic nephropathy, ankylosing spondylitis, chronic pancreatitis, liver fibrosis, kidney fibrosis, as well as metabolic diseases caused by pancreas dysfunctions.

Hereinafter, unless otherwise specified, the collection of diseases, disorders, or dysfunctions that may be treated utilizing the novel compounds disclosed herein are collectively termed "medical conditions." The term "subject," as used herein, comprises any and all organisms and includes the term "patient." A subject to be treated according to the methods described herein may be one who has been diagnosed by a medical practitioner as suffering from a medical condition. Diagnosis may be performed by any suitable means. One skilled in the art will understand that a subject to be treated according to the present disclosure may have been subjected to standard tests to diagnose the medical conditions. As known to the ordinarily skilled artisan, the clinical features of medical conditions of the type disclosed herein vary according to the pathomechanisms.

Herein "treating" refers to utilizing the disclosed compounds for therapeutic purposes. Therapeutic treatment may be administered, for example, to a subject suffering from the medical condition in order to improve or stabilize the subject's condition. Thus, in the claims and embodiments described herein, treating refers to a subject undergoing for therapeutic purposes, the methodologies disclosed herein.

The present invention thus provides a compound of Formula (I):

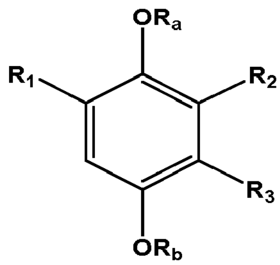

Formula (I)

wherein:

$R_a$, $R_b$ = H, $C_{1-4}$acyl, aryl$C_{1-4}$alkyl;
$R_1$ = $COOR_4$, $(CH_2)_nCOOR_4$, $SO_3H$, $(CH_2)_nSO_3H$, CONH-$R_{10;}$
$R_2$ and $R_3$ are H or $R_5$, with the proviso that when one of $R_2$ and $R_3$ is $R_5$, the other is H;
$R_4$ = H, $C_{1-4}$alkyl, aryl$C_{1-4}$alkyl;
$R_5$ = $R_6$, $R_7$ or $R_{8;}$
$R_6$ = CONH-$R_9$, $CONHCOR_9$, CONH$(CH_2)_n$-$R_9$, CONHCH$(COOR_4)(CH_2)_kR_9$, heteroaryl-$R_9$ wherein k = 0-4;
$R_7$ = benzoheteroaryl optionally substituted with one or more groups selected from: $COOR_4$, $(CH_2)_nCOOR_4$, $SO_3H$, $(CH_2)_nSO_3H$, $OR_4$, azoles [5-membered N-containing heterocycles], fluorine;
$R_8$ = $(CH_2)nX(CH_2)_pR_{9;}$
X = O, S, $SO_2$, NH, NAc, $N(CH_2)_qR_9$;
$R_9$ = aryl, heteroaryl, cycloalkyl optionally substituted with one or more groups selected from $COOR_4$, $(CH_2)_nCOOR_4$, $(CH_2)_nSO_3H$, $OR_4$, $SO_3H$, azoles [5-membered N-containing heterocycles], fluorine, C=O, NHCO-$R_{10}$;
$R_{10}$ = aryl, heteroaryl, optionally substituted with one or more groups selected from $COOR_4$, $(CH_2)_nCOOR_4$, $(CH_2)_nSO_3H$, $OR_4$, $SO_3H$, azoles [5-membered N-containing heterocycles], fluorine; and
n, m, p and q are independently 1-4.

The expression "alkyl" refers to a saturated, straight-chain or branched hydrocarbon group that contains from

1 to 20 carbon atoms, preferably from 1 to 12 carbon atoms, especially from 1 to 6 (e.g. 1, 2, 3 or 4) carbon atoms, for example a methyl, ethyl, propyl, iso-propyl, n-butyl, iso-butyl, sec-butyl or tert-butyl. Furthermore, the term alkyl refers to groups in which one or more hydrogen atoms have been replaced by a halogen atom (preferably F or Cl) such as, for example, a 2,2,2-trichloroethyl or a trifluoromethyl group.

The expression "acyl" refers to the groups (alkyl)-C(O)-, (aryl)-C(O)-, (heteroaryl)-C(O)-, (heteroalkyl)-C(O)-, and (heterocycloalkyl)-C(O)-, wherein the group is attached to the parent structure through the carbonyl functionality. In some embodiments, it is a C1-C4 acyl radical which refers to the total number of chain or ring atoms of the alkyl, aryl, heteroaryl or heterocycloalkyl portion of the acyloxy group plus the carbonyl carbon of acyl, i.e. three other ring or chain atoms plus carbonyl.

The expression "aryl" refers to an aromatic group that contains one or more rings containing from 6 to 14 ring carbon atoms, preferably from 6 to 10 (especially 6) ring carbon atoms. The expression aryl refers furthermore to groups in which one or more hydrogen atoms have been replaced by fluorine, chlorine, bromine or iodine atoms or by OH, SH, NH2, N3 or NO2 groups. Examples are the phenyl, naphthyl, biphenyl, 2-fluorophenyl, anilinyl, 3-nitrophenyl or 4-hydroxyphenyl group.

The expression "aralkyl" or "arylC1-4alkyl" refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, aryl-cycloalkenyl, alkylarylcycloalkyl and alkylarylcycloalkenyl groups. Specific examples of aralkyls are those derived from: methylbenzene, dimethylbenzene, 1,3,5-trimethylbenzene, ethenylbenzene, 1-fluoro-2-methylbenzene, 1H-indene, 1,2,3,4-tetrahydronaphthalene, 1,2-dihydronaphthalene, 2,3-dihydroinden-1-one, phenylcyclopentyl, (propan-2-yl) benzene, cyclohexyl-phenyl, 9H-fluorene and 2,3-dihydro-1H-indene. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression "heteroaryl" refers to an aromatic group that contains one or more rings containing from 5 to 14 ring atoms, preferably from 5 to 10 (especially 5 or 6 or 9 or 10) ring atoms, and contains one or more (preferably 1, 2, 3 or 4) oxygen, nitrogen, phosphorus or sulfur ring atoms (preferably O, S or N). The expression heteroaryl refers furthermore to groups in which one or more hydrogen atoms have been replaced by fluorine, chlorine, bromine or iodine atoms or by OH, SH, N3, NH2or NO2 groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3-bifuryl, pyrazolyl (e.g. 3-pyrazolyl) and isoquinolinyl groups.

The expression "benzoheteroaryl" or "benzoheteroaromatic" refers to bicyclic rings comprising a phenyl ring fused to a monocyclic heteroaromatic ring. Examples of benzoheteroaryl include benzisoxazolyl, benzoxazolyl, benzisothiazolyl, benzothiazolyl, benzimidazolyl, benzofuryl, benzothienyl (including S-oxide and dioxide), quinolyl, isoquinolyl, indazolyl, indolyl, and the like.

The term "azole" refers to a five membered heteroaryl group having a nitrogen ring atom and between 0 and 3 additional ring heteroatoms selected from N, O or S. Imidazole, oxazole, thiazole and tetrazole are representative azole groups.

The expression "cycloalkyl" refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH2, =NOH, N3 or NO2 groups, thus, for example, cyclic ketones substituents such as, for example, oxocyclohexyl, oxocyclohexenyl or oxocyclopentyl. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group.

The expression " optionnally substituted" refers to groups in which at least one, or optionnally two, three or more hydrogen atoms may have been replaced by fluorine, chlorine, bromine or iodine atoms or by OH, =O, SH, =S, NH2, =NOH, N3 or NO2 groups. This expression refers furthermore to groups that may be substituted by at least one, two, three or more unsubstituted C1-C10 alkyl, C2-C10alkenyl, C2-C10 alkynyl, heteroalkyl, C3-C18 cycloalkyl, C2-C17 heterocycloalkyl, C4-C20 alkylcycloalkyl, C2-C19 heteroalkylcycloalkyl, C6-C18 aryl, C1-C17 heteroaryl, C7-C20 aralkyl or C2-C19 heteroaralkyl groups. This expression refers furthermore especially to groups that may be substituted by one, two, three or more unsubstituted C1-C6 alkyl, C2-C6 alkenyl, C2-C6 alkynyl, C1-C6 heteroalkyl, C3-C10 cycloalkyl, C2-C9 heterocycloalkyl, C7-C12 alkylcycloalkyl, C2-C11 heteroalkylcycloalkyl, C6-C10 aryl, C1-C9 heteroaryl, C7-C12 aralkyl or C2-C11 heteroaralkyl groups.

According to a preferred embodiment, all alkyl, alkenyl, alkynyl, heteroalkyl, aryl, heteroaryl, cycloalkyl, heterocycloalkyl, alkylcycloalkyl, heteroalkylcycloalkyl, aralkyl and heteroaralkyl groups described herein may optionally be substituted.

Preferably, the present invention provides a hydroquinone derivative compound of formula (I), wherein:

(A)

$R_a$, $R_b$ = H or $C_{1-4}$acyl;
$R_1$ = $COOR_{4;}$ $R_4$ = H or $C_{1-4}$alkyl;
$R_2$ = H; $R_3$ = $R_{5;}$ and
$R_5$ = $R_6$= CONH-$R_9$;

or
(B)

$R_a$, $R_b$ = H or $C_{1-4}$acyl;
$R_1$ = $COOR_{4;}$ $R_4$ = H or $C_{1-4}$alkyl;
$R_3$ = H; $R_2$ = $R_5$; and
$R_5$ = $R_6$= CONH-$R_9$.

In a first embodiment, the present invention thus includes compounds in accordance with Table 1:

Table 1

| A. | 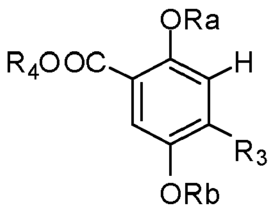 R3 = R5 = CONHR9 | | | | |
|---|---|---|---|---|---|
| **Compound No.** | **Compound chemical names** | **Ra** | **Rb** | **R4** | **R9** |
| Ia-001a | 4-(2-carboxyphenylaminocarbonyl)-2,5-dihydroxybenzoic acid | H | H | H | 2-carboxyphenyl |
| Ia-001a-Tz | 4-(2-(1H-tetrazol-5-yl)phenyl-aminocarbonyl)-2,5-dihydroxybenzoic acid | H | H | H | 2-(1H-tetrazol-5-yl)phenyl |
| Ia-001c | 2,5-dihydroxy-4-(2-sulfophenylaminocarbonyl)benzoic acid | H | H | H | 2-sulfophenyl |
| Ia-002a | 4-(3-carboxyphenylaminocarbonyl)-2,5-dihydroxybenzoic acid | H | H | H | 3-carboxyphenyl |
| Ia-002a-Tz | 4-(3-(1H-tetrazol-5-yl)phenyl-aminocarbonyl)-2,5-dihydroxybenzoic acid | H | H | H | 3-(1H-tetrazol-5-yl)phenyl |
| Ia-002c | 2,5-dihydroxy-4-(3-sulfophenylaminocarbonyl)benzoic acid | H | H | H | 3-sulfophenyl |
| Ia-003a | 4-(4-carboxyphenyl-aminocarbonyl)-2,5-dihydroxybenzoic acid | H | H | H | 4-carboxyphenyl |
| Ia-003a-Tz | 4-(4-(1H-tetrazol-5-yl)pheiryl-aminocarboiryl)-2,5-dihydroxybenzoic acid | H | H | H | 4-(1H-tetrazol-5-yl)phenyl |
| Ia-003c | 2,5-dihydroxy-4-(4-sulfophenylaminocarbonyl)benzoic acid | H | H | H | 4-sulfophenyl |

(continued)

| A. | 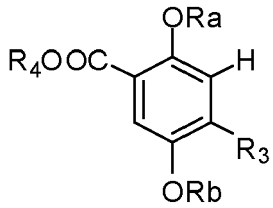<br>R3 = R5 = CONHR9 | | | | |
|---|---|---|---|---|---|
| **Compound No.** | **Compound chemical names** | **Ra** | **Rb** | **R4** | **R9** |
| Ia-004a | 4-(3-carboxy-4-hydroxyphenylaminocarbonyl)-2,5-dihydroxybenzoic acid | H | H | H | 3-carboxy-4-hydroxyphenyl |
| Ia-005a | 4-(4-carboxy-3-hydroxyphenylaminocarbonyl)-2,5-dihydroxybenzoic acid | H | H | H | 4-carboxy-3-hydroxyphenyl |
| Ia-056a | 4-(4-carboxy-2,5-dihydroxyphenylaminocarbonyl)-2,5-dihydroxybenzoic acid | H | H | H | 4-carboxy-2,5-dihydroxyphenyl |
| Ia-006a | 4-(2-carboxy-4-hydroxyphenylaminocarbonyl)-2,5-dihydroxybenzoic acid | H | H | H | 2-carboxy-4-hydroxyphenyl |
| Ib-010a | 3,5-bis(2,5-dihydroxy-4-carboxybenzoylamino)benzoic acid | H | H | H | 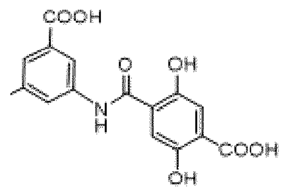 |
| Ia-011a | 3-(4-carboxy-2,5-dihydroxybenzamido) phthalic acid; | H | H | H | 2,3-dicarboxyphenyl |
| Ia-012a | 2-(4-carboxy-2,5-dihydroxybenzamido) terephthalic acid | H | H | H | 2,5-dicarboxyphenyl |
| Ia-013a | Compound 36: 2-(4-carboxy-2,5-dihydroxybenzamido)isophthalic acid | H | H | H | 2,6-dicarboxyphenyl |
| Ia-014a | Compound 38: 4-(4-carboxy-2,5-dihydroxybenzamido)phthalic acid | H | H | H | 3.4-dicarboxyphenyl |
| Ia-015a | 5-(4-carboxy-2,5-dihydroxybenzamido) isophthalic acid | H | H | H | 3.5-dicarboxyphenyl |
| Ia-016a | 2-(4-carboxy-2,5-dihydroxybenzamido) nicotinic acid | H | H | H | 3-carboxypyridin-2-yl |
| Ia-017a | 2-(4-carboxy-2,5-dihydroxybenzamido) isonicotinic acid | H | H | H | 4-carboxypyridin-2-yl |
| Ia-018a | 6-(4-carboxy-2,5-dihydroxybenzamido) nicotinic acid | H | H | H | 5-carboxypyridin-2-yl |
| Ia-019a | 6-(4-carboxy-2,5-dihydroxybenzamido) picolinic acid | H | H | H | 6-carboxypyridin-2-yl |
| Ia-020a | 2-(4-carboxy-2,5-dihydroxybenzamido)-5-fluoronicotinic acid | H | H | H | 3-carboxy-5-fluoropyridin-2-yl |

(continued)

| A. | 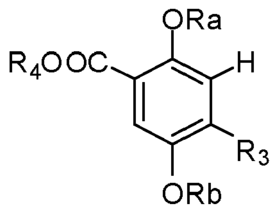<br>R3 = R5 = CONHR9 | | | | |
|---|---|---|---|---|---|
| **Compound No.** | **Compound chemical names** | **Ra** | **Rb** | **R4** | **R9** |
| Ia-021a | 6-(4-carboxy-2,5-dihydroxybenzamido) pyridine-2,5-dicarboxylic acid | H | H | H | 3,6-dicarboxypyridin-2-yl |
| Ia-022a | 2-(4-carboxy-2,5-dihydroxybenzamido) pyridine-3,5-dicarboxylic acid | H | H | H | 3,5-dicarboxypyridin-2-yl |
| Ia-023a | 3-(4-carboxy-2,5-dihydroxybenzamido) isonicotinic acid | H | H | H | 4-carboxypyridin-3-yl |
| Ia-024a | 5-(4-carboxy-2,5-dihydroxybenzamido) nicotinic acid | H | H | H | 5-carboxypyridin-3-yl |
| Ia-025a | 5-(4-carboxy-2,5-dihydroxybenzamido) picolinic acid | H | H | H | 6-carboxypyridin-3-yl |
| Ia-026a | 3-(4-carboxy-2,5-dihydroxybenzamido) picolinic acid | H | H | H | 2-carboxypyridin-3-yl |
| Ia-027a | 5-(4-carboxy-2,5-dihydroxybenzamido)-2-fluoroisonicotinic acid | H | H | H | 4-carboxy-6-fluoropyridin-3-yl |
| Ia-028a | 4-(4-carboxy-2,5-dihydroxybenzamido) nicotinic acid | H | H | H | 3-carboxypyridin-4-yl |
| Ia-029a | 4-(4-carboxy-2,5-dihydroxybenzamido) picolinic acid | H | H | H | 2-carboxypyridin-3-yl |
| Ia-032a | 4-(4-(carboxymethyl) phenylaminocarbonyl)-2,5-dihydroxybenzoic acid | H | H | H | 4-(carboxymethyl)phenyl |
| Ia-033a | 4-(3-(carboxymethyl) phenylaminocarbonyl)-2,5-dihydroxybenzoic acid | H | H | H | 3-(carboxymethyl)phenyl |
| Ia-033a-HyP | 2,5-dihydroxy-4-(3-(1-hydroxy-1H-pyrazol-4-yl)phenylaminocarbonyl) benzoic acid | H | H | H | 3-(1-hydroxy-1H-pyrazol-4-yl) phenyl |
| Ia-034a | 4-(2-(carboxymethyl) phenylaminocarbonyl)-2,5-dihydroxybenzoic acid | H | H | H | 2-(carboxymethyl)phenyl |
| Ia-001a-E2-A2 | ethyl4-(2-(ethoxycarbonyl) phenylaminocarbonyl)-2,5-diacetoxybenzoate | Ac | Ac | Et | 2-(ethoxycarbonyl)phenyl |
| Ia-001a-E2 | ethyl4-(2-(ethoxycarbonyl) phenylaminocarbonyl)-2,5-dihydroxybenzoate | H | H | Et | 2-(ethoxycarbonyl)phenyl |

(continued)

| A. | 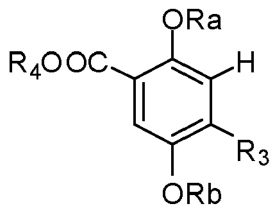 R3 = R5 = CONHR9 | | | | |
|---|---|---|---|---|---|
| **Compound No.** | **Compound chemical names** | **Ra** | **Rb** | **R4** | **R9** |
| Ia-001a-Tz-E1-A2 | ethyl 4-(2-(1H-tetrazol-5-yl) phenylaminocarbonyl)-2,5-diacetoxybenzoate | Ac | Ac | Et | 2-(1H4etrazol-5-yl)phenyl |
| Ia-001a-Tz-E1 | ethyl4-(2-(1H-tetrazol-5-yl) phenylaminocarbonyl)-2,5-dihydroxybenzoate | H | H | Et | 2-(1H4etrazol-5-yl)phenyl |
| Ia-004a-E2 | ethyl 2,5-dihydroxy-4-(4-hydroxy-3-(methoxycarbonyl) phenylaminocarbonyl)benzoate | H | H | Et | 4-hydroxy-3-(methoxycarbonyl)phenyl |
| Ib-010a-E3-A4 | ethyl 3,5-bis(2,5-diacetoxy-4-ethoxycarbonylbenzoylamino)benzoate | Ac | Ac | Et | 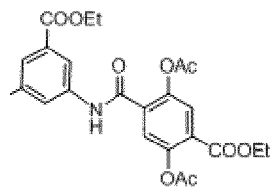 |
| Ib-010a-E3 | ethyl 3,5-bis(2,5-dihydroxy-4-ethoxycarbonylbenzoylamino)benzoate | H | H | Et | 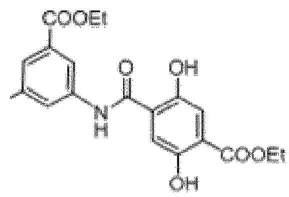 |
| Ia-023a-E2 | ethyl3-(4-(ethoxycarbonyl)-2,5-dihydroxybenzamido)isonicotinate | H | H | Et | 4-ethoxycarbonylpyridin-3-yl |
| Ia-056a-E2 | Compound 390:Ethyl4-(4-ethoxycarbonyl-2,5-dihydroxyphenylaminocarbonyl)-2,5-dihydroxybenzoate | H | H | Et | 4-ethoxycarbonyl-2,5-dihydroxyphenyl |
| B. | 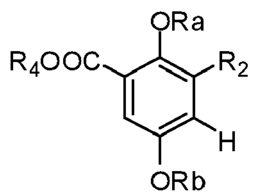 R2 = R5 = CONHR9 | | | | |
| IIa-001a | 3-(2-carboxyphenylaminocarbonyl)-2,5-dihydroxybenzoic acid | H | H | H | 2-carboxyphenyl |
| IIa-001a-Tz | 3-(2-(1H-tetrazol-5-yl) phenylaminocarbonyl)-2,5-dihydroxybenzoic acid | H | H | H | 2-(1H-tetrazol-5-yl)phenyl |

(continued)

| B. | 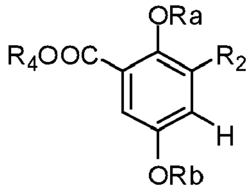<br>R2 = R5 = CONHR9 | | | | |
|---|---|---|---|---|---|
| IIa-001c | 2,5-dihydroxy-3-(2-sulfophenylaminocarbonyl)benzoic acid | H | H | H | 2-sulfophenyl |
| IIa-002a | 3-(3-carboxyphenylaminocarbonyl)-2,5-dihydroxybenzoic acid | H | H | H | 3-carboxyphenyl |
| IIa-002a-Tz | 3-(3-(1H-tetrazol-5-yl) phenylaminocarbonyl)-2,5-dihydroxybenzoic acid | H | H | H | 3-(1H-tetrazol-5-yl)phenyl |
| IIa-002c | 2,5-dihydroxy-3-(3-sulfophenylaminocarbonyl)benzoic acid | H | H | H | 3-sulfophenyl |
| IIa-003a | 3-(4-carboxyphenylaminocarbonyl)-2,5-dihydroxybenzoic acid | H | H | H | 4-carboxyphenyl |
| IIa-003a-Tz | 3-(4-(1H-tetrazol-5-yl) phenylaminocarbonyl)-2,5-dihydroxybenzoic acid | H | H | H | 4-(1H-tetrazol-5-yl)phenyl |
| IIa-003c | 2,5-dihydroxy-3-(4-sulfophenylaminocarbonyl)benzoic acid | H | H | H | 4-sulfophenyl |
| IIa-004a | 3-(3-carboxy-4-hydroxyphenylaminocarbonyl)-2,5-dihydroxybenzoic acid | H | H | H | 3-carboxy-4-hydroxyphenyl |
| IIa-005a | 3-(4-carboxy-3-hydroxyphenylaminocarbonyl)-2,5-dihydroxybenzoic acid | H | H | H | 4-carboxy-3-hydroxyphenyl |
| IIa-006a | 3-(2-carboxy-4-hydroxyphenylaminocarbonyl)-2,5-dihydroxybenzoic acid | H | H | H | 2-carboxy-4-hydroxyphenyl |
| IIb-010a | 3,5-Bis(2,5-dihydroxy-3-carboxybenzoylamino)benzoic acid | H | H | H | 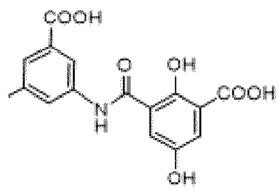 |
| IIa-011a | 3-(3-carboxy-2,5-dihydroxybenzamido) phthalic acid | H | H | H | 2,3-dicarboxyphenyl |
| IIa-012a | 2-(3-carboxy-2,5-dihydroxybenzamido) terephthalic acid | H | H | H | 2,5-dicarboxyphenyl |
| IIa-013a | 2-(3-carboxy-2,5-dihydroxybenzamido) isophthalic acid | H | H | H | 2.6-dicarboxyphenyl |
| IIa-014a | 4-(3-carboxy-2,5-dihydroxybenzamido) phthalic acid | H | H | H | 3.4-dicarboxyphenyl |
| IIa-015a | 5-(3-carboxy-2,5-dihydroxybenzamido) isophthalic acid | H | H | H | 3,5-dicarboxylic acid |

(continued)

| B. | 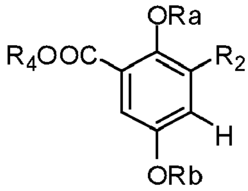<br>R2 = R5 = CONHR9 | | | | |
|---|---|---|---|---|---|
| IIa-016a | 2-(3-carboxy-2,5-dihydroxybenzamido) nicotinic acid | H | H | H | 3-carboxypyridin-2-yl |
| IIa-017a | 2-(3-carboxy-2,5-dihydroxybenzamido) isonicotinic acid | H | H | H | 4-carboxypyridin-2-yl |
| IIa-018a | 6-(3-carboxy-2,5-dihydroxybenzamido) nicotinic acid | H | H | H | 5-carboxypyridin-2-yl |
| IIa-019a | 6-(3-carboxy-2,5 -dihy droxybenzamido) picolinic acid | H | H | H | 6-carboxypyridin-2-yl |
| IIa-020a | 2-(3-carboxy-2,5-dihydroxybenzamido)-5-fluoronicotinic acid | H | H | H | 3-carboxy-5-fluoropyridin-2-yl |
| IIa-021a | 6-(3-carboxy-2,5-dihydroxybenzamido) pyridine-2,5-dicarboxylic acid | H | H | H | 3,6-dicarboxypyridin-2-yl |
| IIa-022a | 2-(3-carboxy-2,5-dihydroxybenzamido) pyridine-3,5-dicarboxylic acid | H | H | H | 3,5-dicarboxyridin-2-yl |
| IIa-023a | 3-(3-carboxy-2,5-dihydroxybenzamido) isonicotinic acid | H | H | H | 4-carboxypyridin-3-yl |
| IIa-024a | 5-(3-carboxy-2,5-dihydroxybenzamido) nicotinic acid | H | H | H | 5-carboxypyridin-3-yl |
| IIa-025a | 5-(3-carboxy-2,5-dihydroxybenzamido) picolinic acid | H | H | H | 6-carboxypyridin-3-yl |
| IIa-026a | 3-(3-carboxy-2,5-dihydroxybenzamido) picolinic acid | H | H | H | 2-carboxypyridin-3-yl |
| IIa-027a | 5-(3-carboxy-2,5-dihydroxybenzamido)-2-fluoroisonicotinic acid | H | H | H | 4-carboxy-6-fluoropyridin-3-yl |
| IIa-028a | 4-(3-carboxy-2,5-dihydroxybenzamido) nicotinic acid | H | H | H | 3-carboxypyridin-4-yl |
| IIa-029a | Compound 172: 4-(3-carboxy-2,5-dihydroxybenzamido)picolinic acid; | H | H | H | 2-carboxypyridin-3-yl |
| IIa-032a | 3-(4-(carboxymethyl) phenylaminocarbonyl)-2,5-dihydroxybenzoic acid | H | H | H | 4-(carboxymethyl)phenyl |
| IIa-033a | 3-(3-(carboxymethyl) phenylaminocarbonyl)-2,5-dihydroxybenzoic acid | H | H | H | 3-(carboxymethyl)phenyl |
| IIa-033a-Hyp | 2,5-dihydroxy-3-(3-(1hydroxy-1H-pyrazol-4-yl)phenylaminocarbonyl) benzoic acid | H | H | H | 3-(1hydroxy-1H-pyrazol-4-yl) phenyl |
| IIa-034a | 3-(2-(carboxymethyl) phenylaminocarbonyl)-2,5-dihydroxybenzoic acid | H | H | H | 2-(carboxymethyl)phenyl |

(continued)

| B. | 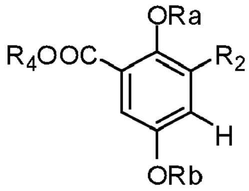 R2 = R5 = CONHR9 | | | | |
|---|---|---|---|---|---|
| IIa-OOlaTz-E1 | ethyl3-(2-(1-tetrazol-5-yl) phenylaminocarbonyl)2,5-dihydroxybenzoate | H | H | Et | 2-(1H-tetrazol-5-yl)phenyl |
| IIa-034a-E2 | ethyl3-(2-(ethoxycarbonylmethyl) phenylaminocarbonyl)-2,5-dihydroxybenzoate | H | H | Et | 2-(2-ethoxycarbonylmethyl) phenyl |

Preferably, the present invention provides a compound of formula (I), wherein:

(A)

$R_a$, $R_b$ = H;
$R_1$ = $SO_3H_;$
$R_2$ = H; $R_3$ = $R_{5;}$ and
$R_5$= $R_6$ = CONH-$R_9$;

or

(B)

$R_a$, $R_b$ = H;
$R_1$ = $SO_3H_;$
$R_3$ = H; $R_2$ = $R_{5;}$ and
$R_5$= $R_6$ = CONH-$R_9$.

In a second embodiment, the present invention includes compounds in accordance with Table 2:

Table 2

| A. | 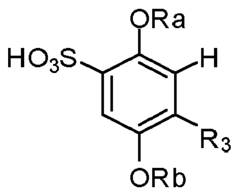 R3 = R5 = CONHR9 | | | |
|---|---|---|---|---|
| Compound No. | **Compound chemical names** | **Ra** | **Rb** | **R9** |
| Ia-001b | 2-(2,5-dihydroxy-4-sulfobenzamido)benzoic acid | H | H | 2-carboxyphenyl |
| Ia-001d | 2,5-dihydroxy-4-(2-sulfophenylaminocarbonyl)benzenesulfoiic acid | H | H | 2-sulfophenyl |
| Ia-002b | 3-(2,5-dihydroxy-4-sulfobenzamido)benzoic acid | H | H | 3-carboxyphenyl |

(continued)

| A. | 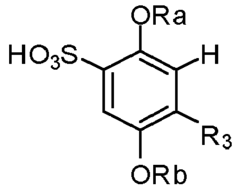<br>R3 = R5 = CONHR9 | | | |
|---|---|---|---|---|
| Compound No. | **Compound chemical names** | **Ra** | **Rb** | **R9** |
| Ia-002d | 2,5-dihydroxy-4-(3-sulfophenylaminocarbonyl)benzenesulfonic acid | H | H | 3-sulfophenyl |
| Ia-003b | 4-(2,5-dihydroxy-4-sulfobenzamido)benzoic acid | H | H | 4-carboxyphenyl |
| Ia-003d | 2,5-dihydroxy-4-(4-sulfophenylaminocarbonyl)benzenesulfonic acid | H | H | 4-sulfophenyl |
| Ia-004b | 5-(2,5-dihydroxy-4-sulfobenzamido)-2-hydroxybenzoic acid | H | H | 3-carboxy-4-hydroxyphenyl |
| Ia-005b | 4-(2,5-dihydroxy-4-sulfobenzamido)-2-hydroxybenzoic acid | H | H | 4-carboxy-3-hydroxyphenyl |
| Ia-006b | 2-(2,5-dihydroxy-4-sulfobenzamido)-5-hydroxybenzoic acid | H | H | 2-carboxy-4-hydroxyphenyl |
| Ib-010b | 3,5-bis(2,5-dihydroxy-4-sulfobenzamido) benzoic acid | H | H | 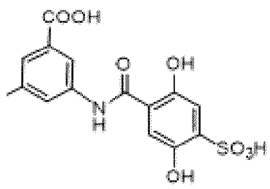 |
| Ia-011b | 3-(2,5-dihydroxy-4-sulfobenzamido)phthalic acid | H | H | 2,3-dicarboxyphenyl |
| Ia-012b | 2-(2,5-dihydroxy-4-sulfobenzamido) terephthalic acid | H | H | 2,5-dicarboxyphenyl |
| Ia-013b | 2-(2,5-dihydroxy-4-sulfobenzamido) isophthalic acid | H | H | 2,6-dicarboxyphenyl |
| Ia-014b | 4-(2,5-dihydroxy-4-sulfobenzamido)phthalic acid | H | H | 3.4-dicarboxyphenyl |
| Ia-015b | 5-(2,5-dihydroxy-4-sulfobenzamido) isophthalic acid | H | H | 3.5-dicarboxyphenyl |
| Ia-016b | 2-(2,5-dihydroxy-4-sulfobenzamido) nicotinic acid | H | H | 3-carboxypyridin-2-yl |
| Ia-017b | 2-(2,5-dihydroxy-4-sulfobenzamido) isonicotinic acid | H | H | 4-carboxypyridin-2-yl |
| Ia-018b | 6-(2,5-dihydroxy-4-sulfobenzamido) nicotinic acid | H | H | 5-carboxypyridin-2-yl |
| Ia-019b | 6-(2,5-dihydroxy-4-sulfobenzamido) picolinic acid | H | H | 6-carboxypyridin-2-yl |

(continued)

| Compound No. | Compound chemical names | Ra | Rb | R9 |
|---|---|---|---|---|
| A. | 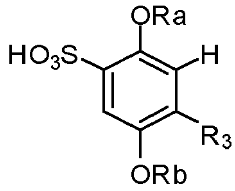 R3 = R5 = CONHR9 | | | |
| Compound No. | **Compound chemical names** | **Ra** | **Rb** | **R9** |
| Ia-020b | 2-(2,5-dihydroxy-4-sulfobenzamido)-5-fluoronicotinic acid | H | H | 3-carboxy-5-fluoropyridin-2-yl |
| Ia-021b | 6-(2,5-dihydroxy-4-sulfobenzamido) pyridine-2,5-dicarboxylic acid | H | H | 3,6-dicarboxypyridin-2-yl |
| Ia-022b | 2-(2,5-dihydroxy-4-sulfobenzamido) pyridine-3,5-dicarboxylic acid | H | H | 3,5-dicarboxypyridin-2-yl |
| Ia-023b | 3-(2,5-dihydroxy-4-sulfobenzamido) isonicotinic acid | H | H | 4-carboxypyridin-3-yl |
| Ia-024b | 5-(2,5-dihydroxy-4-sulfobenzamido) nicotinic acid | H | H | 5-carboxypyridin-3-yl |
| Ia-025b | 5-(2,5-dihydroxy-4-sulfobenzamido) picolinic acid | H | H | 6-carboxypyridin-3-yl |
| Ia-026a | 3-(2,5-dihydroxy-4-sulfobenzamido) picolinic acid | H | H | 2-carboxypyridin-3-yl |
| Ia-027b | 5-(2,5-dihydroxy-4-sulfobenzamido)-2-fluoroisonicotinic acid | H | H | 4-carboxy-6-fluoropyridin-3-yl |
| Ia-028b | 4-(2,5-dihydroxy-4-sulfobenzamido) nicotinic acid | H | H | 3-carboxypyridin-4-yl |
| Ia-029b | 4-(2,5-dihydroxy-4-sulfobenzamido) picolinic acid | H | H | 2-carboxypyridin-3-yl |
| Ia-032b | (4-(2,5-dihydroxy-4-sulfobenzamido) phenyl)acetic acid | H | H | 4-(carboxymethyl)phenyl |
| Ia-033b | (3-(2,5-dihydroxy-4-sulfobenzamido) phenyl)acetic acid | H | H | 3-(carboxymethyl)phenyl |
| Ia-034b | (2-(2,5-dihydroxy-4-sulfobenzamido) phenyl)acetic acid | H | H | 2-(carboxymethyl)phenyl |
| B. | 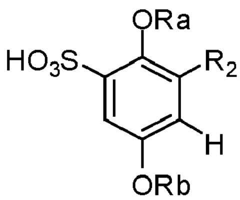 R2 = R5 = CONHR9 | | | |
| IIa-001b | 2-(2,5-dihydroxy-3-sulfobenzamido)benzoic acid | H | H | 2-carboxyphenyl |
| IIa-001d | 2,5-dihydroxy-3-(2-sulfophenylaminocarbonyl)benzenesulfonic acid | H | H | 2-sulfophenyl |

(continued)

| B. | 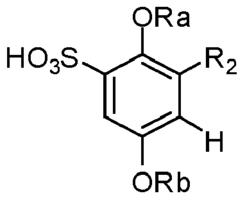 R2 = R5 = CONHR9 | | | |
|---|---|---|---|---|
| IIa-002b | 3-(2,5-dihydroxy-3-sulfobenzamido)benzoic acid | H | H | 3-carboxyphenyl |
| IIa-002d | 2,5-dihydroxy-3-(3-sulfophenylaminocarbonyl)benzenesulfoiic acid | H | H | 3-sulfophenyl |
| IIa-003b | 4-(2,5-dihydroxy-3-sulfobenzamido)benzoic acid | H | H | 4-carboxyphenyl |
| IIa-003d | 2,5-dihydroxy-3-(4-sulfophenylaminocarbonyl)benzenesulfonic acid | H | H | 4-sulfophenyl |
| IIa-004b | 5-(2,5-dihydroxy-3-sulfobenzamido)-2-hydroxybenzoic acid | H | H | 3-carboxy-4-hydroxyphenyl |
| IIa-005b | 4-(2,5-dihydroxy-3-sulfobenzamido)-2-hydroxybenzoic acid; | H | H | 4-carboxy-3-hydroxyphenyl |
| IIa-006b | 2-(2,5-dihydroxy-3-sulfobenzamido)-5-hydroxybenzoic acid | H | H | 2-carboxy-4-hydroxyphenyl |
| IIb-010b | 3-(2,5-dihydroxy-3-sulfobenzamido)-5-(2,5-dihydroxy-4-sulfobenzamido)benzoic acid | H | H | 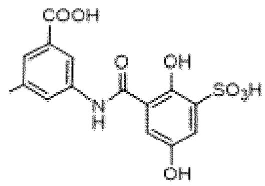 |
| IIa-011b | 3-(2,5-dihydroxy-3-sulfobenzamido)phthalic acid; | H | H | 2,3-dicarboxyphenyl |
| IIa-012b | 2-(2,5-dihydroxy-3-sulfobenzamido) terephthalic acid | H | H | 2,5-dicarboxyphenyl |
| IIa-013b | 2-(2,5-dihydroxy-3-sulfobenzamido) isophthalic acid | H | H | 2,6-dicarboxyphenyl |
| IIa-014b | 4-(2,5-dihydroxy-3-sulfobenzamido)phthalic acid | H | H | 3.4-dicarboxyphenyl |
| IIa-015b | 5-(2,5-dihydroxy-3-sulfobenzamido) isophthalic acid | H | H | 3.5-dicarboxyphenyl |
| IIa-016b | 2-(2,5-dihydroxy-3-sulfobenzamido) nicotinic acid | H | H | 3-carboxypyridin-2-yl |
| IIa-017b | 2-(2,5-dihydroxy-3-sulfobenzamido) isonicotinic acid | H | H | 4-carboxypyridin-2-yl |
| IIa-018b | 6-(2,5-dihydroxy-3-sulfobenzamido) nicotinic acid | H | H | 5-carboxypyridin-2-yl |
| IIa-019b | 6-(2,5-dihydroxy-3-sulfobenzamido) picolinic acid | H | H | 6-carboxypyridin-2-yl |

(continued)

| B. | 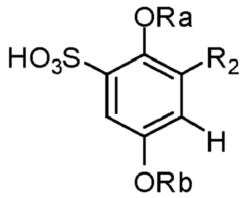 **R2 = R5 = CONHR9** | | | |
|---|---|---|---|---|
| IIa-020b | 2-(2,5-dihydroxy-3-sulfobenzamido)-5-fluoronicotinic acid | H | H | 3-carboxy-5-fluoropyridin-2-yl |
| IIa-021b | 6-(2,5-dihydroxy-3-sulfobenzamido) pyridine-2,5-dicarboxylic acid; | H | H | 3,6-dicarboxypyridin-2-yl |
| IIa-022b | 2-(2,5-dihydroxy-3-sulfobenzamido) pyridine-3,5-dicarboxylic acid | H | H | 3,5-dicarboxypyridin-2-yl |
| IIa-023b | 3-(2,5-dihydroxy-3-sulfobenzamido) isonicotinic acid | H | H | 4-carboxypyridin-3-yl |
| IIa-024b | 5-(2,5-dihydroxy-3-sulfobenzamido) nicotinic acid | H | H | 5-carboxypyridin-3-yl |
| IIa-025b | 5-(2,5-dihydroxy-3-sulfobenzamido) picolinic acid | H | H | 6-carboxypyridin-3-yl |
| IIa-026b | 3-(2,5-dihydroxy-3-sulfobenzamido) picolinic acid | H | H | 2-carboxypyridin-3-yl |
| IIa-027b | 5-(2,5-dihydroxy-3-sulfobenzamido)-2-fluoroisonicotinic acid | H | H | 4-carboxy-6-fluoropyridin-3-yl |
| IIa-028b | 4-(2,5-dihydroxy-3-sulfobenzamido) nicotinic acid | H | H | 3-carboxypyridin-4-yl |
| IIa-029b | Compound 173:4-(2,5-dihydroxy-3-sulfobenzamido)picolinic acid | H | H | 2-carboxypyridin-3-yl |
| IIa-032b | (4-(2,5-dihydroxy-3-sulfobenzamido) phenyl)acetic acid | H | H | 4-(carboxymethyl)phenyl |
| IIa-033b | (3-(2,5-dihydroxy-3-sulfobenzamido) phenyl)acetic acid | H | H | 3-(carboxymethyl)phenyl |
| IIa-034b | (2-(2,5-dihydroxy-3-sulfobenzamido) phenyl)acetic acid | H | H | 2-(carboxymethyl)phenyl |

Preferably, the present invention provides a hydroquinone derivative compound of formula (I), wherein:

(A)

$R_a$, $R_b$ = H;
$R_1$ = $(CH_2)_nCOOR_4$; $R_4$ = H, $C_{1-4}$alkyl, aryl$C_{1-4}$alkyl;
$R_2$ = H; $R_3$ = $R_5$; and
$R_5$ = $R_6$ = CONH-$R_9$;

Or

(B)

$R_a$, $R_b$ = H;
$R_1$ = $(CH_2)_nCOOR_4$; $R_4$ = H, $C_{1-4}$alkyl, aryl$C_1$-$_4$alkyl;

$R_3$ = H; $R_2$ = $R_{5;}$ and
$R_5$ = $R_6$= CONH-$R_9$.

In a third embodiment, the present invention includes compounds in accordance with Table 3:

Table 3

| A. | 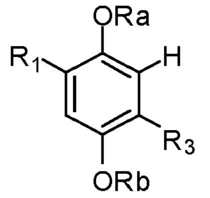<br>**R3 = R5 = R6 = CONH-R9** | | | | |
|---|---|---|---|---|---|
| Compound No. | **Compound chemical names** | **R1** | **Ra** | **Rb** | **R9** |
| IIIa-001a | 2-(4-(carboxymethyl)-2,5-dihydroxybenzamido)benzoic acid | CH2COOH | H | H | 2-carboxyphenyl |
| IIIa-001a-Tz | (2-(1H-tetrazol-5-yl) phenylaminocarbonyl)-2,5-dihydroxyphenyl)acetic acid | CH2COOH | H | H | 2-(1H-tetrazol-5-yl)phenyl |
| IIIa-00 1c | (2,5-dihydroxy-4-(2-sulfophenylaminocarbonyl)phenyl) acetic acid | CH2COOH | H | H | 2-sulfophenyl |
| IIIa-002a | -(4-(carboxymethyl)-2,5-dihydroxybenzamido)benzoic acid; | CH2COOH | H | H | 3-carboxyphenyl |
| IIIa-002c | (2,5-dihydroxy-4-(3-sulfophenylaminocarbonyl)phenyl) acetic acid; | CH2COOH | H | H | 3-sulfophenyl |
| IIIa-003a | 4-(4-(carboxymethyl)-2,5-dihydroxybenzamido)benzoicacid; | CH2COOH | H | H | 4-carboxyphenyl |
| IIIa-003c | 2-(2,5-dihydroxy-4-(4-sulfophenylaminocarbonyl)phenyl) acetic acid; | CH2COOH | H | H | 4-sulfophenyl |
| IIIa-004a | Compound 216:<br>5-(4-(carboxymethyl)-2,5-dihydroxybenzamido)-2-hydroxybenzoic acid | CH2COOH | H | H | 3-carboxy-4-hydroxyphenyl |
| IIIa-005a | 4-(4-(carboxymethyl)-2,5-dihydroxybenzamido)-2-hydroxybenzoic acid | CH2COOH | H | H | 4-carboxy-3-hydroxyphenyl |
| IIIa-006a | 2-(4-(carboxymethyl)-2,5-dihydroxybenzamido)-5-hydroxybenzoic acid | CH2COOH | H | H | 2-carboxy-4-hydroxyphenyl |
| IIIb-010a | 3,5-Bis(2,5-dihydroxy-4-carboxymethylbenzoylamino) benzoic acid | CH2COOH | H | H | 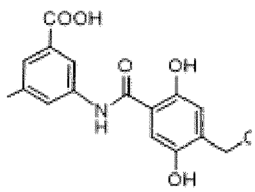 |

(continued)

| Compound No. | **Compound chemical names** | **R1** | **Ra** | **Rb** | **R9** |
|---|---|---|---|---|---|
| IIIa-011a | 3-(4-(carboxymethyl)-2,5-dihydroxybenzamido)phthalic acid | CH2COOH | H | H | 2,3-dicarboxyphenyl |
| IIIa-012a | 2-(4-(carboxymethyl)-2,5-dihydroxybenzamido)terephthalic acid | CH2COOH | H | H | 2,5-dicarboxyphenyl |
| IIIa-013a | 2-(4-(carboxymethyl)-2,5-dihydroxybenzamido)isophthalic acid | CH2COOH | H | H | 2,6-dicarboxyphenyl |
| IIIa-014a | 4-(4-(carboxymethyl)-2,5-dihydroxybenzamido)phthalic acid | CH2COOH | H | H | 3.4-dicarboxyphenyl |
| IIIa-015a | 5-(4-(carboxymethyl)-2,5-dihydroxybenzamido)isophthalic acid | CH2COOH | H | H | 3.5-dicarboxyphenyl |
| IIIa-016a | 2-(4-(carboxymethyl)-2,5-dihydroxybenzamido)nicotinic acid | CH2COOH | H | H | 3-carboxypyridin-2-yl |
| IIIa-017a | 2-(4-(carboxymethyl)-2,5-dihydroxybenzamido)isonicotinic acid | CH2COOH | H | H | 4-carboxypyridin-2-yl |
| IIIa-018a | 6-(4-(carboxymethyl)-2,5-dihydroxybenzamido)nicotinic acid | CH2COOH | H | H | 5-carboxypyridin-2-yl |
| IIIa-019a | 6-(4-(carboxymethyl)-2,5-dihydroxybenzamido)picolinic acid | CH2COOH | H | H | 6-carboxypyridin-2-yl |
| IIIa-020a | 2-(4-(carboxymethyl)-2,5-dihydroxybenzamido)-5-fluoronicotinic acid | CH2COOH | H | H | 3-carboxy-5-fluoropyridin-2-yl |
| IIIa-021a | 6-(4-(carboxymethyl)-2,5-dihydroxybenzamido)pyridine-2,5-dicarboxylic acid | CH2COOH | H | H | 3,6-dicarboxypyridin-2-yl |
| IIIa-022a | 2-(4-(carboxymethyl)-2,5-dihydroxybenzamido)pyridine-3,5-dicarboxylic acid | CH2COOH | H | H | 3,5-dicarboxypyridin-2-yl |
| IIIa-023a | 3-(4-(carboxymethyl)-2,5-dihydroxybenzamido)isonicotinic acid; | CH2COOH | H | H | 4-carboxypyridin-3-yl |
| IIIa-024a | 5-(4-(carboxymethyl)-2,5-dihydroxybenzamido)nicotinic acid | CH2COOH | H | H | 5-carboxypyridin-3-yl |
| IIIa-025a | 5-(4-(carboxymethyl)-2,5-dihydroxybenzamido)picolinic acid | CH2COOH | H | H | 6-carboxypyridin-3-yl |
| IIIa-026a | 3-(4-(carboxymethyl)-2,5-dihydroxybenzamido)picolinic acid | CH2COOH | H | H | 2-carboxypyridin-3-yl |
| IIIa-027a | 5-(4-(carboxymethyl)-2,5-dihydroxybenzamido)-2-fluoroisonicotinic acid | CH2COOH | H | H | 4-carboxy-6-fluoropyridin-3-yl |
| IIIa-028a | 4-(4-(carboxymethyl)-2,5-dihydroxybenzamido)nicotinic acid; | CH2COOH | H | H | 3-carboxypyridin-4-yl |
| IIIa-029a | 4-(4-(carboxymethyl)-2,5-dihydroxybenzamido)picolinic acid; | CH2COOH | H | H | 2-carboxypyridin-3-yl |

(continued)

| Compound No. | **Compound chemical names** | **R1** | **Ra** | **Rb** | **R9** |
|---|---|---|---|---|---|
| IIIa-032a | (4-(4-(carboxymethyl)-2,5-dihydroxybenzamido)phenyl)acetic acid | CH2COOH | H | H | 4-(carboxymethyl)phenyl |
| IIIa-033a | (3-(4-(carboxymethyl)-2,5-dihydroxybenzamido)phenyl)acetic acid | CH2COOH | H | H | 3-(carboxymethyl)phenyl |
| IIIa-034a | (2-(4-(carboxymethyl)-2,5-dihydroxybenzamido)phenyl)acetic acid | CH2COOH | H | H | 2-(carboxymethyl)phenyl |
| IIIa-001a-E2 | methyl 2-(4-(ethoxycarbonylmethyl)-2,5-dihydroxybenzamido)benzoate | CH2COOEt | H | H | 2-(methoxycarbonyl)phenyl |
| IIIa-001aTz-E1 | Compound 383: ethyl (4-(2-(1H-tetrazol-5-yl)phenylaminocarbonyl)-2,5-dihydroxyphenyl)acetate; | CH2COOEt | H | H | 2-(1H-tetrazol-5-yl)phenyl |
| IIIa-015a-E3 | diethyl 5-(4-(ethoxycarbonylmethyl)-2,5-dihydroxybenzamido) isophthalate | CH2COOEt | H | H | 3,5-diethoxycarbonylphenyl |
| IIIb-010a-E3 | methyl 3,5-bis (4-(ethoxycarbonylmethyl)-2,5-dihydroxybenzamido)benzoate | CH2COOEt | H | H | 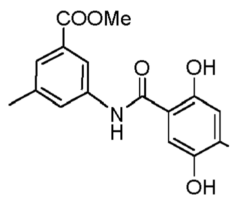 |
| B. | 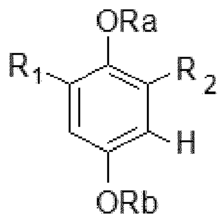 **R2 = R5 = R6 = CONHR9** | | | | |
| IVa-001a | 2-(3-(carboxymethyl)-2,5-dihydroxybenzamido)benzoic acid | CH2COOH | H | H | 2-carboxyphenyl |
| IVa-001c | (2,5-dihydroxy-3-(2-sulfophenylaminocarbonyl)phenyl) acetic acid | CH2COOH | H | H | 2-sulfophenyl |
| IVa-002a | 3-(3-(carboxymethyl)-2,5-dihydroxybenzamido)benzoic acid | CH2COOH | H | H | 3-carboxyphenyl |
| IVa-002c | (2,5-dihydroxy-3-(3-sulfophenylaminocarbonyl)phenyl) acetic acid | CH2COOH | H | H | 3-sulfophenyl |
| IVa-003a | 4-(3-(carboxymethyl)-2,5-dihydroxybenzamido)benzoic acid | CH2COOH | H | H | 4-carboxyphenyl |
| IVa-003c | (2,5-dihydroxy-3-(4-sulfophenylaminocarbonyl)phenyl) acetic acid | CH2COOH | H | H | 4-sulfophenyl |

(continued)

| Compound No. | **Compound chemical names** | **R1** | **Ra** | **Rb** | **R9** |
|---|---|---|---|---|---|
| IVa-004a | 5-(3-(carboxymethyl)-2,5-dihydroxybenzamido)-2-hydroxybenzoic acid | CH2COOH | H | H | 3-carboxy-4-hydroxyphenyl |
| IVa-005a | 4-(3-(carboxymethyl)-2,5-dihydroxybenzamido)-2-hydroxybenzoic acid | CH2COOH | H | H | 4-carboxy-3-hydroxyphenyl |
| IVa-006a | Compound 295: 2-(3-(carboxymethyl)-2,5-dihydroxybenzamido)-5-hydroxybenzoic acid; | CH2COOH | H | H | 2-carboxy-4-hydroxyphenyl |
| IVb-010a | 3,5-Bis(2,5-dihydroxy-3-carboxymethylbenzoylamino) benzoic acid | CH2COOH | H | H | 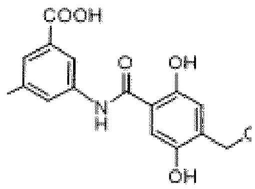 |
| IVa-011a | 3-(3-(carboxymethyl)-2,5-dihydroxybenzamido)phthalic acid | CH2COOH | H | H | 2,3-dicarboxyphenyl |
| IVa-012a | 2-(3-(carboxymethyl)-2,5-dihydroxybenzamido)terephthalic acid | CH2COOH | H | H | 2,5-dicarboxyphenyl |
| IVa-013a | 2-(3-(carboxymethyl)-2,5-dihydroxybenzamido)isophthalic acid | CH2COOH | H | H | 2,6-dicarboxyphenyl |
| IVa-014a | 4-(3-(carboxymethyl)-2,5-dihydroxybenzamido)phthalic acid | CH2COOH | H | H | 3.4-dicarboxyphenyl |
| IVa-015a | 5-(3-(carboxymethyl)-2,5-dihydroxybenzamido)isophthalic acid | CH2COOH | H | H | 3.5-dicarboxyphenyl |
| IVa-016a | 2-(3-(carboxymethyl)-2,5-dihydroxybenzamido)nicotinic acid | CH2COOH | H | H | 3-carboxypyridin-2-yl |
| IVa-017a | 2-(3-(carboxymethyl)-2,5-dihydroxybenzamido)isonicotinic acid | CH2COOH | H | H | 4-carboxypyridin-2-yl |
| IVa-018a | 6-(3-(carboxymethyl)-2,5-dihydroxybenzamido)nicotinic acid | CH2COOH | H | H | 5-carboxypyridin-2-yl |
| IVa-019a | 6-(3-(carboxymethyl)-2,5-dihydroxybenzamido)picolinic acid | CH2COOH | H | H | 6-carboxypyridin-2-yl |
| IVa-020a | 2-(3-(carboxymethyl)-2,5-dihydroxybenzamido)-5-fluoronicotinic acid | CH2COOH | H | H | 3-carboxy-5-fluoropyridin-2-yl |
| IVa-021a | 6-(3-(carboxymethyl)-2,5-dihydroxybenzamido)pyridine-2,5-dicarboxylic acid | CH2COOH | H | H | 3,6-dicarboxypyridin-2-yl |
| IVa-022a | 2-(3-(carboxymethyl)-2,5-dihydroxybenzamido)pyridine-3,5-dicarboxylic acid | CH2COOH | H | H | 3,5-dicarboxypyridin-2-yl |

(continued)

| Compound No. | **Compound chemical names** | **R1** | **Ra** | **Rb** | **R9** |
|---|---|---|---|---|---|
| IVa-023a | 3-(3-(carboxymethyl)-2,5-dihydroxybenzamido)isonicotinic acid | CH2COOH | H | H | 4-carboxypyridin-3-yl |
| IVa-024a | 5-(3-(carboxymethyl)-2,5-dihydroxybenzamido)nicotinic acid | CH2COOH | H | H | 5-carboxypyridin-3-yl |
| IVa-025a | 5-(3-(carboxymethyl)-2,5-dihydroxybenzamido)picolinic acid | CH2COOH | H | H | 6-carboxypyridin-3-yl |
| IVa-026a | 3-(3-(carboxymethyl)-2,5-dihydroxybenzamido)picolinic acid | CH2COOH | H | H | 2-carboxypyridin-3-yl |
| IVa-027a | 5-(3-(carboxymethyl)-2,5-dihydroxybenzamido)-2-fluoroisonicotinic acid | CH2COOH | H | H | 4-carboxy-6-fluoropyridin-3-yl |
| IVa-028a | 4-(3-(carboxymethyl)-2,5-dihydroxybenzamido)nicotinic acid | CH2COOH | H | H | 3-carboxypyridin-4-yl |
| IVa-029a | 4-(3-(carboxymethyl)-2,5-dihydroxybenzamido)picolinic acid | CH2COOH | H | H | 2-carboxypyridin-3-yl |
| IVa-032a | (4-(3-(carboxymethyl)-2,5-dihydroxybenzamido)phenyl)acetic acid | CH2COOH | H | H | 4-(carboxymethyl)phenyl |
| IVa-033a | (3-(3-(carboxymethyl)-2,5-dihydroxybenzamido)phenyl)acetic acid | CH2COOH | H | H | 3-(carboxymethyl)phenyl |
| IVa-034a | (2-(3-(carboxymethyl)-2,5-dihydroxybenzamido)phenyl)acetic acid | CH2COOH | H | H | 2-(carboxymethyl)phenyl |

Preferably, the present invention provides a hydroquinone derivative compound of formula (I), wherein:

(A)

$R_a$, $R_b$ = H; $C_{1-4}$acyl, aryl$C_{1-4}$alkyl;
$R_1$ = CONH-$R_{10}$;
$R_2$ = H; $R_3$ = $R_5$; and
$R_5$ = $R_6$ = CONH-$R_9$;

Or

(B)

$R_a$, $R_b$ = H; $C_{1-4}$acyl, aryl$C_{1-4}$alkyl;

$R_1$ = CONH-$R_{10}$;

$R_3$ = H; $R_2$ = $R_5$; and

$R_5$ = $R_6$ = CONH-$R_9$

In a fourth embodiment, the present invention includes compounds in accordance with Table 4:

Table 4

| A. | 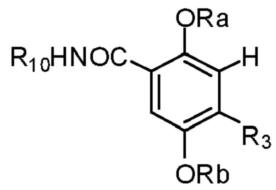<br>**R3 = R5 = R6 = CONH-$R_9$** | | | | |
|---|---|---|---|---|---|
| Compound No. | **Compound chemical names** | **Ra** | **Rb** | **R10** | **R9** |
| Ic-001a-Tz/ 1-004a | 5-(4-(2-(1H-tetrazol-5-yl)phenylaminocarbonyl)-2,5-dihydroxybenzamido)-2-hydroxy benzoic acid | H | H | 3-carboxy-4-hydroxyphenyl | 2-(1H-tetrazol-5-yl)phenyl |
| Ic-001a-Tz(2) | $N_1$,$N_4$-bis(2-(1H-tetrazol-5-yl)phenyl)-2,5-dihydroxyterephthalamide | H | H | 2-(1H-tetrazol-5-yl)phenyl | 2-(1H-tetrazol-5-yl)phenyl |
| Ic-007a | 5-(4-(3-carboxy-4-hydroxyphenylaminocarbonyl)-2,5-dihydroxybenzamido)-2-hydroxy benzoic acid; | H | H | 3-carboxy-4-hydroxyphenyl | 3-carboxy-4-hydroxyphenyl |
| Ic-007b | 5-(2,5-dihydroxy-4-(4-hydroxy-3-sulfophenylaminocarbonyl) benzami do)-2-hydroxy benzenesulphonic acid | H | H | 4-hydroxy-3-sulfonylphenyl | 4-hydroxy-3-sulfonylphenyl |
| Ic-008a | 4-(4-(4-carboxy-3-hydroxyphenylaminocarbonyl)-2,5-dihydroxybenzamido)-2-hydroxy benzoic acid | H | H | 4-carboxy-3-hydroxyphenyl | 4-carboxy-3-hydroxyphenyl |
| Ic-008b | 4-(2,5-dihydroxy-4-(3-hydroxy-4-sulfophenylaminocarbonyl) benzami do)-2-hydroxy benzenesulphonic acid | H | H | 3-hydroxy-4-sulfonylphenyl | 3-hydroxy-4-sulfonylphenyl |
| Ic-009a | 2-(2,5-dihydroxy-4-(4-hydroxy-2-carboxyphenylaminocarbonyl) benza mido)-5-hydroxy benzoic acid | H | H | 2-carboxy-4-hydroxyphenyl | 2-carboxy-4-hydroxyphenyl |
| Ic-009b | 2-(2,5-dihydroxy-4-(4-hydroxy-2-sulfophenylaminocarbonyl) benzami do)-5-hydroxy benzene sulphonic acid | H | H | 4-hydroxy-2-sulfophenyl | 4-hydroxy-2-sulfophenyl |
| Ib-001a-Tz/ 1-004a-E1 | methyl 5-(4-(2-(1H-tetrazol-5-yl)phenylaminocarbonyl)-2,5-dihydroxybenzamido)-2-hydroxybenzoate; | H | H | 4-hydroxy-3-(methoxycarbonyl)ph enyl- | 2-(1H-tetrazol-5-yl)phenyl |
| Ic-007a-E2-A4 | methyl 5-(2,5-diacetoxy-4-(4-acetoxy-3-(methoxycarbonyl) phenylaminocarb onyl) benzamido)-2-acetoxybenzoate; | Ac | Ac | 4-acetoxy-3-(methoxycarbonyl)ph enyl | 4-acetoxy-3-(methoxycarbonyl)phenyl |
| Ic-007a-E2 | methyl 5-(2,5-dihydroxy-4-(4-hydroxy-3-(methoxycarbonyl) phenylaminocarb onyl) benzamido)-2-hydroxybenzoate; | H | H | 4-hydroxy-3-(methoxycarbonyl)ph enyl | 4-hydroxy-3-(methoxycarbonyl)phenyl |

(continued)

| Compound No. | Compound chemical names | Ra | Rb | R10 | R9 |
|---|---|---|---|---|---|
| A. | 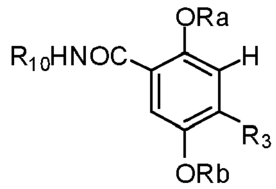 R3 = R5 = R6 = CONH-$R_9$ | | | | |
| Compound No. | **Compound chemical names** | **Ra** | **Rb** | **R10** | **R9** |
| Ic-009a-E2 | : methyl 2-(2,5-dihydroxy-4-(4-hydroxy-2-(methoxycarbonyl) phenylaminocarb onyl) benzamido)-5-hydroxybenzoate | H | H | 4-hydroxy-2-(methoxycarbonyl)ph enyl | 4-hydroxy-2-(methoxycarbonyl)phenyl |
| B. | 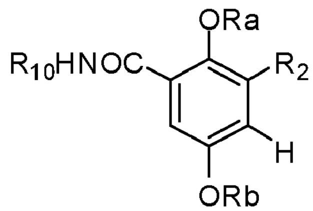 R2 = R5 = R6 = CONH-R9 | | | | |
| IIc-007a | 5-(3-(3-carboxy-4-hydroxyphenylaminocarbonyl)-2,5-dihydroxybenzamido)-2-hydroxy benzoic acid | H | H | 3-carboxy-4-hydroxyphenyl | 3-carboxy-4-hydroxyphenyl |
| IIc-007b | 5-(2,5-dihydroxy-3-(4-hydroxy-3-sulfophenylaminocarbonyl) benzami do)-2-hydroxy benzenesulphonic acid | H | H | 4-hydroxy-3-sulfophenyl | 4-hydroxy-3-sulfophenyl |
| IIc-008a | 4-(3-(3-hydroxy-4-carboxyphenylaminocarbonyl)-2,5-dihydroxybenzamido)-2-hydroxy benzoic acid | H | H | 3-hydroxy-4-carboxyphenyl | 3-hydroxy-4-carboxyphenyl |
| IIc-008b | 4-(2,5-dihydroxy-3-(3-hydroxy-4-sulfophenylaminocarbonyl) benzami do)-2-hydroxy benzenesulphonic acid | H | H | 3-hydroxy-4-sulfophenyl | 3-hydroxy-4-sulfophenyl |
| IIc-009a | 2-(3-(2-carboxy-4-hydroxyphenylaminocarbonyl)-2,5-dihydroxybenzamido)-5-hydroxyl benzoic acid | H | H | 2-carboxy-4-hydroxyphenyl | 2-carboxy-4-hydroxyphenyl |
| IIc-009b | 2-(2,5-dihydroxy-3-(4-hydroxy-2-sulfophenylaminocarbonyl) benzami do)-5-hydroxy benzenesulphonic acid | H | H | 4-hydroxy-2-sulfophenyl | 4-hydroxy-2-sulfophenyl |

Preferably, the present invention provides a hydroquinone derivative compounds of formula (I), wherein:

(A)

$R_a$, $R_b$ = H;

$R_1$ = $COOR_4$; $(CH_2)_nCOOR_4$; $R_4$ = H or $C_{1-4}$alkyl;

$R_2$ = H; $R_3$ = $R_5$; and

$R_5$ = $R_6$ = $CONHCOR_9$;

Or

(B)

$R_a$, $R_b$ = H;

$R_1$ = $COOR_4$; $(CH_2)_nCOOR_4$; $R_4$ = H or $C_{1-4}$alkyl;

$R_3$ = H; $R_2$ = $R_5$; and

$R_5$ = $R_6$ = $CONHCOR_9$.

In a fifth embodiment, the present invention includes compounds in accordance with Table 5:

Table 5

| A. | 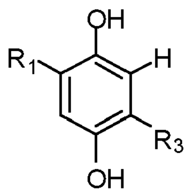 **R3 = R5 = R6 = CONHCOR$_9$** | | |
|---|---|---|---|
| Compound No. | **Compound chemical names** | **R1** | **R9** |
| Ia-040a | N-(1,3,4,5-tetrahydroxycyclohexylcarbonyl) 4-carboxy-2,5-dihydroxybenzamide | COOH | 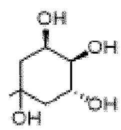 |
| Ia-041a | N-(4-carboxy-2,5-dihydroxybenzoyl) 4-carboxy-2,5-dihydroxy-benzamide | COOH | 2,5-dihydroxy-4-carboxyphenyl |
| Ia-042a | N-(3-carboxy-2,5-dihydroxybenzoyl)4-carboxy-2,5-dihydroxy-benzamide | COOH | 2,5-dihydroxy-3-carboxyphenyl |
| Ia-043a | N-(3,4-dihydroxybenzoyl) 4-carboxy-2,5-dihydroxybenzamide | COOH | 3,4-dihydroxyphenyl |
| Ia-044a | N-(3,5-dihydroxybenzoyl) 4-carboxy-2,5-dihydroxybenzamide | COOH | 3,5-dihydroxyphenyl |
| IIIa-040a | N-(1,3,4,5-tetrahydroxycyclohexylcarbonyl) 3-carboxy-2,5-dihydroxybenzamide | CH2COOH | 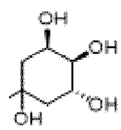 |

(continued)

| | | | |
|---|---|---|---|
| A. | 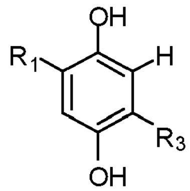 R3 = R5 = R6 = $CONHCOR_9$ | | |
| Compound No. | **Compound chemical names** | **R1** | **R9** |
| IIIa-041a | N-(4-carboxy-2,5-dihydroxybenzoyl) 3-carboxy-2,5-dihydroxy-benzamide | CH2COOH | 2,5-dihydroxy-4-carboxyphenyl |
| IIIa-042a | N-(3-carboxy-2,5-dihydroxybenzoyl)3-carboxy-2,5-dihydroxy-benzamide | CH2COOH | 2,5-dihydroxy-3-carboxyphenyl |
| IIIa-043a | N-(3,4-dihydroxybenzoyl) 3-carboxy-2,5-dihydroxybenzamide | CH2COOH | 3,4-dihydroxyphenyl |
| IIIa-044a | N-(3,5-dihydroxybenzoyl) 3-carboxy-2,5-dihydroxybenzamide | CH2COOH | 3,5-dihydroxyphenyl |
| B. | 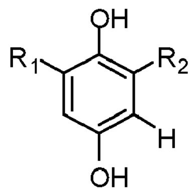 R2 = R5 = R6 = CONHCOR9 | | |
| IIa-040a | N-(1,3,4,5-tetrahydroxycyclohexylcarbonyl) 4-carboxymethyl-2,5-dihydroxybenzamide | COOH | 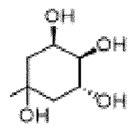 |
| IIa-041a | N-(4-carboxy-2,5-dihydroxybenzoyl) 4-carboxymethyl-2,5-dihydroxy-benzamide | COOH | 2,5-dihydroxy-4-carboxyphenyl |
| IIa-042a | N-(3-carboxy-2,5-dihydroxybenzoyl) 4-carboxymethyl-2,5-dihydroxy-benzamide | COOH | 2,5-dihydroxy-3-carboxyphenyl |
| IIa-043a | N-(3,4-dihydroxybenzoyl) 4-carboxymethyl-2,5-dihydroxybenzamide | COOH | 3,4-dihydroxyphenyl |
| IIa-044a | N-(3,5-dihydroxybenzoyl) 4-carboxymethyl-2,5-dihydroxybenzamide | COOH | 3,5-dihydroxyphenyl |
| IVa-040a | N-(1,3,4,5-tetrahydroxycyclohexylcarbonyl) 3-carboxymethyl-2,5-dihydroxybenzamide | CH2COOH | 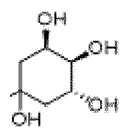 |
| IVa-041a | N-(4-carboxy-2,5-dihydroxybenzoyl) 3-carboxymethyl-2,5-dihydroxy-benzamide | CH2COOH | 2,5-dihydroxy-4-carboxyphenyl |
| IVa-042a | N-(3-carboxy-2,5-dihydroxybenzoyl)3-carboxymethyl-2,5-dihydroxy-benzamide | CH2COOH | 2,5-dihydroxy-3-carboxyphenyl |
| IVa-043a | N-(3,4-dihydroxybenzoyl) 3-carboxymethyl-2,5-dihydroxybenzamide | CH2COOH | 3,4-dihydroxyphenyl |

(continued)

| A. | 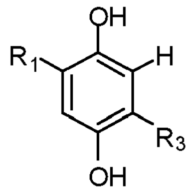 $R3 = R5 = R6 = CONHCOR_9$ | | |
|---|---|---|---|
| Compound No. | **Compound chemical names** | **R1** | **R9** |
| IVa-044a | N-(3,5-dihydroxybenzoyl) 3-carboxymethyl-2,5-dihydroxybenzamide | CH2COOH | 3,5-dihydroxyphenyl |

Preferably, the present invention provides a hydroquinone derivative compound of formula (I), wherein

(A)

$R_a$, $R_b$ = H;

$R_1$ = $COOR_4$; $(CH_2)_nCOOR_4$; $R_4$ = H or $C_{1-4}$alkyl;

$R_2$ = H; $R_3$ = $R_5$; and

$R_5$ = $R_6$ = $CONH(CH_2)_n$-$R_9$;

Or

(B)

$R_a$, $R_b$ = H;

$R_1$ = $COOR_4$; $(CH_2)_nCOOR_4$; $R_4$ = H or $C_{1-4}$alkyl;

$R_3$ = H; $R_2$ = $R_5$; and

$R_5$ = $R_6$ = $CONH(CH_2)_n$-$R_9$.

In a sixth embodiment, the present invention includes compounds in accordance with Table 6:

Table 6

| A. | 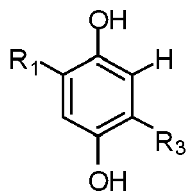 $R3 = R5 = R6 = CONH(CH_2)_n$-$R_9$ | | | |
|---|---|---|---|---|
| Compound No. | **Compound chemical names** | **R1** | **n** | **R9** |
| Ia-035a | 4-(3,4-dihydroxyphenylmethylaminocarbonyl)-2,5-dihydroxybenzoic acid; | COOH | 1 | 3,4-dihydroxyphenyl |

(continued)

| | | | | |
|---|---|---|---|---|
| A. | 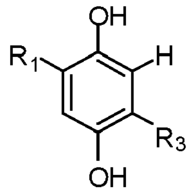<br>**R3 = R5 = R6 = CONH($CH_2$)$_n$-$R_9$** | | | |
| Compound No. | **Compound chemical names** | **R1** | **n** | **R9** |
| Ia-035a-2 | 4-(2-(3,4-dihydroxyphenyl)ethylaminocarbonyl)-2,5-dihydroxybenzoic acid; | COOH | 2 | 3,4-dihydroxyphenyl |
| Ia-037a | 4-(3,5-dihydroxyphenylmethylaminocarbonyl)-2,5-dihydroxybenzoic acid; | COOH | 1 | 3,5-dihydroxyphenyl |
| Ia-038a | 4-((4,5-dihydroxy-3-oxocyclohex-1-enyl) methylaminocarbonyl)-2,5-dihydroxybenzoic acid; | COOH | 1 | 4,5-dihydroxy-3-oxocyclohex-1-enyl) |
| Ia-039a | 2,5-dihydroxy-4-((3,4,5-trihydroxycyclohexyl) methylaminocarbonyl)benzoic acid | COOH | 1 | 3,4,5-trihydroxycyclohexyl) |
| Ia-053a | 4-(2-carboxyphenylmethylaminocarbonyl)-2,5-dihydroxybenzoic acid | COOH | 1 | 2-carboxyphenyl |
| Ia-054a | 4-(3-carboxyphenylmethylaminocarbonyl)-2,5-dihydroxybenzoic acid | COOH | 1 | 3-carboxyphenyl |
| Ia-055a | 4-(4-carboxyphenylmethylaminocarbonyl) 2,5-dihydroxybenzoic acid | COOH | 1 | 4-carboxyphenyl |
| IIIa-035a | 3-(3,4-dihydroxyphenylmethylaminocarbonyl)-2,5-dihydroxybenzoic acid | CH2COOH | 1 | 3,4-dihydroxyphenyl |
| IIIa-037a | 3-(2-(3,4-dihydroxyphenyl)ethylaminocarbonyl)-2,5-dihydroxybenzoic acid | CH2COOH | 1 | 3,5-dihydroxyphenyl |
| IIIa-038a | 3-(3,5-dihydroxyphenylmethylaminocarbonyl)-2,5-dihydroxybenzoic acid | CH2COOH | 1 | 4-((4,5-dihydroxy-3-oxocyclohex-1-enyl) |
| IIIa-039a | 3-((4,5-dihydroxy-3-oxocyclohex-1-enyl) methylaminocarbonyl)-2,5-dihydroxybenzoic acid | CH2COOH | 1 | 3,4,5-trihydroxycyclohexyl) |
| IIIa-053a | 2,5-dihydroxy-3-((3,4,5-trihydroxycyclohexyl) methylaminocarbonyl)benzoic acid | CH2COOH | 1 | 2-carboxyphenyl |
| IIIa-054a | 3-(2-carboxyphenylmethylaminocarbonyl)-2,5-dihydroxybenzoic acid | CH2COOH | 1 | 3-carboxyphenyl |
| IIIa-055a | 3-(3-carboxyphenylmethylaminocarbonyl)-2,5-dihydroxybenzoic acid | CH2COOH | 1 | 4-carboxyphenyl |
| B. | 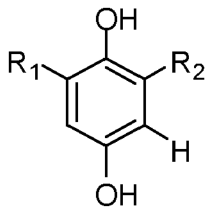<br>**R2 = R5 = R6 = CONH(CH2)n-R9** | | | |

(continued)

| A. | 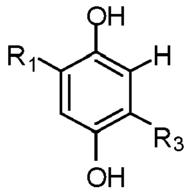 R3 = R5 = R6 = CONH$(CH_2)_n$-$R_9$ | | | |
|---|---|---|---|---|
| Compound No. | **Compound chemical names** | **R1** | **n** | **R9** |
| IIa-035a | 3-(4-carboxyphenylmethylaminocarbonyl)-2,5-dihydroxybenzoic acid | COOH | 1 | 3,4-dihydroxyphenyl |
| IIa-035a-2 | (4-(3,4-dihydroxyphenylmethylaminocarbonyl)-2,5-dihydroxyphenyl)acetic acid | COOH | 2 | 3,4-dihydroxyphenyl |
| IIa-037a | (4-(3,5-dihydroxyphenylmethylaminocarbonyl)-2,5-dihydroxyphenyl)acetic acid | COOH | 1 | 3,5-dihydroxyphenyl |
| IIa-038a | (4-((4,5-dihydroxy-3-oxocyclohex-1-enyl) methylaminocarbonyl)-2,5-dihydroxyphenyl) acetic acid | COOH | 1 | 4,5-dihydroxy-3-oxocyclohex-1-enyl) |
| IIa-039a | Compound 191: (2,5-dihydroxy-4-((3,4,5-trihydroxycyclohexyl)methylaminocarbonyl) phenyl)acetic acid | COOH | 1 | 3,4,5-trihydroxycyclohexyl |
| IIa-053a | (4-(2-carboxyphenylmethylaminocarbonyl)-2,5-dihydroxyphenyl) acetic acid | COOH | 1 | 2-carboxyphenyl |
| IIa-054a | (4-(3-carboxyphenylmethylaminocarbonyl)-2,5-dihydroxyphenyl)acetic acid | COOH | 1 | 3-carboxyphenyl |
| IIa-055a | (4-(4-carboxyphenylmethylaminocarbonyl)-2,5-dihydroxyphenyl)acetic acid | COOH | 1 | 4-carboxyphenyl |
| IVa-035a | (3-(3,4-dihydroxyphenylmethylaminocarbonyl)-2,5-dihydroxyphenyl)acetic acid | CH2COOH | 1 | 3,4-dihydroxyphenyl |
| IVa-037a | (3-(3,5-dihydroxyphenylmethylaminocarbonyl)-2,5-dihydroxyphenyl)acetic acid | CH2COOH | 1 | 3,5-dihydroxyphenyl |
| IVa-038a | (3-((4,5-dihydroxy-3-oxocyclohex-1-enyl) methylaminocarbonyl)-2,5-dihydroxyphenyl) acetic acid | CH2COOH | 1 | 4,5-dihydroxy-3-oxocyclohex-1-enyl) |
| IVa-039a | (2,5-dihydroxy-3-((3,4,5-trihydroxycyclohexyl) methylcarbonylamino)phenyl)acetic acid | CH2COOH | 1 | 3,4,5-trihydroxycyclohexyl |
| IVa-053a | (3-(2-carboxyphenylmethylaminocarbonyl)-2,5-dihydroxyphenyl)acetic acid | CH2COOH | 1 | 2-carboxyphenyl |
| IVa-054a | (3-(3-carboxyphenylmethylaminocarbonyl)-2,5-dihydroxyphenyl)acetic acid | CH2COOH | 1 | 3-carboxyphenyl |
| IVa-055a | (3-(4-carboxyphenylmethylaminocarbonyl)-2,5-dihydroxyphenyl)acetic acid | CH2COOH | 1 | 4-carboxyphenyl |

Preferably, the present invention provides a hydroquinone derivative compound of formula (I), wherein:

$R_a$, $R_b$ = H;

$R_1$ = $SO_3H$;

$R_2$ = H; $R_3$ = $R_5$; and

$R_5$ = $R_6$ = CONH$(CH_2)_n$-$R_9$.

According to a seventh embodiment, the present invention includes compounds in accordance with Table 7 below:

Table 7

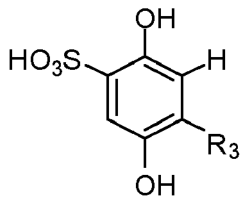

**R3 = R5 = CONH$(CH_2)_n$-$R_9$**

| Compound No. | Compound chemical names | n | R9 |
|---|---|---|---|
| Ia-053b | 2-((2,5-dihydroxy-4-sulfobenzamido)methyl)benzoic acid | 1 | 2-carboxyphenyl |
| Ia-054b | 3-((2,5-dihydroxy-4-sulfobenzamido)methyl)benzoic acid | 1 | 3-carboxyphenyl |
| Ia-055b | 4-((2,5-dihydroxy-4-sulfobenzamido)methyl)benzoic acid | 1 | 4-carboxyphenyl |

Preferably, the present invention relates to a hydroquinone derivative compound of formula (I) wherein:

(A)

$R_a$, $R_b$ = H;
$R_1$ = $COOR_4$; $(CH_2)_nCOOR_4$; $R_4$ = H or $C_{1-4}$alkyl;
$R_2$ = H; $R_3$ = $R_5$; and
$R_5$ = $R_6$ = CONHCH$(COOR_4)(CH_2)_k$-$R_9$;

Or

(B)

$R_a$, $R_b$ = H;
$R_1$ = $COOR_4$; $(CH_2)_nCOOR_4$; $R_4$ = H or $C_{1-4}$alkyl;
$R_3$ = H; $R_2$ = $R_5$; and
$R_5$ = $R_6$ = CONHCH$(COOR_4)(CH_2)_k$-$R_9$.

According to an eight embodiment, the present invention includes compounds in accordance with Table 8 below:

Table 8

| | | | | | |
|---|---|---|---|---|---|
| A. | 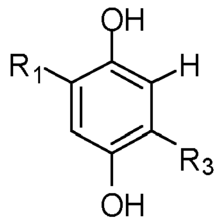 $R_3 = R_5 = R_6 = CONHCH(COOR_4)(CH_2)_kR_9$ | | | | |
| Compound No. | **Compound chemical names** | **R1** | **R4** | **k** | **R9** |
| Ia-035a-3 | 4-(1-carboxy-2-(3,4-dihydroxyphenyl) ethylaminocarbonyl)-2,5-dihydroxybenzoic acid | COOH | H | 1 | 3,4-dihydroxyphenyl |
| Ia-036a | 4-(carboxy(3,4-dihydroxyphenyl) methylaminocarbonyl)-2,5-dihydroxybenzoic acid | COOH | H | 0 | 3,4-dihydroxyphenyl |
| IIIa-036a | 3-(1-carboxy-2-(3,4-dihydroxyphenyl) ethylaminocarbonyl)-2,5-dihydroxybenzoic acid | CH2COOH | H | 0 | 3,4-dihydroxyphenyl |
| B. | 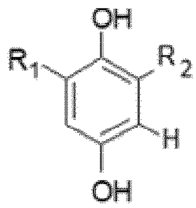 $R_2 = R_5 = R_6 = CONHCH(COOR_4)(CH_2)_kR_9$ | | | | |
| IIa-035a-3 | 3-(carboxy(3,4-dihydroxyphenyl) methylaminocarbonyl)-2,5-dihydroxybenzoic acid | COOH | H | 1 | 3,4-dihydroxyphenyl |
| IIa-036a | (4-(carboxy(3,4-dihydroxyphenyl) methylaminocarbonyl)-2,5-dihydroxyphenyl)acetic acid | COOH | H | 0 | 3,4-dihydroxyphenyl |
| IVa-036a | (3-(carboxy(3,4-dihydroxyphenyl) methylaminocarbonyl)-2,5-dihydroxyphenyl)acetic acid | CH2COOH | H | 0 | 3,4-dihydroxyphenyl |

In a specific embodiment, the present invention provides a hydroquinone derivative compounds of formula (I), wherein

(A)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl,
$R_2$ = H; $R_3$ = $R_5$; and
$R_5$ = $R_6$ = heteroaryl-$R_9$;

Or

(B)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl;
$R_3$ = H; $R_2$ = $R_5$; and
$R_5$ = $R_6$ = heteroaryl-$R_9$.

According to a ninth embodiment, the present invention includes compounds in accordance with Table 9:

Table 9

| A. | 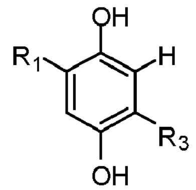 **R3 = R5 = R6 = heteroaryl-R9** | | |
|---|---|---|---|
| Compound No. | **Compound chemical names** | **R1** | **Heteroaryl-R9** |
| Ia-045a | 4,6-Bis(4-carboxy-2,5-dihydroxyphenyl)-1,3,5-triazin-2-one | COOH | 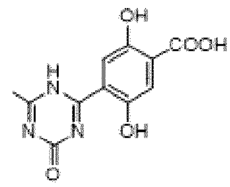 |
| Ia-046a | 4-(4-carboxy-2,5-dihydroxyphenyl)-6-(3-carboxy-2,5-dihydroxyphenyl)-1,3,5-triazin-2-one | COOH | 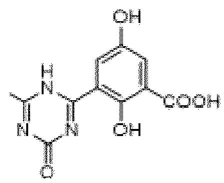 |
| Ia-047a | 4-(4-carboxy-2,5-dihydroxyphenyl)-6-(3,4-dihydroxyphenyl)-1,3,5-triazin-2-one | COOH | 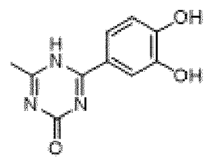 |
| Ia-048a | 4-(4-carboxy-2,5-dihydroxyphenyl)-6-(3,5-dihydroxyphenyl)-1,3,5-triazin-2-one | COOH | 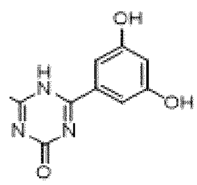 |
| Ia-049a | 4,6-Bis(4-carboxy-2,5-dihydroxyphenyl)-1,3,5-triazine | COOH | 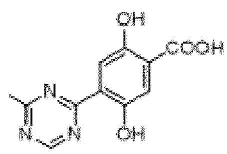 |
| Ia-050a | 2-(4-Carboxy-2,5-dihydroxyphenyl)-4-(3-carboxy-2,5-dihydroxyphenyl)-1,3,5-triazine | COOH | 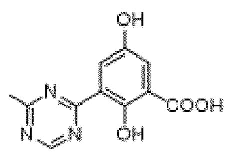 |

(continued)

| A. | 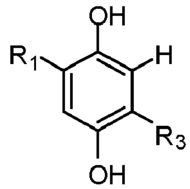<br>**R3 = R5 = R6 = heteroaryl-R9** | | |
|---|---|---|---|
| Compound No. | **Compound chemical names** | **R1** | **Heteroaryl-R9** |
| Ia-051a | 2-(4-Carboxy-2,5-dihydroxyphenyl)-4-(3,4-dihydroxyphenyl)-1,3,5-triazine | COOH | 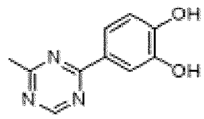 |
| Ia-052a | 2-(4-Carboxy-2,5-dihydroxyphenyl)-4-(3,5-dihydroxyphenyl)-1,3,5-triazine | COOH | 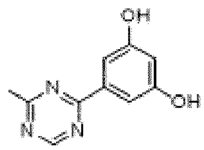 |
| IIa-046a | 4,6-Bis(3-carboxy-2,5-dihydroxyphenyl)-1,3,5-triazin-2-one | COOH | 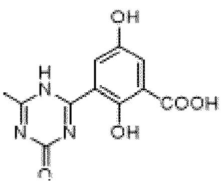 |
| IIa-050a | 4-(3-Carboxy-2,5-dihydroxyphenyl)-6-(4-carboxy-2,5-dihydroxyphenyl)-1,3,5-triazin-2-one | COOH | 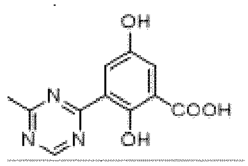 |
| IIIa-045a | 4-(3-Carboxy-2,5-dihydroxyphenyl)-6-(3,4-dihydroxyphenyl)-1,3,5-triazin-2-one | CH2COOH | 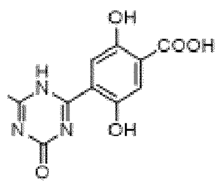 |
| IIIa-046a | 4-(3-Carboxy-2,5-dihydroxyphenyl)-6-(3,5-dihydroxyphenyl)-1,3,5-triazin-2-one | CH2COOH | 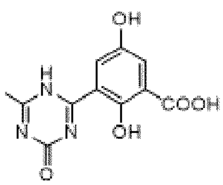 |
| IIIa-047a | 4,6-Bis(3-carboxy-2,5-dihydroxyphenyl)-1,3,5-triazine | CH2COOH | 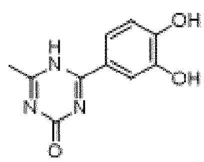 |

(continued)

| A. | 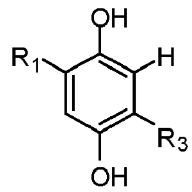<br>R3 = R5 = R6 = heteroaryl-R9 | | |
|---|---|---|---|
| Compound No. | **Compound chemical names** | **R1** | **Heteroaryl-R9** |
| IIIa-048a | 2-(3-Carboxy-2,5-dihydroxyphenyl)-4-(4-carboxy-2,5-dihydroxyphenyl)-1,3,5-triazine | CH2COOH | 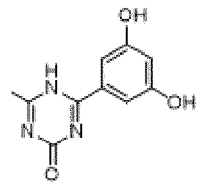 |
| IIIa-049a | 2-(3-Carboxy-2,5-dihydroxyphenyl)-4-(3,4-dihydroxyphenyl)-1,3,5-triazine | CH2COOH | 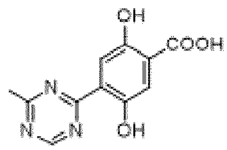 |
| IIIa-050a | 2-(3-Carboxy-2,5-dihydroxyphenyl)-4-(3,5-dihydroxyphenyl)-1,3,5-triazine | CH2COOH | 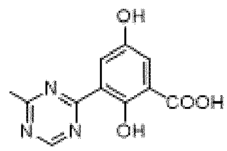 |
| IIIa-051a | 2-(3-Carboxymethyl-2,5-dihydroxyphenyl)-4-(3,4-dihydroxyphenyl)-1,3,5-triazine | CH2COOH | 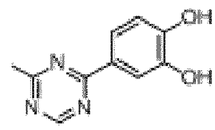 |
| IIIa-052a | 2-(3-Carboxymethyl-2,5-dihydroxyphenyl)-4-(3,5-dihydroxyphenyl)-1,3,5-triazine | CH2COOH | 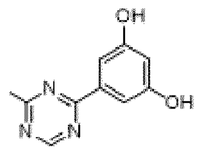 |
| B. | 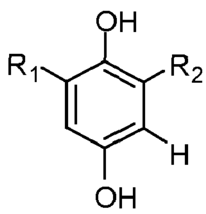<br>R2 = R5 = R6 = heteroaryl-R9 | | |
| IIa-045a | 4-(4-Carboxymethyl-2,5-dihydroxyphenyl)-6-(3,4-dihydroxyphenyl)-1,3,5-triazin-2-one | COOH | 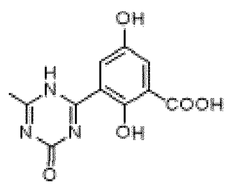 |

(continued)

| B. | 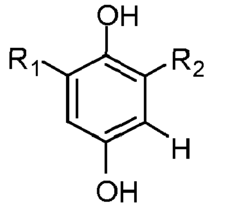<br>R2 = R5 = R6 = heteroaryl-R9 | | |
|---|---|---|---|
| IIa-047a | 4-(4-Carboxymethyl-2,5-dihydroxyphenyl)-6-(3,5-dihydroxyphenyl)-1,3,5-triazin-2-one | COOH | 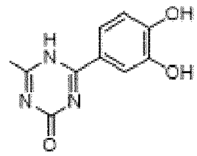 |
| IIa-048a | 2-(4-Carboxy-2,5-dihydroxyphenyl)-4-(4-carboxymethyl-2,5-dihydroxyphenyl)-1,3,5-triazine | COOH | 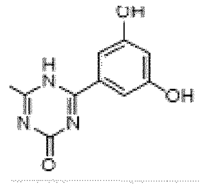 |
| IIa-049a | 2-(3-Carboxy-2,5-dihydroxyphenyl)-4-(4-carboxymethyl-2,5-dihydroxyphenyl)-1,3,5-triazine | COOH | 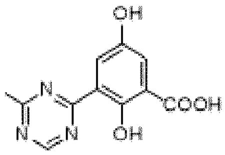 |
| IIa-051a | 2-(4-Carboxymethyl-2,5-dihydroxyphenyl)-4-(3,4-dihydroxyphenyl)-1,3,5-triazine | COOH | 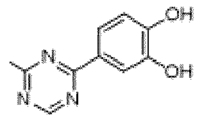 |
| IIa-052a | 2-(4-Carboxymethyl-2,5-dihydroxyphenyl)-4-(3,5-dihydroxyphenyl)-1,3,5-triazine | COOH | 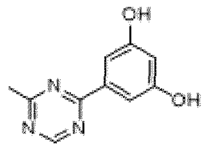 |
| IVa-045a | 4-(4-Carboxy-2,5-dihydroxyphenyl)-6-(3-carboxymethyl-2,5-dihydroxyphenyl)-1,3,5-triazin-2-one | CH2COOH | 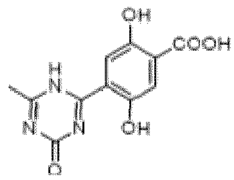 |
| IVa-046a | 4-(3-Carboxy-2,5-dihydroxyphenyl)-6-(3-carboxymethyl-2,5-dihydroxyphenyl)-1,3,5-triazin-2-one | CH2COOH | 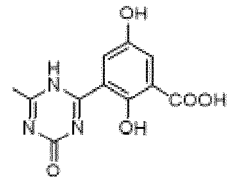 |
| IVa-047a | 4-(3-Carboxymethyl-2,5-dihydroxyphenyl)-6-(3,4-dihydroxyphenyl)-1,3,5-triazin-2-one | CH2COOH | 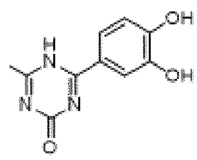 |

(continued)

| | | | |
|---|---|---|---|
| B. | 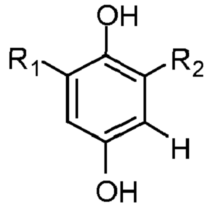 R2 = R5 = R6 = heteroaryl-R9 | | |
| IVa-048a | 4-(3-Carboxymethyl-2,5-dihydroxyphenyl)-6-(3,5-dihydroxyphenyl)-1,3,5-triazin-2-one | CH2COOH | 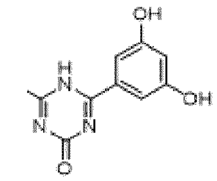 |
| IVa-049a | 2-(4-Carboxy-2,5-dihydroxyphenyl)-4-(3-carboxymethyl-2,5-dihydroxyphenyl)-1,3,5-triazine | CH2COOH | 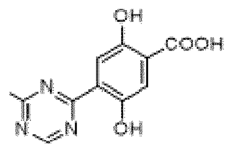 |
| IVa-050a | 2-(3-Carboxy-2,5-dihydroxyphenyl)-4-(3-carboxymethyl-2,5-dihydroxyphenyl)-1,3,5-triazine | CH2COOH | 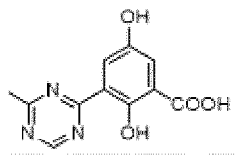 |
| IVa-051a | 2-(3-Carboxymethyl-2,5-dihydroxyphenyl)-4-(3,4-dihydroxyphenyl)-1,3,5-triazine | CH2COOH | 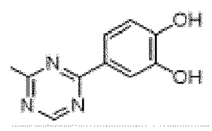 |
| IVa-052a | 2-(3-Carboxymethyl-2,5-dihydroxyphenyl)-4-(3,5-dihydroxyphenyl)-1,3,5-triazine | CH2COOH | 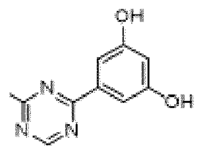 |

Preferably, the present invention provides a hydroquinone derivative compound of formula (I), wherein:

(A)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl,
$R_2$ = H; $R_3$ = $R_5$; and
$R_5$ = $R_7$ = benzoheteroaryl optionally substituted with one or more groups selected from: $COOR_4$, $(CH_2)_nCOOR_4$, $SO_3H$, $(CH_2)_nSO_3H$, $OR_4$, azoles [5-membered N-containing heterocycles], fluorine;

Or

(B)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl;

$R_3$ = H; $R_2$ = $R_5$; and
$R_5$ = $R_7$ = benzoheteroaryl optionally substituted with one or more groups selected from: $COOR_4$, $(CH_2)_nCOOR_4$, $SO_3H$, $(CH_2)_nSO_3H$, $OR_4$, azoles [5-membered N-containing heterocycles], fluorine.

According to a tenth embodiment, the present invention includes compounds in accordance with Table 10:

Table 10

| | | | |
|---|---|---|---|
| A. | 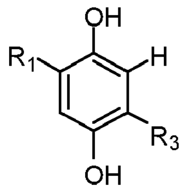 **R3 = R5 = R7** | | |
| Compound No. | **Compound chemical names** | **R1** | **R3** |
| Id-030a | 2-(4-Carboxy-2,5-dihydroxyphenyl)-1H-benzo[d] imidazole-4-carboxylic acid | COOH | 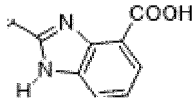 |
| Id-030a-E2 | Methyl 2-(4-ethoxycarbonyl-2,5-dihydroxyphenyl)-1H-benzo[d]imidazole-4-carboxylate | COOEt | 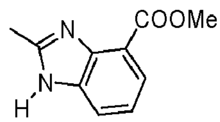 |
| Id-030b | 2-(2,5-Dihydroxy-4-sulfophenyl)-1H-benzo[d] imidazole-4-carboxylic acid | SO3H | 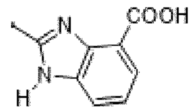 |
| Id-031a | 2-(4-Carboxy-2,5-dihydroxyphenyl)-1H-benzo[d] imidazole-5-carboxylic acid | COOH | 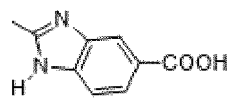 |
| Id-031b | 2-(2,5-Dihydroxy-4-sulfophenyl)-1H-benzo[d] imidazole-5-carboxylic acid | SO3H | 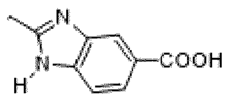 |
| IIId-030a | 2-(3-Carboxy-2,5-dihydroxyphenyl)-1H-benzo[d] imidazole-4-carboxylic acid | CH2COOH | 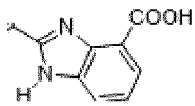 |
| IIId-031a | 2-(2,5-Dihydroxy-3-sulfophenyl)-1H-benzo[d] imidazole-4-carboxylic acid | CH2COOH | 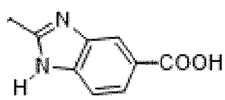 |
| B. | 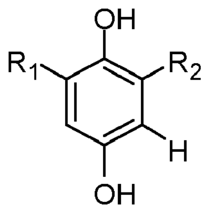 **R2 = R5 = R7** | | |
| IId-030a | 2-(3-Carboxy-2,5-dihydroxyphenyl)-1H-benzo[d] imidazole-5-carboxylic acid | COOH | 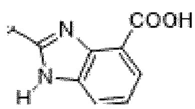 |

(continued)

| B. | 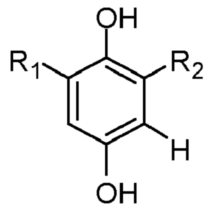 **R2 = R5 = R7** | | |
|---|---|---|---|
| IId-030b | 2-(2,5-Dihydroxy-3-sulfophenyl)-1H-benzo[d] imidazole-5-carboxylic acid | SO3H | 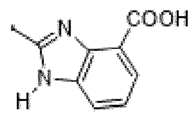 |
| IId-031a | 2-(4-(Carboxymethyl)-2,5-dihydroxyphenyl)-1H-benzo[d]imidazole-4-carboxylic acid | COOH | 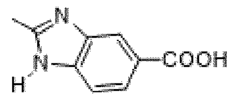 |
| IId-030a | 2-(4-(Carboxymethyl)-2,5-dihydroxyphenyl)-1H-benzo[d]imidazole-5-carboxylic acid | SO3H | 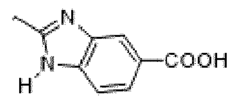 |
| IVd-030a | 2-(3-(Carboxymethyl)-2,5-dihydroxyphenyl)-1H-benzo[d]imidazole-4-carboxylic acid | CH2COOH | 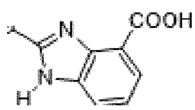 |
| IVd-031a | 2-(3-(Carboxymethyl)-2,5-dihydroxyphenyl)-1H-benzo[d]imidazole-5-carboxylic acid | CH2COOH | 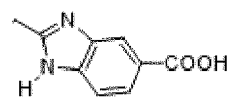 |

Preferably, the present invention provides a hydroquinone derivative compound of formula (I), wherein:

(A)

$R_a$, $R_b$ = H, $C_{1-4}$acyl, aryl$C_{1-4}$alkyl;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl;
$R_2$ = H; $R_3$ = $R_5$; and
$R_5$ = $R_8$ = $(CH_2)_mX(CH_2)_pR_9$;
wherein X = O, S, $SO_2$, NH, NAc, $N(CH_2)_qR_9$;

Or

(B)

$R_a$, $R_b$ = H, $C_{1-4}$acyl, aryl$C_{1-4}$alkyl;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl, aryl$C_{1-4}$alkyl,
$R_3$ = H; $R_2$ = $R_5$; and
$R_5$ = $R_8$ = $(CH_2)_mX(CH_2)_pR_9$;
wherein X = O, S, $SO_2$, NH, NAc, $N(CH_2)_qR_9$.

According to an eleventh embodiment, the present invention includes compounds in accordance with Table 11:

Table 11

| A. | 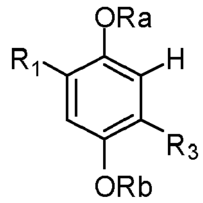 $R3 = (CH_2)_mX(CH_2)_pR_9$ | | | | | | | |
|---|---|---|---|---|---|---|---|---|
| No. | **Compound chemical names** | **R1** | **Ra** | **Rb** | **m** | **X** | **p** | **R9** |
| IIIc-056a | Bis(4-carboxy-2,5-dihydroxyphenylmethyl)amine | COOH | H | H | 1 | NH | 1 | 4-carboxy-2,5-dihydroxyphenyl |
| IIIc-056b | 4-((2,5-Dihydroxy-4-sulfophenylmethylamino) methyl)-2,5-dihydroxybenzoic acid | SO3H | H | H | 1 | NH | 1 | 4-carboxy-2,5-dihydroxyphenyl |
| IIIc-056c | Compound 267: 4-((2,5-Dihydroxy-3-sulfophenylmethylamino)methyl)-2,5-dihydroxybenzoic acid | COOH | H | H | 1 | NH | 1 | 2,5-dihydroxy-3-sulfophenyl |
| IIIc-056d | Bis(2,5-dihydroxy-4-sulfophenylmethyl)amine | SO3H | H | H | 1 | NH | 1 | 2,5-dihydroxy-4-sulfophenyl |
| IIIc-057a | N,N-Bis(4-carboxy-2,5-dihydroxyphenylmethyl) acetamide | COOH | H | H | 1 | NH(COCH3) | 1 | 4-carboxy-2,5-dihydroxyphenyl |
| IIIc-057b | N-(2,5-dihydroxy-4-sulfophenylmethyl) N-(4-carboxy-2,5-dihydroxyphenylmethyl) acetamide | SO3H | H | H | 1 | NH(COCH3) | 1 | 4-carboxy-2,5-dihydroxyphenyl |
| IIIc-057c | N-(2,5-dihydroxy-3-sulfophenylmethyl) N-(4-carboxy-2,5-dihydroxyphenylmethyl) acetamide | COOH | H | H | 1 | NH(COCH3) | 1 | 2,5-dihydroxy-3-sulfophenyl |
| IIIc-057d | N,N-Bis(2,5-dihydroxy-4-sulfophenylmethyl) acetamide | SO3H | H | H | 1 | NH(COCH3) | 1 | 2,5-dihydroxy-4-sulfophenyl |
| IIIc-058a | 4-((2,5-Dihydroxy-4-carboxyphenyl) methylthiomethyl)-2,5-dihydroxybenzoic acid | COOH | H | H | 1 | S | 1 | 4-carboxy-2,5-dihydroxyphenyl |
| IIIc-058b | 4-((2,5-Dihydroxy-4-sulfophenyl)methylthiomethyl)-2,5-dihydroxybenzoic acid | SO3H | H | H | 1 | S | 1 | 4-carboxy-2,5-dihydroxyphenyl |
| IIIc-058c | 4-((2,5-Dihydroxy-3-sulfophenyl)methylthiomethyl)-2,5-dihydroxybenzoic acid | COOH | H | H | 1 | S | 1 | 2,5-dihydroxy-3-sulfophenyl |

(continued)

| A. | 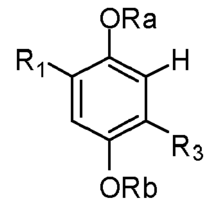 R3 = $(CH_2)_mX(CH_2)_pR_9$ | | | | | | | |
|---|---|---|---|---|---|---|---|---|
| No. | Compound chemical names | R1 | Ra | Rb | m | X | p | R9 |
| IIIc-058d | 4-((2,5-Dihydroxy-4-sulfophenyl)methylthiomethyl)-2,5-dihydroxybenzenesulfonic acid; | SO3H | H | H | 1 | S | 1 | 2,5-dihydroxy-4-sulfophenyl |
| IIIc-059a | 4-((2,5-Dihydroxy-4-carboxyphenyl) methylsulfonylmethyl)-2,5-dihydroxybenzoic acid; | COOH | H | H | 1 | SO2 | 1 | 4-carboxy-2,5-dihydroxyphenyl |
| IIIc-059b | 4-((2,5-Dihydroxy-4-sulfophenyl) methylsulfonylmethyl)-2,5-dihydroxybenzoic acid; | SO3H | H | H | 1 | SO2 | 1 | 4-carboxy-2,5-dihydroxyphenyl |
| IIIc-059c | 4-((2,5-Dihydroxy-3-sulfophenyl) methylsulfonylmethyl)-2,5-dihydroxybenzoic acid | COOH | H | H | 1 | SO2 | 1 | 2,5-dihydroxy-3-sulfophenyl |
| IIIc-059d | 4-((2,5-Dihydroxy-4-sulfophenyl) methylsulfonylmethyl)-2,5-dihydroxybenzenesulfonic acid | SO3H | H | H | 1 | SO2 | 1 | 2,5-dihydroxy-4-sulfophenyl |
| IIIc-060a | 4-((2,5-Dihydroxy-4-carboxyphenyl) methoxymethyl)-2,5-dihydroxybenzoic acid | COOH | H | H | 1 | O | 1 | 4-carboxy-2,5-dihydroxyphenyl |
| IIIc-060b | 4-((2,5-dihydroxy-4-sulfophenyl)methoxymethyl)-2,5-dihydroxybenzoic acid; | SO3H | H | H | 1 | O | 1 | 4-carboxy-2,5-dihydroxyphenyl |
| IIIc-060c | 4-((2,5-dihydroxy- 3-sulfophenyl)methoxymethyl)-2,5-dihydroxybenzoic acid | COOH | H | H | 1 | O | 1 | 2,5-dihydroxy-3-sulfophenyl |
| IIIc-060d | 4-((2,5-Dihydroxy-4-sulfophenyl)methoxymethyl)-2,5-dihydroxybenzenesulfonic acid | SO3H | H | H | 1 | O | 1 | 2,5-dihydroxy-4-sulfophenyl |
| IIIc-061a | Tris (4-carboxy-2,5-dihydroxyphenylmethyl)amine | COOH | H | H | 1 | N(CH2)qR9 q = 1: R9 = 4-carboxy-2,5-dihydroxyphenyl | 1 | 4-carboxy-2,5-dihydroxyphenyl |

(continued)

| A. | 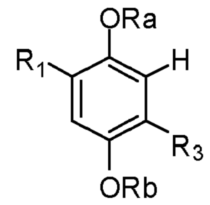 R3 = $(CH_2)_mX(CH_2)_pR_9$ | | | | | | | |
|---|---|---|---|---|---|---|---|---|
| No. | **Compound chemical names** | **R1** | **Ra** | **Rb** | **m** | **X** | **p** | **R9** |
| IIIc-061a-E3 | Tris (4-ethoxycarbonyl-2,5-dihydroxyphenylmethyl) amine | COOEt | H | H | | (CH2)qR9 q = 1: R9 = 4-ethoxycarboxy-2,5-dihydroxyphenyl | 1 | 4-ethoxycarbonyl-2,5-dihydroxyphenyl |
| B. | 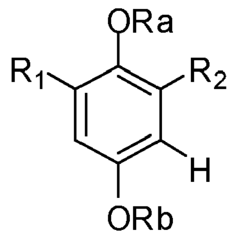 R2 = $(CH_2)_mX(CH_2)_pR_9$ | | | | | | | |
| IVc-056a | Bis(3-carboxy-2,5-dihydroxyphenylmethyl)amine | COOH | H | H | 1 | NH | 1 | 3-carboxy-2,5-dihydroxyphenyl |
| IVc-056b | 3-((2,5-Dihydroxy-3-sulfophenylmethylamino) methyl)-2,5-dihydroxybenzoic acid | SO3H | H | H | 1 | NH | 1 | 3-carboxy-2,5-dihydroxyphenyl |
| IVc-056c | 3-((2,5-Dihydroxy-4-sulfophenylmethylamino) methyl)-2,5-dihydroxybenzoic acid | COOH | H | H | 1 | NH | 1 | 2,5-dihydroxy-3-sulfophenyl |
| IVc-056d | Bis(2,5-dihydroxy-3-sulfophenylmethyl)amine | SO3H | H | H | 1 | NH | 1 | 2,5-dihydroxy-4-sulfophenyl |
| IVc-057a | N,N-Bis(3-carboxy-2,5-dihydroxyphenylmethyl) acetamide | COOH | H | H | 1 | NH(COCH3) | 1 | 3-carboxy-2,5-dihydroxyphenyl |
| IVc-057b | N-(2,5-dihydroxy-3-sulfophenylmethyl) N-(3-carboxy-2,5-dihydroxyphenylmethyl) acetamide | SO3H | H | H | 1 | NH(COCH3) | 1 | 3-carboxy-2,5-dihydroxyphenyl |

(continued)

| B. | 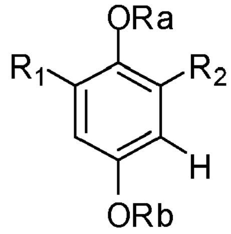 R2 = $(CH_2)_mX(CH_2)_pR_9$ | | | | | | | |
|---|---|---|---|---|---|---|---|---|
| IVc-057c | N-(2,5-dihydroxy-4-sulfophenylmethyl) N-(3-carboxy-2,5-dihydroxyphenylmethyl) acetamide | COOH | H | H | 1 | NH(COCH3) | 1 | 2,5-dihydroxy-3-sulfophenyl |
| IVc-057d | N,N-Bis(2,5-dihydroxy-3-sulfophenylmethyl) acetamide | SO3H | H | H | 1 | NH(COCH3) | 1 | 2,5-dihydroxy-4-sulfophenyl |
| IVc-058a | 3-((2,5-Dihydroxy-3-carboxyphenyl) methylthiomethyl)-2,5-dihydroxybenzoic acid | COOH | H | H | 1 | S | 1 | 3-carboxy-2,5-dihydroxyphenyl |
| IVc-058b | 3-((2,5-Dihydroxy-3-sulfophenyl)methylthiomethyl)-2,5-dihydroxybenzoic acid | SO3H | H | H | 1 | S | 1 | 3-carboxy-2,5-dihydroxyphenyl |
| IVc-058c | 3-((2,5-Dihydroxy-4-sulfophenyl)methylthiomethyl)-2,5-dihydroxybenzoic acid | COOH | H | H | 1 | S | 1 | 2,5-dihydroxy-3-sulfophenyl |
| IVc-058d | 3-((2,5-Dihydroxy-3-sulfophenyl)methylthiomethyl)-2,5-dihydroxybenzenesulfonic acid | SO3H | H | H | 1 | S | 1 | 2,5-dihydroxy-4-sulfophenyl |
| IVc-059a | 3-((2,5-Dihydroxy-3-carboxyphenyl) methylsulfonylmethyl)-2,5-dihydroxybenzoic acid | COOH | H | H | 1 | SO2 | 1 | 3-carboxy-2,5-dihydroxyphenyl |
| IVc-059b | 3-((2,5-Dihydroxy-3-sulfophenyl) methylsulfonylmethyl)-2,5-dihydroxybenzoic acid | SO3H | H | H | 1 | SO2 | 1 | 3-carboxy-2,5-dihydroxyphenyl |
| IVc-059c | 3-((2,5-Dihydroxy-4-sulfophenyl) methylsulfonylmethyl)-2,5-dihydroxybenzoic acid | COOH | H | H | 1 | SO2 | 1 | 2,5-dihydroxy-3-sulfophenyl |
| IVc-059d | 3-((2,5-Dihydroxy-3-sulfophenyl) methylsulfonylmethyl)-2,5-dihydroxybenzenesulfonic acid | SO3H | H | H | 1 | SO2 | 1 | 2,5-dihydroxy-4-sulfophenyl |
| IVc-060a | 3-((2,5-Dihydroxy-3-carboxyphenyl) methoxymethyl)-2,5-dihydroxybenzoic acid | COOH | H | H | 1 | O | 1 | 3-carboxy-2,5-dihydroxyphenyl |

(continued)

| B. | 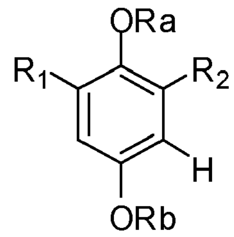 $R2 = (CH_2)_mX(CH_2)_pR_9$ | | | | | | | |
|---|---|---|---|---|---|---|---|---|
| IVc-060b | 3-((2,5-dihydroxy-3-sulfophenyl)methoxymethyl)-2,5-dihydroxybenzoic acid | SO3H | H | H | 1 | O | 1 | 3-carboxy-2,5-dihydroxyphenyl |
| IVc-060c | 3-((2,5-dihydroxy-4-sulfophenyl)methoxymethyl)-2,5-dihydroxybenzoic acid | COOH | H | H | 1 | O | 1 | 2,5-dihydroxy-3-sulfophenyl |
| IVc-060d | 3-((2,5-Dihydroxy-3-sulfophenyl)methoxymethyl)-2,5-dihydroxybenzenesulfonic acid | SO3H | H | H | 1 | O | 1 | 2,5-dihydroxy-4-sulfophenyl |
| IVc-061a | Tris (3-carboxy-2,5-dihydroxyphenylmethyl)amine | COOH | H | H | 1 | $N(CH_2)qR_9$ q = 1: $R_9$ = 3-carboxy-2,5-dihydroxyph enyl | 1 | 3-carboxy-2,5-dihydroxyphenyl |
| IVc-059a-E2-A4 | Methyl 3-((2,5-diacetoxy-3-methoxycarbonylphenyl) methylsulfonylmet hyl)-2,5-diacetoxybenzoate | COOMe | Ac | Ac | 1 | SO2 | 1 | 2,5-diacetoxy-3-(methoxycarbonyl)phenyl |
| IVc-059a-E2 | Methyl 3-((2,5-dihydroxy-3-methoxycarbonylphenyl) methylsulfonylmet hyl)-2,5-hydro xybenzoate | COOMe | H | H | 1 | SO2 | 1 | 2,5-dihydroxy-3-(methoxycarbonyl)phenyl |
| IVc-061a-E3 | Tris (3-carboxy-2,5-dihydroxyphenylmethyl)amine | COOEt | H | H | 1 | $N(CH_2)qR_9$ q = 1: $R_9$ = 3-ethoxycarbo nyl-2,5-dihydroxyphenyl | 1 | 3-ethoxycarbonyl-2,5-dihydroxyphenyl |

According to yet another embodiment, the present invention includes compounds in accordance with Table 12:

Table 12

| Compound No. | Compound chemical names | Structures |
|---|---|---|
| V-001a | 4-(3-(2,5-dihydroxy-4-carboxyphenylcarbamoyl)-5-hydroxybenzamido)-2,5-dihydroxy benzoic acid | 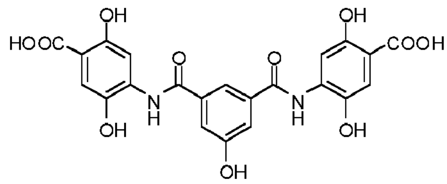 |
| V-001a diethyl ester | 4-(3-(4-(ethoxycarbonyl)-2,5-dihydroxyphenylcarbamoyl)-5-hydroxybenzamido)-2,5-dihydroxybenzoic acid ethyl ester | 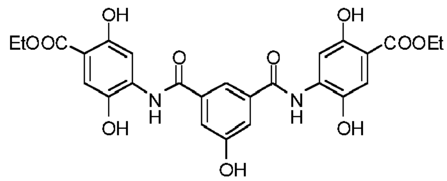 |
| V-002a | 2-hydroxy-5-(3-hydroxy-5-(4-hydroxy-3-carboxyphenylcarbamoyl)benzamido)benzoic acid | 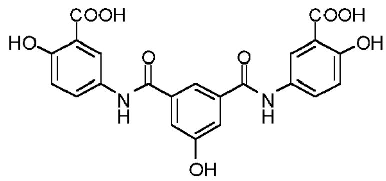 |
| V-002a diethyl ester | 5-(3-(3-(ethoxycarbonyl)-4-hydroxyphenylcarbamoyl)-5-hydroxybenzamido)-2-hydroxy benzoic acid ethyl ester | 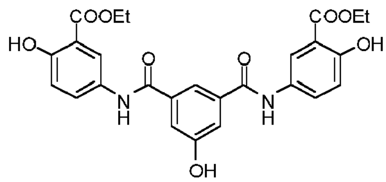 |
| V-003a | 4,4'-carbonylbis(azanediyl)bis(2,5-dihydroxybenzoic acid) | 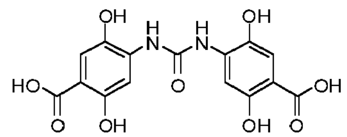 |
| V-003a diethyl ester | Diethyl 4,4'-carbonylbis(azanediyl)bis(2,5-dihydroxybenzoate) | 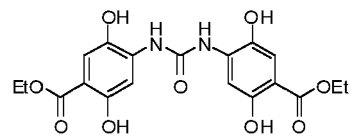 |

The present invention also relates to a process for the preparation of the hydroquinone derivative compound of formula (I), which comprises:

Step (a): Coupling of hydroquinone-derived carboxylic acid, protected at the phenolic functions, with an aniline carrying diverse functional groups, by way of the corresponding acyl chloride or using an amide coupling agent such as TCFH in the presence of NMI;
Step (b): Deprotection of the ester functions typically by a saponification reaction; and
Step (c): Deprotection of the phenolic functions typically by catalytic hydrogenation.

Or, alternatively,

Step (d): Reduction of the hydroquinone-derived carboxylic acid, protected at the phenolic functions, to produce a benzylic alcohol
(e) Activation of the benzylic alcohol function and substitution with a benzylic nucleophile (alcohol, thiol, primary or

secondary amine) carrying diverse functional groups;

(f) Deprotection of the ester functions typically by a saponification reaction; and

(g) Deprotection of the phenolic functions typically by catalytic hydrogenation

The process is schematically represented below:

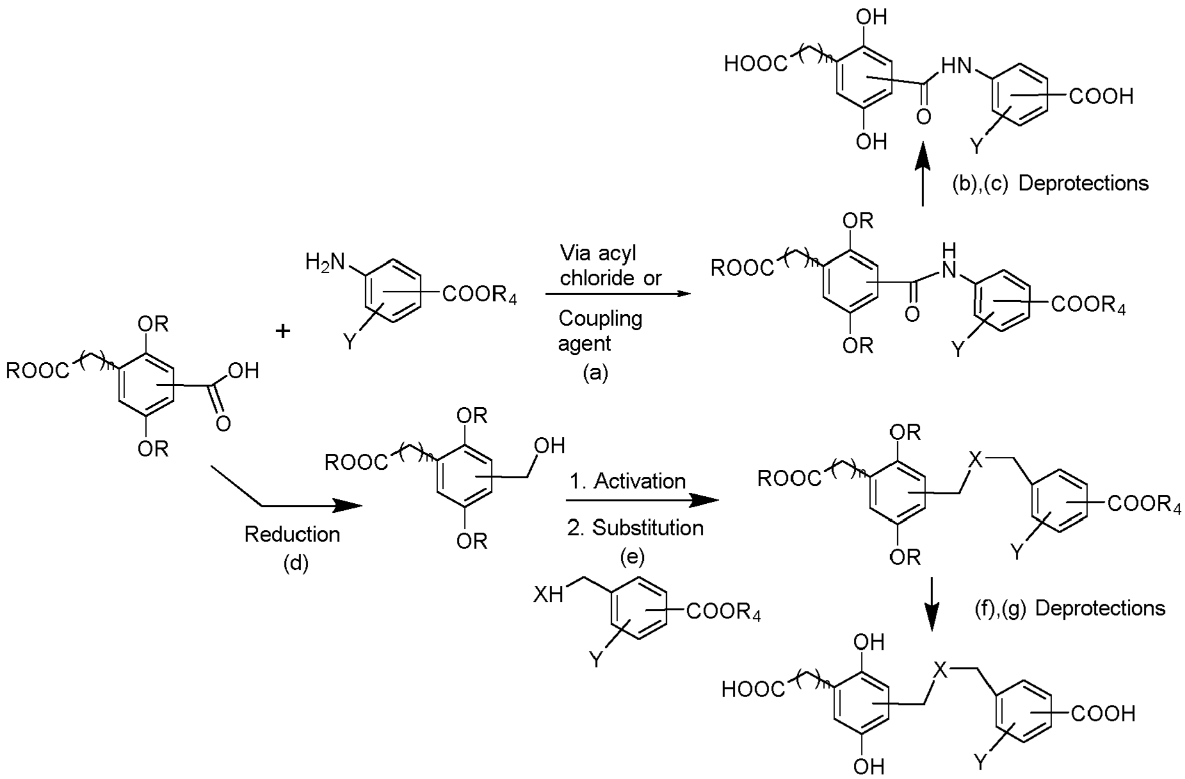


The hydroquinone derivative compounds of the present invention may be present as optically active forms (stereoisomers), E/Z isomers, enantiomers, racemates, diastereoisomers thereof, and hydrates and solvates thereof. Solvates of the compounds are due to the mutual attraction between the molecules of the compound and the inert solvent used. Solvates e.g. monohydrate, dihydrates or alcoholates.

The hydroquinone derivatives of the present invention may also be present as pharmaceutically acceptable salts, wherein the parent hydroquinone derivatives are modified by preparing acidic or basic salts thereof.

Examples of pharmaceutically acceptable salts include, among others, mineral or organic acid salts of basic residues, such as amines; and alkaline or organic salts of acidic residues, such as carboxylic acids and the like. Pharmaceutically acceptable salts may include conventional non-toxic salts or quaternary ammonium salts which are suitable for all routes of administration of the compounds, for example, from non-toxic organic or inorganic acids. For example, said conventional non-toxic salts include those derived from inorganic acids, such as hydrochloric, hydrobromic, sulfuric, sulfamic, phosphoric, nitric and the like; and salts prepared from organic acids such as acetic, propionic, succinic, glycolic, stearic, lactic, malic, tartaric, citric, ascorbic, pamoic, maleic, hydroxyleleic, phenylacetic, glutamic, benzoic, salicylic, sulfanyl, 2-acetoxy -benzoic, fumaric, toluenesulfonic, methanesulfonic, ethanedisulfonic, oxalic, isethionic, trifluoroacetic and the like.

Pharmaceutically acceptable salts are described for cations and anions "Pharmaceutical salts: A summary on doses of salt formers from the Orange Book. Saal C, European Journal of Pharmaceutical Sciences, 2013, 49, 614-623. By way of examples of cations, we may cite Aluminum, Arginine, Benzathine, Calcium, Chloroprocaine, Choline, Diethanolamine, Ethanolamine, Ethylenediamine, Lysine, Magnesium, Histidine, Lithium, Meglumine, Potassium, Procaine, Sodium, Triethylamine, Zinc. By way of examples of anions, we may cite Acetate, Aspartate, Benzenesulfonate, Benzoate, Besylate, Bicarbonate, Bitartrate, Bromide, Camsylate, Carbonate, Chloride, Citrate, Decanoate, Edetate, Esylate, Fumarate, Gluceptate, Gluconate, Glutamate, Glycolate, Hexanoate, Hydroxynaphthoate, Iodide, Isethionate, Lactate, Lactobionate, Malate, Maleate, Mandelate, Mesylate, Methylsulfate, Mucate, Napsylate, Nitrate, Octanoate, Oleate, Pamoate, Pantothenate, Phosphate, Polygalacturonate, Propionate, Salicylate, Stearate, Acetate, Succinate, Sulfate, Tartrate, Teoclate, Tosylate. Alternatively, zwiterions may be used as salts such as free amino-acids Arginine or Lysine as cationic counterions and Glutamate or Aspartate as anionic counterions. Short peptides (2 to 20 amino acids) as

repeated units of Arginine or Lysine and their combinations with other neutral amino acids. These cationic cell penetrating peptides (CPP) may be used as enhancers for the penetration of drug into cells.

Pharmaceutically acceptable salts of the compounds useful in the present invention may be for example synthesized from the parent compound containing a basic or acidic moiety by conventional chemical procedures. Generally, said salts can be prepared by reacting the free acid or the basic forms of these compounds with a stoichiometric amount of the appropriate base or acid in water or in an organic solvent, or in a mixture of the two; In general, non-aqueous media such as ether, ethyl acetate, ethanol, isopropanol or acetonitrile are preferred. In Remington's Pharmaceutical Sciences, 17th ed., Mack Publishing Company, Easton, PA, 1985, p. 1418, whose disclosure is incorporated herein by reference, lists of suitable salts are presented.

Suitable dosages according to the invention and as described herein in the various embodiments will vary depending upon the condition, age and species of the subject, and can be readily determined by those skilled in the art. The total daily dosages employed in both veterinary and human medicine will suitably be in the range 0.01 -2000 mg/kg body-weight, preferably from 0.1-1000 mg/kg body-weight, preferably from 1-100 mg/kg and these may be administered as single or divided doses, and in addition, the upper limit can also be exceeded when this is found to be indicated. Such dosage will be adjusted to the individual requirements in each particular case including the specific compound(s) being administered, the route of administration, the condition being treated, as well as the patient being treated. However, the compounds can also be administered as depot preparations (implants, slow-release formulations, etc.) weekly, monthly or at even longer intervals. In such cases the dosage will be much higher than the daily one and has to be adapted to the administration form, the body weight and the concrete indication. The appropriate dosage can be determined by conducting conventional model tests, preferably animal models. In general, in the case of oral or parenteral administration to adult humans weighing approximately 70 kg, a daily dosage of about 10 mg to about 10.000 mg, preferably from about 200 mg to about 1 .000 mg, should be appropriate, although the upper limit may be exceeded when indicated. It is to be understood that the above therapeutically effective dosages need not be the result of a single administration and are usually the result of the administration of a plurality of unit doses. Those unit doses can in turn comprise portions of a daily or weekly dosage, and thus, the therapeutically effective dose is determined over the period of treatment (contacting). For example, for oral administration, the daily dose can be about 0.04 to about 1.0 mg/kg of body weight, more preferably about 0.04 to about 0.20 mg/kg/day, more preferably still at about 0.05 to about 0.15 mg/kg/day, and most preferably about 0.1 mg/kg body weight. In general, the amount of active substance administered can vary over a relatively wide range to achieve, and preferably maintain, the desired plasma concentration. Unit dosage forms of the active ingredient can contain about 0.1 milligrams to about 15 milligrams thereof. A preferred unit dosage form contains about 0.1 to about 1 milligram of agent and can be administered 2 to 5 times per day. However, it should be noted that other alternative routes like continuous infusion at a rate designed to maintain the above described plasma concentration is also contemplated. Duration of a particular treatment can also vary, depending on severity of the disease, whether the treatment is intended for an acute manifestation or for prophylactic purposes, and like considerations. Typical administration lasts for a time period of about 5 to about 14 days, with a 7-day time course being usual. Courses (cycles) of administration can also be repeated at monthly intervals, or parenteral unit dosages can be delivered at weekly intervals. Oral unit dosages can be administered at intervals of one to several days to provide the determined therapeutically effective dose. The appropriate dosage of the compounds of the invention will depend on the type of disease to be treated, on the severity and course of the disease, on whether the agent is administered for preventive or therapeutic purposes, of the patient's medical history, and of the response to the compounds, and of the criteria of the responsible physician. The determination of the appropriate dose or route of administration is clearly within the capabilities of a current physician. Animal experiments provide a reliable guide for the determination of effective doses for human therapy. The inter-species scaling of effective doses can be carried out following the principles established by Mordenti, J. and Chappell, W. "The use of interspecies scaling in toxicokinetics", in Toxicokinetics and New Drug Development, editors Yacobi et al., Pergamon Press, New York, 1989, pp. 42-96.

The present invention further provides a pharmaceutical composition comprising (i) a therapeutically effective amount of the hydroquinone derivative compound of formula (I) or a pharmaceutically acceptable salt, or a prodrug, or stereoisomer thereof, and (ii) a pharmaceutically acceptable excipient. The pharmaceutical excipients according to the present invention may be selected from the group consisting of conventional excipients, such as binders, fillers and lubricants and combinations thereof.

Pharmaceutical composition of the compounds of the present invention with one or more pharmaceutical carriers, may be formulated with said excipients as liquid medicines, solid or semisolid medicines, and/or gaseous medicines.

When they are present in solid formulations, these may include without any limitations, granules, pellets, tablets, and capsules, powders, crushable tablets, dissolvable tablets, film coated tablets, controlled, sustained, extended or modified release tablets etc. Such formulations may also be in the forms of immediate release, delayed release, or modified release. Further, immediate release compositions may be conventional, dispersible, chewable, mouth dissolving, or flash melt preparations, and modified release compositions that may comprise hydrophilic or hydrophobic, or combinations of hydrophilic and hydrophobic, release rate controlling substances to form matrix or reservoir or combination of matrix and reservoir systems. The compositions may be prepared using any one or more of techniques such as direct blending, dry granulation, wet granulation, and extrusion and spheronization. Compositions may be presented as uncoated, film coated, sugar coated, powder coated, enteric coated, and modified release coated. When they are present in liquid formulations, these may include without any limitations, any liquid forms such as solutions, suspensions, dispersions, colloids, emulsions, lotions, creams, tinctures, and freeze-dried compositions. When they are present in gaseous formulations, these may include without any limitations, aerosols, sprays, droplets, mists, nebulas, and/or atomized vapors.

Pharmaceutical compositions according to the present invention may further comprise any additives such as vehicles, binding agents, perfumes, flavoring agents, sweeteners, colorants, antiseptics, antioxidants, stabilizing agents, and surfactants, if desired.

Pharmaceutical compositions and compounds according to the present invention may be formulated for administration via enteral routes, such as oral routes; parenteral routes via injections such as intravenous injections; topical routes, such as ocular routes, mucosal and transmucosal routes, including sublingual/buccal routes; as well as as pulmonary, nasal, intranasal, intrabronchial, intrapulmonary routes.

Preferred formulations of the compounds according to the present invention comprise liquid oral formulations, occular formulations and intravenous formulations.

Liquid oral formulations are solution and suspension formats for oral drug administration are common, convenient and deemed safe. They can be used to deliver relatively large quantities of drug and are frequently used for the paediatric population and for those who experience difficulties in swallowing tablets or capsules. Introduction via the gastrointestinal tract provides fast dissolution and drug absorption however tolerability and interaction with other materials in the tract must be considered. Properties of the active pharmaceutical ingredient (API) can lend themselves to be formulated in simple aqueous based oral solutions however when API's are poorly soluble, formulations need to be designed around the molecule to enhance solubility and bioavailability. Introduction of surfactant, solvents, buffers and oils increase solubility and reduce precipitation on interaction with gastric fluids. Where suspensions are required, particle size is often reduced and controlled to enhance drug permeation and absorption. As formulation development progresses, preservatives and flavours are introduced to enable patient conformity and shelf life appropriateness.

Ocular formulations are ophthalmic formulations are delivered directly to the eye and are frequently liquids in solution or suspension formats. Administration to the eye avoids metabolism by the gastrointestinal tract and can be used for drugs which are poorly absorbed when given orally. Eye drops are sterile and isotonic with a nominal pH of 4 - 8 to avoid eye irritation. Excipients including chelating agents, polymers, surfactants and cyclodextrins are added to the formulation to improve stability, modify viscosity or increase solubility, permeability, and bioavailability of poorly soluble drugs.

Intravenous formulations allow rapid absorption after parenteral drug delivery of the entire dose reaching the patients system for an immediate response. This mode of introduction avoids metabolism by the gastrointestinal tract and can be used for drugs which are poorly absorbed when given orally. Injections are typically sterile. isotonic water-based solutions and are designed to not induce pain on administration. For poorly soluble drugs, formulations are modified with organic co-solvents, surfactants and buffers to increase solubility with nano-suspension formulations also being acceptable. For those molecules which are unstable in solution, formulations can be lyophilised for dilution at the point of use.

The present invention also provides kits, comprising a composition comprising a therapeutically effective dose of one or more compounds of the present invention or the formulations thereof as well as a delivery device for administration of the said composition or formulation and further comprising a package insert incorporating manual instructions for usage.

To prepare the present pharmaceutical compositions, the active ingredient according to the invention may be mixed with a pharmaceutical acceptable carrier, adjuvant and/or excipient, according to conventional pharmaceutical compounding techniques. Pharmaceutically acceptable carriers that can be used in the present compositions encompass any of the standard pharmaceutical carriers, such as a phosphate buffered saline solution, water, and emulsions, such as an oil/water or water/oil emulsion, and various types of wetting agents. Tire compositions can additionally contain solid pharmaceutical excipients such as starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Liquid carriers, particularly for injectable solutions, include water, saline, aqueous dextrose, and glycols. For examples of carriers, stabilizers and adjuvants, see Remington's Pharmaceutical Sciences, edited by E. W. Martin (Mack Publishing Company, 18th ed., 1990). The compositions also can include stabilizers and preservatives. Examples of pharmaceutically-acceptable solvents that may be used in the present composition may be found in reference books such as the Handbook of Pharmaceutical Excipients (Fifth Edition, Pharmaceutical Press, Londn and American Pharmacists Association, Washington, 2006), " Ullmann's Encyclopedia of Industrial Chemistry, 6th Ed." (various editors, 1989-1998,

Marcel Dekker) and in "Pharmaceutical Dosage Forms and Drug Delivery Systems" (ANSEL et al., 1994 et 2011, WILLIAMS & WILKINS). Non-limiting examples of pharmaceutically-acceptable solvents that may be used in the present composition include, but are not limited to, propylene glycol (also known as 1 ,2-dihydroxypropane, 2-hydroxypropanol, methyl ethylene glycol, methyl glycol or propane- 1 ,2-diol), ethanol, methanol, propanol, isopropanol, butanol, glycerol, polyethylene glycol (PEG), glycol, Cremophor EL or any forms of polyethoxylated castor oil, dipropylene glycol, dimethyl isosorbide, propylene carbonate, N-methylpyrrolidone, glycofurol, tetraethyleneglycol, propylene glycol fatty acid esters, and mixtures thereof.

The compounds according to the invention have demonstrated anti-fibrotic, anti-inflammatory and antiangiogenic effects. Several diseases listed herein after involving not only one but two or even the three targets. Disease Categories showing pre-dominant angiogenic (vascular), inflammatory and/or fibrotic pathobiology.

Therefore, the present invention provides a method of treating and/or preventing a disease or disorder autoimmune, immunological, rheumatology, vascular disorders, ophthalmologic disorders, fibrotic disorders, metabolic and gastro-intestinal disorders, neuroinflammatory and neurodegenerative diseases, neoplasms and cancer associated disorders, hormone related diseases and immunological disorders resulting from viral and bacterial infectious diseases and complications thereof, comprising administering a subject in a need a therapeutically effective dose of the compounds and/or pharmaceutical compositions of the present invention as described above.

Pharmaceutical compositions and methods according to the present invention are particularly suitable for a subject which is a human or non-human animal subject.

Depending on their structure, compounds according to the invention may target diseases basis on their individual cytokine profiles as described in BIODATA provided for several compounds (See Figures 3A-D). The following web site Open Targets Platform (URL: https://www.targetvalidation.org/, Denise Carvalho et al, Nucleic Acids Research, Volume 47, Issue D1, 08 January 2019, D1056-D1065, https://doi.org/10.1093/nar/gky1133) gives a complete picture based on current bibliographic information and can be searched by disease or cytokine.

Two typical examples: (a) disease specific search for diabetic retinopathy leads to the following cytokines described with a high correlation with the disease (Figure 3C). The three top cytokines are As an example for diabetic retinopathy search indicate VEGFA score = 1 (vascular endothelial growth factor A), HFE score = 1 (homeostatic iron regulator) and TNF score = 0.86 (tumor necrosis factor) and (b) searching for VEGFA: leads to nervous system disease, vascular disease, eye disease, retinopathy and several other diseases shown in Figure 3D

To illustrate the potency of compounds on small group of inflammatory cytokines, the effects of the compounds were assessed on human whole blood and their IC50 values on inhibition of LPS induced inflammation (Table 13).

Table 13:

| Compound | GM-CSF | IFNγ | | IL-1b | IL-2 | IL-4 | IL-5 | IL-6 | IL-9 | IL-10 | IL-12p70 | IL-13 | IL-17A | IL-17F | IL-18 | IL-21 | IL-33 | TGFbeta | TNFα | TNFβ |
|---|---|---|---|---|---|---|---|---|---|---|---|---|---|---|---|---|---|---|---|---|
| Ia-001a | 100 | 30.5 | | 34.4 | 94.3 | 100 | 100 | 1.24 | 5.63 | 100 | 1.5 | 26.4 | 0.1 | 14.7 | 100 | 100 | 19.3 | 100 | 0.02 | 13.20 |
| Ia-001aTz | 100 | 100 | | 100 | 7.99 | 100 | 100 | 0.75 | 8.63 | 26.1 | 32.4 | 32.1 | 0.11 | 46.5 | 100 | 100 | 23.6 | 100 | 0.63 | 53.00 |
| Ia-001aTz/1-004a | 3.4 | 0.61 | | 100 | 4.91 | 100 | 100 | 3.04 | 8.48 | 19.5 | 12.2 | 1.5 | 0.02 | 2.4 | 46.6 | 0.3 | 1.67 | 100 | 0.002 | 3.02 |
| Ia-001aTz2 | 100 | 1.38 | | 100 | 9.45 | 100 | 100 | 0.52 | 9.13 | 100 | 29.1 | 0.12 | 0.31 | 0.15 | 33.9 | 32 | 0.23 | 100 | 0.1 | 0.41 |
| 1-001c | 100 | 0.44 | | 100 | 12.8 | 100 | 100 | 0.95 | 46.9 | 1.9 | 0.44 | 86.1 | 0.09 | 89.7 | 100 | 100 | 100 | 100 | 0.05 | 29.70 |
| 1-003aTz | 100 | 2.95 | | 100 | 43 | 100 | 100 | 1.41 | 3.62 | 6.94 | 1.26 | 89.6 | 0.07 | 83.9 | 100 | 1.49 | 72.2 | 100 | 0.18 | 55.00 |
| Ic-007a | 100 | 1.44 | | 100 | 100 | 100 | 100 | 0.09 | 27.1 | 12.5 | 0.49 | 30.1 | 0.04 | 22.9 | 100 | 1.9 | 18.4 | 100 | 0.27 | 6.52 |
| Ib-010a | 100 | 2.22 | | 33.3 | 2.74 | 100 | 100 | 1.02 | 11.58 | 100 | 0.27 | 100 | 0.42 | 100 | 100 | 67.8 | 31.3 | 100 | 0.31 | 49.30 |
| Ib-010a-E3 | 100 | 2.79 | | 100 | 36.2 | 100 | 100 | 0.24 | 26.8 | 100 | 0.84 | 33 | 0.52 | 34.2 | 100 | 100 | 20 | 100 | 0.98 | 38.20 |
| 1-015a | 2.76 | 100 | | 100 | 7.08 | 100 | 100 | 0.77 | 1.47 | 5.27 | 0.12 | 0.15 | 0.05 | 0.15 | 100 | 57.2 | 0.24 | 100 | 0.17 | 0.30 |
| IIc-007a | 26 | 0.11 | | 100 | 12.8 | 100 | 100 | 0.05 | 2.14 | 17.9 | 3.37 | 0.25 | 0.01 | 0.26 | 100 | 9.41 | 0.18 | 100 | 0.03 | 0.29 |
| 2-009a | 100 | 0.54 | | 100 | 19.6 | 100 | 100 | 0.27 | 18.9 | 11.31 | 100 | 0.13 | 0.001 | 0.13 | 2.95 | 8.01 | 0.29 | 100 | 0.11 | 6.94 |
| 2-053a | 100 | 1.04 | | 100 | 1.67 | 100 | 100 | 2.2 | 1.41 | 100 | 0.27 | 29.9 | 100 | 28.8 | 100 | 100 | 12.1 | 100 | 0.02 | 91.50 |
| 3-001aTz | 100 | 18.9 | | 100 | 100 | 100 | 100 | 1.98 | 3.01 | 7.78 | 0.48 | 0.15 | 0.17 | 0.16 | 27.7 | 3 | 0.14 | 100 | 0.31 | 0.23 |
| 3-013a | 27.8 | 0.15 | | 100 | 57.1 | 100 | 100 | 3.55 | 11.9 | 37.3 | 0.5 | 0.37 | 0.15 | 0.41 | 100 | 1.63 | 1.11 | 100 | 0.51 | 0.89 |
| 3-057a | 100 | 0.9 | | 16.2 | 24.2 | 100 | 100 | 0.19 | 11.1 | 100 | 0.73 | 4.73 | 1.32 | 5.06 | 100 | 1.56 | 2.93 | 100 | 0.15 | 1.58 |
| IIIc-061a | 31.2 | 2.48 | | 100 | 100 | 100 | 100 | 0.18 | 8.85 | 17.9 | 37.2 | 0.31 | 0.04 | 0.32 | 32.4 | 6.38 | 0.31 | 100 | 0.03 | 0.81 |
| 4-058a | 100 | 10.6 | | 27.5 | 3.82 | 100 | 100 | 0.21 | 31.1 | 16.4 | 4.23 | 55.9 | 0.1 | 86.8 | 100 | 3.2 | 33.9 | 100 | 0.18 | 59.30 |
| IVc-059a | 100 | 4.34 | | 38.6 | 21.2 | 100 | 100 | 0.11 | 0.4 | 1.31 | 42 | 100 | 0.16 | 100 | 100 | 1.87 | 27.5 | 100 | 0.16 | 0.33 |
| IVc-059a-E2-A4 | 1.83 | 3.5 | | 14.1 | 10.4 | 100 | 100 | 0.99 | 2.9 | 16.8 | 51.4 | 18.7 | 0.02 | 25.8 | 100 | 100 | 23.2 | 100 | 0.3 | 19.40 |

Immunological/Rheumatology/Vascular disorders may include for example peritonitis, Scleroderma, Kawasaki Disease (KD), Takayasu Arteritis (TA), Microscopic Polyangiitis (MP), Giant Cell arteritis - Horton's disease (GCA), Antiphospholipid syndrome (APS), Behçet's Disease (BD), Granulomatosis with polyangiitis (GPA), Wegener's granulomatosis (WG), Eosinophilic granulomatosis with polyangiitis - Churg-Strauss syndrome (EGPA), and Rosacea (RO).

Ophthalmologic disorders may include for example and without any limitations, Age Related Macular Degeneration (AMD, ARMD, Dry and Wet forms), Pterygium (PTE), Diabetic Retinopathy (DR), Diabetic Macular Edema (DME), Stargardt's disease (SD), Proliferative vitreoretinopathy, Dry Eye Syndrome (DYS), Endophtalmitis. Pterygium (PTE), Central serous chorioretinopathy (CSC), Glaucoma associated complications, and Uveitis (UVE).

According to a preferred embodiment, the compounds and pharmaceutical compositions according to the present invention are used in a method of treating and/or preventing retinal neurodegenerative disease selected from the group consisting of diabetic retinopathy, age-related macular degeneration, glaucoma and retinitis pigmentosa. Retinal neurodegenerative diseases refer to retinal conditions characterized by progressive neuronal loss. Diabetic retinopathy, age-related macular degeneration, glaucoma and retinitis pigmentosa are considered retinal diseases in which neurodegeneration plays an essential role.

According to this preferred embodiment, the compounds according to the present invention may be combined with other active agents to enhance the therapeutic efficacy of the condition to be treated and particularly with dipeptidyl

peptidase-4 inhibitor(DPPIV) or a pharmaceutically acceptable salt thereof, for use in the topical eye treatment and/or prevention of a retinal neurodegenerative disease. The DPPIV inhibitor may be selected from the group consisting of sitagliptin, saxagliptin, vildagliptin, linagliptin, anagliptin, teneligliptin, alogliptin, trelagliptin, gemigliptin, omarigliptin, its pharmaceutically acceptable salts, and mixtures thereof.

Fibrotic disorders may include for example and without any limitations, cystic fibrosis, retroperitoneal fibrosis, Idiopathic pulmonary fibrosis, combined pulmonary fibrosis & emphysema (CPFE), Eosinophilic angiocentric fibrosis, intestinal fibrosis, ovarian fibrosis, oral submucous fibrosis (OSMF) and liver fibrosis.

Metabolic & gastro-intestinal disorders may include for example and without any limitations, diabetes induced complications: Diabetic Nephropathy (DN), Diabetic Retinopathy (DR), Diabetic Cardiomyopathy (DCDM), Diabetic Foot Ulcer (DFU); hepatic diseases: non alcoholic steatic hepatosis (NASH), Primary biliary cholangitis (PBC), Hepatic fibrosis - Cirrhosis (HF); Inflammatory Bowell diseases: Ulcerative Colitis, Crohn's disease, Intestinal fibrosis; Kidney diseases: Diabetic Nephropathy or Diabetic Kidney Disease (DN or DKD), Polycystic Kidney Disease (PKD), Chronic Kidney Disease (CKD), Nephrogenic Systemic Fibrosis (NSF); and Pancreatitis (Acute and Chronic).

Neoplasms and cancer associated disorders may include for example and without any limitations, pancreatic cancer: mostly fibrotic, renal cell carcinoma, radiation induced fibrosis, primary myelofibrosis, desmoplasia, fibrosarcoma, hepatocellular carcinoma, retinoblastoma, intra-ocular lymphoma, and melanoma (desmoplastic, conjunctival, uveal).

Viral and bacterial infectious diseases including septic shock, inflammation in the eye such as endophtalmitis due to surgery or infection.

## EXAMPLES

### EXAMPLE 1: Synthesis of the compounds and intermediates

**Example 1.1: Molecules of type Ia: Monoamides**, 25 examples

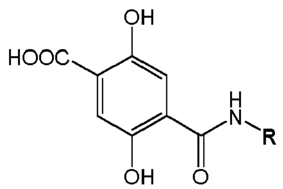

### SYNTHESIS OF PRECURSORS

**Scheme 1.** Synthetic Intermediates **KI-1** and **KI-6**

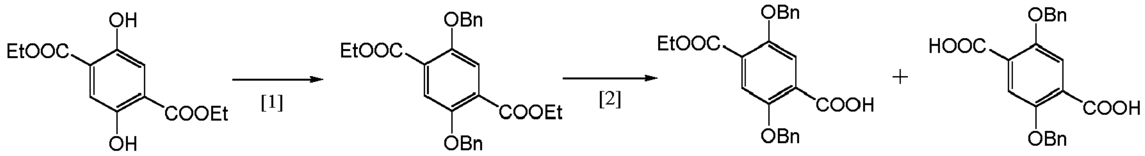


**KI-0** **KI-1** **KI-6**

***Procedures:***

Synthesis of **KI-1** an **KI-6** (steps [1],[2])

***Diethyl 2,5-bis(benzyloxy)terephthalate* (KI-0) (Step [1]).** Potassium carbonate-325 mesh (326.1 g, 2.4 mol) was added portion wise to a stirred solution of diethyl 2,5-dihydroxyterephthalate (200.0 g, 0.79 mol) in DMF (800.0 mL) at ambient temperature over 15 min. Benzyl bromide (280.0 mL, 2.4 mol) was then added to the reaction flask in a dropwise manner over 30 min, and the resulting mixture was heated at 100 °C for 2 h, resulting in the formation of a thick cream-coloured precipitate. The reaction mixture was cooled down to ambient temperature and treated with a solution of saturated aqueous ammonium chloride (2 L). The resulting suspension was stirred for 30 min, then filtered. The solid material was washed with a solution of saturated aqueous ammonium chloride (2 × 200 mL). The solid was

then suspended in ethanol (400 mL), filtered and dried in the vacuum oven to give the desired product as a white solid (341.0 g, 0.785 mol, 99.8%)
UPLC-MS (acidic method, 2 min): rt = 1.36 min, no ionization observed, peak area >95%
$^1$H NMR (400 MHz, DMSO-$d_6$) δ 7.51-7.44 (m, 6H), 7.44-7.36 (m, 4H), 7.36-7.28 (m, 2H), 5.17 (s, 4H), 4.29 (q, $J$ = 7.1 Hz, 4H), 1.26 (t, $J$ = 7.1 Hz, 6H).

***2,5-Bis(benzyloxy)-4-(ethoxycarbonyl)benzoic acid (*KI-1*) and 2,5-bis(benzyloxy)terephthalic acid (*KI-6*)* (step [2])**

A solution of potassium hydroxide (14.2 g, 0.25 mol) in water (200.0 mL) was rapidly added to a solution of diethyl 2,5-bis(benzyloxy)terephthalate (100 g, 0.23 mol) in 1,4-dioxane (1 L), and the resulting mixture was stirred overnight at ambient temperature. The solvent was removed *in vacuo*, resulting in the formation of a white slurry. The crude was suspended in EtOH (500 mL) and filtered and the collected solid was dried in vacuum oven to afford pure diethyl 2,5-bis(benzyloxy)terephthalate (23.0 g, 0.053 mol, 23%)
UPLC-MS (acidic method, 2 min): rt = 1.36 min, no ionization observed, peak area 98%
The ethanolic filtrate was concentrated to give an oil. Ethyl acetate (500 mL) was added to form a precipitate which was filtered and washed with ethyl acetate (200 mL) to afford 2,5-bis(benzyloxy)terephthalic acid **(KI-6)** as white solid (18.9 g, 0.042 mol, 18%).
UPLC-MS (acidic method, 2 min): rt = 1.22 min, $m/z$ 377.1 $[M-H]^-$, peak area 95%
$^1$H NMR (400 MHz, DMSO-$d_6$) δ 7.52-7.45 (m, 4H), 7.41-7.33 (m, 4H), 7.33-7.26 (m, 4H), 5.13 (s, 4H).
The filtrate was washed with a saturated solution of aqueous sodium carbonate (200 mL), aqueous 1 M hydrochloric acid (500 mL) and brine (500 mL), then dried ($Na_2SO_4$), filtered and concentrated to dryness to give 2,5-bis(benzyloxy)-4-(ethoxycarbonyl)benzoic acid (**KI-1**) as a white solid (53 g, 0.130 mol, 57.0%).
UPLC-MS (acidic method, 2 min): rt = 1.22 min, $m/z$ 405.3 $[M-H]^-$, peak area 96%
$^1$H NMR (400 MHz, DMSO-$d_6$) δ 7.52-7.27 (m, 13H), 5.17 (s, 4H), 4.28 (q, $J$ = 7.1 Hz, 2H), 1.26 (t, $J$ = 7.1 Hz, 3H).

**Scheme 2**: Synthetic Intermediate **KI-1Bn**

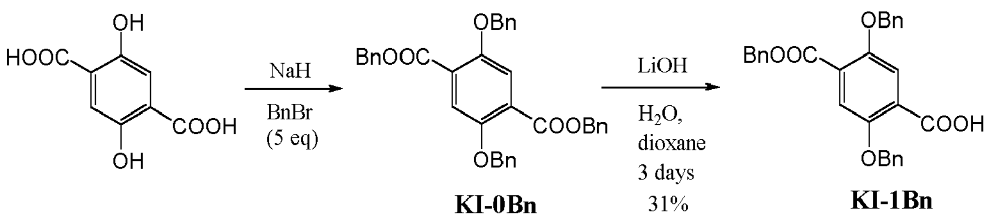


**KI-0Bn** **KI-1Bn**

contains 16% KI-0Bn

*Procedure:*

Synthesis of **KI-1Bn*:***

***2,5-Bis(benzyloxy)-4-(benzyloxycarbonyl)benzoic acid* (KI-1Bn).** To a solution of 2,5-dihydroxyterephthalic acid (1.97 g, 10 mmol) in DMF (20 mL) at 0°C was added sodium hydride (2.0 g, 50 mmol) under stirring, then benzyl bromide (5.95 mL, 50 mmol, 5 eq) dropwise over 5 min, and the resulting mixture kept at room temp. for 2h. The mixture was then heated at 60°C for 18 h. The reaction mixture was cooled down to ambient temperature and treated cautiously with MeOH (5 mL). The mixture was concentrated under reduced pressure, and the residual material co-evaporated with heptane (3x). The residue was taken in DCM (30 mL), the organic phase washed with 1M HCl (25 mL), the separated aqueous layer was extracted with DCM (3x30 mL) and the combined organic phases dried (MgSO4) and concentrated. The crude solid was recrystallized from EtOH (~30 mL) to afford pure **KI-0Bn** (2.286g, 41%). A sample of KI-0Bn (282 mg, 0.5 mmol) was added to a mixture of 1,4-dioxane (10 mL) and lithium hydroxide hydrate (22 mg, 0.52 mmol), and the mixture was heated to 80°C for 3 d.
The mixture was cooled down to room temp., taken in AcOEt (30 mL) and the solution washed with 1M HCl (2 mL). The organic phase was dried and concentrated, and the residue submitted to column chromatography to afford monoester **KI-1Bn** contaminated by about 16% of diester **KI-0Bn**.
**KI-0Bn**: $^1$H NMR (400 MHz, $CDCl_3$): δ 7.51 (s, 2H), 7.36-7.30 (m, 20H), 5.34 (s, 4H), 5.11 (s, 4H).
**KI-1Bn**: $^1$H NMR (250 MHz, $CDCl_3$): δ 7.86 (s, 1H), 7.61 (s, 1H), 7.41-7.30 (m, 20H), 5.36 (s, 2H), 5.26 (s, 2H), 5.16 (s, 2H).

**SYNTHESIS OF MONOAMIDES**

**Scheme 3.** Synthesis of Monoamides **Ia**

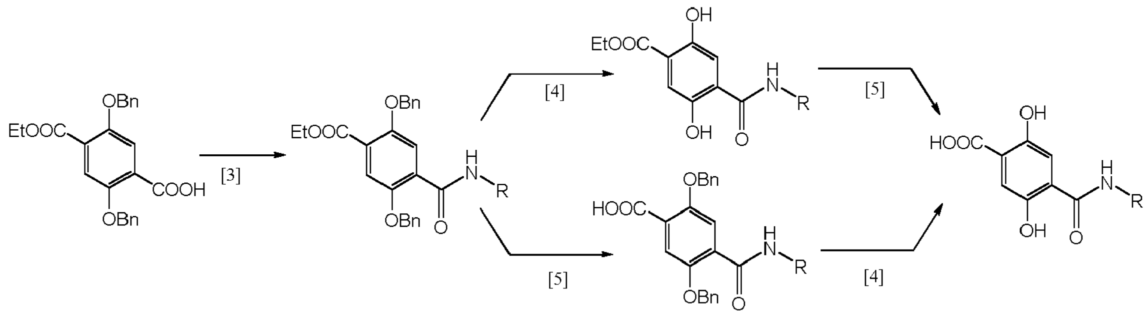


**General procedure A for Amide Coupling [3A]**

➢ Coupling *via* acyl chloride, from **KI-1** or **KI-6**

**Model Reaction (Ia-013a)**

***Dimethyl 2-(2,5-bis(benzyloxy)-4-(ethoxycarbonyl)benzoylamino)isophthalate.*** 2,5-Bis(benzyloxy)-4-(ethoxycarbonyl)benzoic acid (**KI-1**, 1.1 g, 2.8 mmol) was dissolved in thionyl chloride (6 mL), and the resulting mixture was stirred for 5 min. A few drops of DMF were added, and the resulting mixture was stirred at ambient temperature for 18 h. Excess thionyl chloride was removed *in vacuo* and the residue was co-distilled with toluene (3 × 20 mL) to afford the acyl chloride. A solution of the acyl chloride in DCM (10 mL) was rapidly added to a solution of amine (dimethyl 2-aminoisophthalate, 210 mg, 3.1 mmol) and *N,N*-diisopropylethylamine (0.53 mL, 3.045 mmol) in DCM (10 mL) at ambient temperature and the reaction was stirred for 18 h. The reaction was diluted with DCM (70 mL) and was washed with a 1 M aqueous hydrochloric acid (100 mL), water (100 mL), dried ($Na_2SO_4$), filtered and concentrated to give the crude product, which was purified by silica gel chromatography eluting with a gradient of ethyl acetate (0 to 25%) in iso-hexane to the desired product as a white solid (1.24 g, 2.075 mmol, 75%).
UPLC-MS (acidic method, 2 min): rt = 1.37 min; *m*/*z* = 598.2 $[M+H]^+$, peak area >95%
$^1$H NMR (400 MHz, DMSO- $d_6$) δ 11.60 (s, 1H), 8.02 (d, *J* = 7.8 Hz, 2H), 7.69 (s, 1H), 7.59 - 7.23 (m, 12H), 5.49 (s, 2H), 5.19 (s, 2H), 4.28 (q, *J* = 7.1 Hz, 2H), 3.71 (s, 6H), 1.26 (t, *J* = 7.1 Hz, 3H).

**General procedure B for Amide Coupling [3B]**

➢ Coupling using a condensation agent, from **KI-1** or **KI-6**

**Model Reaction (Ia-001a)**

***Methyl 2-(2,5-bis(benzyloxy)-4-(ethoxycarbonyl)benzoylamino)benzoate.*** 1-Methylimidazole (13.0 mL, 163 mmol) was added to a mixture of 2,5-bis(benzyloxy)-4-(ethoxycarbonyl)benzoic acid (**KI-1**, 12.95 g, 32 mmol) and methyl anthranilate (5.729 g, 38 mmol, 1.190 eq) in MeCN (150 mL) at ambient temperature. Chloro-*N,N,N',N'*-tetramethylformamidinium hexafluorophosphate(TCFH) (10.728 g, 0.038 mol) was added portionwise over 30 min, and the resulting mixture was stirred overnight at ambient temperature. The reaction mixture was treated with 1 M aqueous hydrochloric acid (700 mL). The resulting suspension was stirred for 10 min, then filtered. The solid material was washed with water (2 × 200 mL). The solid was then suspended in ethanol (200 mL), filtered, washed with ether (100 mL) and dried in the vacuum oven to give the desired product as a white solid (14.7 g, 27 mmol, 86%)
UPLC-MS (acidic method, 2 min): rt = 1.43 min; *m*/*z* = 540.2 $[M+H]^+$, peak area >99%
$^1$H NMR (400 MHz, DMSO-$d_6$) δ 11.87 (s, 1H), 8.65 (d, *J* = 8.4 Hz, 1H), 7.98 (dd, *J* = 8.0, 1.7 Hz, 1H), 7.81 - 7.61 (m, 2H), 7.59 - 7.12 (m, 12H), 5.41 (s, 2H), 5.20 (s, 2H), 4.28 (q, *J* = 7.1 Hz, 2H), 3.74 (s, 3H), 1.27 (t, *J* = 7.1 Hz, 3H).0

**General Deprotection procedures [4], [5]**

**Saponification**

**Model reaction (Ia-013a)**

***2-(2,5-Bis(benzyloxy)-4-carboxybenzoylamino)isophthalic acid.*** The solution of lithium hydroxide (42 mg, 1 mmol) in water (4 mL) was added to the solution of dimethyl 2-(2,5-bis(benzyloxy)-4-(ethoxycarbonyl)benzoylamino)isophthalate (100 mg, 0.167 mmol) in THF (6 mL) and the resulting mixture was stirred at ambient temperature for 18 h. Additional LiOH (6 eq) in water (4 mL) was added to the reaction mixture which was stirred for an additional 23 h. After completion, water (50 mL) was added to the reaction and the mixture was washed with ethyl acetate (50 mL). The aqueous layer was acidified with a 1 M aqueous hydrochloric acid to pH=2 and extracted with ethyl acetate (2X 50 mL). The organic layers were collected, dried ($Na_2SO_4$), filtered and concentrated to give the title compound as a white solid (82 mg, 0.151 mmol, 91%).
UPLC-MS (acidic method, 2 min): rt = 1.02 min; *m/z* = 542.1 $[M+H]^+$, peak area >95%
$^1$H NMR (400 MHz, DMSO-$d_6$) δ 13.18 (s, 2H), 11.74 (s, 1H), 8.00 (d, *J* = 7.8 Hz, 2H), 7.71 (s, 1H), 7.58 - 7.43 (m, 5H), 7.40 - 7.24 (m, 7H), 5.47 (s, 2H), 5.14 (s, 2H).

**Debenzylation**

**Model reaction (Ia-013a)**

***2-(2,5-Dihydroxy-4-carboxybenzoylamino)isophthalic acid (*Ia-013a).** Pd/C (94 mg, 10%) was added to a solution of 2-(2,5-bis(benzyloxy)-4-carboxybenzoylamino)isophthalic acid (0.94 g, 1.74 mmol) in a 10:30 mixture of EtOH and DCM. The mixture was placed under hydrogen at atmospheric pressure at ambient temperature and stirred for 18 h. After completion, the mixture was filtered through Celite, which was washed with DCM and MeOH. The filtrate and washings were combined and concentrated under reduced pressure to afford the desired product **Ia-013a** as a yellow solid (644 mg, 1.69 mmol, 98%).
UPLC-MS (acidic method, 4 min): rt = 0.91 min; *m/z* =362.0 $[M+H]^+$, peak area >98%
$^1$H NMR (DMSO-$d_6$) δ: 13.11 (s, 2H), 11.70 (s, 1H), 11.13 (s, 1H), 7.97 (d, *J* = 7.8 Hz, 2H), 7.47 (s, 1H), 7.41 (s, 1H), 7.38 (t, *J* = 7.7 Hz, 1H)

**Scheme 4a.** Synthesis of Monoamides **Ia** from **KI-1Bn**

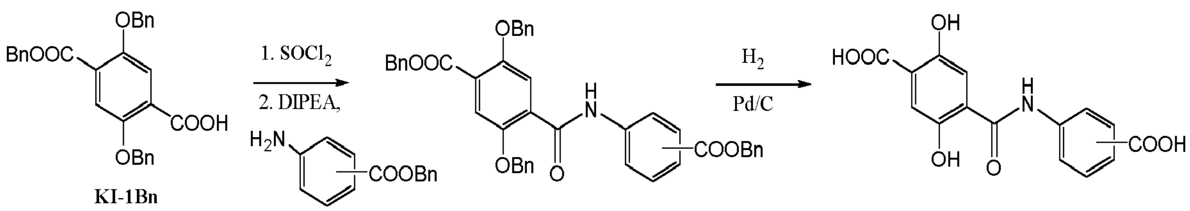


KI-1Bn

**General procedure from KI-1Bn**

- Coupling *via* acyl chloride

**Model Reaction : synthesis of Ia-032a**

***Benzyl 4-(2,5-bis(benzyloxy)-4-(benzyloxycarbonyl)benzoylamino)phenylacetate.*** To a solution of **KI-1Bn** (233 mg, 84% purity) in DCM (10 mL) was added $SOCl_2$ (1.8 mL, 60 eq) and the mixture was heated at 50°C for 3h. The solvents were evaporated and the residue co-evaporated with toluene (3x10 mL), to give the crude acyl chloride (237 mg, 99%). This material was dissolved in DCM (3 mL), and diisopropylethylamine (0.08 mL, 1.15 eq) was added, followed by a solution of benzyl 4-aminophenylacetate (92 mg, 0.95 eq) in DCM (3 mL). Final reaction volume was 10 mL. The reaction mixture was stirred at room temp. for 18h. The mixture was diluted with DCM (15 mL), washed with sat aqueous ammonium chloride (12 mL). The separated aqueous phase was extracted with DCM (2x2 mL), the organic phases were combined, dried (MgSO4), and concentrated. The crude product was submitted to column chromatography (Pet. Et./DCM 1:1 then gradient to 100% DCM) to give a first fraction of pure KI-0Bn, and second fraction containing the pure desired product (237 mg, 86%, corrected for the purity of KI-1Bn).

$^1H$ NMR (400 MHz, $CDCl_3$): δ 10.10 (s, 1H), 8.08 (s, 1H), 7.65 (s, 1H), 7.53-7.30 (m, 20H), 7.22-7.13 (br AB, 4H), 5.39, 5.22, 5.21, 5.12 (4s, 4x 2H), 3.61 (s, 2H).

***4-(4-(Carboxymethyl)phenylaminocarbonyl)-2,5-dihydroxybenzoic acid*** **(Ia-032a).** To a solution of precursor prepared above (225 mg, 0.33 mmol) in DCM (20 mL) was added 5% Pd on charcoal (72 mg), followed by EtOH (20 mL). The flask was flushed with Ar (3x) then filled with hydrogen (atmospheric pressure) and the mixture stirred for 2h at room temperature. The catalyst was removed by filtration over a millipore filter, the filtrate was evaporated and the residue dried to afford the product **Ia-032a** as a light yellow solid (107 mg, 99%).

$^1H$ NMR (250 MHz, DMSO-$d_6$): δ 12.28 (br, 1H), 10.92 (s, 1H), 10.44 (s, 1H), 7.65 (br d, 2H), 7.41 (s, 1H), 7.38 (s, 1H), 7.25 (br d, 2H), 3.55 (s, 2H).

**Examples:**

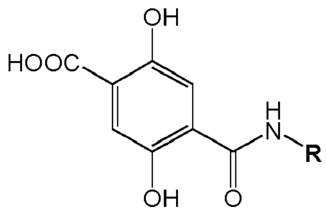

R =

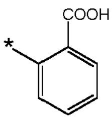
Ia-001a

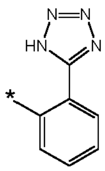
Ia-001aTz

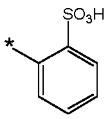
Ia-001c

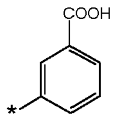
Ia-002a

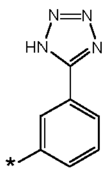
Ia-002aTz

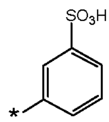
Ia-002c

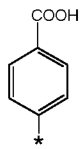
Ia-003a

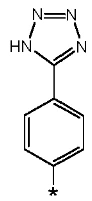
Ia-003aTz

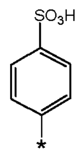
Ia-003c

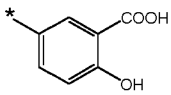
Ia-004a

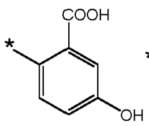
Ia-006a

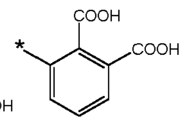
Ia-011a

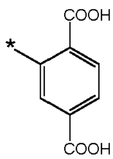
Ia-012a

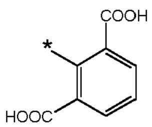
Ia-013a

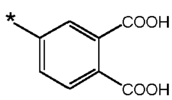
Ia-014a

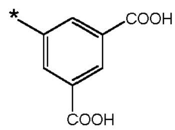
Ia-015a

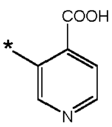
Ia-023a

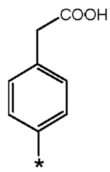
Ia-032

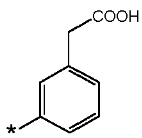
Ia-033a

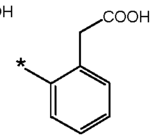
Ia-034a

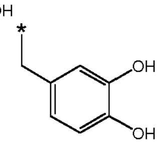
Ia-035a

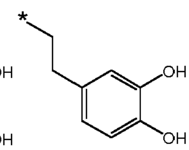
Ia-035a-2

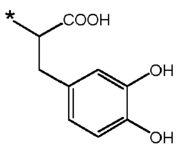
Ia-035a-3

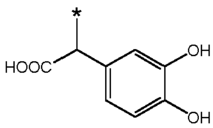
Ia-036a

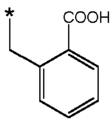
Ia-053a

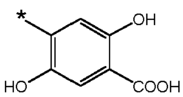
Ia-056a

For conditions and yields: See Table 1 (Figures 4A-D)

**1) 4-(2-Carboxyphenylaminocarbonyl)-2,5-dihydroxybenzoic acid,** compound **Ia-001a**

UPLC-MS (acidic method, 2 min): r.t = 0.95 mins, $m/z$ = 316 $[M-H]^-$, peak area > 97% $^1H$ NMR (400 MHz, DMSO-$d_6$) δ 12.22 (s, 1H), 10.85 (s, 1H), 8.65 (d, $J$ = 8.4 Hz, 1H), 8.01 (dd, $J$ = 8.0, 1.7 Hz, 1H), 7.71 -7.56 (m, 1H), 7.41 (d, $J$ = 6.9 Hz, 2H), 7.22 (t, $J$ = 7.6 Hz, 1H).
HRMS: $[M+H]^+$, calc. for $C_{15}H_{12}NO_7$: 318.06083, found: 318.06080.
Biodata: **Ia-001a**: FGF-1 IC50 [μM] = 41; FGF-2 IC50 [μM] = 39; VEGF-A1 IC50 [μM] = 7.3; VEGFR-Phosphorylation

inhibition IC50 [μM] =2.25; PMN ROS [inhibition at 0.3 μM [%] = 48; PMN ROS inhibition IC50 [μM] = 0.355; Neutrophil adhesion inhibition [%] = 44.3; Whole Blood: GM-CSF IC50 [μM] = >100; IFNγ IC50 [μM] = 30.5; IL-1β IC50 [μM] = 34.4; IL-2 IC50 [μM] = 94.3; IL-4 IC50 [μM] = >100; IL-5 IC50 [μM] = >100; IL-6 IC50 [μM] = 1.24; IL-9 IC50 [μM] = 5.63; IL-10 IC50 [μM] = >100; IL-12p70 IC50 [μM] = 1.5; IL-13 IC50 [μM] = 26.4; IL-17A IC50 [μM] = 0.1; IL-17F IC50 [μM] = 14.7; IL-18 IC50 [μM] = >100; IL-21 IC50 [μM] = >100; IL-33 IC50 [μM] = 19.3; TGFβ IC50 [μM] = >100; TNF α IC50 [μM] = 0.02; TNFβ IC50 [μM] = 13.2

**Diethyl ester:**

UPLC-MS (acidic method, 2 min): r.t = 1.39 mins, *m/z* = 372.2 $[M-H]^-$, peak area > 98%
$^1$H NMR (400 MHz, DMSO-$d_6$) δ 11.96 (s, 1H), 11.05 (s, 1H), 9.87 (s, 1H), 8.57 (dd, *J* = 8.5, 1.2 Hz, 1H), 7.99 (dd, *J* = 7.9, 1.7 Hz, 1H), 7.65 (ddd, J= 8.7, 7.3, 1.7 Hz, 1H), 7.49 (s, 1H), 7.40 (s, 1H), 7.25 (td, *J* = 7.6, 1.2 Hz, 1H), 4.35 (m, 4H), 1.33 (m, 6H).
HRMS: $[M+H]^+$, calc. for $C_{19}H_{20}NO_7$: 374.12342, found: 374.12354
Biodata: **Ia-001a-E2:** FGF-1 IC50 [μM] = 9.2; FGF-2 IC50 [μM] = 200; VEGF-A1 IC50 [μM] = 123; VEGFR-Phosphorylation inhibition IC50 [μM] =1.25; PMN ROS [inhibition at 0.3 μM [%] = 51.47; PMN ROS inhibition IC50 [μM] = 2.58; Neutrophil adhesion inhibition [%] = 50.5

2) **4-(2-(1H-Tetrazol-5-yl)phenylaminocarbonyl)-2,5-dihydroxybenzoic acid**, compound **Ia-001aTz:**

UPLC-MS (acidic method, 4 min): rt = 1.17 min; *m/z* =342.0 $[M+H]^+$, peak area >96%
$^1$H NMR (400 MHz, DMSO-$d_6$) δ 11.60 (s, 1H), 10.90 (s, 2H), 8.47 (d, *J* = 8.4 Hz, 1H), 7.93 (d, *J* = 7.9 Hz, 1H), 7.61 (t, *J* = 7.9 Hz, 1H), 7.50 - 7.32 (m, 3H).
HRMS: $[M+H]^+$, calc. for $C_{15}H_{12}N_5O_5$: 342.08329, found: 342.08318
Biodata: **Ia-001a-Tz**: FGF-1 IC50 [μM] = 6.2; FGF-2 IC50 [μM] = 20; VEGF-A1 IC50 [μM] = 17; VEGFR-Phosphorylation inhibition IC50 [μM] =0.23; PMN ROS [inhibition at 0.3 μM [%] = 54.33; PMN ROS inhibition IC50 [μM] = 1.54; Neutrophil adhesion inhibition [%] = 69.75; Whole Blood: GM-CSF IC50 [μM] = >100; IFNγ IC50 [μM] = >100; IL-1β IC50 [μM] = >100; IL-2 IC50 [μM] = 7.99; IL-4 IC50 [μM] = >100; IL-5 IC50 [μM] = >100; IL-6 IC50 [μM] = 0.75; IL-9 IC50 [μM] = 8.63; IL-10 IC50 [μM] = 26.1; IL-12p70 IC50 [μM] = 32.4; IL-13 IC50 [μM] = 32.1; IL-17A IC50 [μM] = 0.11; IL-17F IC50 [μM] = 46.5; IL-18 IC50 [μM] = >100; IL-21 IC50 [μM] = >100; IL-33 IC50 [μM] = 23.6; TGFβ IC50 [μM] = >100; TNF α IC50 [μM] = 0.63; TNF β IC50 [μM] = 53

**Ethyl ester:**

UPLC-MS (acidic method, 2 min): r.t = 1.08 mins, *m/z* = 370.1 $[M+H]^+$, peak area > 97%
$^1$H NMR (400 MHz, DMSO-$d_6$) δ 11.48 (s, 1H), 9.88 (s, 1H), 8.46 (d, *J* = 8.4 Hz, 1H), 7.89 (dd, *J* = 7.8, 1.6 Hz, 1H), 7.62 (ddd, *J* = 8.6, 7.4, 1.6 Hz, 1H), 7.47 (s, 1H), 7.42 - 7.34 (m, 2H), 4.37 (q, *J* = 7.1 Hz, 2H), 1.34 (t, *J* = 7.1 Hz, 3H).
HRMS: $[M+H]^+$, calc. for $C_{17}H_{16}N_5O_5$: 370.11460, found: 370.11458
Biodata: **Ia-001a-Tz-E1**: FGF-1 IC50 [μM] = 200; FGF-2 IC50 [μM] = 200; VEGF-A1 IC50 [μM] = 200; VEGFR-Phosphorylation inhibition IC50 [μM] =3.8; PMN ROS [inhibition at 0.3 μM [%] = 75.97; PMN ROS inhibition IC50 [μM] = N.D.; Neutrophil adhesion inhibition [%] = 98.88

**Ethyl ester Diacetate:**

UPLC-MS (acidic method, 4 min): r.t = 1.57 mins, *m/z* = 454.1 $[M+H]^+$, peak area > 97%
$^1$H NMR (400 MHz, DMSO-$d_6$) δ 11.23 (s, 1H), 8.23 (d, *J* = 8.6 Hz, 1H), 7.96 (dd, *J* = 7.9, 1.7 Hz, 1H), 7.84 (s, 1H), 7.73 (s, 1H), 7.64 (t, *J* = 7.5 Hz, 1H), 7.43 (td, *J* = 7.6, 1.2 Hz, 1H), 4.32 (q, *J* = 7.1 Hz, 2H), 2.34 (s, 3H), 2.18 (s, 3H), 1.32 (t, *J* = 7.1 Hz, 3H).
HRMS: $[M+H]^+$, calc. for $C_{21}H_{20}N_5O_7$: 454.13572, found: 454.13542
Biodata: **Ia-001a-Tz-El-A2**: FGF-1 IC50 [μM] = 60; FGF-2 IC50 [μM] = 200; VEGF-A1 IC50 [μM] = 200; VEGFR-Phosphorylation inhibition IC50 [μM] =ND; PMN ROS [inhibition at 0.3 μM [%] = 49.85; PMN ROS inhibition IC50 [μM] = N.D.; Neutrophil adhesion inhibition [%] = 98.2

3) **2,5-Dihydroxy-4-(2-sulfophenylaminocarbonyl)benzoic acid**, compound **Ia-001c**:

UPLC-MS (acidic method, 2 min): rt = 0.73 min; *m/z* =352.0 $[M-H]^-$, peak area >99%
$^1$H NMR (400 MHz, DMSO-$d_6$) δ 11.54 (s, 1H), 10.99 (s, 1H), 8.34 (d, J = 8.2 Hz, 1H), 7.74 (dd, J = 7.7, 1.7 Hz, 1H), 7.42 - 7.28 (m, 3H), 7.12 (td, J = 7.5, 1.2 Hz, 1H).

HRMS: $[M+H]^+$, calc. for $C_{14}H_{12}NO_8S$: 354.02781, found: 354.02754.
Biodata: **Ia-001c**: FGF-1 IC50 [μM] = 21; FGF-2 IC50 [μM] = 13; VEGF-A1 IC50 [μM] = 100; VEGFR-Phosphorylation inhibition IC50 [μM] =3.31; PMN ROS [inhibition at 0.3 μM [%] = 40.36; PMN ROS inhibition IC50 [μM] = 2.4; Neutrophil adhesion inhibition [%] = 31.33; Whole Blood: GM-CSF IC50 [μM] = >100; IFNγ IC50 [μM] = 0.44; IL-1β IC50 [μM] = >100; IL-2 IC50 [μM] = 12.8; IL-4 IC50 [μM] = >100; IL-5 IC50 [μM] = >100; IL-6 IC50 [μM] = 0.95; IL-9 IC50 [μM] = 46.9; IL-10 IC50 [μM] = 1.9; IL-12p70 IC50 [μM] = 0.44; IL-13 IC50 [μM] = 86.1; IL-17A IC50 [μM] = 0.09; IL-17F IC50 [μM] = 89.7; IL-18 IC50 [μM] = >100; IL-21 IC50 [μM] = >100; IL-33 IC50 [μM] = >100; TGFβ IC50 [μM] = >100; TNF α IC50 [μM] = 0.05; TNF β IC50 [μM] = 29.7

4) **4-(3-Carboxyphenylaminocarbonyl)-2,5-dihydroxybenzoic acid**, compound **Ia-002a**:

$^1$H-NMR (250 MHZ, DMSO-$d_6$) δ ~13 (v br, 1H), 10.82 (br s, 1H), 10.60 (br s, 1H), 8.38 (s, 1H). 7.92 (d, 1H), 7.71 (d, 1H), 7.49 (t, 1H), 7.39 (s, 2H)
Biodata: **1a-002a:** FGF-1 IC50 [μM] = 20; FGF-2 IC50 [μM] = 59; VEGF-A1 IC50 [μM] = 71; VEGFR-Phosphorylation inhibition IC50 [μM] =5.7; PMN ROS [inhibition at 0.3 μM [%] = 42.1; PMN ROS inhibition IC50 [μM] = 0.312; Neutrophil adhesion inhibition [%] = 27.92

5) **4-(3-(1H-Tetrazol-5-yl)phenylaminocarbonyl)-2,5-dihydroxybenzoic acid**, compound **Ia-002aTz:**

UPLC-MS (acidic method, 4 min): rt = 1.13 min; *m/z* =342.0 $[M+H]^+$, peak area >99%
$^1$H NMR (400 MHz, DMSO-$d_6$) δ 10.81 (s, 1H), 10.67 (s, 1H), 8.54 (s, 1H), 7.91 - 7.84 (m, 1H), 7.79 (dt, J = 7.8, 1.4 Hz, 1H), 7.60 (t, J = 7.9 Hz, 1H), 7.41 (d, J = 2.2 Hz, 2H).
HRMS: $[M+H]^+$, calc. for $C_{15}H_{12}N_5O_5$: 342.08329, found: 342.08317
Biodata: **Ia-002a-Tz:** FGF-1 IC50 [μM] = 10; FGF-2 IC50 [μM] = 53; VEGF-A1 IC50 [μM] = 57; VEGFR-Phosphorylation inhibition IC50 [μM] =100; PMN ROS [inhibition at 0.3 μM [%] = 66.61; PMN ROS inhibition IC50 [μM] = 1.86; Neutrophil adhesion inhibition [%] = 38.5

6) **2,5-Dihydroxy-4-(3-sulfophenylaminocarbonyl)benzoic acid**, compound **Ia-002c**:

$^1$H-NMR (250 MHZ, DMSO-$d_6$) δ 11.04 (s, 1H), 10.53 (s, 1H), 7.96 (s, 1H), 7.69 (d, 1H), 7.43 (s, 2H) and 7.40-7.28 (m, 2H)
Biodata: Ia-002c: FGF-1 IC50 [μM] = 14; FGF-2 IC50 [μM] = 150; VEGF-A1 IC50 [μM] = 150; VEGFR-Phosphorylation inhibition IC50 [μM] =100; PMN ROS [inhibition at 0.3 μM [%] = 38.9; PMN ROS inhibition IC50 [μM] = 0.405; Neutrophil adhesion inhibition [%] = 1.87

7) **4-(4-Carboxyphenylaminocarbonyl)-2,5-dihydroxybenzoic acid**, compound **Ia-003a**:

$^1$H-NMR (250 MHz, DMSO-$d_6$) δ 12.8 (br, 1H), 10.73 (s, 1H), 10.68 (s, 1H), 7.94 (d of AB, 2H), 7.84 (d of AB, 2H), 7.39 (s, 1H), 7.34 (s, 1H)
HRMS (AX033)
Biodata: **Ia-003a:** FGF-1 IC50 [μM] = 12; FGF-2 IC50 [μM] = 34; VEGF-A1 IC50 [μM] = 150; VEGFR-Phosphorylation inhibition IC50 [μM] =ND; PMN ROS [inhibition at 0.3 μM [%] = 44.4; PMN ROS inhibition IC50 [μM] = 0.414; Neutrophil adhesion inhibition [%] = 39.1

8) **4-(4-(1H-Tetrazol-5-yl)phenylaminocarbonyl)-2,5-dihydroxybenzoic acid**, compound **Ia-003aTz**:

UPLC-MS (acidic method, 2 min): rt = 0.79 min; *m/z* =342.0 $[M+H]^+$, peak area >97%
$^1$H NMR (400 MHz, DMSO-$d_6$) δ 10.77 (s, 1H), 10.70 (s, 1H), 8.04 (d, J = 8.7 Hz, 2H), 7.96 (d, J = 8.5 Hz, 2H), 7.41 (s, 1H), 7.37 (s, 1H).
HRMS: $[M+H]^+$, calc. for $C_{15}H_{12}N_5O_5$: 342.08329, found: 342.08326
Biodata: **Ia-003a-Tz**: FGF-1 IC50 [μM] = 6.7; FGF-2 IC50 [μM] = 45; VEGF-A1 IC50 [μM] = 13; VEGFR-Phosphorylation inhibition IC50 [μM] =6.4; PMN ROS [inhibition at 0.3 μM [%] = 62.21; PMN ROS inhibition IC50 [μM] = 2.49; Neutrophil adhesion inhibition [%] = 30.33; Whole Blood: GM-CSF IC50 [μM] = >100; IFNγ IC50 [μM] = 2.95; IL-1β IC50 [μM] = >100; IL-2 IC50 [μM] = 43; IL-4 IC50 [μM] = >100; IL-5 IC50 [μM] = >100; IL-6 IC50 [μM] = 1.41; IL-9 IC50 [μM] = 3.62; IL-10 IC50 [μM] = 6.94; IL-12p70 IC50 [μM] = 1.26; IL-13 IC50 [μM] = 89.6; IL-17A IC50 [μM] = 0.07; IL-17F IC50 [μM] = 83.9; IL-18 IC50 [μM] = >100; IL-21 IC50 [μM] = 1.49; IL-33 IC50 [μM] = 72.2; TGFβ IC50 [μM] = >100; TNF α IC50 [μM] = 0.18; TNF β IC50 [μM] = 55.

9) **2,5-Dihydroxy-4-(4-sulfophenylaminocarbonyl)benzoic acid**, compound **Ia-003c**:

$^1$H-NMR (400 MHz, DMSO-$d_6$) δ 10.90 (s, 1H), 10.51 (s, 1H), 7.66 (d of AB, 2H), 7.58 (d of AB, 2H), 7.40 (s, 1H), 7.39 (S, 1H)
Biodata: **Ia-003c**: FGF-1 IC50 [μM] = 35; FGF-2 IC50 [μM] = 150; VEGF-A1 IC50 [μM] = 150; VEGFR-Phosphorylation inhibition IC50 [μM] =0.4; PMN ROS [inhibition at 0.3 μM [%] = N.D.; PMN ROS inhibition IC50 [μM]-; Neutrophil adhesion inhibition [%] = N.D.

10) **4-(3-Carboxy-4-hydroxyphenylaminocarbonyl)-2,5-dihydroxybenzoic acid**, compound **Ia-004a**:

UPLC-MS (acidic method, 6.5 min): rt = 1.40 min; *m/z* =332.0 [M-H]$^-$, peak area >96%
$^1$H NMR (400 MHz, DMSO-$d_6$) δ 10.94 (s, 1H), 10.37 (s, 1H), 8.20 (d, J = 2.7 Hz, 1H), 7.74 (dd, J = 8.9, 2.8 Hz, 1H), 7.39 (s, 1H), 7.34 (s, 1H), 6.93 (d, J = 8.9 Hz, 1H).
HRMS: [M+H]$^+$, calc. for $C_{15}H_{12}NO_8$: 334.05574, found: 334.05572
Biodata: **Ia-004a**: FGF-1 IC50 [μM] = 32; FGF-2 IC50 [μM] = 15; VEGF-A1 IC50 [μM] = 28; VEGFR-Phosphorylation inhibition IC50 [μM] =7.7; PMN ROS [inhibition at 0.3 μM [%] = 64.61; PMN ROS inhibition IC50 [μM] = 0; Neutrophil adhesion inhibition [%] = 28.38

**Ethyl Methyl ester**:

UPLC-MS (acidic method, 2 min): r.t = 1.16 mins, *m/z* = 376.1 [M+H]$^+$, peak area > 95%
$^1$H NMR (400 MHz, DMSO-d6) δ 10.90 (s, 1H), 10.50 (s, 1H), 10.36 (s, 1H), 9.91 (s, 1H), 8.26 (d, *J* = 2.7 Hz, 1H), 7.76 (dd, *J* = 8.9, 2.8 Hz, 1H), 7.43 (s, 1H), 7.36 (s, 1H), 7.01 (d, *J* = 8.9 Hz, 1H), 4.37 (q, *J* = 7.1 Hz, 2H), 3.91 (s, 3H), 1.34 (t, *J* = 7.1 Hz, 3H).
HRMS: [M+H]$^+$, calc. for $C_{18}H_{18}NO_8$: 376.10269, found: 376.10259
Biodata: **Ia-004a-E2**: FGF-1 IC50 [μM] = 35; FGF-2 IC50 [μM] = 36; VEGF-A1 IC50 [μM] = 200; VEGFR-Phosphorylation inhibition IC50 [μM] =6.1; PMN ROS [inhibition at 0.3 μM [%] = 85.34; PMN ROS inhibition IC50 [μM] = N.D.; Neutrophil adhesion inhibition [%] = 97.65

11) **4-(2-Carboxy-4-hydroxyphenylaminocarbonyl)-2,5-dihydroxybenzoic acid**, compound **Ia-006a**:

UPLC-MS (acidic method, 2 min): rt = 0.81 min; *m/z* =334.0 [M+H]$^+$, peak area >96%
$^1$H NMR (400 MHz, DMSO-$d_6$) δ 13.33 (s, 1H), 11.86 (s, 1H), 10.90 (s, 1H), 9.66 (s, 1H), 8.39 (d, *J* = 9.0 Hz, 1H), 7.40 (d, *J* = 6.8 Hz, 2H), 7.03 (dd, *J* = 9.0, 3.0 Hz, 1H).
HRMS: [M+H]$^+$, calc. for $C_{15}H_{12}NO_8$: 334.05574, found: 334.05562.
Biodata: **Ia-006a**: FGF-1 IC50 [μM] = 89; FGF-2 IC50 [μM] = 224; VEGF-A1 IC50 [μM] = 100; VEGFR-Phosphorylation inhibition IC50 [μM] =11.8; PMN ROS [inhibition at 0.3 μM [%] = 60.56; PMN ROS inhibition IC50 [μM] = 1.2; Neutrophil adhesion inhibition [%] = 25.33

12) **3-(4-Carboxy-2,5-dihydroxybenzamido)phthalic acid**, compound **Ia-011a:**

UPLC-MS (acidic method, 2 min): rt = 0.69 min; *m/z* = 360.1 [M-H]$^-$, peak area >89%
$^1$H NMR (400 MHz, DMSO-$d_6$) δ 13.46 (s, 2H), 11.37 (s, 1H), 10.97 (s, 1H), 8.33 (dd, *J* = 8.2, 1.3 Hz, 1H), 7.63 (dd, *J* = 7.7, 1.3 Hz, 1H), 7.57 (t, *J* = 7.9 Hz, 1H), 7.52 (s, 1H), 7.44 (s, 1H).
HRMS: [M+H]$^+$, calc. for $C_{16}H_{12}NO_9$: 362.05065, found: 362.05036
Biodata: **Ia-011a**: FGF-1 IC50 [μM] = 35; FGF-2 IC50 [μM] = 110; VEGF-A1 IC50 [μM] = 200; VEGFR-Phosphorylation inhibition IC50 [μM] =100; PMN ROS [inhibition at 0.3 μM [%] = 57.71; PMN ROS inhibition IC50 [μM] = N.D.; Neutrophil adhesion inhibition [%] = 42.32

13) **2-(4-Carboxy-2,5-dihydroxybenzamido)terephthalic acid**, compound **Ia-012a**:

UPLC-MS (acidic method, 4 min): rt = 1.12 min; *m/z* = 362.1 [M+H]$^+$, peak area >96%
$^1$H NMR (400 MHz, DMSO-$d_6$) δ 13.41 (brs, 2H), 12.29 (s, 1H), 10.94 (s, 1H), 9.26 (d, *J* = 1.7 Hz, 1H), 8.09 (d, *J* = 8.2 Hz, 1H), 7.73 (dd, *J* = 8.2, 1.7 Hz, 1H), 7.42 (s, 2H).
HRMS: [M+H]$^+$, calc. for $C_{16}H_{12}NO_9$: 362.05065, found: 362.05046.
Biodata: **Ia-012a**: FGF-1 IC50 [μM] = 38; FGF-2 IC50 [μM] = 36; VEGF-A1 IC50 [μM] = 30; VEGFR-Phosphorylation inhibition IC50 [μM] =100; PMN ROS [inhibition at 0.3 μM [%] = 59.52; PMN ROS inhibition IC50 [μM] = 1.85; Neutrophil adhesion inhibition [%] = 28.5

14) **2-(4-Carboxy-2,5-dihydroxybenzamido)isophthalic acid**, compound **Ia-013a**:

UPLC-MS (acidic method, 4 min): rt = 0.91 min; *m/z* = 362.0 $[M+H]^+$, peak area >98% $^1H$ NMR (400 MHz, DMSO-$d_6$) δ 13.11 (s, 2H), 11.70 (s, 1H), 11.13 (s, 1H), 7.97 (d, J = 7.8 Hz, 2H), 7.47 (s, 1H), 7.41 (s, 1H), 7.38 (t, J = 7.7 Hz, 1H)
HRMS: $[M+H]^+$, calc. for $C_{16}H_{12}NO_9$: 362.05065, found: 362.05041
Biodata: **Ia-013a**: FGF-1 IC50 [μM] = 29; FGF-2 IC50 [μM] = 18; VEGF-A1 IC50 [μM] = 17; VEGFR-Phosphorylation inhibition IC50 [μM] =100; PMN ROS [inhibition at 0.3 μM [%] = 38.52; PMN ROS inhibition IC50 [μM] = 2.38; Neutrophil adhesion inhibition [%] = 27.67

15) **4-(4-Carboxy-2,5-dihydroxybenzamido)phthalic acid**, compound **Ia-014a**:

UPLC-MS (acidic method, 4 min): rt = 0.98 min; *m/z* = 362.1 $[M+H]^+$, peak area >94%
$^1H$ NMR (400 MHz, DMSO-$d_6$) δ 10.76 (s, 1H), 10.70 (s, 1H), 8.14 (s, 1H), 7.94 - 7.84 (m, 2H), 7.38 (s, 1H), 7.30 (s, 1H).
HRMS: $[M+H]^+$, calc. for $C_{16}H_{12}NO_9$: 362.05065, found: 362.05047
Biodata: **Ia-014a**: FGF-1 IC50 [μM] = 20; FGF-2 IC50 [μM] = 20; VEGF-A1 IC50 [μM] = 88; VEGFR-Phosphorylation inhibition IC50 [μM] =100; PMN ROS [inhibition at 0.3 μM [%] = 61.3; PMN ROS inhibition IC50 [μM] = 1; Neutrophil adhesion inhibition [%] = 9.667

16) **5-(4-Carboxy-2,5-dihydroxybenzamido)isophthalic acid**, compound **Ia-015a**:

$^1H$ NMR (250 MHz, DMSO-$d_6$) δ 10.74 (s, 1H), 8.56 (narrow d, 2H), 8.22 (narrow t, 1H), 7.41 (s, 1H) and 7.37 (s, 1H)
Biodata: **Ia-015a**: FGF-1 IC50 [μM] = 20; FGF-2 IC50 [μM] = 9.3; VEGF-A1 IC50 [μM] = 19; VEGFR-Phosphorylation inhibition IC50 [μM] =0.15; PMN ROS [inhibition at 0.3 μM [%] = 43.8; PMN ROS inhibition IC50 [μM] = 0.387; Neutrophil adhesion inhibition [%] = 17.38;
Whole Blood: GM-CSF IC50 [μM] = 2.76; IFNγ IC50 [μM] = >100; IL-1β IC50 [μM] = >100; IL-2 IC50 [μM] = 7.08; IL-4 IC50 [μM] = >100; IL-5 IC50 [μM] = >100; IL-6 IC50 [μM] = 0.77; IL-9 IC50 [μM] = 1.47; IL-10 IC50 [μM] = 5.27; IL-12p70 IC50 [μM] = 0.12; IL-13 IC50 [μM] = 0.15; IL-17A IC50 [μM] = 0.05; IL-17F IC50 [μM] = 0.15; IL-18 IC50 [μM] = >100; IL-21 IC50 [μM] = 57.2; IL-33 IC50 [μM] = 0.24; TGFβ IC50 [μM] = >100; TNF α IC50 [μM] = 0.17; TNF β IC50 [μM] = 0.3.

17) **3-(4-carboxy-2,5-dihydroxybenzamido)isonicotinic acid**, compound **Ia-023a**:

UPLC-MS (acidic method, 4 min): rt = 0.63 min; *m/z* = 319.1 $[M+H]^+$, peak area >93%
$^1H$ NMR (400 MHz, DMSO-$d_6$) δ 12.17 (s, 1H), 11.03 (s, 1H), 9.78 (s, 1H), 8.46 (d, J = 5.0 Hz, 1H), 7.82 (d, *J* = 5.0 Hz, 1H), 7.45 (s, 1H), 7.43 (s, 1H).
HRMS: $[M+H]^+$, calc. for $C_{14}H_{11}N_2O_7$: 319.05608, found: 319.05610
Biodata: **Ia-023a**: FGF-1 IC50 [μM] = N.D.; FGF-2 IC50 [μM] = 63; VEGF-A1 IC50 [μM] = 143; VEGFR-Phosphorylation inhibition IC50 [μM] =100; PMN ROS [inhibition at 0.3 μM [%] = 61.95; PMN ROS inhibition IC50 [μM] = 0; Neutrophil adhesion inhibition [%] = 13.37

**Diethyl ester:**

UPLC-MS (acidic method, 4 min): r.t = 1.87 mins, *m/z* = 375.1 $[M+H]^+$, peak area > 98%
$^1H$ NMR (400 MHz, DMSO-d6) δ 11.79 (s, 1H), 11.23 (s, 1H), 9.87 (s, 1H), 9.68 (s, 1H), 8.50 (d, J = 5.0 Hz, 1H), 7.81 (dd, J = 5.1, 0.7 Hz, 1H), 7.53 (s, 1H), 7.41 (s, 1H), 4.46 - 4.27 (m, 4H), 1.41 - 1.25 (m, 6H).
HRMS: $[M+H]^+$, calc. for $C_{18}H_{19}N_2O_7$: 375.11867, found: 375.11867
Biodata: **Ia-023a-E2**: FGF-1 IC50 [μM] = N.D.; FGF-2 IC50 [μM] = 76; VEGF-A1 IC50 [μM] = 141; VEGFR-Phosphorylation inhibition IC50 [μM] =6.6; PMN ROS [inhibition at 0.3 μM [%] = 69.47; PMN ROS inhibition IC50 [μM] = 0; Neutrophil adhesion inhibition [%] = 34

18) **4-(4-(carboxymethyl)phenylaminocarbonyl)-2,5-dihydroxybenzoic acid**, compound **Ia-032a:**

$^1H$ NMR (250 MHz, DMSO-d6): δ 12.28 (br, 1H), 10.92 (s, 1H), 10.44 (s, 1H), 7.65 (br d, 2H), 7.41 (s, 1H), 7.38 (s, 1H), 7.25 (br d, 2H), 3.55 (s, 2H).
Biodata: **Ia-032a**: FGF-1 IC50 [μM] = N.D.; FGF-2 IC50 [μM] = 91; VEGF-A1 IC50 [μM] = N.D.; VEGFR-Phosphorylation inhibition IC50 [μM] =100; PMN ROS [inhibition at 0.3 μM [%] = N.D.; PMN ROS inhibition IC50 [μM]-; Neutrophil

adhesion inhibition [%] = N.D.

19) **4-(3-(Carboxymethyl)phenylaminocarbonyl)-2,5-dihydroxybenzoic acid**, compound **Ia-033a**:

UPLC-MS (acidic method, 6.5 min): rt = 1.71 min; *m/z* = 332.1 $[M+H]^+$, peak area >99%
$^1H$ NMR (400 MHz, DMSO-$d_6$) δ 10.89 (s, 1H), 10.46 (s, 1H), 7.65 (s, 1H), 7.59 (d, *J* = 8.1 Hz, 1H), 7.40 (s, 1H), 7.38 (s, 1H), 7.03 (d, *J* = 7.6 Hz, 1H), 3.57 (s, 2H).
HRMS: $[M+H]^+$, calc. for $C_{16}H_{14}NO_7$: 332.07647, found: 332.07644
Biodata: **Ia-033a**: FGF-1 IC50 [μM] = 60; FGF-2 IC50 [μM] = 165; VEGF-A1 IC50 [μM] = 281; VEGFR-Phosphorylation inhibition IC50 [μM] =100; PMN ROS [inhibition at 0.3 μM [%] = 57.73; PMN ROS inhibition IC50 [μM] = 1.32; Neutrophil adhesion inhibition [%] = 38

20) **4-(2-(Carboxymethyl)phenylaminocarbonyl)-2,5-dihydroxybenzoic acid**, compound **Ia-034a**:

UPLC-MS (acidic method, 2 min): rt = 0.82 min; *m/z* = 332.1 $[M+H]^+$, peak area >98%
$^1H$ NMR (400 MHz, DMSO-$d_6$) δ 7.78 (d, *J* = 8.1 Hz, 1H), 7.31 (d, *J* = 3.0 Hz, 2H), 7.28 (d, *J* = 7.6 Hz, 2H), 7.15 (t, *J* = 7.4 Hz, 1H), 3.63 (s, 2H).
HRMS: $[M+H]^+$, calc. for $C_{16}H_{14}NO_7$: 332.07648, found: 332.07641
Biodata: **Ia-034a**: FGF-1 IC50 [μM] = 79; FGF-2 IC50 [μM] = 14; VEGF-A1 IC50 [μM] = 102; VEGFR-Phosphorylation inhibition IC50 [μM] =100; PMN ROS [inhibition at 0.3 μM [%] = 49.07; PMN ROS inhibition IC50 [μM] = 2.28; Neutrophil adhesion inhibition [%] = 15

21) 4-(3,4-**Dihydroxyphenylmethylaminocarbonyl)-2,5-dihydroxybenzoic acid**, compound **Ia-035a**:

UPLC-MS (acidic method, 2 min): rt = 1.02 min; *m/z* = 320.1 $[M+H]^+$, peak area >91%
$^1H$ NMR (400 MHz, DMSO- $d_6$) δ 11.56 (s, 1H), 9.21 (t, *J* = 5.9 Hz, 1H), 8.91 (s, 1H), 8.79 (s, 1H), 7.46 (s, 1H), 7.28 (s, 1H), 6.72 (d, *J* = 2.1 Hz, 1H), 6.67 (d, *J* = 8.0 Hz, 1H), 6.57 (dd, *J* = 8.0, 2.1 Hz, 1H), 4.32 (d, *J* = 5.8 Hz, 2H).
HRMS: $[M+H]^+$, calc. for $C_{15}H_{14}NO_7$: 320.07648, found: 320.07650
Biodata: **Ia-035a**: FGF-1 IC50 [μM] = 20; FGF-2 IC50 [μM] = 71; VEGF-A1 IC50 [μM] = 200; VEGFR-Phosphorylation inhibition IC50 [μM] =36; PMN ROS [inhibition at 0.3 μM [%] = 75.13; PMN ROS inhibition IC50 [μM] = 0.41; Neutrophil adhesion inhibition [%] = 27

22) **4-(2-(3,4-Dihydroxyphenyl)ethylaminocarbonyl)-2,5-dihydroxybenzoic acid**, compound **Ia-035a-2**:

UPLC-MS (acidic method, 2 min): rt = 0.77 min; *m/z* = 332.0 $[M-H]^-$, peak area >99%
$^1H$ NMR (400 MHz, DMSO-$d_6$) δ 11.50 (s, 1H), 8.85 (t, J = 5.6 Hz, 1H), 8.76 (s, 1H), 8.66 (s, 1H), 7.41 (s, 1H), 7.28 (s, 1H), 6.67 - 6.60 (m, 2H), 6.48 (dd, J = 8.0, 2.1 Hz, 1H), 3.44 (q, J = 6.8 Hz, 2H), 2.67 (dd, J = 8.6, 6.0 Hz, 2H).δ 11.56 (s, 1H), 9.21 (t, J = 5.9 Hz, 1H), 8.91 (s, 1H), 8.79 (s, 1H), 7.46 (s, 1H), 7.28 (s, 1H), 6.72 (d, J = 2.1 Hz, 1H), 6.67 (d, J = 8.0 Hz, 1H), 6.57 (dd, J = 8.0, 2.1 Hz, 1H), 4.32 (d, J = 5.8 Hz, 2H).
HRMS: $[M+H]^+$, calc. for $C_{16}H_{16}NO_7$: 334.09213, found: 334.09242
Biodata: **Ia-035a-2**: FGF-1 IC50 [μM] = 10; FGF-2 IC50 [μM] = 65; VEGF-A1 IC50 [μM] = 109; VEGFR-Phosphorylation inhibition IC50 [μM] =10; PMN ROS [inhibition at 0.3 μM [%] = 58.81; PMN ROS inhibition IC50 [μM] = 1.1; Neutrophil adhesion inhibition [%] = 21.5

23) **4-(1-Carboxy-2-(3,4-dihydroxyphenyl)ethylaminocarbonyl)-2,5-dihydroxybenzoic acid**, compound **Ia-035a-3**:

UPLC-MS (acidic method, 2 min): rt = 0.70 min; *m/z* = 376.0 $[M-H]^-$, peak area >99%
$^1H$ NMR (400 MHz, DMSO-$d_6$) δ 12.90 (s, 1H), 11.11 (s, 1H), 8.98 (d, J = 7.4 Hz, 1H), 8.73 (d, J = 13.1 Hz, 2H), 7.45 (s, 1H), 7.31 (s, 1H), 6.61 (dd, J = 5.1, 2.9 Hz, 2H), 6.47 (dd, J = 8.1, 2.1 Hz, 1H), 3.01 (dd, J = 13.9, 4.9 Hz, 1H), 2.96 - 2.84 (m, 1H).
HRMS: $[M+H]^+$, calc. for $C_{17}H_{16}NO_9$: 378.08196, found: 378.08195
Biodata: **Ia-035a-3:** FGF-1 IC50 [μM] = 34; FGF-2 IC50 [μM] = 207; VEGF-A1 IC50 [μM] = 198; VEGFR-Phosphorylation inhibition IC50 [μM] =10; PMN ROS [inhibition at 0.3 μM [%] = 79.62; PMN ROS inhibition IC50 [μM] = 0.66; Neutrophil adhesion inhibition [%] = 22

24) **4-(Carboxy(3,4-dihydroxyphenyl)methylaminocarbonyl)-2,5-dihydroxybenzoic acid**, compound **Ia-036a**:

UPLC-MS (acidic method, 2 min): rt = 0.62 min; *m/z* = 362.1 $[M-H]^-$, peak area >92%

$^1H$ NMR (400 MHz, DMSO-$d_6$) δ 11.00 (s, 1H), 9.32 (d, *J* = 6.7 Hz, 1H), 9.05 (s, 1H), 8.97 (s, 1H), 7.40 (s, 1H), 7.34 (s, 1H), 6.81 (d, J = 2.1 Hz, 1H), 6.75 - 6.64 (m, 2H), 5.29 (d, J = 6.6 Hz, 1H).
HRMS: $[M+H]^+$, calc. for $C_{16}H_{14}NO_9$: 364.06630, found: 364.06615
Biodata: **Ia-036a**: FGF-1 IC50 [μM] = N.D.; FGF-2 IC50 [μM] = 37; VEGF-A1 IC50 [μM] = 123; VEGFR-Phosphorylation inhibition IC50 [μM] =1.6; PMN ROS [inhibition at 0.3 μM [%] = 87.17; PMN ROS inhibition IC50 [μM] = N.D.; Neutrophil adhesion inhibition [%] = 30.77

25) **4-(2-Carboxyphenylmethylaminocarbonyl)-2,5-dihydroxybenzoic acid**, compound **Ia-053a**:

UPLC-MS (acidic method, 4 min): rt = 1.08 min; *m/z* = 332.0 $[M+H]^+$, peak area >97%
$^1H$ NMR (400 MHz, DMSO-$d_6$) δ 13.09 (s, 1H), 11.19 (s, 1H), 9.34 - 9.27 (m, 1H), 7.91 (dd, J = 7.8, 1.4 Hz, 1H), 7.55 (dd, J = 7.5, 1.5 Hz, 1H), 7.46 (d, J = 7.9 Hz, 2H), 7.39 (td, J = 7.5, 1.4 Hz, 1H), 7.32 (s, 1H), 4.82 (d, J = 5.9 Hz, 2H).
HRMS: $[M+H]^+$, calc. for $C_{16}H_{14}NO_7$: 332.07648, found: 332.07622
Biodata: **Ia-053a**: FGF-1 IC50 [μM] = 150; FGF-2 IC50 [μM] = 124; VEGF-A1 IC50 [μM] = 210; VEGFR-Phosphorylation inhibition IC50 [μM] =100; PMN ROS [inhibition at 0.3 μM [%] = 52.92; PMN ROS inhibition IC50 [μM] = 2.5; Neutrophil adhesion inhibition [%] = 18.67

**Scheme 4b.** Synthesis of ethyl 4-amino-2,5-dibenzyloxybenzoate (aniline **A**)

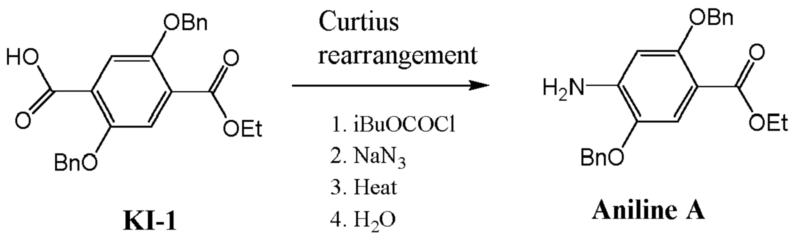


**KI-1** **Aniline A**

Protocole: synthesis of Aniline **A**

**Ethyl 4-amino-2,5-dibenzyloxybenzoate** (Aniline **A**). To a cooled solution (ice bath) of **2,5-bis(benzyloxy)-4-(ethoxycarbonyl)benzoic acid (KI-1**) (2.0 g, 4.9 mmol) and $Et_3N$ (1.6 mL, 11.3 mmol) in THF (25 mL) under inert atmosphere ($N_2$), was slowly added DPPA (1.1 mL, 5.2 mmol). The mixture was slowly warmed up to r.t and stirred at this temperature for 3 h. Then water (8 mL) was added and the reaction mixture was heated to 70 °C for 2 h. The reaction mixture was poured into a saturated solution of sodium hydrogencarbonate (250 mL) and stirred for 5 min before being extracted with EtOAc (3 x 100 mL). The combined organic layers were dried over sodium sulphate, filtered, and concentrated under reduced pressure to give a colourless oil. The residue was purified by flash column chromatography (iso-Hexane/EtOAc 1:0 then gradient to 30% EtOAc) to yield the title compound **Aniline A** (1.0 g, 53%) as an off-white solid.
UPLC-MS (acidic method, 2 min): rt = 1.26 min; *m/z* = 378.2 $[M+H]^+$, peak area 94%
$^1H$ NMR (400 MHz, DMSO-$d_6$) δ 7.54 - 7.46 (m, 4H), 7.44 - 7.36 (m, 4H), 7.35 - 7.25 (m, 3H), 6.46 (s, 1H), 5.65 (s, $NH_2$), 5.06 (s, 2H), 5.02 (s, 2H), 4.16 (q, J = 7.1 Hz, 2H), 1.22 (t, J = 7.1 Hz, 3H).

2 6) **4-(4-Carboxy-2,5-dihydroxyphenylaminocarbonyl)-2,5-dihydroxybenzoic acid**, compound **Ia-056a**:

UPLC-MS (acidic method, 4 min): rt = 0.90 min; *m/z* = 348.0 [M-H]-, peak area 97%
1H NMR (400 MHz, DMSO-d6) δ 11.28 (brs, 1H), 11.26 (s, 1H), 9.97 (s, 1H), 8.09 (s, 1H), 7.48 (s, 1H), 7.42 (s, 1H), 7.26 (s, 1H).

**Diethyl ester:**

UPLC-MS (acidic method, 4 min): rt = 1.86 min; *m/z* = 404.2 $[M-H]^-$, peak area 92%
$^1H$ NMR (400 MHz, DMSO-$d_6$) δ 11.48 (brs, 1H), 11.32 (brs, 1H), 10.28 (s, 1H), 10.07 (brs, 1H), 9.88 (s, 1H), 8.12 (s, 1H), 7.58 (s, 1H), 7.39 (s, 1H), 7.28 (s, 1H), 4.46 - 4.27 (m, 4H), 1.46 - 1.12 (m, 6H).

**Example 1.2 - Molecules of type Ib**: Diamides from diamines, 1 example

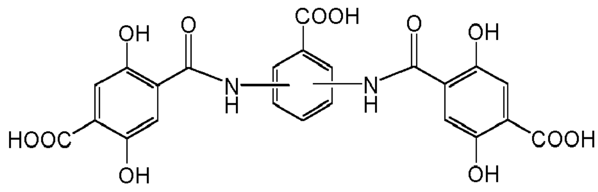

**Scheme 5.** Synthesis of Diamides of type **Ib**

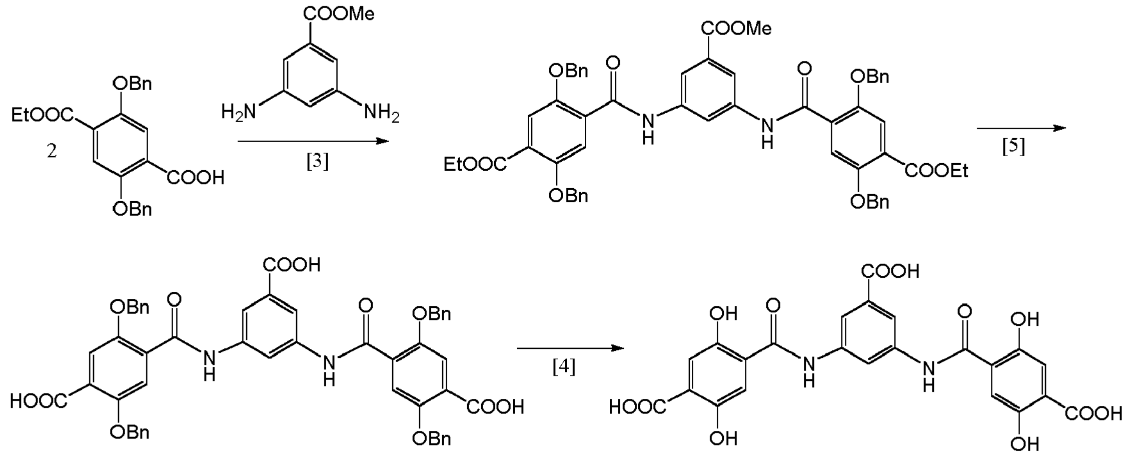


**Synthetic Procedures**: see general procedures, steps [3], [4], [5]

Example:

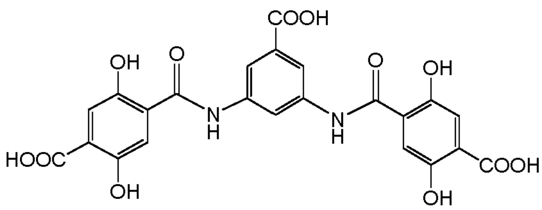

**Ib-010a**

For conditions and yields: See Table 1 (Figures 4A-D)

**27)3,5-Bis(2,5-dihydroxy-4-carboxybenzoylamino)benzoic acid**, compound **Ib-010a:**

UPLC-MS (acidic method, 4 min): rt = 0.98 min; *m/z* = 511.0 $[M-H]^-$, peak area >93%
$^1H$ NMR (400 MHz, DMSO-$d_6$) δ 10.81 (s, 2H), 10.65 (s, 2H), 8.38 (d, *J* = 2.0 Hz, 1H), 8.12 (d, *J* = 2.0 Hz, 2H), 7.39 (d, *J* = 2.3 Hz, 4H).
HRMS: $[M+H]^+$, calc. for $C_{23}H_{17}N_2O_{12}$: 513.07760, found: 513.07736,
Biodata: **Ib-010a**: FGF-1 IC50 [μM] = 14; FGF-2 IC50 [μM] = 3.8; VEGF-A1 IC50 [μM] = 15; VEGFR-Phosphorylation inhibition IC50 [μM] =1.6; PMN ROS [inhibition at 0.3 μM [%] = 71.84; PMN ROS inhibition IC50 [μM] = 1.06; Neutrophil adhesion inhibition [%] = 1.5; Whole Blood: GM-CSF IC50 [μM] = >100 IFNγ IC50 [μM] = 2.22; IL-1β IC50 [μM] = 33.3; IL-2 IC50 [μM] = 2.74; IL-4 IC50 [μM] = >100; IL-5 IC50 [μM] = >100; IL-6 IC50 [μM] = 1.02; IL-9 IC50 [μM] = 11.58; IL-10 IC50 [μM] = >100; IL-12p70 IC50 [μM] = 0.27; IL-13 IC50 [μM] = >100; IL-17A IC50 [μM] = 0.42; IL-17F IC50 [μM] = >100; IL-18 IC50 [μM] = >100; IL-21 IC50 [μM] = 67.8; IL-33 IC50 [μM] = 31.3; TGFβ IC50 [μM] = >100; TNF α IC50 [μM] = 0.31; TNF β IC50 [μM] = 49.3

**Triethyl ester:**

UPLC-MS (acidic method, 2 min): r.t = 1.29 mins, *m/z* = 597.1 $[M+H]^+$, peak area > 96%
$^1H$ NMR (400 MHz, DMSO-$d_6$) δ 10.80 (s, 2H), 10.68 (s, 2H), 9.95 (s, 2H), 8.40 (t, *J* = 2.0 Hz, 1H), 8.15 (d, *J* = 2.0 Hz, 2H), 7.42 (s, 2H), 7.39 (s, 2H), 4.42 - 4.33 (m, 6H), 1.38 - 1.33 (m, 9H). HRMS: $[M+H]^+$, calc. for $C_{29}H_{29}N_2O_{12}$: 597.17150, found: 597.17131
Biodata: **Ib-010a-E3**: FGF-1 IC50 [μM] = 26; FGF-2 IC50 [μM] = 226; VEGF-A1 IC50 [μM] = 200; VEGFR-Phosphorylation inhibition IC50 [μM] =0.09; PMN ROS [inhibition at 0.3 μM [%] = 36.41; PMN ROS inhibition IC50 [μM] = 6.52; Neutrophil adhesion inhibition [%] = 79.5; Whole Blood: GM-CSF IC50 [μM] = >100; IFNγ IC50 [μM] = 2.79; IL-1β IC50 [μM] = >100; IL-2 IC50 [μM] = 36.2; IL-4 IC50 [μM] = >100; IL-5 IC50 [μM] = >100; IL-6 IC50 [μM] = 0.24; IL-9 IC50 [μM] = 26.8; IL-10 IC50 [μM] = >100; IL-12p70 IC50 [μM] = 0.84; IL-13 IC50 [μM] = 33; IL-17A IC50 [μM] = 0.52; IL-17F IC50 [μM] = 34.2; IL-18 IC50 [μM] = >100; IL-21 IC50 [μM] = >100; IL-33 IC50 [μM] = 20; TGFβ IC50 [μM] = >100; TNF α IC50 [μM] = 0.98; TNF β IC50 [μM] = 38.2.

**Triethyl ester Tetraacetate:**

UPLC-MS (acidic method, 2 min): r.t = 1.19 mins, *m/z* = 782.1 $[M+NH_4]^+$, peak area 91%
$^1H$ NMR (400 MHz, DMSO-$d_6$) δ 10.84 (s, 2H), 8.40 (m, 1H), 8.08 (d, J = 2.0 Hz, 2H), 7.80 (s, 2H), 7.63 (s, 2H), 4.45 - 4.22 (m, 6H), 2.32 (s, 6H), 2.23 (s, 6H), 1.38 - 1.27 (m, 9H).
Biodata: **Ib-010a-E3-A4**: FGF-1 IC50 [μM] = 200; FGF-2 IC50 [μM] = 200; VEGF-A1 IC50 [μM] = 200; VEGFR-Phosphorylation inhibition IC50 [μM] =0.54; PMN ROS [inhibition at 0.3 μM [%] = 54.86; PMN ROS inhibition IC50 [μM] = 1.49; Neutrophil adhesion inhibition [%] = 96

**Example 1.3. - Molecules of type Ic**: Diamides from diacids, 4 examples

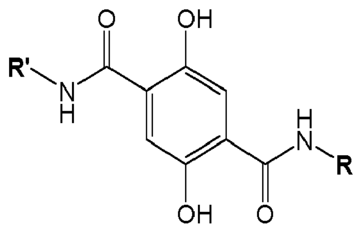

**Scheme 6.** Synthesis of Amides of type **Ic** with R = R'

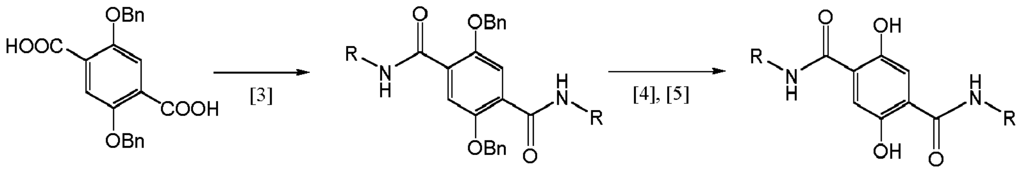


Procedures: see general procedures from **KI-6** [3], [4], [5]

Examples:

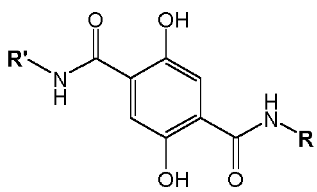

R, R' = 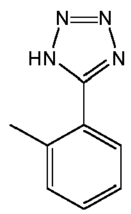 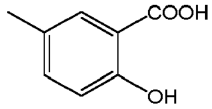 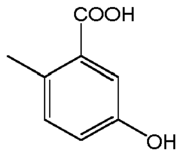

Ic-001aTz2 Ic-007a Ic-009a

For conditions and yields: See Table 1 (Figures 4A-D)

28) **$N_1$,$N_4$-Bis(2-(1H-tetrazol-5-yl)phenyl)-2,5-dihydroxyterephthalamide**, compound **Ic-001aTz2**:

UPLC-MS (acidic method, 2 min): rt = 0.97 min; *m/z* = 485.1 $[M+H]^+$, peak area >97%
$^1H$ NMR (400 MHz, DMSO-$d_6$) δ 11.53 (s, 1H), 10.98 (s, 1H), 8.49 (dd, J = 8.4, 1.2 Hz, 1H), 7.90 (dd, J = 7.8, 1.6 Hz, 1H), 7.62 (ddd, J = 8.7, 7.4, 1.6 Hz, 1H), 7.58 (s, 1H), 7.37 (td, J = 7.6, 1.2 Hz, 1H).
HRMS: $[M+H]^+$, calc. for $C_{22}H_{17}N_{10}O_4$: 485.14287, found: 485.17274 Biodata: **Ic-001aTz2**: FGF-1 IC50 [μM] = 200; FGF-2 IC50 [μM] = 70; VEGF-A1 IC50 [μM] = 45; VEGFR-Phosphorylation inhibition IC50 [μM] =2.2; PMN ROS [inhibition at 0.3 μM [%] = 60.64; PMN ROS inhibition IC50 [μM] = N.D.; Neutrophil adhesion inhibition [%] = 97.15; Whole Blood: GM-CSF IC50 [μM] = >100; IFNγ IC50 [μM] = 1.38; IL-1β IC50 [μM] = >100; IL-2 IC50 [μM] = 9.45; IL-4 IC50 [μM] = >100; IL-5 IC50 [μM] = >100; IL-6 IC50 [μM] = 0.52; IL-9 IC50 [μM] = 9.13; IL-10 IC50 [μM] = >100; IL-12p70 IC50 [μM] = 29.1; IL-13 IC50 [μM] = 0.12; IL-17A IC50 [μM] = 0.31; IL-17F IC50 [μM] = 0.15; IL-18 IC50 [μM] = 33.9; IL-21 IC50 [μM] = 32; IL-33 IC50 [μM] = 0.23; TGFβ IC50 [μM] = >100; TNF α IC50 [μM] = 0.1; TNF β IC50 [μM] = 0.41.

29) **5-(4-(3-Carboxy-4-hydroxyphenylaminocarbonyl)-2,5-dihydroxybenzamido)-2-hydroxybenzoic acid**, compound **Ic-007a:**

UPLC-MS (acidic method, 2 min): rt = 0.80 min; *m/z* = 469.1 $[M+H]^+$, peak area >91%
$^1H$ NMR (400 MHz, DMSO-$d_6$) δ 8.08 (s, 2H), 7.65 (s, 2H), 7.59 (s, 2H), 7.30 - 6.89 (m, 5H), 6.79 (d, *J* = 8.8 Hz, 2H).
HRMS: $[M+H]^+$, calc. for $C_{22}H_{17}N_2O_{10}$: 469.08777, found: 469.08739
Biodata: **Ic-007a**: FGF-1 IC50 [μM] = 13; FGF-2 IC50 [μM] = 5.4; VEGF-A1 IC50 [μM] = 3.2; VEGFR-Phosphorylation inhibition IC50 [μM] =0.09; PMN ROS [inhibition at 0.3 μM [%] = 57.99; PMN ROS inhibition IC50 [μM] = 2.7; Neutrophil adhesion inhibition [%] = 57.5; Whole Blood: GM-CSF IC50 [μM] = >100; IFNγ IC50 [μM] = 1.44; IL-1β IC50 [μM] = >100; IL-2 IC50 [μM] = >100; IL-4 IC50 [μM] = >100; IL-5 IC50 [μM] = >100; IL-6 IC50 [μM] = 0.09; IL-9 IC50 [μM] = 27.1; IL-10 IC50 [μM] = 12.5; IL-12p70 IC50 [μM] = 0.49; IL-13 IC50 [μM] = 30.1; IL-17A IC50 [μM] = 0.04; IL-17F IC50 [μM] = 22.9; IL-18 IC50 [μM] = >100; IL-21 IC50 [μM] = 1.9; IL-33 IC50 [μM] = 18.4; TGFβ IC50 [μM] = >100; TNF α IC50 [μM] = 0.27; TNF β IC50 [μM] = 6.52

**Dimethyl ester:**

UPLC-MS (acidic method, 2 min): r.t = 1.13 mins, *m/z* = 497.0 $[M+H]^+$, peak area > 95%
$^1H$ NMR (400 MHz, DMSO-d6) δ 11.08 (s, 2H), 10.48 (s, 2H), 10.38 (s, 2H), 8.27 (d, *J* = 2.8 Hz, 2H), 7.89 - 7.74 (m, 2H), 7.56 (s, 2H), 7.03 (d, *J* = 8.9 Hz, 2H), 3.93 (s, 6H).
HRMS: $[M+H]^+$, calc. for $C_{24}H_{21}N_2O_{10}$: 497.11907, found: 497.11874.
Biodata: **Ic-007a-E2**: FGF-1 IC50 [μM] = 23; FGF-2 IC50 [μM] = 49; VEGF-A1 IC50 [μM] = 200; VEGFR-Phosphorylation inhibition IC50 [μM] =0.25; PMN ROS [inhibition at 0.3 μM [%] = 21.33; PMN ROS inhibition IC50 [μM] = 7.9; Neutrophil adhesion inhibition [%] = 2

30) **2-(2,5-Dihydroxy-4-(4-hydroxy-2-carboxyphenylaminocarbonyl)benzamido)-5-hydroxybenzoic acid**, compound **Ic-009a:**

UPLC-MS (acidic method, 2 min): rt = 0.85 min; *m/z* = 469.0 $[M+H]^+$, peak area >98%
$^1H$ NMR (400 MHz, DMSO-$d_6$) δ 13.31 (s, 2H), 11.93 (s, 2H), 10.85 (s, 2H), 9.64 (s, 2H), 8.41 (d, *J* = 9.0 Hz, 2H), 7.55 (s, 2H), 7.39 (d, *J* = 3.0 Hz, 2H), 7.03 (dd, *J* = 9.0, 3.0 Hz, 2H).
HRMS: calc. for $C_{22}H_{17}N_2O_{10}$: 469.08777, found: 469.08755
Biodata: **Ic-009a**: FGF-1 IC50 [μM] = 32; FGF-2 IC50 [μM] = 202; VEGF-A1 IC50 [μM] = 208; VEGFR-Phosphorylation inhibition IC50 [μM] =ND; PMN ROS [inhibition at 0.3 μM [%] = 72.85; PMN ROS inhibition IC50 [μM] = N.D.; Neutrophil

adhesion inhibition [%] = 72.21

**Dimethyl ester:**

UPLC-MS (acidic method, 2 min): r.t = 1.03 mins, *m/z* = 497.1 $[M+H]^+$, peak area > 92%
$^1H$ NMR (400 MHz, DMSO-d6) δ 11.69 (s, 2H), 11.01 (s, 2H), 9.75 (s, 2H), 8.36 (d, *J* = 9.0 Hz, 2H), 7.62 (s, 2H), 7.37 (d, *J* = 3.0 Hz, 2H), 7.07 (dd, *J* = 9.0, 3.0 Hz, 2H), 3.87 (s, 6H).
HRMS: calc. for $C_{24}H_{21}N_2O_{10}$: 497.11907, found: 497.11913
Biodata: **Ic-009a-E2:** FGF-1 IC50 [μM] = 23; FGF-2 IC50 [μM] = 30; VEGF-A1 IC50 [μM] = 200; VEGFR-Phosphorylation inhibition IC50 [μM] =0.02; PMN ROS [inhibition at 0.3 μM [%] = 24.12; PMN ROS inhibition IC50 [μM] = N.D.; Neutrophil adhesion inhibition [%] = 41.05

**Scheme 7.** Synthesis of Amides of type **Ic** with R ≠ R'

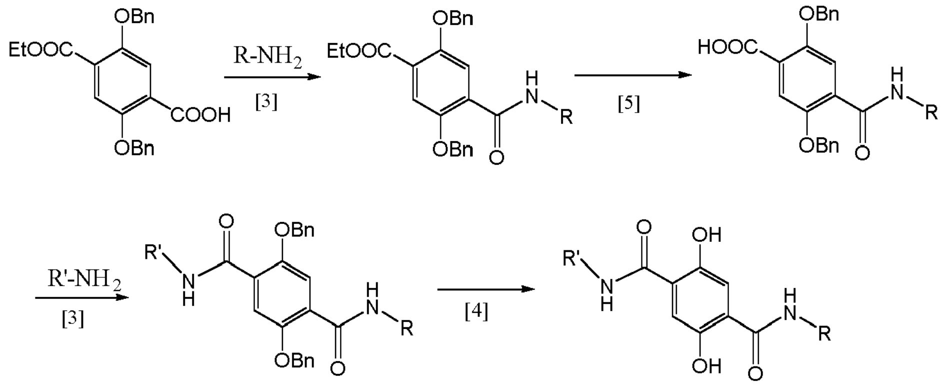


Synthetic Procedures: See general procedure from **KI-1**.

Example:

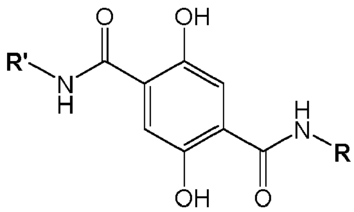

**Ic-001aTz/004a**

**R** =
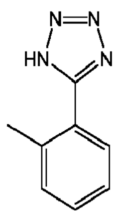
**R'** =
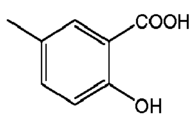

For conditions and yields: See Table 1 (Figures 4A-D)

31) **5-(4-(2-(1H-Tetrazol-5-yl)phenylaminocarbonyl)-2,5-dihydroxybenzamido)-2-hydroxybenzoic acid**, compound **Ic-001aTz/004a**:

UPLC-MS (acidic method, 4 min): rt = 1.40 min; *m/z* = 477.2 [M-H]$^-$, peak area >99%
$^1$H NMR (400 MHz, DMSO-$d_6$) δ 10.92 (s, 1H), 10.42 (s, 1H), 8.47 (dd, J = 8.4, 1.2 Hz, 1H), 8.25 (d, J = 2.7 Hz, 1H), 7.98 - 7.88 (m, 1H), 7.76 (dd, J = 8.9, 2.8 Hz, 1H), 7.63 - 7.56 (m, 2H), 7.51 (s, 1H), 7.37 (td, J = 7.6, 1.2 Hz, 1H), 6.97 (d, J = 8.9 Hz, 1H).
HRMS: [M+H]$^+$, calc. for $C_{22}H_{17}N_6O_7$: 477.11532, found: 477.11475
Biodata: **Ic-001a-Tz/004a**: FGF-1 IC50 [μM] = 19; FGF-2 IC50 [μM] = 87; VEGF-A1 IC50 [μM] = 25; VEGFR-Phosphorylation inhibition IC50 [μM] =2.6; PMN ROS [inhibition at 0.3 μM [%] = 82.83; PMN ROS inhibition IC50 [μM] = N.D.; Neutrophil adhesion inhibition [%] = 96.99; Whole Blood: GM-CSF IC50 [μM] = 3.4; IFNγ IC50 [μM] = 0.61; IL-1β IC50 [μM] = >100; IL-2 IC50 [μM] = 4.91; IL-4 IC50 [μM] = >100; IL-5 IC50 [μM] = >100; IL-6 IC50 [μM] = 3.04; IL-9 IC50 [μM] = 8.48; IL-10 IC50 [μM] = 19.5; IL-12p70 IC50 [μM] = 12.2; IL-13 IC50 [μM] = 1.5; IL-17A IC50 [μM] = 0.02; IL-17F IC50 [μM] = 2.4; IL-18 IC50 [μM] = 46.6; IL-21 IC50 [μM] = 0.3; IL-33 IC50 [μM] = 1.67; TGFβ IC50 [μM] = >100; TNF α IC50 [μM] = 0.002; TNF β IC50 [μM] = 3.02

**Methyl ester**:

UPLC-MS (acidic method, 2 min): r.t = 1.06 mins, *m/z* = 491.1 [M+H]$^+$, peak area > 89%
$^1$H NMR (400 MHz, DMSO-d6) δ 11.55 (s, 1H), 10.90 (s, 1H), 10.45 (s, 1H), 10.37 (s, 1H), 8.47 (dd, J = 8.4, 1.2 Hz, 1H), 8.30 (d, J = 2.7 Hz, 1H), 7.91 (dd, J = 7.8, 1.6 Hz, 1H), 7.76 (dd, J = 8.9, 2.7 Hz, 1H), 7.63 (ddd, J = 8.7, 7.4, 1.6 Hz, 1H), 7.58 (s, 1H), 7.51 (s, 1H), 7.38 (td, J = 7.6, 1.2 Hz, 1H), 7.02 (d, J = 8.8 Hz, 1H), 3.93 (s, 4H).
HRMS: [M+H]$^+$, calc. for $C_{23}H_{19}N_6O_7$: 491.13097, found: 491.13071
Biodata: **Ic-001a-Tz/004a-E1**: FGF-1 IC50 [μM] = 104; FGF-2 IC50 [μM] = 200; VEGF-A1 IC50 [μM] = 35; VEGFR-Phosphorylation inhibition IC50 [μM] =0.1; PMN ROS [inhibition at 0.3 μM [%] = 73.97; PMN ROS inhibition IC50 [μM] = N.D.; Neutrophil adhesion inhibition [%] = 97.56

**Example 1.4. Molecules** of **type Id**: Benzimidazole-linked, 1 example

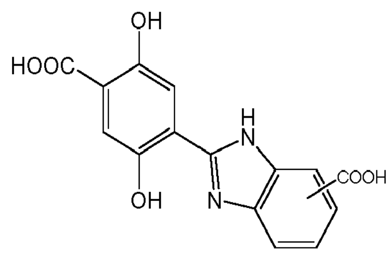

**Scheme 8.** Synthesis of compounds of type **Id**

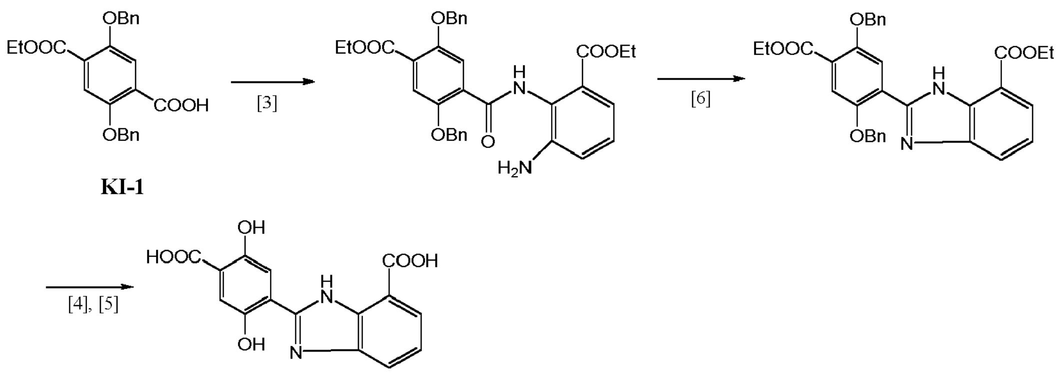

Synthetic procedure: see general procedure from **KI-1** [3], [4], [5]

Cyclization step: see Table 1 (Figures 4A-D)

Example:

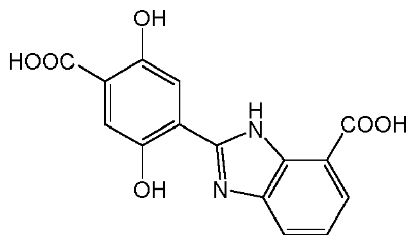

**1d-030a**

32) **2-(4-Carboxy-2,5-dihydroxyphenyl)-1H-benzo[d]imidazole-4-carboxylic acid**, compound **Id-030a**:

UPLC-MS (acidic method, 4 min): rt = 0.99 min; *m/z* = 313.1 $[M-H]^-$, peak area >97%
$^1H$ NMR (400 MHz, DMSO-$d_6$) δ 8.10 (d, J = 8.2 Hz, 1H), 8.05 (d, J = 7.7 Hz, 1H), 7.80 (s, 1H), 7.68 (t, J = 8.0 Hz, 1H), 7.62 (s, 1H)
Biodata - unstable in DMSO

**Ethyl Methyl ester**:

UPLC-MS (acidic method, 4 min): r.t = 2.09 mins, *m/z* = 357.1 $[M+H]^+$, peak area > 89%
$^1H$ NMR (400 MHz, DMSO-$d_6$+$D_2O$ 10%) δ 7.97 (d, *J* = 8.0 Hz, 1H), 7.90 - 7.83 (m, 2H), 7.44 - 7.36 (m, 2H), 4.35 (q, *J* = 7.1 Hz, 2H), 3.94 (s, 3H), 1.33 (t, *J* = 7.1 Hz, 3H).
HRMS: $[M+H]^+$, calc. for $C_{18}H_{17}N_2O_6$: 357.10811, found: 357.10827
Biodata: **Id-030a-E2:** FGF-1 IC50 [μM] = 115; FGF-2 IC50 [μM] = 200; VEGF-A1 IC50 [μM] = 200; VEGFR-Phosphorylation inhibition IC50 [μM] =0.33; PMN ROS [inhibition at 0.3 μM [%] = 0; PMN ROS inhibition IC50 [μM] = 0; Neutrophil adhesion inhibition [%] = 43

**Example 1.5: Molecules of type IIa: Monoamides,** 16 examples

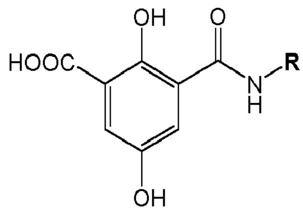

<u>**SYNTHESIS OF PRECURSORS**</u>

**Scheme 9a.** Synthesis of Intermediates **KI-2** and **KI-7**

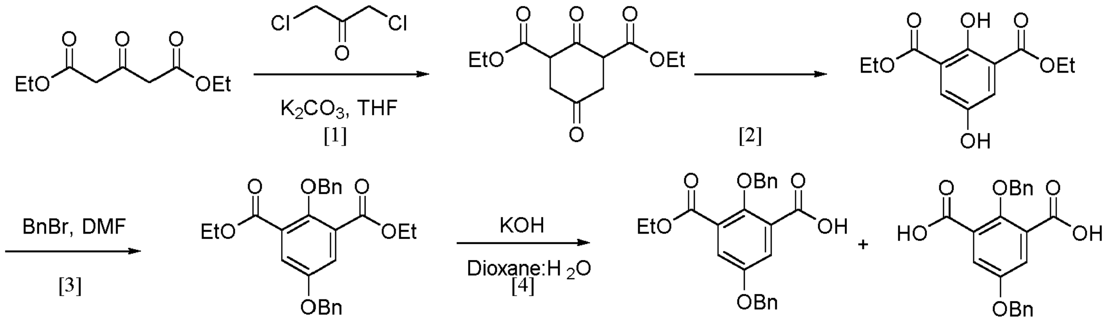


**KI-2** **KI-7**

*Procedures:*

Synthesis of **KI-2** an **KI-7**

**2,6-Di(ethoxycarbonyl)cyclohexane-1,4-dione** [Step 1][Ref: Rodriguez et al. Synth. Comm. 28 (1998) 2259-69]: 1,3-Dichloroacetone (12.5 g, 0.1 mol) in THF (500 mL) was added dropwise, over a period of 30 min, to a suspension of diethyl 1,3-acetonedicarboxylate (18 mL, 0.1 mol) and potassium carbonate-325 mesh (21.5 g, 0.15 mol) in THF (1 L) at reflux temperature. After 2 h, the reaction was complete and the mixture was cooled down to room temperature then filtered thought Celite©; the solid residue was washed with THF (200 mL), the solvent was then removed under reduced pressure. The crude product was purified by flash column chromatography (Hexanes/EtOAc 0 to 20%) to give the title compound (9.3 g, 37% yield).
UPLC-MS (acidic method, 2 min): rt = 1.01 min; *m/z* = 257.1 $[M+H]^-$, peak area >74%
$^1$H NMR (400 MHz, DMSO-$d_6$) Complex mixture of enol and cis/trans isomers $\delta$ 12.10 (s), 4.23 (q, *J* = 7.1 Hz), 4.11 (m), 3.85 - 3.77 (m), 3.70 (s), 3.10 - 2.89 (m), 2.89 - 2.80 (m), 2.62 - 2.58 (m), 1.25 (t, *J* = 7.1 Hz), 1.22 - 1.15 (m, 9H).

**Diethyl 2,5-dihydroxyisophthalate** [Step 2]: [Protocole taken from Zhong et al.: Chem Eur. J. 25 (2019) 8177-8169]: To a stirred solution of 2,6-di(ethoxycarbonyl)-cyclohexane-1,4-dione (5.0 g, 20 mmol) in AcOH (17 mL) at room temperature was added NBS (3.5 g, 20 mmol) in portions over 30 min. After 20 additional min, the reaction was quenched by the addition of water (100 ml). The desired product separated as a solid. After filtration and drying, the title compound was isolated as a white solid (4.6 g, 93% yield).
UPLC-MS (acidic method, 2 min): rt = 1.00 min; *m/z* = 253.1 $[M-H]^-$, peak area >90%
$^1$H NMR (400 MHz, DMSO-$d_6$) $\delta$ 10.85 (s, 1H), 9.57 (s, 1H), 7.39 (s, 2H), 4.32 (q, *J* = 7.1 Hz, 4H), 1.31 (t, *J* = 7.1 Hz, 6H).

**Diethyl 2,5-bis(benzyloxy)isophthalate** [Step 3]: To a suspension of diethyl 2,5-dihydroxyisophthalate (19.0 g, 75 mmol) and potassium carbonate-325 mesh (82.6 g, 598 mmol) in DMF (83 mL) was added dropwise benzyl bromide (43.0 mL, 362 mmol) over 10 min. The reaction mixture was heated at 100 °C during 2 h. After cooling down to room temperature the solvent was removed under reduced pressure. Water (500 mL) was then added to the residue. The mixture was extracted with EtOAc (3 x 250 mL), the gathered organic layers were washed with brine (300 mL), dried over $Na_2SO_4$, filtered then concentrated under reduced pressure. The crude material was purified by flash column chromatography (Hexanes/EtOAc 0 to 15%) to give the title compound (29.4 g, 90% yield).
UPLC-MS (acidic method, 2 min): rt = 1.38 min; *m/z* = 435.2 $[M+H]^+$, peak area >90%
$^1$H NMR (400 MHz, DMSO-d6) $\delta$ 7.50 (s, 2H), 7.49 - 7.45 (m, 2H), 7.44 - 7.37 (m, 5H), 7.37 - 7.29 (m, 3H), 5.17 (s, 2H), 4.95 (s, 2H), 4.26 (q, *J* = 7.1 Hz, 4H), 1.22 (t, J = 7.1 Hz, 6H).

**2,5-bis(benzyloxy)-3-(ethoxycarbonyl)benzoic acid (KI-2)** [Step 4]: A solution of potassium hydroxide (4.4 g, 79 mmol) in water (66 mL) was rapidly added to a solution of diethyl 2,5-bis(benzyloxy)isophthalate (31.3 g, 72 mmol) in 1,4-dioxane (430 mL). The reaction mixture was stirred at room temperature for 1.5 h. 1,4-Dioxane was removed under reduced pressure, an aqueous saturated solution of $Na_2CO_3$ (1 L) was then added, the aqueous layer was extracted with EtOAc (3x 500 mL), dried over $Na_2SO_4$, filtered and the solvent was removed in *vacuo.* The residue was purified by filtration over a Silica Pad using Hexanes/EtOAc (1/1) then EtOAc (1% v/v AcOH) as eluent to yield two different fractions:
The starting material: Diethyl 2,5-bis(benzyloxy)isophthalate (20.4 g, 65% yield).
UPLC-MS (acidic method, 2 min): rt = 1.38 min; *m/z* = 435.2 $[M+H]^+$, peak area >78%
$^1$H NMR (400 MHz, DMSO-d6) $\delta$ 7.50 (s, 2H), 7.49 - 7.45 (m, 2H), 7.44 - 7.37 (m, 5H), 7.37 - 7.29 (m, 3H), 5.17 (s, 2H), 4.95 (s, 2H), 4.26 (q, *J* = 7.1 Hz, 4H), 1.22 (t, *J* = 7.1 Hz, 6H).

The desired product, 2,5-bis(benzyloxy)-3-(ethoxycarbonyl)benzoic acid as a white fluffy solid, (**KI-2**) (3.5 g, 12% yield).

UPLC-MS (acidic method, 2 min): rt = 1.22 min; *m/z* = 405.1 $[M-H]^-$, peak area >90%
$^1H$ NMR (400 MHz, DMSO-$d_6$) δ 13.30 (s, 1H), 7.48 (t, *J* = 3.0 Hz, 2H), 7.46 - 7.42 (m, 5H), 7.39 (m, 3H), 7.37 - 7.31 (m, 2H), 5.17 (s, 2H), 4.96 (s, 2H), 4.25 (q, *J* = 7.1 Hz, 2H), 1.22 (t, *J* = 7.1 Hz, 3H).

The original saturated aqueous solution of $Na_2CO_3$ was then acidified to pH~7 using concentrated hydrochloric acid. Then extracted with EtOAc (2 x 500 mL), dried over sodium sulfate, filtered and concentrated under reduced pressure to give a mixture of **KI-2** and **KI-7** (3.0 g, 11% yield).
UPLC-MS (acidic method, 2 min): rt = 1.00 min; *m/z* = 377.1 $[M-H]^-$, peak area 25%, rt = 1.22 min; *m/z* = 405.1 $[M-H]^-$, peak area 56%
$^1H$ NMR (400 MHz, DMSO-$d_6$) δ 13.23 (s, 2H), 7.52 - 7.29 (m, 12H), 5.17 (s, 2H), 4.97 (s, 2H).

**Scheme 9b:** Alternative Synthesis of **KI-7**

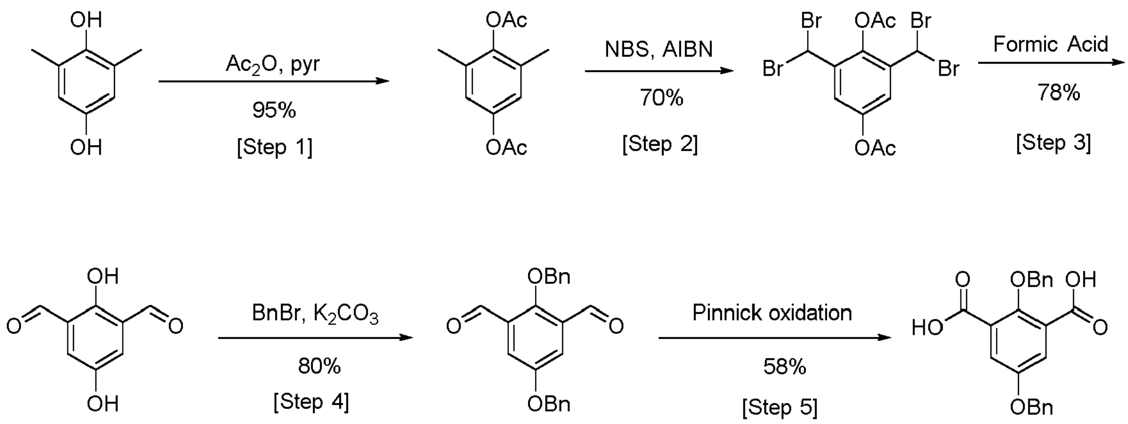


**KI-7**

**2,6-Dimethyl-1,4-phenylene diacetate [Step 1]**: To a stirred solution of 2,6-dimethylhydroquinone (5.0 g, 36 mmol) in pyridine (10 mL) was rapidly added acetic anhydride (10 mL). The solution was stirred at room temperature for 18 h. The solvent was then removed under reduced pressure and the residue was dissolved with EtOAc (250 mL) and subsequently washed with an aqueous solution of hydrochloric acid (1 M, 250 mL), a saturated aqueous solution of $NaHCO_3$ (250 mL) and water (250 mL), dried over $Na_2SO_4$, filtered and the solvent was removed in *vacuo,* to afford the title compound (7.4 g, 92%) as a white solid.
UPLC-MS (acidic method, 2 min): rt = 1.07 min; *m/z* = 240.2 $[M+NH_4]^+$, peak area >90%
$^1H$ NMR (400 MHz, DMSO-$d_6$) δ 6.88 ($H_{Ar}$, 2H), 2.37 - 2.30 (m, 3H), 2.27 - 2.21 (m, 3H), 2.12 - 2.02 (m, 6H).

**2,6-Bis(dibromomethyl)-1,4-phenylene diacetate [Step 2]**: To a stirred solution of **2,6-dimethyl-1,4-phenylene diacetate** (6.34 g, 29 mmol) in 1,2-Dichloroethane (130 mL) was sequentially added 2,2'-Azobis(2-methylpropionitrile) (AIBN) (0.93 g, 6 mmol) and *N*-Bromosuccinimide (NBS) (25.39 g, 143 mmol). The solution was stirred under reflux for 24 h. Additional 2,2'-Azobis(2-methylpropionitrile) (AIBN) (0.93 g, 6 mmol) and *N*-Bromosuccinimide (NBS) (5.08 g, 28.6 mmol) were added and the reaction mixture was stirred under reflux for another 24 h. The reaction was cooled down to room temperature and the residual solid was removed by filtration and washed with DCM (250 mL). The filtrate was washed with an aqueous saturated solution of sodium hydrogen carbonate (250 mL), an aqueous solution of hydrochloric acid (1 M, 250 mL) and brine (250 mL). The organic layer was then dried with sodium sulfate, filtered and concentrated under reduced pressure to afford the title compound (9.38 g, 61%) as an orange oil.
UPLC-MS (acidic method, 2 min): rt = 1.23 min; *m/z* = 553.1 $[M+NH_4]^+$, peak area >88%
$^1H$ NMR (400 MHz, DMSO-$d_6$) δ 7.69 (s, 2H), 7.31 (s, 2H), 2.49 (s, 3H), 2.32 (s, 3H).

**2,5-Dihydroxyisophthalaldehyde [Step 3]**: To a stirred suspension of **2,6-bis(dibromomethyl)-1,4-phenylene diacetate** (3.89 g, 7.23 mmol) in formic acid (50 mL) was added water (5 mL). The mixture was stirred under reflux for 18 h. The reaction mixture was then slowly poured into aqueous saturated sodium hydrogen carbonate (300 mL). The formed precipitate was then isolated by filtration to give the title compound (0.94 g, 78%) as a brown solid.
UPLC-MS (acidic method, 2 min): rt = 0.74 min; *m/z* = 165.0 $[M-H]^-$, peak area >98%

**2,5-Bis(benzyloxy)isophthalaldehyde [Step 4]**: Potassium carbonate-325 mesh (2.34 g, 17.0 mmol) was added to a stirred solution of **2,5-dihydroxyisophthalaldehyde** (0.94 g, 5.7 mmol) in DMF (6 mL) at ambient temperature. Benzyl bromide (2.0 mL, 17.0 mmol) was then added to the reaction flask, and the resulting mixture was heated at 100 °C for 18 h. The reaction mixture was cooled down to ambient temperature and treated with a solution of saturated aqueous ammonium chloride (100 mL). The resulting suspension was stirred for 30 min, then filtered. The solid material was washed with a solution of saturated aqueous ammonium chloride (2 × 50 mL). The solid was then triturated with ethanol (5 mL), dried by suction to give the desired product as a brown solid (1.58 g, 68%)

UPLC-MS (acidic method, 2 min): rt = 1.28 min, no ionization observed, peak area 78%
$^1H$ NMR (400 MHz, DMSO-$d_6$) δ 10.14 (s, 2H), 7.63 (s, 2H), 7.53 - 7.28 (m, 10H), 5.23 (s, 2H), 5.21 (s, 2H).

**2,5-Bis(benzyloxy)isophthalic acid (KI-7) [Step 5]: 2,5-bis(benzyloxy)isophthalaldehyde** (1.58 g, 4.5 mmol) was dissolved in a 2-methyl-2-butene solution in THF (2.0 M, 25 mL). A solution of sodium chlorite (5.1 g, 45.0 mmol) and potassium dihydrogen phosphate (4.6 g, 33.8 mmol) in water (25 mL) was then added and the reaction mixture was stirred at room temperature for 18 h. The reaction mixture was then poured into a saturated aqueous solution of $NaHCO_3$ (400 mL). The aqueous layer was washed with EtOAc (3 x 100 mL) followed by acidification with conc. hydrochloric acid (pH~1). The aqueous layer was then extracted with DCM (2 x 150 mL) and $Et_2O$ (2 x 200 mL), the gathered organic layer was dried with sodium sulfate, filtered and concentrated under reduced pressure.

The residue was then triturated with *n*-pentane (3 x 50 mL) to give the title compound **KI-7** as a white solid (0.98 g, 58%).
UPLC-MS (acidic method, 2 min): rt = 1.03 min, *m/z* = 379.1 $[M+H]^+$, peak area >96%
$^1H$ NMR (400 MHz, DMSO-$d_6$) δ 13.23 (s, 2H), 7.57 - 7.28 (m, 12H), 5.17 (s, 2H), 4.97 (s, 2H).

**Scheme 9c:** Synthesis of **KI-2Ac$_2$**

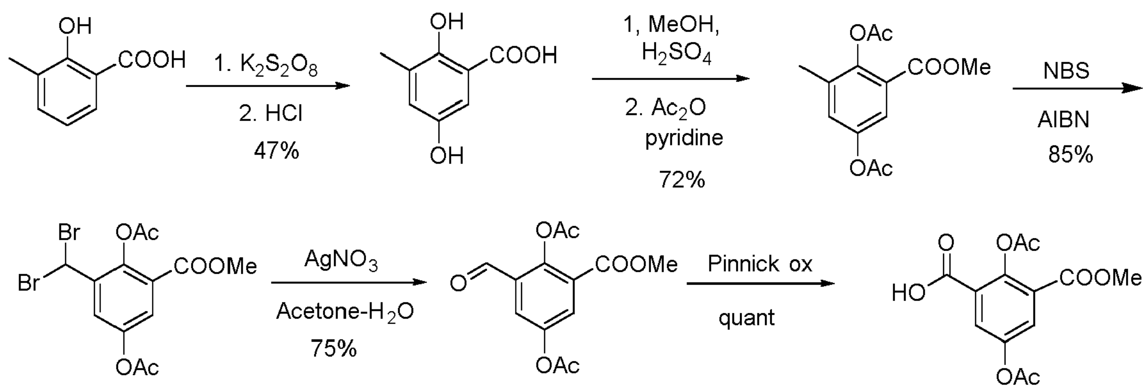


**KI-2Ac$_2$**

**Methyl 2,5-diacetoxy-3-methylbenzoate.** 3-Methylsalicylic acid was oxidized with persulfate as reported by Nudenberg et al (J. Org. Chem. 1943, 8, 500-508). 3-Methylsalicylic acid (15.0 g, 98.6 mmol) was dissolved in a solution of sodium hydroxide (15.0 g, 375 mmol, 3.8 eq.) in water (37.5 mL). The light brown solution was cooled to 20 °C and treated, while stirring, with 7.75 mL portions of 40% sodium hydroxide and 33.8 mL portions of 10% potassium persulfate solutions, beginning with the hydroxide, at such a rate that a temperature of 30-35 °C was maintained and until ten portions of each were added. After the addition was completed, stirring was continued for 1 h, the mixture was allowed to stand at room temperature for 16-20 h, and conc HCl was then added until blue to Congo. Unreacted 3-methylsalicylic acid separated at this point as a solid and was removed by filtration. The filtrate was extracted with ether several times (5x50 mL) to recover the remainder of 3-methylsalicylic acid. The aqueous solution was then treated with conc HCl (100 mL) and then refluxed for 2 h to decompose the intermediate monosulfate. The warm solution was allowed to cool to r.t. and the almost black crystalline solid which precipitated was filtered, washed with water, and dried. Extraction of the aqueous filtrate with ether gave an additional batch of **2,5-dihydroxy-3-methylbenzoic acid** as a brown solid (total: 7.71 g, 47%). Mp 212-214 °C;$^1H$ NMR (250 MHz, DMSO): δ 10.94 (br. s., 1H), 7.00 (dd, *J* = 3.0 and 0.75, $1H_{ar}$), 6.86 (dd, *J* = 3.0 and 0.75, $1H_{ar}$), 2.12 (s, 3H).

Esterification. Concentrated sulfuric acid (0.7 mL) was carefully added at 0 °C and under nitrogen to a solution of 2,5-dihydroxy-3-methylbenzoic acid (2.05 g, 12.2 mmol) in MeOH (7 mL). The reaction mixture was then stirred for 6 h at 100 °C. After cooling down to r.t., the solvent was removed under reduced pressure. The crude product obtained was dissolved in ethyl acetate (50 mL) and the solution washed with water (20 mL), 10% aq.$NaHCO_3$ (20 mL), 5% aq. HCl (20 mL), and brine (20 mL), and was then dried ($MgSO_4$). After filtering, the organic layer was concentrated in vacuo to give the residue which was purified by flash chromatography on silica gel (petroleum ether/ethyl acetate = 9/1) to afford **methyl 2,5-dihydroxy-3-methylbenzoate** as a white solid (370 mg, 75%). Mp 101-103 °C; $^1H$ NMR (250 MHz, $CDCl_3$): δ 10.56 (d, *J* = 0.50 Hz, 1H), 7.12 (dd, *J* = 3.25 and 0.50 Hz, $1H_{ar}$), 6.90 (dt, *J* = 3.00 and 0.50 Hz, $1H_{ar}$), 4.45 (s, 3H), , 2.24 (s, 3H); HRMS (ESI): m/z calcd for $C_9H_{11}O_4$ $[M+H]^+$: 183.0652; found 183.0651.

Acetylation. Excess acetic anhydride (10 mL) was added to a solution of methyl 2,5-dihydroxy-3-methyl-benzoate (4.43 g, 24.3 mmol) in pyridine (10 mL) under argon. After 12 h of stirring at r.t., pyridine and acetic anhydride were eliminated by co-evaporation with toluene (25 mL) under reduced pressure. The crude product was then dissolved in ethyl acetate (15 mL) and the solution washed successively with a 2% aq HCl (5 mL), saturated aq $NaHCO_3$ (5 mL)

and brine (5 mL). The organic phase was dried ($MgSO_4$) and the solvent was evaporated under reduced pressure. The colorless oil obtained was purified by flash chromatography on silica gel (petroleum ether/ethyl acetate = 8/2) to afford the acetylated title product (2,5-acetoxy-3-methyl-benzoate) as a white solid (6.25 g, 97%). Mp 69-71 °C; $^1$H NMR (250 MHz, $CDCl_3$): δ 7.58 (dd, *J* = 3.00 and 0.50 Hz, $1H_{ar}$), 7.18 (dd, *J* = 3.00 and 0.75 Hz, $1H_{ar}$), 3.85 (s, 3H), , 2.37 (s, 3H), 2.30 (s, 3H), 2.22 (s, 3H); HRMS (ESI$^+$): m/z calcd for $C_{13}H_{18}NO_6$ $[M+NH_4]^+$: 284.1129; found 284.1128.

**Methyl 2,5-diacetoxy-3-dibromomethylbenzoate**. A solution of methyl 2,5-acetoxy-3-methylbenzoate (2.15 g, 8.08 mmol), *N*-bromosuccinimide (2.87 mg, 16.2 mmol, 2.0 eq.) and azobisisobutyronitrile (AIBN, 27.0 mg, 0.162 mmol, 0.02 eq.) in carbon tetrachloride (45 mL) was refluxed for about 12 h until a white solid was floating on the surface. After cooling down, the mixture was filtered and the filtrate was then concentrated under reduced pressure. The colorless oil obtained was purified by flash chromatography on silica gel (petroleum ether/ethyl acetate = 9/1) to afford the dibrominated product as a white solid (3.29 g, 84%). Mp 117-119 °C; $^1$H NMR (250 MHz, $CDCl_3$): δ 7.86 (d, *J* = 2.75 Hz, $1H_{ar}$), 7.78 (d, *J* = 2.75 Hz, $1H_{ar}$), 6.81 (s, 1H), 3.86 (s, 3H), 2.42 (s, 3H), 2.33 (s, 3H); HRMS (ESI$^+$): m/z calcd for $C_{13}H_{12}Br_2NaO_6$ $[M+Na]^+$: 444.8893; found 444.8896.

**Methyl 2,5-diacetoxy-3-formylbenzoate.** A solution of methyl 2,5-diacetoxy-3-dibromo-methylbenzoate (2.30 g, 5.42 mmol) and silver nitrate (2.29 g, 13.5 mmol, 2.5 eq.) in a mixture acetone - $H_2O$ (4.7 : 1, 40 mL) was stirred at r.t. and in the dark during 12 h. The mixture was then filtered and the filtrate was extracted with ethyl acetate (2 x 20 mL). The combined organic phases were washed with brine (5 mL), dried ($MgSO_4$) and concentrated under reduced pressure. The colorless oil thus obtained was purified by flash chromatography on silica gel (petroleum ether/ethyl acetate = 9/1) to afford the aldehyde as a yellow solid (1.14 g, 75%). Mp 102-104 °C; 1H NMR (250 MHz, $CDCl_3$): □ 10.17 (s, 1H), 8.00 (d, J = 3.00 Hz, 1Har), 7.82 (d, J = 3.00 Hz, 1Har), 3.90 (s, 3H), 2.44 (s, 3H), 2.34 (s, 3H).

**2,5-Diacetoxyisophthalic acid monomethyl ester (KI-2Ac$_2$).** A solution of sodium hydrogen phosphate (1.35 g, 9.81 mmol, 2.5 eq.) in water (3.5 mL) was added dropwise to a solution of methyl 2,5-diacetoxy-3-formylbenzoate (1.10 g, 3.93 mmol) in DMSO (14 mL). The mixture was then cooled to 0 °C and a solution of sodium chlorite (1.06 g, 9.34 mmol, 2.4 eq.) in water (3.5 mL) was slowly added. After 72 h of stirring at r.t., the mixture was quenched with aqueous saturated $NaHCO_3$ (10 mL) and extracted with ethyl acetate (2 x 30 mL). The aqueous phase was then acidified with 1M HCl to pH = 1 and extracted ethyl acetate (2 x 30 mL). The combined organic phases were washed with brine (5 mL), dried ($MgSO_4$) and concentrated under reduced pressure. The orange oil obtained was used in the next step without any purification.

## SYNTHESIS OF MONOAMIDES

**Scheme 10.** Synthesis of Monoamides **IIa**

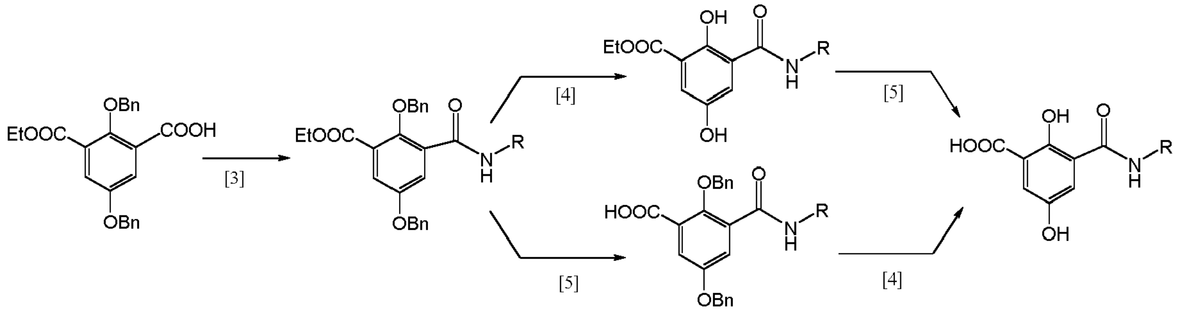


*Protocoles: Synthesis of Monoamides*

Amide Coupling and Deprotection Procedures

**See General Procedures from KI-1** and **KI-6** steps [3], [4], [5], same procedures from **KI-2, KI-2Ac$_2$** and **KI-7**

**Examples**

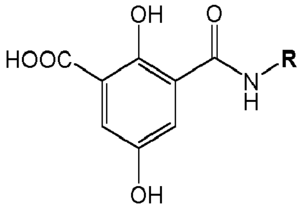

R =

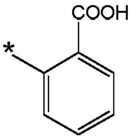

IIa-001a

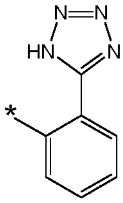

IIa-001aTz

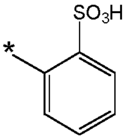

IIa-001c

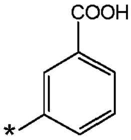

IIa-002a

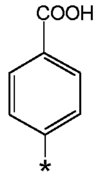

IIa-003a

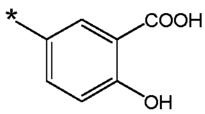

IIa-004a

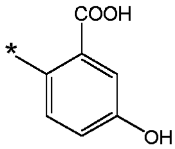

IIa-006a

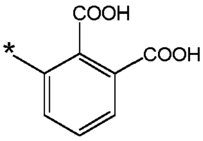

IIa-011a

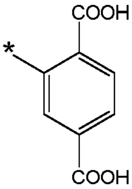

IIa-012a

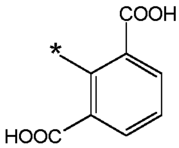

IIa-013a

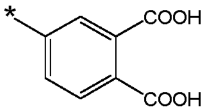

IIa-014a

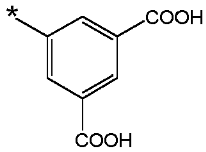

IIa-015a

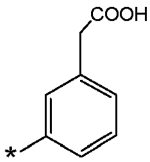

IIa-033a

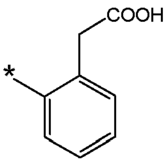

IIa-034a

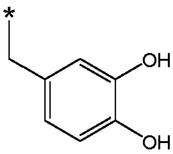

IIa-035a

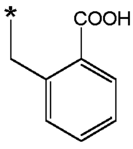

IIa-053a

For conditions and yields: See Table 2 (Figures 5A-C)

33) **3-(2-Carboxyphenylaminocarbonyl)-2,5-dihydroxybenzoic acid**, compound **IIa-001a**:

UPLC-MS (acidic method, 2 min): rt = 0.86 min; *m/z* = 318.0 $[M+H]^+$, peak area >92%
$^1H$ NMR (400 MHz, DMSO-d6) δ 13.49 (s, 1H), 12.16 (s, 1H), 9.54 (s, 1H), 8.70 (dd, *J* = 8.5, 1.2 Hz, 1H), 7.99 (dd, *J* = 7.9, 1.7 Hz, 1H), 7.70 - 7.51 (m, 2H), 7.42 (d, *J* = 3.3 Hz, 1H), 7.20 (td, *J* = 7.6, 1.2 Hz, 1H).
HRMS: $[M+H]^+$, calc. for $C_{15}H_{12}NO_7$: 318.06083, found: 318.06082
Biodata: **IIa-001a**: FGF-1 IC50 [μM] = 8.6; FGF-2 IC50 [μM] = 11; VEGF-A1 IC50 [μM] = 150; VEGFR-Phosphorylation inhibition IC50 [μM] =100; PMN ROS [inhibition at 0.3 μM [%] = 30.5; PMN ROS inhibition IC50 [μM] = 0.408; Neutrophil adhesion inhibition [%] = 36.24

34) **3-(2-(1H-Tetrazol-5-yl)phenylaminocarbonyl)-2,5-dihydroxybenzoic acid**, compound **IIa-001aTz:**

UPLC-MS (acidic method, 2 min): rt = 0.71 min; *m/z* = 342.1 $[M+H]^+$, peak area >99%
$^1H$ NMR (400 MHz, DMSO-$d_6$) δ 11.40 (s, 1H), 9.49 (s, 1H), 8.51 (d, J = 8.3 Hz, 1H), 7.87 (d, J = 7.7 Hz, 1H), 7.72 - 7.51 (m, 2H), 7.51 - 7.26 (m, 2H)
HRMS: $[M+H]^+$, calc. for $C_{15}H_{12}N_5O_5$: 342.08329, found: 342.08317
Biodata: **IIa-001a-Tz:** FGF-1 IC50 [μM] = N.D.; FGF-2 IC50 [μM] = 9.8; VEGF-A1 IC50 [μM] = 187; VEGFR-Phosphorylation inhibition IC50 [μM] =ND; PMN ROS [inhibition at 0.3 μM [%] = 74.15; PMN ROS inhibition IC50 [μM] = N.D.; Neutrophil adhesion inhibition [%] = 24.18

**Ethyl ester**

UPLC-MS (acidic method, 4 min): rt = 1.46 min; *m/z* = 370.1 $[M+H]^+$, peak area >92%
$^1H$ NMR (400 MHz, DMSO-$d_6$) δ 11.51 (s, 1H), 11.37 (s, 1H), 9.63 (s, 1H), 8.47 (d, *J*= 8.3 Hz, 1H), 7.92 (dd, *J* = 7.8, 1.6 Hz, 1H), 7.66 - 7.57 (m, 2H), 7.43 (d, *J* = 3.2 Hz, 1H), 7.38 (td, *J* = 7.6, 1.2 Hz, 1H), 4.40 (q, *J* = 7.1 Hz, 2H), 1.36 (t, *J* = 7.1 Hz, 3H),
HRMS: $[M+H]^+$, calc. for $C_{17}H_{16}N_5O_5$: 370.11460, found: 370.11444
Biodata: **IIa-001aTz-E1:** FGF-1 IC50 [μM] = 200; FGF-2 IC50 [μM] = 42; VEGF-A1 IC50 [μM] = 200; VEGFR-Phosphorylation inhibition IC50 [μM] =100; PMN ROS [inhibition at 0.3 μM [%] = 68.86; PMN ROS inhibition IC50 [μM] = N.D.; Neutrophil adhesion inhibition [%] = 81.94

35) **2,5-Dihydroxy-3-(2-sulfophenylaminocarbonyl)benzoic acid**, compound **IIa-001c**:

UPLC-MS (acidic method, 2 min): rt = 0.63 min; *m/z* = 354.0 $[M+H]^+$, peak area >80%
$^1H$ NMR (400 MHz, DMSO-$d_6$) δ 11.18 (s, 1H), 9.48 (s, 1H), 8.34 - 8.27 (m, 1H), 7.72 (dd, J = 7.7, 1.7 Hz, 1H), 7.52 (d, J = 3.2 Hz, 1H), 7.41 - 7.31 (m, 2H), 7.08 (td, J = 7.5, 1.2 Hz, 1H).
HRMS: $[M+H]^+$, calc. for $C_{14}H_{12}NO_8S$: 354.02781, found: 354.02781
Biodata: **IIa-001c**: FGF-1 IC50 [μM] = N.D.; FGF-2 IC50 [μM] = 71; VEGF-A1 IC50 [μM] = 283; VEGFR-Phosphorylation inhibition IC50 [μM] =ND; PMN ROS [inhibition at 0.3 μM [%] = 72.62; PMN ROS inhibition IC50 [μM] = N.D.; Neutrophil adhesion inhibition [%] = 43.53

36) **3-(3-Carboxyphenylaminocarbonyl)-2,5-dihydroxybenzoic acid**, compound **IIa-002a**:

$^1H$ NMR (250 MHz, $CD_3OD$): δ 8.37 (t, 1H), 7.93 (br d, 1H), 7.81 (br d, 1), 7.76 (d, 1H), 7.52 (d, 1H), 7.48 (t, 1H).
HRMS (ESI-): $[M-H]^-$, calc. for $C_{15}H_{10}NO_7$: 316.0461, found: 318.0463
Biodata: **IIa-002a**: FGF-1 IC50 [μM] = 19; FGF-2 IC50 [μM] = 131; VEGF-A1 IC50 [μM] = 150; VEGFR-Phosphorylation inhibition IC50 [μM] =ND; PMN ROS [inhibition at 0.3 μM [%] = 43.3; PMN ROS inhibition IC50 [μM] = 0.299; Neutrophil adhesion inhibition [%] = 17.39

37) **3-(4-Carboxyphenylaminocarbonyl)-2,5-dihydroxybenzoic acid)amide**, compound **IIa-003a**:

$^1H$ NMR (250 MHz, $CD_3OD$): □ 8.06 (BB' of AA'BB', 2H), 7.85 (AA' of AA'BB', 2H), 7.75 (d, 1H), 7.58 (d, 1H)
HRMS (ESI-): calcd for $C_{15}H_{10}NO_7$ $[M-H]^-$: 316.0461; found 316.0462
Biodata: **IIa-003a**: FGF-1 IC50 [μM] = 22; FGF-2 IC50 [μM] = 13; VEGF-A1 IC50 [μM] = 150; VEGFR-Phosphorylation inhibition IC50 [μM] =2.5; PMN ROS [inhibition at 0.3 μM [%] = 59.3; PMN ROS inhibition IC50 [μM] = N.D.; Neutrophil adhesion inhibition [%] = 35.27

38) **3-(3-Carboxy-4-hydroxyphenylaminocarbonyl)-2,5-dihydroxybenzoic acid**, compound **IIa-004a**:

UPLC-MS (acidic method, 2 min): rt = 0.76 min; *m/z* = 334.0 $[M+H]^+$, peak area >99%
$^1H$ NMR (400 MHz, DMSO-$d_6$) δ 14.02 (brs, 1H), 11.07 (brs, 1H), 10.32 (s, 1H), 9.47 (brs, 1H), 8.27 (d, *J* = 2.7 Hz, 1H), 7.76 (dd, *J* = 8.9, 2.7 Hz, 1H), 7.42 (d, *J* = 3.2 Hz, 1H), 7.36 (d, *J* = 3.2 Hz, 1H), 6.96 (d, *J* = 8.9 Hz, 1H).
HRMS: $[M+H]^+$, calc. for $C_{15}H_{12}NO_8$: 334.05574, found: 334.05571
Biodata: **IIa-004a**: FGF-1 IC50 [μM] = 47; FGF-2 IC50 [μM] = 33; VEGF-A1 IC50 [μM] = 200; VEGFR-Phosphorylation inhibition IC50 [μM] =100; PMN ROS [inhibition at 0.3 μM [%] = 63.5; PMN ROS inhibition IC50 [μM] = 1.002; Neutrophil adhesion inhibition [%] = 36

39) **3-(2-Carboxy-4-hydroxyphenylaminocarbonyl)-2,5-dihydroxybenzoic acid**, compound **IIa-006a**:

UPLC-MS (acidic method, 2 min): rt = 0.65 min; *m/z* = 334.0 $[M+H]^+$, peak area >99%
$^1H$ NMR (400 MHz, DMSO-$d_6$) δ 13.36 (s, 1H), 11.83 (s, 1H), 9.62 (s, 1H), 9.47 (s, 1H), 8.47 (d, *J* = 9.1 Hz, 1H), 7.64 (d, *J* = 3.3 Hz, 1H), 7.41 (d, *J* = 3.2 Hz, 1H), 7.37 (d, *J* = 30 Hz, 1H), 7.03 (dd, *J* = 9.1, 3.0 Hz, 1H).
HRMS: $[M+H]^+$, calc. for $C_{15}H_{12}NO_8$: 334.05574, found: 334.05548
Biodata: **IIa-006a**: FGF-1 IC50 [μM] = N.D.; FGF-2 IC50 [μM] = 43; VEGF-A1 IC50 [μM] = 121; VEGFR-Phosphorylation inhibition IC50 [μM] =2.9; PMN ROS [inhibition at 0.3 μM [%] = 84.29; PMN ROS inhibition IC50 [μM] = N.D.; Neutrophil adhesion inhibition [%] = 20.29

40) **3-(3-Carboxy-2,5-dihydroxybenzamido)phthalic acid,** compound **IIa-011a**:

UPLC-MS (acidic method, 2 min): rt = 0.72 min; *m/z* = 360.1 $[M-H]^-$, peak area >77%
$^1H$ NMR (400 MHz, DMSO-$d_6$) δ 10.83 (s, 1H), 9.55 (s, 1H), 8.43 (dd, *J* = 7.9, 1.5 Hz, 1H), 7.73 (d, *J* = 3.3 Hz, 1H), 7.63 - 7.53 (m, 2H), 7.46 (d, *J* = 3.3 Hz, 1H),
HRMS: $[M+H]^+$, calc. for $C_{16}H_{12}NO_9$: 362.05066, found: 362.05045,
Biodata: **IIa-011a**: FGF-1 IC50 [μM] = 141; FGF-2 IC50 [μM] = 123; VEGF-A1 IC50 [μM] = 200; VEGFR-Phosphorylation inhibition IC50 [μM] =100; PMN ROS [inhibition at 0.3 μM [%] = 58.1; PMN ROS inhibition IC50 [μM] = 1.18; Neutrophil adhesion inhibition [%] = 26

41) **2-(3-Carboxy-2,5-dihydroxybenzamido)terephthalic acid**, compound **IIa-012a**:

UPLC-MS (acidic method, 2 min): rt = 0.73 min; *m/z* = 362.0 [M+H], peak area >97%
$^1H$ NMR (DMSO-$d_6$) δ: 13.81 (s, 1H), 13.34 (s, 1H), 12.23 (s, 1H), 9.50 (s, 1H), 9.30 (d, *J* = 1.6 Hz, 1H), 8.06 (d, *J* = 8.2 Hz, 1H), 7.71 (dd, *J* = 8.2, 1.7 Hz, 1H), 7.64 (d, *J* = 3.3 Hz, 1H), 7.43 (d, J = 3.3 Hz, 1H),
HRMS: $[M+H]^+$, calc. for $C_{16}H_{12}NO_9$: 362.05066, found: 362.05046,
Biodata: **IIa-012a:** FGF-1 IC50 [μM] = 150; FGF-2 IC50 [μM] = 150; VEGF-A1 IC50 [μM] = 32; VEGFR-Phosphorylation inhibition IC50 [μM] =3.31; PMN ROS [inhibition at 0.3 μM [%] = 44.8; PMN ROS inhibition IC50 [μM] = 0.313; Neutrophil adhesion inhibition [%] = 7.5

42)**2-(3-Carboxy-2,5-dihydroxybenzamido)isophthalic acid**, compound **IIa-013a:**

UPLC-MS (acidic method, 2 min): rt = 1.68 min; *m/z* = 362.0 $[M+H]^+$, peak area >96%
$^1H$ NMR (400 MHz, DMSO-$d_6$) δ 13.12 (brs, 3H), 11.71 (s, 1H), 9.47 (s, 1H), 7.96 (s, 1H), 7.94 (s, 1H), 7.66 (d, *J* = 3.3 Hz, 1H), 7.44 (d, *J* = 3.3 Hz, 1H), 7.35 (t, *J* = 7.8 Hz, 1H).
HRMS: $[M+H]^+$, calc. for $C_{16}H_{12}NO_9$: 362.05066, found: 362.05047
Biodata: **IIa-013a:** FGF-1 IC50 [μM] = 137; FGF-2 IC50 [μM] = 12; VEGF-A1 IC50 [μM] = 34; VEGFR-Phosphorylation inhibition IC50 [μM] =100; PMN ROS [inhibition at 0.3 μM [%] = 57.68; PMN ROS inhibition IC50 [μM] = 2.54; Neutrophil adhesion inhibition [%] = 15.75

43)**4-(3-Carboxy-2,5-dihydroxybenzamido)phthalic acid**, compound **IIa-014a:**

UPLC-MS (acidic method, 2 min): rt = 0.63 min; *m/z* = 362.1 $[M+H]^+$, peak area >88%
$^1H$ NMR (400 MHz, DMSO-d6) δ 10.80 (s, 1H), 9.45 (s, 1H), 8.01 (d, *J* = 2.2 Hz, 1H), 7.86 (dd, *J* = 8.5, 2.2 Hz, 1H), 7.74 (d, *J* = 8.5 Hz, 1H), 7.45 - 7.33 (m, 2H),
HRMS: $[M+H]^+$, calc. for $C_{16}H_{12}NO_9$: 362.05066, found: 362.05048
Biodata: **IIa-014a:** FGF-1 IC50 [μM] = 40; FGF-2 IC50 [μM] = 61; VEGF-A1 IC50 [μM] = 91; VEGFR-Phosphorylation inhibition IC50 [μM] =17.6; PMN ROS [inhibition at 0.3 μM [%] = 73.94; PMN ROS inhibition IC50 [μM] = N.D.; Neutrophil adhesion inhibition [%] = 21.5

44) **5-(3-Carboxy-2,5-dihydroxybenzamido)isophthalic acid**, compound **IIa-015a:**

$^1H$ NMR (250 MHz, $CD_3OD$): δ 8.60 (d, *J* = 1.5 Hz, 2H), 8.44 (t, *J* = 1.5 Hz, 1H), 7.75 (d, *J* = 3.2 Hz, 1H), 7.56 (d, *J* = 3.2 Hz, 1)
HRMS (ESI+): *m/z* calcd for C16H12NO9 [M+H]+: 362.0507; found 362.0505 [LM-163]
Biodata: **IIa-015a:** FGF-1 IC50 [μM] = N.D.; FGF-2 IC50 [μM] = 125; VEGF-A1 IC50 [μM] = N.D.; VEGFR-Phosphorylation inhibition IC50 [μM] =ND; PMN ROS [inhibition at 0.3 μM [%] = N.D.; PMN ROS inhibition IC50 [μM] = N.D.; Neutrophil adhesion inhibition [%] = N.D.

45) **(3-(3-(Carboxymethyl)phenylaminocarbonyl)-2,5-dihydroxybenzoic acid**, compound **IIa-033a:**

UPLC-MS (acidic method, 2 min): rt = 0.78 min; *m/z* = 330.1 $[M-H]^-$, peak area >98%
$^1H$ NMR (400 MHz, DMSO-$d_6$) δ 10.40 (s, 1H), 9.45 (s, 1H), 7.66 (t, J = 1.9 Hz, 1H), 7.63 - 7.57 (m, 1H), 7.45 (d, J = 3.3 Hz, 1H), 7.38 (d, J = 3.2 Hz, 1H), 7.29 (t, J = 7.8 Hz, 1H), 7.05 - 6.95 (m, 1H), 3.56 (s, 2H).
HRMS: $[M+H]^+$, calc. for $C_{16}H_{l4}NO_7$: 332.07648, found: 332.07644,
Biodata: **IIa-033a:** FGF-1 IC50 [μM] = 35; FGF-2 IC50 [μM] = 32; VEGF-A1 IC50 [μM] = 200; VEGFR-Phosphorylation inhibition IC50 [μM] =4.9; PMN ROS [inhibition at 0.3 μM [%] = 50.64; PMN ROS inhibition IC50 [μM] = 0.914; Neutrophil adhesion inhibition [%] = 11.75

46)**3-(2-(Carboxymethyl)phenylaminocarbonyl)-2,5-dihydroxybenzoic acid**, compound **IIa-034a:**

UPLC-MS (acidic method, 2 min): rt = 0.74 min; *m/z* = 332.0 $[M+H]^+$, peak area >95%
$^1H$ NMR (400 MHz, DMSO-$d_6$) δ 12.48 (s, 1H), 10.65 (s, 1H), 9.30 (s, 1H), 7.97 (d, *J* = 8.3 Hz, 1H), 7.64 (d, *J* = 3.2 Hz, 1H), 7.40 (d, *J* = 3.3 Hz, 1H), 7.33 - 7.26 (m, 2H), 7.13 (td, *J* = 7.5, 1.3 Hz, 1H), 3.70 (s, 2H).
HRMS: $[M+H]^+$, calc. for $C_{16}H_{14}NO_7$: 332.07648, found: 332.07653
Biodata: **IIa-034a:** FGF-1 IC50 [μM] = 22; FGF-2 IC50 [μM] = 5.7; VEGF-A1 IC50 [μM] = 86; VEGFR-Phosphorylation inhibition IC50 [μM] =100; PMN ROS [inhibition at 0.3 μM [%] = 69.2; PMN ROS inhibition IC50 [μM] = N.D.; Neutrophil adhesion inhibition [%] = 26.58

**Diethyl ester**

UPLC-MS (acidic method, 2 min): rt = 1.12 min; *m/z* = 388.1 $[M+H]^+$, peak area >99%
$^1H$ NMR (400 MHz, DMSO-$d_6$) δ 11.81 (s, 1H), 9.58 (s, 1H), 7.77 (t, *J* = 8.5 Hz, 1H), 7.65 (d, *J* = 3.4 Hz, 1H), 7.42 (d, *J* = 3.4 Hz, 1H), 7.36 - 7.31 (m, 2H), 7.20 (t, *J* = 7.4 Hz, 1H), 4.39 (q, *J* = 7.1 Hz, 2H), 4.05 (q, *J* = 7.1 Hz, 2H), 3.77 (s, 2H), 1.36 (t, *J* = 7.1 Hz, 3H), 1.13 (t, *J* = 7.1 Hz, 3H).
HRMS: $[M+H]^+$, calc. for $C_{20}H_{22}NO_7$: 388.13907, found: 388.13887
Biodata: **IIa-034a-E2:** FGF-1 IC50 [μM] = N.D.; FGF-2 IC50 [μM] = 164; VEGF-A1 IC50 [μM] = 200; VEGFR-Phosphorylation inhibition IC50 [μM] =ND; PMN ROS [inhibition at 0.3 μM [%] = 68.73; PMN ROS inhibition IC50 [μM] = N.D.; Neutrophil adhesion inhibition [%] = 11.86

47)**3-(3,4-Dihydroxyphenylmethylaminocarbonyl)-2,5-dihydroxybenzoic acid**, compound **IIa-035a**:

UPLC-MS (acidic method, 2 min): rt = 0.69 min; *m/z* = 318.0 $[M-H]^-$, peak area >99%
$^1H$ NMR (400 MHz, DMSO-$d_6$) δ 9.38 (s, 1H), 8.84 (s, 1H), 8.75 (s, 1H), 7.55 (d, J = 3.2 Hz, 1H), 7.34 (d, J = 3.2 Hz, 1H), 6.74 (d, J = 2.1 Hz, 1H), 6.67 (d, J = 8.0 Hz, 1H), 6.58 (dd, J = 8.1, 2.1 Hz, 1H), 4.34 (d, J = 5.7 Hz, 2H).
HRMS: $[M+H]^+$, calc. for $C_{15}H_{14}NO_7$: 320.07647, found: 320.07625.
Biodata: **IIa-035a**: FGF-1 IC50 [μM] = 16; FGF-2 IC50 [μM] = 61; VEGF-A1 IC50 [μM] = 45; VEGFR-Phosphorylation inhibition IC50 [μM] =0.52; PMN ROS [inhibition at 0.3 μM [%] = 76.99; PMN ROS inhibition IC50 [μM] = 1; Neutrophil adhesion inhibition [%] = 36.67

48)**3-(2-Carboxyphenylmethylaminocarbonyl)-2,5-dihydroxybenzoic acid**, compound **IIa-053a:**

UPLC-MS (acidic method, 4 min): rt = 1.08 min; *m/z* = 332.0 $[M+H]^+$, peak area >97%
$^1H$ NMR (DMSO-$d_6$) δ: 13.13 (brs, 1H), 12.73 (brs, 1H), 9.40 (s, 1H), 9.05 (s, 1H), 7.91 (dd, J = 7.8, 1.4 Hz, 1H), 7.65 - 7.29 (m, 5H), 4.80 (d, J = 6.1 Hz, 2H).
HRMS: $[M+H]^+$, calc. for $C_{16}H_{l4}NO_7$: 332.07648, found: 332.07626
Biodata: **IIa-053a:** FGF-1 IC50 [μM] = 84; FGF-2 IC50 [μM] = 18; VEGF-A1 IC50 [μM] = 37; VEGFR-Phosphorylation inhibition IC50 [μM] =0.27; PMN ROS [inhibition at 0.3 μM [%] = 63.13; PMN ROS inhibition IC50 [μM] = 0.62; Neutrophil adhesion inhibition [%] = 20; Whole Blood: GM-CSF IC50 [μM] = >100; IFNγ IC50 [μM] = 1.04; IL-1β IC50 [μM] = >100; IL-2 IC50 [μM] = 1.67; IL-4 IC50 [μM] = >100; IL-5 IC50 [μM] = >100; IL-6 IC50 [μM] = 2.2; IL-9 IC50 [μM] = 1.41; IL-10 IC50 [μM] = >100; IL-12p70 IC50 [μM] = 0.27; IL-13 IC50 [μM] = 29.9; IL-17A IC50 [μM] = >100; IL-17F IC50 [μM] = 28.8; IL-18 IC50 [μM] = >100; IL-21 IC50 [μM] = >100; IL-33 IC50 [μM] = 12.1; TGFβ IC50 [μM] = >100; TNF α IC50 [μM] = 0.02; TNF β IC50 [μM] = 91.5.

**Example 1.6. Molecules of type IIb:** Diamides from diamines, 1 example

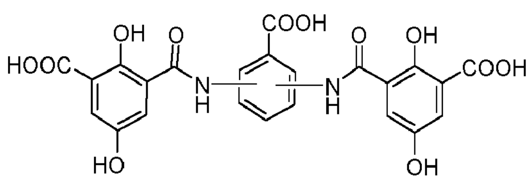

**Scheme 11.** Synthesis of Diamides of type **IIb**

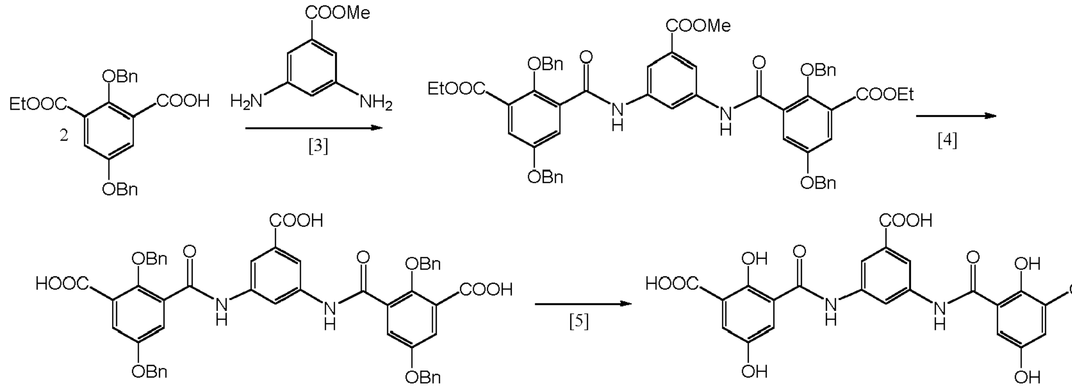


Procedure: see general procedure from **KI-1** [3], [4], [5]

Example:

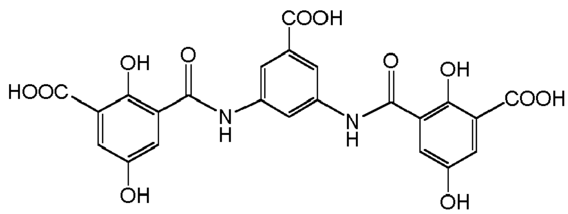

**IIb-010a**

For conditions and yields: See Table 2 (Figures 5A-C)

49)**3,5-Bis(2,5-dihydroxy-3-carboxybenzoylamino)benzoic acid**, compound **IIb-010a:**

UPLC-MS (acidic method, 4 min): rt = 0.98 min; *m/z* = 511.1 $[M-H]^-$, peak area >93%
$^1H$ NMR (400 MHz, DMSO-$d_6$) δ 10.81 (s, 2H), 10.65 (s, 2H), 8.38 (m, 1H), 8.12 (d, J = 2.0 Hz, 2H), 7.39 (d, J = 2.3 Hz, 4H).
HRMS: $[M+H]^+$, calc. for $C_{23}H_{17}N_2O_{12}$: 513.07760, found: 513.07774
Biodata: **IIb-010a:** FGF-1 IC50 [μM] = 200; FGF-2 IC50 [μM] = 102; VEGF-A1 IC50 [μM] = 28; VEGFR-Phosphorylation inhibition IC50 [μM] =ND; PMN ROS [inhibition at 0.3 μM [%] = 81.87; PMN ROS inhibition IC50 [μM] = N.D.; Neutrophil adhesion inhibition [%] = 59.84

**Example 1.7. Molecules of type IIc:** Diamides from diacids, 2 examples

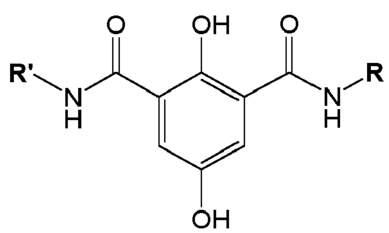

**Scheme 12.** Synthesis of Amides of type **IIc** with R = R'

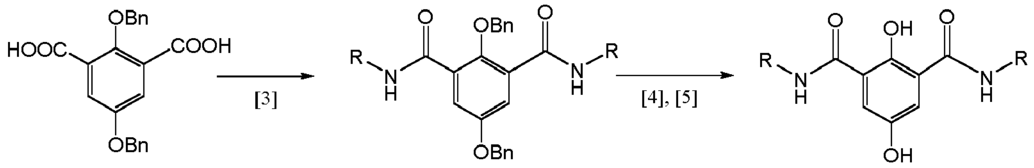


Procedure: see general procedure from KI-6 [3], [4], [5]

**Examples**

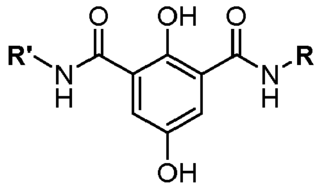

R, R' = 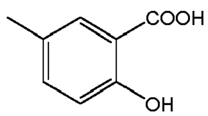 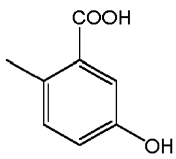

IIc-007a IIc-009a

For conditions and yields: See Table 2 (Figures 5A-C)

50)**5-(3-(3-Carboxy-4-hydroxyphenylaminocarbonyl)-2,5-dihydroxybenzamido)-2-hydroxybenzoic acid,** compound **IIc-007a:**

UPLC-MS (acidic method, 4 min): rt = 1.20 min; *m/z* = 469.1 $[M+H]^+$, peak area >91%
$^1H$ NMR (400 MHz, DMSO-d6) δ 10.41 (s, 2H), 9.51 (s, 1H), 8.22 (d, J = 2.7 Hz, 2H), 7.79 (dd, J = 9.0, 2.7 Hz, 2H), 7.58 (s, 2H), 6.98 (d, J = 8.9 Hz, 2H),
HRMS: $[M+H]^+$, calc. for $C_{22}H_{17}N_2O_{10}$: 469.08777, found: 469.08803
Biodata: **IIc-007a:** FGF-1 IC50 [μM] = 11; FGF-2 IC50 [μM] = 91; VEGF-A1 IC50 [μM] = 8.2; VEGFR-Phosphorylation inhibition IC50 [μM] =0.44; PMN ROS [inhibition at 0.3 μM [%] = 93.04; PMN ROS inhibition IC50 [μM] = N.D.; Neutrophil adhesion inhibition [%] = 64.61 ; Whole Blood: GM-CSF IC50 [μM] = 26; IFNγ IC50 [μM] = 0.11; IL-1β IC50 [μM] = >100; IL-2 IC50 [μM] = 12.8; IL-4 IC50 [μM] = >100; IL-5 IC50 [μM] = >100; IL-6 IC50 [μM] = 0.05; IL-9 IC50 [μM] = 2.14; IL-10 IC50 [μM] = 17.9; IL-12p70 IC50 [μM] = 3.37; IL-13 IC50 [μM] = 0.25; IL-17A IC50 [μM] = 0.01; IL-17F IC50 [μM] = 0.26; IL-18 IC50 [μM] = >100; IL-21 IC50 [μM] = 9.41; IL-33 IC50 [μM] = 0.18; TGFβ IC50 [μM] = >100; TNF α IC50 [μM] = 0.03; TNF β IC50 [μM] = 0.29

51)**2-(3-(2-Carboxy-4-hydroxyphenylaminocarbonyl)-2,5-dihydroxybenzamido)-5-hydroxylbenzoic acid,** compound **IIc-009a:**

UPLC-MS (acidic method, 2 min): rt = 0.79 min; *m/z* = 469.1 $[M+H]^+$, peak area >95%
$^1H$ NMR (400 MHz, DMF-d7) δ 10.11 (s,1H), 10.01 (s,2H), 8.71 (d, *J* = 9.0 Hz, 2H), 8.01 (s, 2H), 7.82 (d, *J* = 3.0 Hz, 2H), 7.46 (d, *J* = 8.9 Hz, 2H).
HRMS: $[M+H]^+$, calc. for $C_{22}H_{17}N_2O_{10}$: 469.08777, found: 469.08714
Biodata: **IIc-009a:** FGF-1 IC50 [μM] = 30; FGF-2 IC50 [μM] = N.D.; VEGF-A1 IC50 [μM] = 182; VEGFR-Phosphorylation inhibition IC50 [μM] =1.7; PMN ROS [inhibition at 0.3 μM [%] = 75.31; PMN ROS inhibition IC50 [μM] = N.D.; Neutrophil adhesion inhibition [%] = 53.15; Whole Blood: GM-CSF IC50 [μM] = >100; IFNγIC50 [μM] = 0.54; IL-1β IC50 [μM] = >100; IL-2 IC50 [μM] = 19.6; IL-4 IC50 [μM] = >100; IL-5 IC50 [μM] = >100; IL-6 IC50 [μM] = 0.27; IL-9 IC50 [μM] = 18.9; IL-10 IC50 [μM] = 11.31; IL-12p70 IC50 [μM] = >100; IL-13 IC50 [μM] = 0.13; IL-17A IC50 [μM] = 0.001; IL-17F

IC50 [μM] = 0.13; IL-18 IC50 [μM] = 2.95; IL-21 IC50 [μM] = 8.01; IL-33 IC50 [μM] = 0.29; TGFβ IC50 [μM] = >100; TNF α IC50 [μM] = 0.11; TNF β IC50 [μM] = 6.94.

**Example 1.8: Molecules of type IIIa: Monoamides, 4 examples**

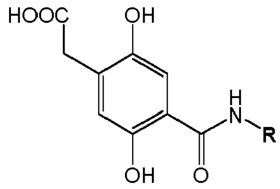

**Synthesis of Precursors**

**Scheme 13.** Synthetic Intermediates: **KI-8** and **KI-9** from **KI-1**

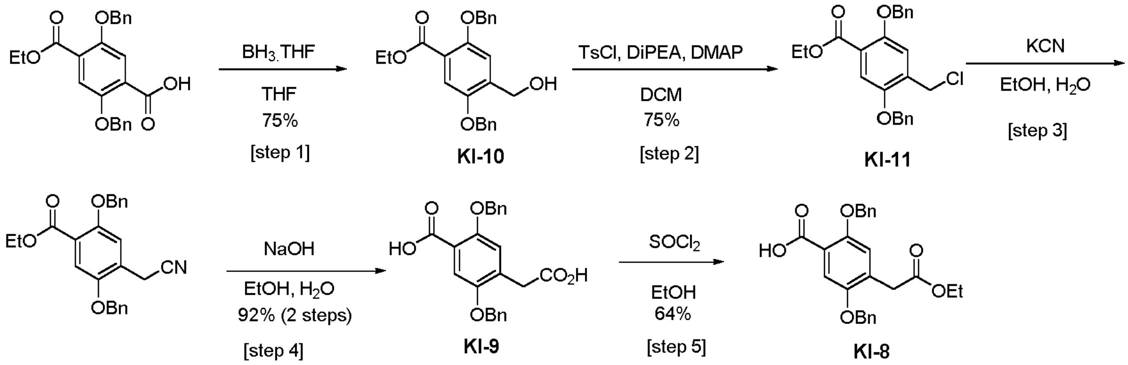


*Synthetic Protocoles*

Intermediates **KI-8** and **KI-9** (by way of **KI-10** and **KI-11**)

**Ethyl 2,5-bis(benzyloxy)-4-(hydroxymethyl)benzoate (KI-10)** [Step 1]: To a cooled solution at -20 °C of **KI-1** (62.3 g, 153 mmol) in THF (700 mL) under positive flow of inert gas ($N_2$) was slowly added a pre-cooled solution of $BH_3$.THF (615.0 mL, 615 mmol) (at -10 °C). The addition was done in such a way that the internal temperature of the reaction never exceeded -15 °C. The reaction was slightly warmed , the internal temperature being not allowed to exceed -7 °C. The mixture was maintained at this temperature for 2.5 h. The reaction mixture was then poured into ice/water (8 L). The blurry white mixtures obtained were left to stir at room temp. for 2 h and the residual white suspension was filtered through a fritted funnel (Porosity 3). The white solid thus obtained was further dried into vaccum oven at 40 °C overnight, to afford ethyl 2,5-bis(benzyloxy)-4-(hydroxymethyl)benzoate **(KI-10)** (60.5 g, 97% yield) as a white solid.
UPLC-MS (acidic method, 2 min): rt = 1.25 min; m/z = 391.2 $[M-H]^-$, peak area >68%
$^1H$ NMR (400 MHz, DMSO-$d_6$) δ 7.54 - 7.48 (m, 2H), 7.48 - 7.43 (m, 2H), 7.43 - 7.35 (m, 4H), 7.35 - 7.27 (m, 4H), 5.28 (t, *J* = 5.4 Hz, 1H), 5.13 (s, 2H), 5.11 (s, 2H), 4.58 (d, *J* = 5.4 Hz, 2H), 4.25 (q, *J* = 7.1 Hz, 2H), 1.26 (*t*, J = 7.1 Hz, 3H).

**Ethyl 2,5-bis(benzyloxy)-4-(chloromethyl)benzoate (KI-11)** [Step 2]: Tosyl chloride (17.0 g, 70 mmol) was added slowly to a cooled solution (ice bath) of **KI-10** (25.0 g, 64 mmol), DIPEA (12.2 mL, 70 mmol) and DMAP (0.778 g, 6 mmol) in DCM (260 mL). The reaction mixture was stirred at 60 °C for 30 h, whereupon the reaction was judged complete. DCM (100 mL) was added and the organic layer was separated, washed with saturated aqueous sodium hydrogen carbonate (4 x 50 mL), water (2 x 50 mL) and brine (50 mL), dried over sodium sulphate and concentrated. The crude product was submitted to column chromatography (Hexanes/EtOAc 0-20%) to give ethyl 2,5-bis(benzyloxy)-4-(chloromethyl)benzoate **(KI-11)** (23.5 g, 75%).
UPLC-MS (acidic method, 2 min): rt = 1.39 min; *m*/*z* = no ion, peak area >84%.
$^1H$ NMR (400 MHz, DMSO-$d_6$) δ 7.54 - 7.44 (m, 4H), 7.43 - 7.36 (m, 6H), 7.36 - 7.28 (m, 2H), 5.18 (s, 2H), 5.13 (s, 2H), 4.76 (s, 2H), 4.26 (q, J = 7.1 Hz, 2H), 1.25 (t, J = 7.1 Hz, 3H).

**Ethyl 2,5-bis(benzyloxy)-4-(cyanomethyl)benzoate** [Step 3]: A suspension of **KI-11** (7.5 g, 18.2 mmol) in a mixture of EtOH (90 mL) and $H_2O$ (45 mL) was treated with potassium cyanide (1.8 g, 27.4 mmol). The reaction mixture was heated to 75 °C and stirred at this temperature for 16 h. The reaction mixture was diluted with water (500 mL) and extracted with EtOAc (2 x 200 mL), the combined organic phases were then washed with brine (200 mL), dried over sodium sulfate, filtered and concentrated in vacuo. The crude product was isolated as a mixture of ethyl 2,5-bis(benzyloxy)-4-(cyanomethyl)benzoate and 2,5-bis(benzyloxy)-4-(cyanomethyl)benzoic acid (7.1 g), the product was used in the next step without further purification.
UPLC-MS (acidic method, 2 min): rt = 1.30 min; *m/z* = 402.2 $[M+H]^+$, peak area >65% and rt = 1.15 min; *m/z* = 374.1 $[M+H]^+$, peak area >8%.
$^1H$ NMR (400 MHz, DMSO-$d_6$) δ 7.56 - 7.27 (m, 12H), 5.19 (s, 2H), 5.14 (s, 2H), 4.26 (q, J = 7.1 Hz, 2H), 3.95 (s, 2H), 1.25 (t, J = 7.1 Hz, 3H).

**2,5-Bis(benzyloxy)-4-(carboxymethyl)benzoic acid (KI-9)** [Step 4]: To a suspension of ethyl 2,5-bis(benzyloxy)-4-(cyanomethyl)benzoate (7.1 g, 13.2 mmol) in EtOH (20 mL) was added a solution of sodium hydroxide (8.5 g, 212.5 mmol) in water (50 mL) and the reaction mixture was stirred under reflux for 18 h. The reaction mixture was diluted with water (100 mL) and the resulting solution was washed with EtOAc (2 x 100 mL), the thus obtained organic layer was extracted with water (100 mL) and all aqueous layers were gathered. Then the aqueous layer was acidified to pH ~ 3 with a saturated aqueous solution of citric acid, the formed precipitate was filtered and dried under reduced pressure. The solid was further dried in the vac. oven at 40 °C overnight to give 2,5-bis(benzyloxy)-4-(carboxymethyl)benzoic acid (**KI-9**) (6.4 g, 92% yield) as a yellowish solid.
UPLC-MS (acidic method, 2 min): rt = 1.07 min; *m/z* = 393.2 $[M+H]^+$, peak area >74%.
$^1H$ NMR (400 MHz, DMSO-d6) δ 12.46 (s, 2H), 7.55 - 7.27 (m, 11H), 7.19 (s, 1H), 5.11 (s, 2H), 5.09 (s, 2H), 3.61 (s, 2H).

**2,5-Bis(benzyloxy)-4-(2-ethoxycarbonylmethyl)benzoic acid (KI-8)** [Step 5]: **KI-9** (5.5 g, 14.0 mmol) was suspended in EtOH (30 mL)and the suspension was treated with thionyl chloride (0.52 mL, 7.1 mmol) and the reaction mixture was stirred at room temperature for 24 h. Then, the reaction mixture was poured into a saturated solution of sodium bicarbonate (1 L), leading to a white suspension. The solid was isolated by filtration and further triturated with $Et_2O$ (2 x 20 mL). The solid was further dried in the vac. oven overnight to give 2,5-bis(benzyloxy)-4-(2-ethoxycarbonylmethyl)benzoic acid **(KI-8)** (4.2 g, 64%) as a white solid.
UPLC-MS (acidic method, 2 min): rt = 1.23 min; *m/z* = 419.3 $[M-H]^-$, peak area >79%.
$^1H$ NMR (400 MHz, DMSO-$d_6$) δ 12.68 (brs, 1H), 7.58 - 7.27 (m, 11H), 7.19 (s, 1H), 5.11 (s, 2H), 5.08 (s, 2H), 4.01 (q, J = 7.1 Hz, 2H), 3.66 (s, 2H), 1.11 (t, J = 7.1 Hz, 3H).

## Synthesis of Monoamides

**Scheme 14.** Synthesis of Monoamides **IIIa**

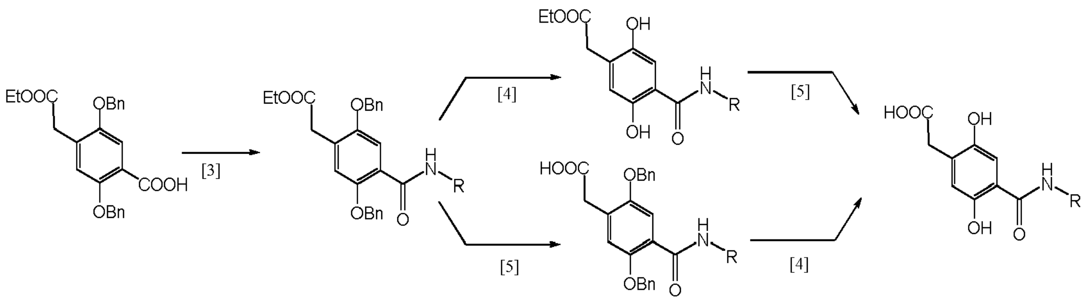


*Synthetic Protocoles*

Amide Coupling and Deprotection Procedures:

See General Procedures from **KI-1** and **KI-6** steps [3], [4], [5]

Examples

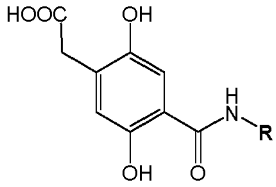

R = 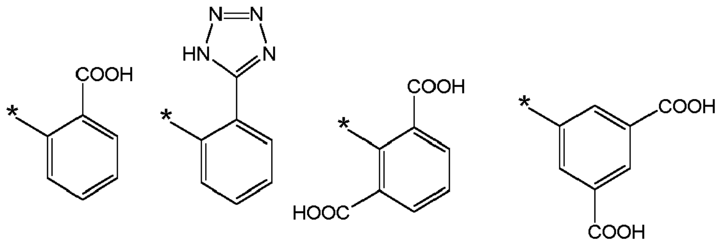

IIIa-001a IIIa-001aTz IIIa-013a IIIa-015a

For conditions and yields: See Table 3 (Figure 6)

52) **2-(4-(Carboxymethyl)-2,5-dihydroxybenzamido)benzoic acid**, compound **IIIa-001a:**

UPLC-MS (acidic method, 2 min): rt = 0.89 min; *m/z* = 332.1 $[M+H]^+$, peak area >98%
$^1H$ NMR (400 MHz, DMSO-d6) δ 13.38 (s, 1H), 12.17 (s, 1H), 10.67 (s, 1H), 9.23 (s, 1H), 8.64 (dd, *J* = 8.5, 1.2 Hz, 1H), 8.00 (dd, *J* = 7.9, 1.7 Hz, 1H), 7.62 (ddd, *J* = 8.7, 7.3, 1.7 Hz, 1H), 7.33 (s, 1H), 7.19 (td, *J* = 7.6, 1.2 Hz, 1H), 6.80 (s, 1H), 3.49 (s, 2H).
HRMS: $[M+H]^+$, calc. for $C_{16}H_{14}NO_7$: 332.07648, found: 332.07666
Biodata: **IIIa-001a:** FGF-1 IC50 [μM] = 62; FGF-2 IC50 [μM] = 10; VEGF-A1 IC50 [μM] = 32; VEGFR-Phosphorylation inhibition IC50 [μM] =ND; PMN ROS [inhibition at 0.3 μM [%] = 74.79; PMN ROS inhibition IC50 [μM] = N.D.; Neutrophil adhesion inhibition [%] = 30.4

**Ethyl methyl ester**

UPLC-MS (acidic method, 2 min): rt = 1.14 min; *m/z* = 374.2 $[M+H]^+$, peak area >96%.
$^1H$ NMR (400 MHz, DMSO-d6) δ 11.91 (s, 1H), 10.78 (s, 1H), 9.25 (s, 1H), 8.60 (dd, J = 8.5, 1.2 Hz, 1H), 7.97 (dd, J = 8.0, 1.7 Hz, 1H), 7.64 (ddd, J = 8.7, 7.3, 1.7 Hz, 1H), 7.38 (s, 1H), 7.26 - 7.18 (m, 1H), 6.81 (s, 1H), 4.08 (q, *J* = 7.1 Hz, 2H), 3.88 (s, 3H), 3.56 (s, 2H), 1.19 (t, *J* = 7.1 Hz, 3H).
HRMS: $[M+H]^+$, calc. for $C_{19}H_{20}NO_7$: 374.12342, found: 374.12333
Biodata: **IIIa-001a-E2:** FGF-1 IC50 [μM] = 200; FGF-2 IC50 [μM] = 33; VEGF-A1 IC50 [μM] = 43; VEGFR-Phosphorylation inhibition IC50 [μM] =ND; PMN ROS [inhibition at 0.3 μM [%] = 69.66; PMN ROS inhibition IC50 [μM] = N.D.; Neutrophil adhesion inhibition [%] = 44.46

53) **(2-(1H-Tetrazol-5-yl)phenylaminocarbonyl)-2,5-dihydroxyphenyl)acetic acid**, compound **IIIa-001aTz:**

UPLC-MS (acidic method, 2 min): rt = 0.87 min; *m/z* = 356.1 [M+H], peak area >95%.
$^1H$ NMR (400 MHz, DMSO-$d_6$) δ 12.22 (s, 1H), 11.43 (s, 1H), 10.74 (s, 1H), 9.21 (s, 1H), 8.46 (dd, J= 8.5, 1.2 Hz, 1H), 7.87 (d, *J* = 8.1 Hz, 1H), 7.60 (td, *J* = 8.6, 7.9, 1.6 Hz, 1H), 7.39 - 7.30 (m, 2H), 6.80 (s, 1H), 3.48 (s, 2H).
HRMS: $[M+H]^+$, calc. for $C_{16}H_{14}N_5O_5$: 356.09894, found: 356.09889
Biodata: **IIIa-001aTz:** FGF-1 IC50 [μM] = 51; FGF-2 IC50 [μM] = 14; VEGF-A1 IC50 [μM] = 12; VEGFR-Phosphorylation inhibition IC50 [μM] =0.71; PMN ROS [inhibition at 0.3 μM [%] = 69.35; PMN ROS inhibition IC50 [μM] = N.D.; Neutrophil adhesion inhibition [%] = 39.74; Whole Blood: GM-CSF IC50 [μM] = >100; IFNγIC50 [μM] = 18.9; IL-1β IC50 [μM] = >100; IL-2 IC50 [μM] = >100; IL-4 IC50 [μM] = >100; IL-5 IC50 [μM] = >100; IL-6 IC50 [μM] = 1.98; IL-9 IC50 [μM] = 3.01; IL-10 IC50 [μM] = 7.78; IL-12p70 IC50 [μM] = 0.48; IL-13 IC50 [μM] = 0.15; IL-17A IC50 [μM] = 0.17; IL-17F IC50 [μM] = 0.16; IL-18 IC50 [μM] = 27.7; IL-21 IC50 [μM] = 3; IL-33 IC50 [μM] = 0.14; TGFβ IC50 [μM] = >100; TNF α IC50 [μM] = 0.31; TNF β IC50 [μM] = 0.23.

**Ethyl ester**:

UPLC-MS (acidic method, 4 min): rt = 1.56 min; *m/z* = 384.2 $[M+H]^+$, peak area >95%.

$^1$H NMR (400 MHz, DMSO-*d$_6$*) δ 11.34 (s, 1H), 10.73 (s, 1H), 9.25 (s, 1H), 8.45 (dd, *J* = 8.5, 1.2 Hz, 1H), 7.85 (dd, *J* = 7.8, 1.6 Hz, 1H), 7.61 (ddd, *J* = 8.7, 7.4, 1.6 Hz, 1H), 7.39 - 7.30 (m, 2H), 6.80 (s, 1H), 4.08 (q, *J* = 7.1 Hz, 2H), 3.56 (s, 2H), 1.19 (t, *J* = 7.1 Hz, 3H).
HRMS: [M+H]$^+$, calc. for $C_{18}H_{18}N_5O_5$: 384.13024, found: 384.12999
Biodata: **IIIa-001aTz-E1:** FGF-1 IC50 [μM] = 200; FGF-2 IC50 [μM] = 200; VEGF-A1 IC50 [μM] = 200; VEGFR-Phosphorylation inhibition IC50 [μM] =0.32; PMN ROS [inhibition at 0.3 μM [%] = 70.03; PMN ROS inhibition IC50 [μM] = N.D.; Neutrophil adhesion inhibition [%] = 91.76

54) **2-(4-(Carboxymethyl)-2,5-dihydroxybenzamido)isophthalic acid**, compound **IIIa-013a:**

UPLC-MS (acidic method, 4 min): rt = 0.82 min; *m/z* = 376.0 [M+H]$^+$, peak area >89%.
$^1$H NMR (400 MHz, DMSO-d6) δ 13.67 - 11.48 (m, 3H), 10.82 (s, 1H), 9.19 (s, 1H), 7.95 (d, *J* = 7.8 Hz, 2H), 7.36 (s, 1H), 7.33 (t, *J* = 7.8 Hz, 1H), 6.79 (s, 1H), 3.48 (s, 2H).
HRMS: [M+H]$^+$, calc. for $C_{17}H_{14}NO_9$: 376.06630, found: 376.06638 Biodata: **IIIa-013a:** FGF-1 IC50 [μM] = 17; FGF-2 IC50 [μM] = 31; VEGF-A1 IC50 [μM] = 43; VEGFR-Phosphorylation inhibition IC50 [μM] =1.3; PMN ROS [inhibition at 0.3 μM [%] = 76.55; PMN ROS inhibition IC50 [μM] = N.D.; Neutrophil adhesion inhibition [%] = 30.667; Whole Blood: GM-CSF IC50 [μM] = 27.8; IFNγ IC50 [μM] = 0.15; IL-1β IC50 [μM] = >100; IL-2 IC50 [μM] = 57.1; IL-4 IC50 [μM] = >100; IL-5 IC50 [μM] = >100; IL-6 IC50 [μM] = 3.55; IL-9 IC50 [μM] = 11.9; IL-10 IC50 [μM] = 37.3; IL-12p70 IC50 [μM] = 0.5; IL-13 IC50 [μM] = 0.37; IL-17A IC50 [μM] = 0.15; IL-17F IC50 [μM] = 0.41; IL-18 IC50 [μM] = >100; IL-21 IC50 [μM] = 1.63; IL-33 IC50 [μM] = 1.11; TGFβ IC50 [μM] = >100; TNF α IC50 [μM] = 0.51; TNF β IC50 [μM] = 0.89.

55) **5-(4-(Carboxymethyl)-2,5-dihydroxybenzamido)isophthalic acid**, compound **IIIa-015a:**

UPLC-MS (acidic method, 4 min): rt = 0.93 min; *m/z* = 376.0 [M+H]$^+$, peak area >96%.
$^1$H NMR (400 MHz, DMSO-d6+10% $D_2O$) δ 8.34 (s, 2H), 8.18 (s, 1H), 7.38 (s, 1H), 6.71 (s, 1H), 3.39 (s, 2H).
HRMS: [M+H]$^+$, calc. for $C_{17}H_{14}NO_9$: 376.06630, found: 376.06665
Biodata: **IIIa-015a:** FGF-1 IC50 [μM] = 16; FGF-2 IC50 [μM] = 114; VEGF-A1 IC50 [μM] = 202; VEGFR-Phosphorylation inhibition IC50 [μM] =0.89; PMN ROS [inhibition at 0.3 μM [%] = 78.76; PMN ROS inhibition IC50 [μM] = N.D.; Neutrophil adhesion inhibition [%] = 12.13

**Ethyl dimethyl ester**

UPLC-MS (acidic method, 4 min): rt = 1.69 min; *m/z* = 432.1 [M+H]$^+$, peak area >97%.
$^1$H NMR (400 MHz, DMSO-d6) δ 9.23 (s, 1H), 8.58 (s, 1H), 8.58 (s, 1H), 8.23 - 8.21 (m, 1H), 7.31 (s, 1H), 6.81 (s, 1H), 4.08 (q, J = 7.0 Hz, 2H), 3.91 (s, 6H), 3.58 (s, 2H), 1.19 (t, J = 7.1 Hz, 3H),
HRMS: [M+H]$^+$, calc. for $C_{21}H_{22}NO_9$: 432.12891, found: 432.02903
Biodata: **IIIa-015a-E3:** FGF-1 IC50 [μM] = N.D.; FGF-2 IC50 [μM] = 191; VEGF-A1 IC50 [μM] = 87; VEGFR-Phosphorylation inhibition IC50 [μM] =7.2; PMN ROS [inhibition at 0.3 μM [%] = 91.37; PMN ROS inhibition IC50 [μM] = N.D.; Neutrophil adhesion inhibition [%] = 61.22

**Example 1.9. Molecules of type IIIb:** Diamides from diamines, 1 example

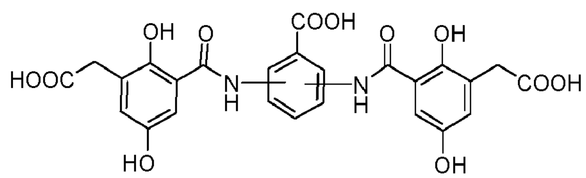

**Scheme 15.** Synthesis of Diamides of type **IIIb**

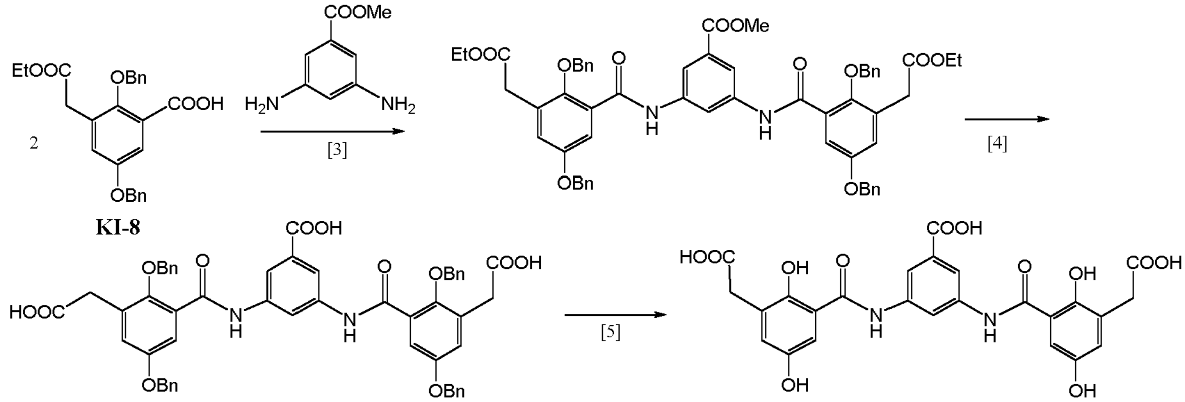


Procedure: see general procedure from **KI-1** steps [3], [4], [5]

Example:

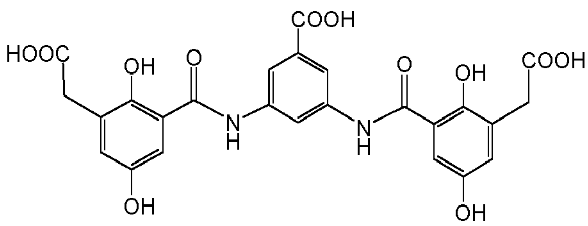

**IIIb-010a**

For conditions and yields: See Table 3 (Figure 6)

56)**3,5-Bis(2,5-dihydroxy-4-carboxymethylbenzoylamino)benzoic acid**, compound **IIIb-010a:**

UPLC-MS (acidic method, 4 min): rt = 1.08 min; m/z = 539.2.1 $[M-H]^-$, peak area 80%.
$^1$H NMR (400 MHz, DMSO-$d_6$) δ 10.85 (brs, 2H), 10.57 (brs, 2H), 9.20 (s, 2H), 8.26 (m, 1H), 8.09 (d, *J* = 2.0 Hz, 2H), 7.36 (s, 2H), 6.82 (s, 2H), 3.50 (s, 4H).
HRMS: [M+H]+, calc. for C23H17N2O12: 513.07760, found: 513.07774

**Triethyl ester**

UPLC-MS (acidic method, 4 min): rt = 1.81 min; m/z = 623.3 $[M-H]^-$, peak area 94%.
$^1$H NMR (400 MHz, DMSO-$d_6$) δ 10.87 (s, 2H), 10.65 (s, 2H), 9.23 (s, 2H), 8.31 - 8.28 (m, 1H), 8.10 (d, J = 2.0 Hz, 2H), 7.35 (s, 2H), 6.81 (s, 2H), 4.35 (q, J = 7.1 Hz, 2H), 4.08 (q, J = 7.1 Hz, 4H), 3.58 (s, 4H), 1.35 (t, J = 7.1 Hz, 3H), 1.19 (t, J = 7.1 Hz, 6H).
Biodata: **IIIb-010a-E3:** FGF-1 IC50 [μM] = 200; FGF-2 IC50 [μM] = 200; VEGF-A1 IC50 [μM] = 200; VEGFR-Phosphorylation inhibition IC50 [μM] =0.65; PMN ROS [inhibition at 0.3 μM [%] = N.D.; PMN ROS inhibition IC50 [μM] = N.D.; Neutrophil adhesion inhibition [%] = 0

**Example 1.10: MOLECULES OF TYPE IIIc. Dimers, 6 examples**

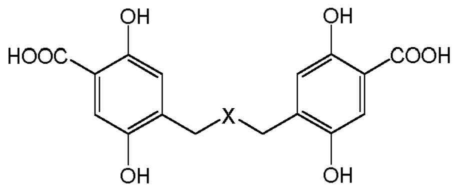

Synthetic Schemes and Procedures

**Scheme 16: X=O**

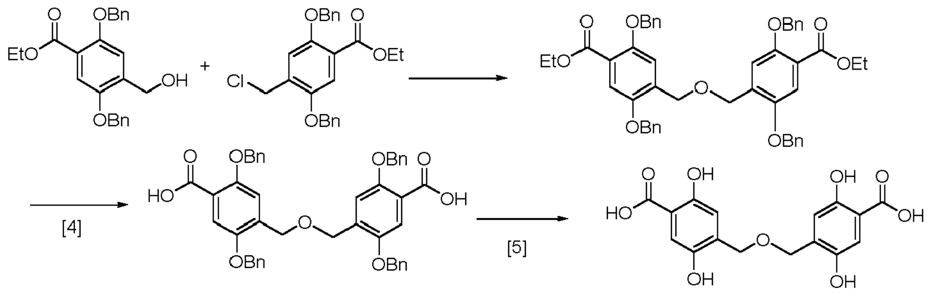


Synthetic procedures

*Formation of ether linkage from **KI-10** and **KI-11***

**Ethyl 2,5-dibenzyloxy-4- [(2,5-dibenzyloxy-4-ethoxycarbonyphenyl)methoxymethyl] benzoate**

[Step 1]: To a solution of **KI-11** (340 mg, 0.83 mmol) and **KI-10** (250 mg, 0.64 mmol) in DMF (6 mL), cooled down in an ice bath, was added portionwise sodium hydride (76 mg, 1.9 mmol). After 1 h, the reaction mixture was poured into a saturated aqueous solution of ammonium chloride (50 mL) and the material was extracted with EtOAc (3 x 30 mL), the organic phase was further washed with water (2 x 50 mL) and brine (50 mL), dried with sodium sulfate and concentrated under reduced pressure. The crude product was purified by flash column chromatography (Hexanes/EtOAc 0 to 20%) to give th title compound (161 mg, 33% yield) as a brown solid.

Deprotection procedures:

See General procedures [4], [5]

Examples

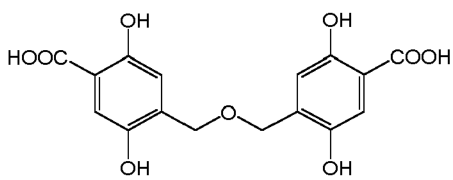

IIIb-060a

For conditions and yields: See Table 4 (deprotections) (Figure 7)

57) **4-((2,5-Dihydroxy-4-carboxyphenyl)methoxymethyl)-2,5-dihydroxybenzoic acid**, compound **IIIc-060a:**

UPLC-MS (acidic method, 4 min): rt = 0.94 min; *m/z* = 349.1 $[M-H]^-$, peak area >90%.
$^1H$ NMR (400 MHz, DMSO-d6) δ 7.20 (s, 2H), 6.92 (s, 2H), 4.56 (s, 4H),

Biodata: **IIIc-060a:** FGF-1 IC50 [μM] = 200; FGF-2 IC50 [μM] = 51; VEGF-A1 IC50 [μM] = 114; VEGFR-Phosphorylation inhibition IC50 [μM] =1.3; PMN ROS [inhibition at 0.3 μM [%] = 86.84; PMN ROS inhibition IC50 [μM] = N.D.; Neutrophil adhesion inhibition [%] = 41.38

**Scheme 17: X = NH**

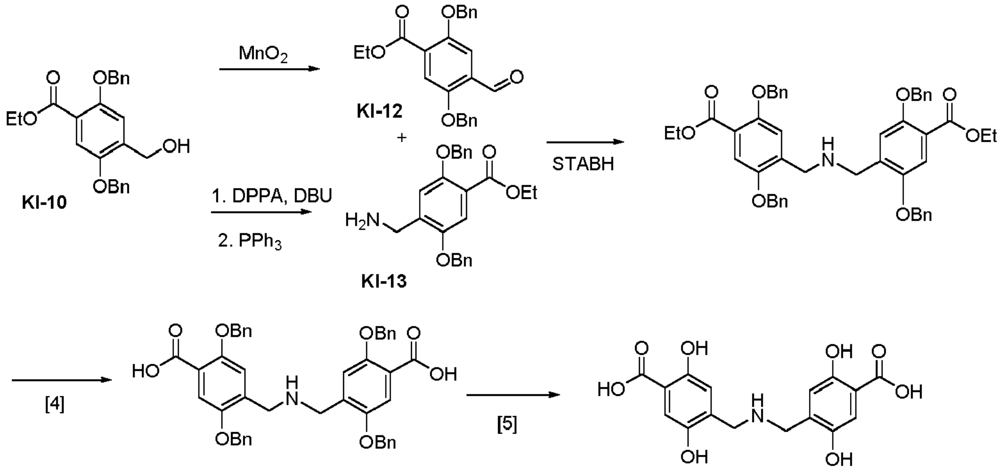


Synthetic procedures

*Formation of amine linkage from **KI-12** and **KI-13** via **KI-10** and **KI-11***

**Ethyl 2,5-Bis(benzyloxy)-4-formylbenzoate (KI-12)**: To a solution of **KI-10** (3.4 g, 8.6 mmol) in dichloromethane (50 mL) was added manganese dioxide (4.5 g, 51.9 mmol). The resulting suspension was stirred under reflux for 4 h. The reaction was filtered through a pad of Celite® and the solid washed with dichloromethane (300 mL), the solvent was then removed under reduced pressure to give the title compound (3.2 g, 94% yield) as a yellowish solid.
UPLC-MS (acidic method, 2 min): rt = 1.33 min; *m*/*z* = no ion, peak area >93%
$^1$H NMR (400 MHz, DMSO-$d_6$) δ 10.39 (s, 1H), 7.56 (s, 1H), 7.54 - 7.26 (m, 11H), 5.28 (s, 2H), 5.20 (s, 2H), 4.30 (q, *J* = 7.1 Hz, 2H), 1.27 (t, *J* = 7.1 Hz, 3H).

**Ethyl 4-(azidomethyl)-2,5-bis(benzyloxy)benzoate:** To a suspension of KI-10 (3.0 g, 7.6 mmol) and 1,8-Diazabicyclo[5.4.0]undec-7-ene (DBU) (1.5 mL. 9.9 mmol) in toluene (30 mL) was added diphenyl phosphoryl azide (DPPA) (2 mL, 9.1 mmol). The resulting mixture was stirred at room temperature for 18 h. An aqueous solution of 1M hydrogen chloride (200 mL) was added and the product was extracted with EtOAc (3 x 100 mL), the combined organic layers were washed with water (2 x 50 mL), dried over sodium sulfate and concentrated under reduced pressure. The residue was purified by flash column chromatography (Hexanes/EtOAc 0 to 20%) to yield the title compound (3.1 g, 98%) as a colourless oil that became a white solid over time.
UPLC-MS (acidic method, 2 min): rt = 1.37 min; *m*/*z* = 390.2, $[M+H-N_2]^+$, peak area >93%
$^1$H NMR (400 MHz, DMSO-$d_6$) δ 7.53 - 7.46 (m, 4H), 7.44 - 7.26 (m, 8H), 5.16 (s, 2H), 5.14 (s, 2H), 4.48 (s, 2H), 4.26 (q, J = 7.1 Hz, 2H), 1.25 (t, J = 7.1 Hz, 3H).

**Ethyl 4-(aminomethyl)-2,5-bis(benzyloxy)benzoate (KI-13):** To a solution of ethyl 4-(azidomethyl)-2,5-bis(benzyloxy)benzoate (2.8 g, 6.7 mmol) in THF (60 mL) and water (6 mL) was added polymer-bound triphenylphosphine (4.7 g, ~3mmol/g loading) and the resulting mixture was left to stir at room temperature for 18 h. The resin was removed by filtration and washed with water (100 mL) and EtOAc (2 x 100 mL). The phases were separated, and the aqueous layer was further extracted with EtOAc (100 mL), the gathered organic layers were washed with brine (200 mL), dried over sodium sulfate and concentrated to give the title compound **(KI-13)** (1.8 g, 70% yield) as a white solid.
UPLC-MS (basic method, 2 min): rt = 1.20 min; *m*/*z* = 392.2, $[M+H]^+$, peak area >93%
$^1$H NMR (400 MHz, DMSO-$d_6$) δ 7.60 - 7.22 (m, 12H), 5.14 (s, 2H), 5.10 (s, 2H), 4.24 (q, J = 7.1 Hz, 2H), 3.75 (s, 2H), 1.25 (t, *J* = 7.1 Hz, 3H).

**Ethyl 2,5-dibenzyloxy-4- [(2,5-dibenzyloxy-4-ethoxycarbonylphenyl)methylaminomethyl] benzoate: KI-12** (500 mg, 1.3 mmol) and **KI-13** (641 mg, 1.6 mmol) were dissolved in DCM (35 mL) followed by addition of activated molecular sieves (10 beads). The resulting mixture was stirred at room temperature for 5 h. Sodium triacetoxyborohydride (654 mg, 3.1 mmol) was then added and the resulting mixture was stirred at room temperature for 18 h. DCM (25 mL) was added, the reaction mixture was decanted into a separating funnel and washed with water (50 mL), dried with sodium sulfate and concentrated under reduced pressure. The residue was purified by flash column chromatography

((Hexanes/EtOAc 0 to 50%) to yield the titled compound (631 mg, 64% yield) as a colourless oil that became solid over time.
UPLC-MS (acidic method, 2 min): rt = 1.37 min; *m*/*z* = 766.3, $[M+H]^+$, peak area >92%
$^1H$ NMR (400 MHz, DMSO-$d_6$) δ 7.47 - 7.42 (m, 4H), 7.38 - 7.25 (m, 20H), 5.08 (s, 4H), 5.04 (s, 4H), 4.25 (q, *J* = 7.1 Hz, 4H), 3.76 (s, 4H), 1.25 (t, *J* = 7.1 Hz, 6H).

Deprotection procedures: See General procedures [4], [5]

For conditions and yields: See Table 4 [(Figure 7)

**Examples**

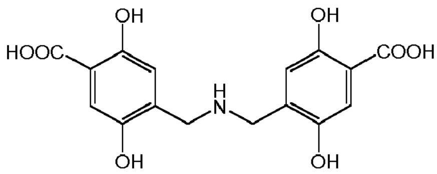

58)**Bis(4-carboxy-2,5-dihydroxyphenylmethyl)amine**, compound **IIIc-056a:**

UPLC-MS (acidic method, 4 min): rt = 0.57 min; *m*/*z* = 350.0 $[M+H]^+$, peak area >96%.
$^1H$ NMR (400 MHz, DMSO-d6) δ 9.97 (s, 2H), 9.08 (s, 2H), 7.31 (s, 2H), 7.02 (s, 2H), 4.11 (s, 4H).
HRMS: $[M+H]^+$, calc. for $C_{16}H_{16}NO_8$: 350.08704, found: 350.08706
Biodata: **IIIc-056a:** FGF-1 IC50 [μM] = 200; FGF-2 IC50 [μM] = 35; VEGF-A1 IC50 [μM] = 200; VEGFR-Phosphorylation inhibition IC50 [μM] =ND; PMN ROS [inhibition at 0.3 μM [%] = 90.07; PMN ROS inhibition IC50 [μM] = N.D.; Neutrophil adhesion inhibition [%] = 35

**Scheme 18: X = NAc**

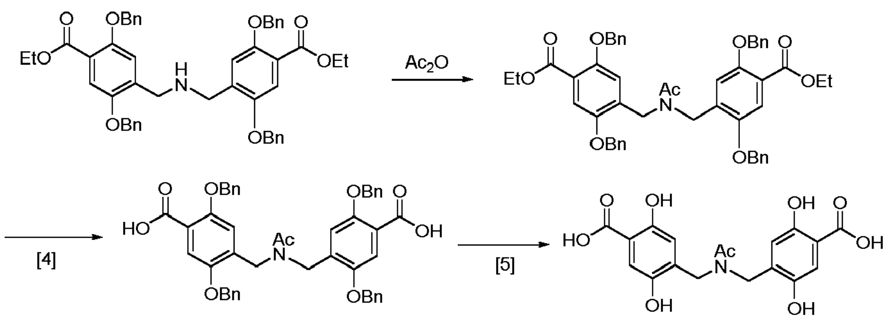


Synthetic procedures

**N,N-Bis-[2,5-dibenzyloxy-4-ethoxycarbonylphenylmethyl]acetamide:** To a solution of N,N-bis-[2,5-dibenzyloxy-4-ethoxycarbonylphenylmethyl]amine (300 mg, 0.39 mmol) and pyridine (0.2 mL, 2.4 mmol) in DCM (6 mL), under an atmosphere of nitrogen, was added acetic anhydride (0.2 mL, 2.1 mmol). The reaction mixture was stirred at room temperature for 1 h and concentrated under reduced pressure. The residue was purified by flash column chromatography (Hexanes/EtOAc, 0 to 50 %) to give the title compound (316 mg, 92% yield) as a colourless oil.
UPLC-MS (acidic method, 2 min): rt = 1.44 min; *m*/*z* = 808.2 $[M+H]^+$, peak area >95%.
$^1H$ NMR (400 MHz, DMSO-d6) δ 7.49 - 7.17 (m, 22H), 6.92 (s, 1H), 6.72 (s, 1H), 5.03 (s, 2H), 5.00 (s, 2H), 4.96 (s, 2H), 4.94 (s, 2H), 4.46 (s, 4H), 4.31 - 4.16 (m, 4H), 1.96 (s, 3H), 1.28 - 1.20 (m, 6H).

Deprotection procedures:

See General procedures [4], [5]

**Examples**

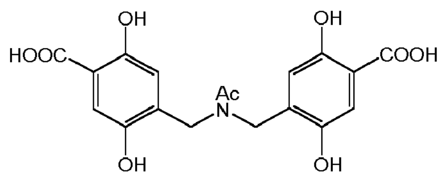

For conditions and yields: See Table 4 (deprotections) (Figure 7)

59)**N,N-Bis(4-carboxy-2,5-dihydroxyphenylmethyl)acetamide**, compound **IIIc-057a:**

UPLC-MS (acidic method, 4 min): rt = 0.84 min; *m/z* = 392.1 $[M+H]^+$, peak area >99%.
$^1H$ NMR (400 MHz, DMSO-d6) δ 9.50 (s, 1H), 9.34 (s, 1H), 7.22 (s, 1H), 7.17 (s, 1H), 6.63 (s, 1H), 6.53 (s, 1H), 4.48 (s, 2H), 4.39 (s, 2H), 2.11 (s, 3H).
HRMS: $[M+H]^+$, calc. for $C_{18}H_{18}NO_9$: 392.09761, found: 392.09766
Biodata: **IIIc-057a:** FGF-1 IC50 [μM] = 87; FGF-2 IC50 [μM] = 77; VEGF-A1 IC50 [μM] = 38; VEGFR-Phosphorylation inhibition IC50 [μM] =0.2; PMN ROS [inhibition at 0.3 μM [%] = 90.34; PMN ROS inhibition IC50 [μM] = N.D.; Neutrophil adhesion inhibition [%] = 40.04; Whole Blood: GM-CSF IC50 [μM] = >100; IFNγ IC50 [μM] = 0.9; IL-1β IC50 [μM] = 16.2; IL-2 IC50 [μM] = 24.2; IL-4 IC50 [μM] = >100; IL-5 IC50 [μM] = >100; IL-6 IC50 [μM] = 0.19; IL-9 IC50 [μM] = 11.1; IL-10 IC50 [μM] = >100; IL-12p70 IC50 [μM] = 0.73; IL-13 IC50 [μM] = 4.73; IL-17A IC50 [μM] = 1.32; IL-17F IC50 [μM] = 5.06; IL-18 IC50 [μM] = >100; IL-21 IC50 [μM] = 1.56; IL-33 IC50 [μM] = 2.93; TGFβ IC50 [μM] = >100; TNF α IC50 [μM] = 0.15; TNF β IC50 [μM] = 1.58.

**Scheme 19: X = $NCH_2C_6H_3(OH)_2COOH$**

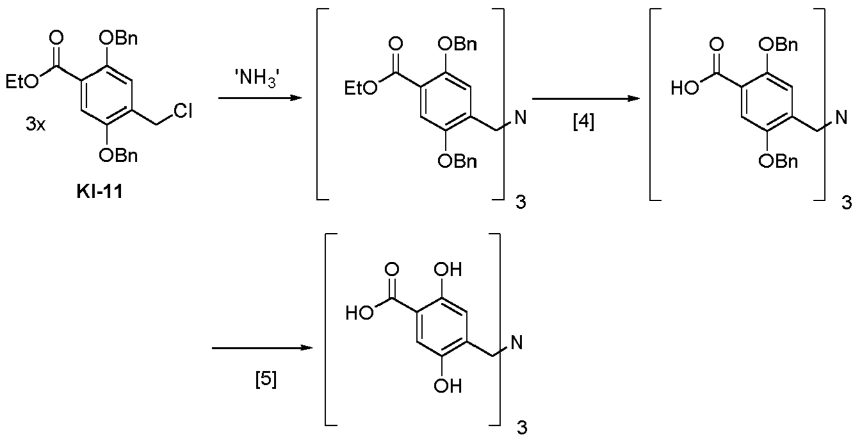


Synthetic procedures

*Formation of tertiary amine from **KI-11** and $NH_3$*

**Tris(4-ethoxycarbony-2,5-dibenzyloxyphenylmethyl)amine (Step 1):** A sealed tube was charged with **KI-11** (32.5 g, 79 mmol), sodium iodide (0.79 g, 5 mmol), a solution of ammonia in MeOH (7 N) (38 mL, 264 mmol) and EtOAc (80 mL). The tube was then sealed and the reaction mixture was heated at 60 °C for 24 h. After 24 h, the starting material (**KI-11)** was consumed and the reaction mixture contained the desired product but also monomeric and dimeric structures. Water (100 mL) was added and the resulting mixture was extracted with EtOAc (3 x 100 mL), the gathered organic layer was dried over $Na_2SO_4$, filtered and concentrated under reduced pressure. The residue obtained was

dissolved in EtOAc (165 mL) and **KI-11** (2.5 g, 6 mmol), sodium iodide (0.79 g, 5 mmol), DIPEA (10 mL, 58 mmol) were added and the resulting solution was heated at 60 °C. After 18h, water (100 mL) was added and the resulting mixture was extracted with EtOAc (3 x 100 mL), the gathered organic layer was dried over $Na_2SO_4$, filtered and concentrated under reduced pressure. The residue was purified by recrystallisation using EtOH/EtOAc 95/5 (20 mL) as solvent to yield to the title compound (15.0 g, 50%) as white crystals.
UPLC-MS (basic method, 2 min): rt = 1.71 min; m/z = 1140.3 $[M+H]^+$, peak area >99%.
$^1$H NMR (400 MHz, DMSO-$d_6$) δ 6.56 - 6.49 (m, 12H), 6.48 - 6.38 (m, 24H), 4.22 (s, 6H), 4.04 (s, 6H), 3.42 (q, J = 7.1 Hz, 6H), 3.00 (s, 6H), 0.42 (t, J = 7.1 Hz, 9H).

Deprotection procedures: See General procedures [4], [5]

**Examples**

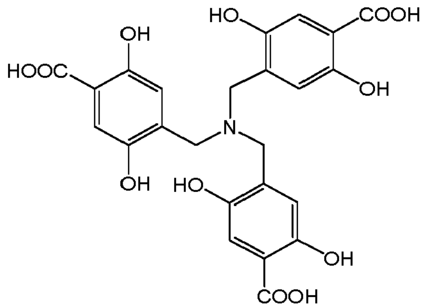

For conditions and yields: See Table 4 (deprotections) (Figure 7)

60) **Tris(4-carboxy-2,5-dihydroxyphenylmethyl)amine**, compound **IIIc-061a:**

UPLC-MS (acidic method, 4 min): rt = 0.74 min; *m/z* = 516.0 $[M+H]^+$, peak area >86%.
$^1$H NMR (400 MHz, DMSO-d6) δ 7.27 (s, 3H), 6.83 (s, 3H), 4.33 (s, 6H).
HRMS: $[M+H]^+$, calc. for $C_{24}H_{22}NO_{12}$: 516.11365, found: 516.11389
Biodata: **IIIc-061a:** FGF-1 IC50 [μM] = 9; FGF-2 IC50 [μM] = 7.6; VEGF-A1 IC50 [μM] = 21; VEGFR-Phosphorylation inhibition IC50 [μM] =4.3; PMN ROS [inhibition at 0.3 μM [%] = 92.5; PMN ROS inhibition IC50 [μM] = N.D.; Neutrophil adhesion inhibition [%] = 58.69; Whole Blood: GM-CSF IC50 [μM] = 31.2; IFNγ IC50 [μM] = 2.48; IL-1β IC50 [μM] = >100; IL-2 IC50 [μM] = >100; IL-4 IC50 [μM] = >100; IL-5 IC50 [μM] = >100; IL-6 IC50 [μM] = 0.18; IL-9 IC50 [μM] = 8.85; IL-10 IC50 [μM] = 17.9; IL-12p70 IC50 [μM] = 37.2; IL-13 IC50 [μM] = 0.31; IL-17A IC50 [μM] = 0.04; IL-17F IC50 [μM] = 0.32; IL-18 IC50 [μM] = 32.4; IL-21 IC50 [μM] = 6.38; IL-33 IC50 [μM] = 0.31; TGFβ IC50 [μM] = >100; TNF α IC50 [μM] = 0.03; TNF β IC50 [μM] = 0.81

**Triethyl ester**

UPLC-MS (acidic method, 2 min): rt = 1.05 min; *m/z* = 600.2 $[M+H]^+$, peak area >82%.
$^1$H NMR (400 MHz, DMSO-d6) δ 10.02 (s, 3H), 9.54 (s, 3H), 7.17 (s, 3H), 7.00 (s, 3H), 4.33 (q, J = 7.1 Hz, 6H), 3.59 (s, 6H), 1.31 (t, J = 7.1 Hz, 9H).
HRMS: $[M+H]^+$, calc. for $C_{30}H_{34}NO_{12}$: 600.20755, found: 600.20790
Biodata: **IIIc-061a-E3:** FGF-1 IC50 [μM] = 200; FGF-2 IC50 [μM] = 200; VEGF-A1 IC50 [μM] = 200; VEGFR-Phosphorylation inhibition IC50 [μM] =0.34; PMN ROS [inhibition at 0.3 μM [%] = N.D.; PMN ROS inhibition IC50 [μM] = N.D.; Neutrophil adhesion inhibition [%] = 10.4

**Scheme 20: X = S**

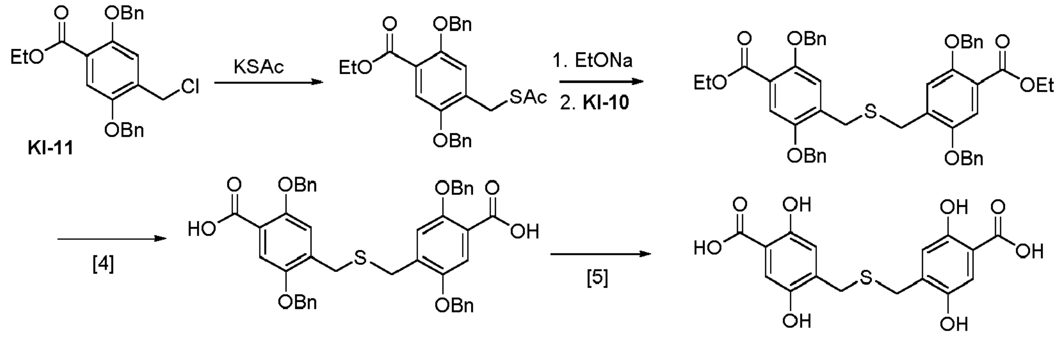


Synthetic procedures

*Formation of thioether linkage from KI-11*

See **Scheme 21**. Same conditions to create the thioether linkage.

Deprotection procedures: see General procedures [4], [5]

**Examples**

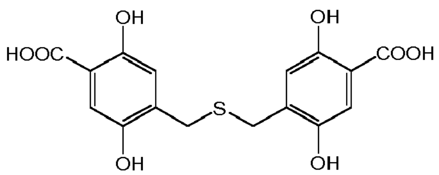

For conditions and yields: See Table 4 (deprotections) (Figure 7)

6 1)**4-((2,5-Dihydroxy-4-carboxyphenyl)methylthiomethyl)-2,5-dihydroxybenzoic acid**, compound **IIIc-058a:**

UPLC-MS (acidic method, 2 min): rt = 0.84 min; *m/z* = 365.1 $[M-H]^-$, peak area >79%
$^1H$ NMR (400 MHz, Methanol-$d_4$) δ 7.25 (s, 2H), 6.83 (s, 2H), 3.69 (s, 4H).
HRMS: $[M+H]^+$, calc. for $C_{16}H_{15}O_8S$: 367.04822, found: 367.04768,
Biodata: **IIIc-058a:** FGF-1 IC50 [μM] = 12; FGF-2 IC50 [μM] = 12; VEGF-A1 IC50 [μM] = 124; VEGFR-Phosphorylation inhibition IC50 [μM] =0.29; PMN ROS [inhibition at 0.3 μM [%] = 61.22; PMN ROS inhibition IC50 [μM] = 1.17; Neutrophil adhesion inhibition [%] = 23

**Scheme 21: X = $SO_2$**

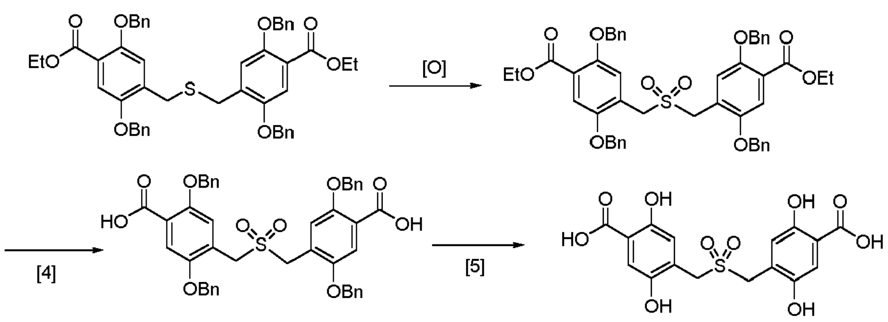

Synthetic procedures

*Oxidation of thioether linkage*

See preparation of **IVb-059a:** same conditions of oxidation.
Deprotection procedures: see General procedures [4], [5]

**Examples**

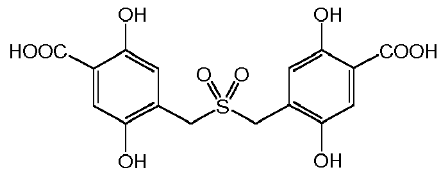

For conditions and yields: See Table 4 (deprotections) (Figure 7)

62)**4-((2,5-Dihydroxy-4-carboxyphenyl)methylsulfonylmethyl)-2,5-dihydroxybenzoic acid,** compound **IIIc-059a:**

UPLC-MS (acidic method, 2 min): rt = 0.72 min; m/z = 397.1 $[M-H]^-$, peak area >97%
$^1H$ NMR (400 MHz, DMSO-$d_6$) δ 13.94 (brs, 2H), 10.63 (brs, 2H), 9.71 (s, 2H), 7.27 (s, 2H), 6.91 (s, 2H), 4.44 (s, 4H).
HRMS: $[M+H]^+$, calc. for $C_{16}H_{15}O_{10}S$: 399.03804, found: 399.03768
Biodata: **IIIc-059a:** FGF-1 IC50 [μM] = 47; FGF-2 IC50 [μM] = 50; VEGF-A1 IC50 [μM] = 200; VEGFR-Phosphorylation inhibition IC50 [μM] =3.61; PMN ROS [inhibition at 0.3 μM [%] = 66.39; PMN ROS inhibition IC50 [μM] = 1; Neutrophil adhesion inhibition [%] = 14.67

**Example 1.11: MOLECULES OF TYPE IVc**

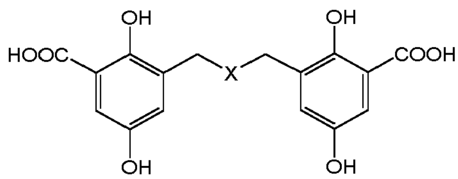

**Scheme 22: X=S**

Synthesis *via* dimethyl ether

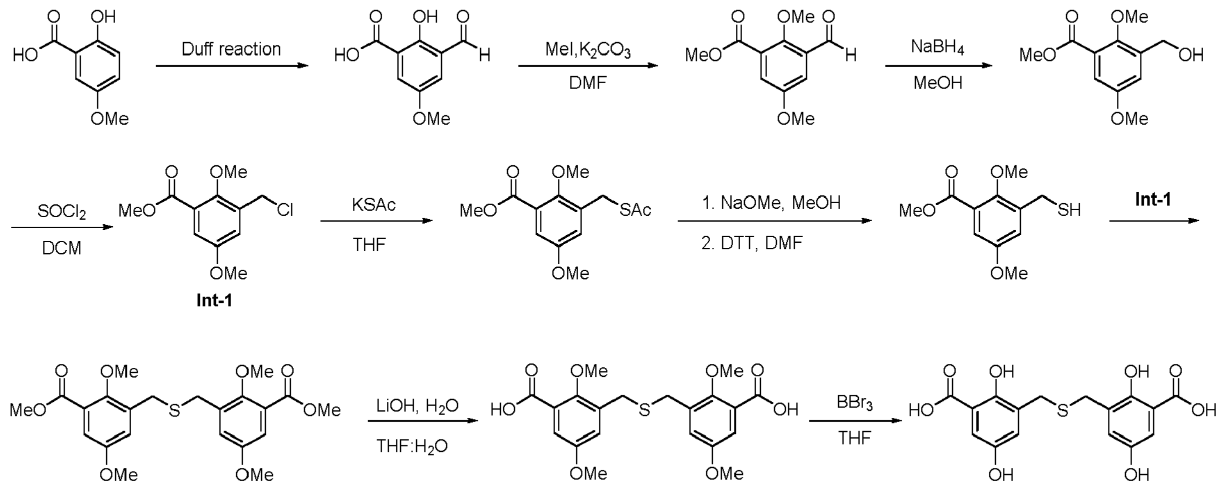


## Synthetic procedures

### *Synthesis from 5-O-methyl gentisic acid*

**3-Formyl-2-hydroxy-5-methoxybenzoic acid.** Hexamethylenetetramine (40.019 g, 0.285 mol) was added to the mixture of 2-hydroxy-5-methoxybenzoic acid (24.0 g, 0.143 mol) in trifluoroacetic acid (190.0 mL). The reaction was refluxed for 18 h. After completion, the reaction was cooled down to ambient temperature and a solution of 2M hydrochloric acid (700 mL) was added to the mixture which was stirred at ambient temperature for 24 h. The precipitate was filtered, washed with water (500 mL) and dried in the vacuum oven to give 3-formyl-2-hydroxy-5-methoxybenzoic acid as a pale yellow solid (21.30 g, 0.11 mol, 77.0%)
UPLC-MS (acidic method, 2 min): rt = 0.69 min; *m/z* = 195.1 $[M-H]^-$, peak area >98%
$^1H$ NMR (400 MHz, DMSO-$d_6$) δ 10.36 (s, 1H), 7.62 (d, *J* = 3.4 Hz, 1H), 7.44 (d, *J* = 3.4 Hz, 1H), 3.78 (s, 3H).

**Methyl 3-formyl-2,5-dimethoxybenzoate.** Methyl iodide (6.7 mL, 0.107 mol) was added to a mixture of 3-formyl-2-hydroxy-5-methoxybenzoic acid (10.0 g, 0.05 mol) and potassium carbonate-325 mesh (28.18 g, 0.20 mol) in DMF (100.0 mL). The resulting mixture was stirred at ambient temperature for 18 h. The reaction was cooled down to ambient temperature and treated with cold water (400 mL) resulting in the formation of a precipitate. The resulting suspension was stirred for 10 min, then filtered and dried in the vacuum oven to give methyl 3-formyl-2,5-dimethoxybenzoate as a yellow solid (8.90 g, 0.04 mol, 78.0%).
UPLC-MS (acidic method, 2 min): rt = 0.95 min; *m/z* = 225.1 $[M+H]^+$, peak area >98%
$^1H$ NMR (400 MHz, DMSO-$d_6$) δ 10.27 (s, 1H), 7.55 (d, *J* = 3.4 Hz, 1H), 7.41 (d, *J* = 3.4 Hz, 1H), 3.88 (s, 3H), 3.87 (s, 3H), 3.83 (s, 3H).

**Methyl 3-(hydroxymethyl)-2,5-dimethoxybenzoate.** Sodium borohydride (16.198 g, 0.428 mol) was added slowly to a solution of methyl 3-formyl-2,5-dimethoxybenzoate (48.0 g, 0.214 mol) in MeOH (900 mL) at 0 °C and the resulting mixture was stirred at ambient temperature for 15 min. The solvent was removed *in vacuo* and the residue was dissolved in DCM (200 mL) then washed with water (200 mL). The organic layer was dried over $Na_2SO_4$, filtered and concentrated to dryness to give methyl 3-(hydroxymethyl)-2,5-dimethoxybenzoate as white solid (45.6 g, 0.20 mol, 95.0%)
UPLC-MS (acidic method, 2 min): rt = 0.84 min; no ionization, peak area >98%
$^1H$ NMR (400 MHz, DMSO-$d_6$) δ 7.20 (d, *J* = 3.2 Hz, 1H), 7.08 (d, *J* = 3.3 Hz, 1H), 5.24 (t, *J* = 5.7 Hz, 1H), 4.54 (d, *J* = 5.6 Hz, 2H), 3.83 (s, 3H), 3.76 (s, 3H), 3.68 (s, 3H).

**Methyl 3-(chloromethyl)-2,5-dimethoxybenzoate.** Thionyl chloride (17.2 mL, 0.235 mol) was added dropwise to a solution of methyl 3-(hydroxymethyl)-2,5-dimethoxybenzoate (45.60 g, 0.20 mol) over 30 min at ambient temperature. The resulting mixture was stirred for 18 h at ambient temperature. After completion, the reaction mixture was washed with a saturated solution of aqueous sodium carbonate (3 x 500 mL) and brine (500 mL). The organic layer was dried over $Na_2SO_4$, filtered and concentrated to dryness to give methyl 3-(chloromethyl)-2,5-dimethoxybenzoate as a brown solid (49.1 g, 0.18 mol, 90%,)
UPLC-MS (acidic method, 2 min): rt = 1.10 min; no ionization, peak area >92%
$^1H$ NMR (400 MHz, DMSO-$d_6$) δ 7.29 (d, *J* = 3.2 Hz, 1H), 7.22 (d, *J* = 3.3 Hz, 1H), 4.74 (s, 2H), 3.85 (s, 3H), 3.77 (s, 3H), 3.76 (s, 3H).

**Methyl 3-(acetylthiomethyl)-2,5-dimethoxybenzoate.** Potassium thioacetate (15.54 g, 0.136 mol) was added to the solution of methyl 3-(chloromethyl)-2,5-dimethoxybenzoate (22.20 g, 0.091 mol) in THF (500.0 mL) at ambient temperature. The reaction mixture was stirred a 75 °C for 18 h. After completion, the solvent was removed under reduced

pressure to give a red oily residue which was treated with a saturated brine solution (500 mL), then extracted with diethyl ether (2 x 250 mL). The organic phases were combined, dried over $Na_2SO_4$, filtered and concentrated to dryness to yield methyl 3-(acetylthiomethyl)-2,5-dimethoxybenzoate as a brown solid (25.50 g, 0.09 mol, 99%).
UPLC-MS (acidic method, 2 min): rt = 1.10 min; no ionization, peak area >92%
$^1$H NMR (400 MHz, DMSO-$d_6$) δ 7.12 (d, *J* = 3.3 Hz, 1H), 7.10 (d, *J* = 3.2 Hz, 1H), 4.10 (s, 2H), 3.84 (s, 3H), 3.74 (s, 3H), 3.70 (s, 3H), 2.35 (s, 3H).

**Methyl 3-(mercaptomethyl)-2,5-dimethoxybenzoate.** To a solution of methyl 3-(acetylthiomethyl)-2,5-dimethoxybenzoate (25.5 g, 89.68 mmol) in dry methanol (800 mL) was added in a dropwise manner at 0 °C, a solution of sodium methanolate (5.81 g, 107.62 mmol) under a nitrogen atmosphere. The mixture was stirred at room temperature for 45 min before being quenched with Dowex X8($H^+$) ion-exchange resin and stirred for 15 min. After filtration, the solvent was evaporated under reduced pressure to give the crude product as a brown oil (which contained the desired product and the corresponding disulphide compound in a 2:1 ratio). The residue was dissolved in DMF (400.0 mL) and treated with dithiothreitol (DTT) (33.95 g, 0.26 mol) under a nitrogen atmosphere. The reaction mixture was stirred at 75 °C for 18 h. After completion of the disulphide to thiol conversion, the solvent was evaporated. The resulting residue was dissolved in DCM (1 L), washed with brine (7 x 500 mL), dried over $Na_2SO_4$, filtered and concentrated to dryness to yield homogeneous methyl 3-(mercaptomethyl)-2,5-dimethoxybenzoate as a brown oil (21.40 g, 88.32 mmol, 99%)
UPLC-MS (acidic method, 2 min): rt = 1.05 min; *m/z* = 243.1 $[M+H]^+$, peak area >95%
$^1$H NMR (400 MHz, DMSO-$d_6$) δ 7.19 (d, *J* = 3.2 Hz, 1H), 7.09 (d, *J* = 3.3 Hz, 1H), 3.83 (s, 3H), 3.76 (s, 3H), 3.73 (s, 3H), 3.71 (d, *J* = 8.0 Hz, 2H), 2.93 (t, *J* = 8.0 Hz, 1H).

**Dimethyl 3,3'-(thiobis(methylene))bis(2,5-dimethoxybenzoate).** Triethylamine (19.0 mL, 136.61 mmol) was added to a solution of methyl 3-(mercaptomethyl)-2,5-dimethoxybenzoate (21.40 g, 88.32 mmol) and methyl 3-(chloromethyl)-2,5-dimethoxybenzoate (22.69 g, 92.74 mmol) under a nitrogen atmosphere. The reaction was stirred at ambient temperature for 18 h. After completion, the solvent was removed under vacuum to give a brown oil residue which was treated with water (1 L), then extracted with diethyl ether (3 x 500 mL). The organic phases were combined, washed with brine (5 x 250 mL), dried over $Na_2SO$, filtered and concentrated to dryness to yield the crude product as a brown oil, which was purified by silica gel chromatography: elution was made with a gradient of ethyl acetate (0 to 20%) in iso-hexane to yield dimethyl 3,3'-(thiobis(methylene))bis(2,5-dimethoxybenzoate) as a brown solid (29.9 g, 66.37 mmol, 76%)
UPLC-MS (acidic method, 2 min): rt = 1.22 min; *m/z* = 451.2 $[M+H]^+$, peak area >92%
$^1$H NMR (400 MHz, DMSO-$d_6$) δ 7.12 - 7.08 (m, 4H), 3.83 (s, 6H), 3.76 (s, 4H), 3.73 (s, 6H), 3.67 (s, 6H).

**3,3'-(thiobis(methylene))bis(2,5-dimethoxybenzoic acid).** A solution of Lithium hydroxide monohydrate (1.0 g, 23.83 mmol) in water (30.0 mL) was added to the solution of dimethyl 3,3'-(thiobis(methylene))bis(2,5-dimethoxybenzoate) (2.0 g, 4.44 mmol) in THF (100.0 mL). The reaction was stirred at ambient temperature for 48 h. After completion, water (100 mL) was added to the reaction and the mixture was washed with ethyl acetate (100 mL). The aqueous layer was acidified with a 1 M aqueous hydrochloric acid to pH=2 and extracted with ethyl acetate (3 X 100 mL). The organic layers were collected, dried ($Na_2SO_4$), filtered and concentrated to give 3,3'-(thiobis(methylene))bis(2,5-dimethoxybenzoic acid) as a brown solid (1.91 g, 4.11 mmol, 93%)
UPLC-MS (acidic method, 2 min): rt = 0.93 min; m/z = 421.1 $[M-H]^-$, peak area >95%
$^1$H NMR (400 MHz, DMSO-$d_6$) δ 12.96 (s, 2H), 7.10 (d, *J* = 3.3 Hz, 2H), 7.08 (d, *J* = 3.2 Hz, 2H), 3.76 (s, 4H), 3.73 (s, 6H), 3.69 (s, 6H).

**3-[(2,5-Dihydroxy-3-carboxyphenyl)methylthiomethyl]-2,5-dihydroxybenzoic acid.** To a solution of preceding thioether (5.0 g, 0.01 mol) in DCM (200.0 mL) was added slowly a 1M solution of tribromoborane in DCM (100.7 mL, 0.101 mol) at 0 °C and the reaction mixture was refluxed for 48 h. After this time, the solid was filtered, washed with DCM (500 mL) and then suspended in a solution of 1M hydrochloric acid (50 mL). The resulting mixture was refluxed for 2 h. The resulting brown suspension was filtered and dried to give the crude product, which was purified by reverse phase column chromatography (25 g, MeCN:$H_2O$ 5% to 95% over 12 CV) to yield 2,5-dihydroxy-3-[(2,5-dihydroxy-3-carboxyphenyl)methylthiomethyl]benzoic acid as white solid (1.1 g, 0.003 mol, 26%).
UPLC-MS (acidic method, 2 min): rt = 0.70 min; m/z = 365.1 $[M-H]^-$, peak area >95%
$^1$H NMR (400 MHz, DMSO-$d_6$) δ 13.86 (s, 2H), 11.09 (s, 2H), 9.14 (s, 2H), 7.08 (d, *J* = 3.1 Hz, 2H), 7.02 (d, *J* = 3.1 Hz, 2H), 3.65 (s, 4H).

Example:

63)**3-((2,5-Dihydroxy-3-carboxyphenyl)methylthiomethyl)-2,5-dihydroxybenzoic acid**, compound **IVc-058a:**

UPLC-MS (acidic method, 2 min): rt = 0.70 min; m/z = 365.1 $[M-H]^-$, peak area >95%
$^1$H NMR (400 MHz, DMSO-$d_6$) δ 13.86 (s, 2H), 11.09 (s, 2H), 9.14 (s, 2H), 7.08 (d, *J* = 3.1 Hz, 2H), 7.02 (d, *J* = 3.1 Hz, 2H), 3.65 (s, 4H).

HRMS: $[M+H]^+$, calc. for $C_{16}H_{15}O_8S$: 367.04822, found: 367.04734
Biodata: **IVc-058a:** FGF-1 IC50 [μM] = 36; FGF-2 IC50 [μM] = 4.9; VEGF-A1 IC50 [μM] = 83; VEGFR-Phosphorylation inhibition IC50 [μM] =0.53; PMN ROS [inhibition at 0.3 μM [%] = 77.04; PMN ROS inhibition IC50 [μM] = 0.29; Neutrophil adhesion inhibition [%] = 43.5; Whole Blood: GM-CSF IC50 [μM] = >100; IFNγ IC50 [μM] = 10.6; IL-1β IC50 [μM] = 27.5; IL-2 IC50 [μM] = 3.82; IL-4 IC50 [μM] = >100; IL-5 IC50 [μM] = >100; IL-6 IC50 [μM] = 0.21; IL-9 IC50 [μM] = 31.1; IL-10 IC50 [μM] = 16.4; IL-12p70 IC50 [μM] = 4.23; IL-13 IC50 [μM] = 55.9; IL-17A IC50 [μM] = 0.1; IL-17F IC50 [μM] = 86.8; IL-18 IC50 [μM] = >100; IL-21 IC50 [μM] = 3.2; IL-33 IC50 [μM] = 33.9; TGFβ IC50 [μM] = >100; TNF α IC50 [μM] = 0.18; TNF β IC50 [μM] = 59.3.

**Scheme 23: X = $SO_2$**

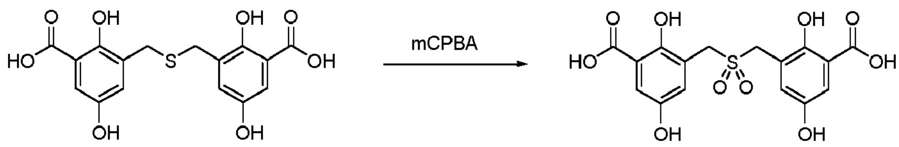


*Synthesis from compound **IVc-058a***

**3-((2,5-Dihydroxy-3-carboxyphenyl)methylsulfonylmethyl)-2,5-dihydroxybenzoic acid.** To a solution of thioether **IVc-058a** (4.25 g, 9.28 mmol) in DMF (20.0 mL) was added slowly a solution of 3-chlorobenzene-1-carboperoxy acid (mCPBA purity grade ≤77%, 5.80 g, 25.88 mmol) in DMF (20.0 mL). The reaction mixture was stirred for 18 h at ambient temperature. The crude product was submitted for HPLC purification (see general conditions) to give a white solid (3.20 g) which was triturated with 1M HCl (50 mL) to yield the desired product as white solid (2.55 g, 6.40 mmol, 56%) UPLC-MS (acidic method, 4 min): rt = 0.68 min; m/z = 397.1 $[M-H]^-$, peak area >95%

64)**3-((2,5-Dihydroxy-3-carboxyphenyl)methylsulfonylmethyl)-2,5-dihydroxybenzoic acid**, compound **IVc-059a**:

UPLC-MS (acidic method, 4 min): rt = 0.68 min; m/z = 397.1 $[M-H]^-$, peak area >95%
$^1$HNMR (400 MHz, DMSO-$d_6$) δ 11.24 (s, 1H), 9.32 (s, 1H), 7.21 (d, *J* = 3.1 Hz, 1H), 7.09 (d, *J* = 3.1 Hz, 1H), 4.44 (s, 2H).
HRMS: $[M+H]^+$, calc. for $C_{16}H_{15}O_{10}S$: 399.03804, found: 399.03775
Biodata: **IVc-059a**: FGF-1 IC50 [μM] = 6.7; FGF-2 IC50 [μM] = 2.7; VEGF-A1 IC50 [μM] = 12; VEGFR-Phosphorylation inhibition IC50 [μM] =4.8; PMN ROS [inhibition at 0.3 μM [%] = 74; PMN ROS inhibition IC50 [μM] = 0.147; Neutrophil adhesion inhibition [%] = 10; Whole Blood: GM-CSF IC50 [μM] = >100; IFNγ IC50 [μM] = 4.34; IL-1β IC50 [μM] = 38.6; IL-2 IC50 [μM] = 21.2; IL-4 IC50 [μM] = >100; IL-5 IC50 [μM] = >100; IL-6 IC50 [μM] = 0.11; IL-9 IC50 [μM] = 0.4; IL-10 IC50 [μM] = 1.31; IL-12p70 IC50 [μM] = 42; IL-13 IC50 [μM] = >100; IL-17A IC50 [μM] = 0.16; IL-17F IC50 [μM] = >100; IL-18 IC50 [μM] = >100; IL-21 IC50 [μM] = 1.87; IL-33 IC50 [μM] = 27.5; TGFβ IC50 [μM] = >100; TNF α IC50 [μM] = 0.16; TNF β IC50 [μM] = 0.33

**Dimethyl ester**

UPLC-MS (acidic method, 2 min): rt = 0.94 min; m/z = 427.1 $[M+H]^+$, peak area >95%
$^1$H NMR(400 MHz, DMSO-$d_6$) δ 10.28 (s, 2H), 9.43 (s, 2H), 7.21 (d, *J* = 3.1 Hz, 2H), 7.13 (d, *J* = 3.1 Hz, 2H), 4.47 (s, 4H), 3.91 (s, 6H).
HRMS: $[M+H]^+$, calc. for $C_{18}H_{19}O_{10}S$: 427.06934, found: 427.06942
Biodata: **IVc-059a-E2**: FGF-1 IC50 [μM] = 78; FGF-2 IC50 [μM] = 94; VEGF-A1 IC50 [μM] = 200; VEGFR-Phosphorylation inhibition IC50 [μM] =2.7; PMN ROS [inhibition at 0.3 μM [%] = 45.67; PMN ROS inhibition IC50 [μM] = 2.64; Neutrophil adhesion inhibition [%] = 50.5

**Dimethyl ester tetraacetate**

UPLC-MS (acidic method, 2 min): rt = 1.05 min; m/z = 593.1 $[M-H]^-$, peak area >95%
$^1$H NMR (400 MHz, DMSO-$d_6$) δ 7.75 (d, *J* = 2.8 Hz, 2H), 7.53 (d, *J* = 2.9 Hz, 2H), 4.65 (s, 4H), 3.81 (s, 6H), 2.31 (s, 6H), 2.23 (s, 6H).
HRMS: $[M+H]^+$, calc. for $C_{26}H_{27}O_{14}S$: 595.11160, found: 595.11149
Biodata: **IVc-059a-E2-A4**: FGF-1 IC50 [μM] = 54; FGF-2 IC50 [μM] = 200; VEGF-A1 IC50 [μM] = 25; VEGFR-Phosphorylation inhibition IC50 [μM] =ND; PMN ROS [inhibition at 0.3 μM [%] = 29.17; PMN ROS inhibition IC50 [μM] =

4.13; Neutrophil adhesion inhibition [%] = 87.5; Whole Blood: GM-CSF IC50 [μM] = 1.83; IFNγ IC50 [μM] = 3.5; IL-1β IC50 [μM] = 14.1; IL-2 IC50 [μM] = 10.4; IL-4 IC50 [μM] = >100; IL-5 IC50 [μM] = >100; IL-6 IC50 [μM] = 0.99; IL-9 IC50 [μM] = 2.9; IL-10 IC50 [μM] = 16.8; IL-12p70 IC50 [μM] = 51.4; IL-13 IC50 [μM] = 18.7; IL-17A IC50 [μM] = 0.02; IL-17F IC50 [μM] = 25.8; IL-18 IC50 [μM] = >100; IL-21 IC50 [μM] = >100; IL-33 IC50 [μM] = 23.2; TGFβ IC50 [μM] = >100; TNFα IC50 [μM] = 0.3; TNF β IC50 [μM] = 19.4

**Example 1.12 - Molecules of type V: Reference compounds**

Diamides derived from 5-hydroxyisophthalic acid

2 examples

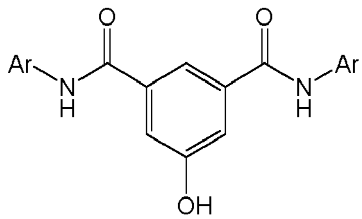

Synthetic Schemes and Procedures

**Scheme 23: synthesis of V-001a**

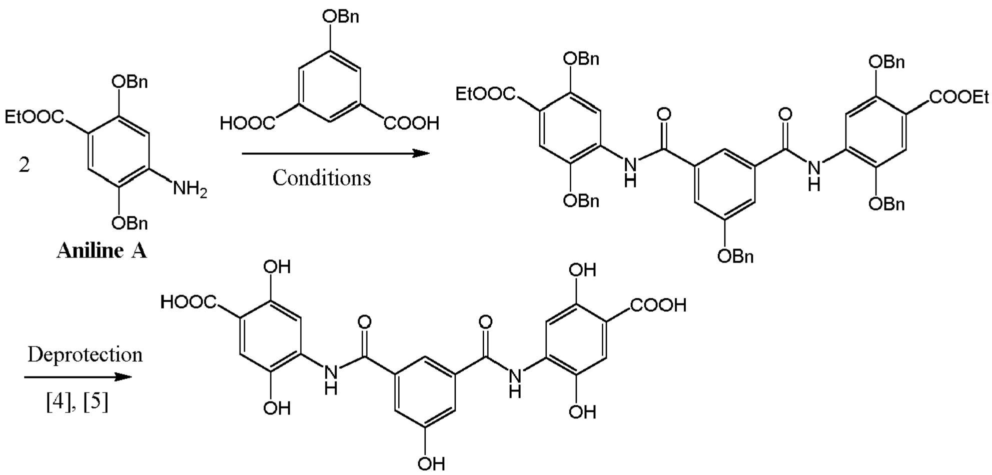


*Formation of amide Linkage*

See **General procedure B for Amide Coupling [3B]** using aniline **A** (2 moles) or methyl 5-aminosalicylate

Deprotection procedures: See General procedures [4], [5]

For details see Table 5 (Figure 8)

**Examples**

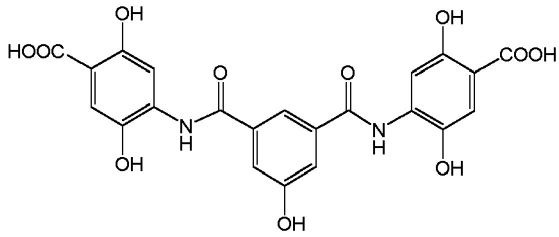

V-001a

65) **5-Hydroxyisophthalic acid bis-N-(4-carboxy-2,5-dihydroxyphenyl)amide (V-001a)**

UPLC-MS (acidic method, 2 min): rt = 0.72 min; m/z = 483.0 $[M+H]^+$, peak area >95%
$^1H$ NMR (400 MHz, DMSO-d6) δ 10.28 (s, 1H), 9.83 (s, 2H), 9.48 (s, 2H), 7.92 (s, 1H), 7.68 (s, 2H), 7.52 (d, J = 1.5 Hz, 2H), 7.30 (s, 2H).

**Diethyl ester**

UPLC-MS (acidic method, 2 min): rt = 1.09 min; m/z = 539.2 $[M-H]^-$, peak area 94%
$^1H$ NMR (400 MHz, DMSO-d6) δ 10.23 (s, 2H), 9.92 (s, 2H), 9.49 (s, 2H), 7.91 (s, 1H), 7.73 (s, 2H), 7.51 (d, J = 1.5 Hz, 2H), 7.32 (s, 2H), 4.35 (q, J = 7.1 Hz, 4H), 1.34 (t, J = 7.1 Hz, 6H).

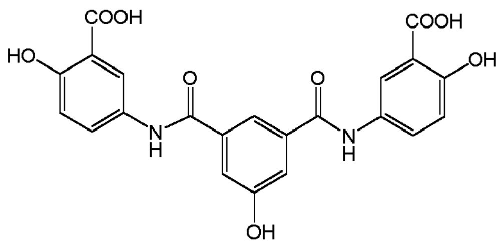

V-002a

66)**5-Hydroxyisophthalic acid bis-N-(3-carboxy-4-hydroxyphenyl)amide (V-002a)**

UPLC-MS (acidic method, 4 min): rt = 1.00 min; m/z = 451.1 $[M-H]^-$, peak area 97%
$^1H$ NMR (400 MHz, DMSO-d6) δ 10.31 (s, 2H), 10.11 (s, 1H), 8.28 (d, J = 2.7 Hz, 2H), 7.98 (m, 1H), 7.89 (dd, J = 9.0, 2.7 Hz, 2H), 7.50 (d, J = 1.5 Hz, 2H), 6.97 (d, J = 9.0 Hz, 2H).

Urea-linked Gentisic Acid Units 1 example

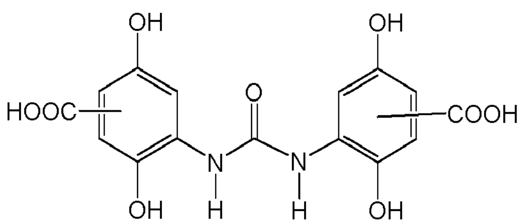

Synthetic Scheme and Procedures

**Scheme 24: synthesis of V-003a**

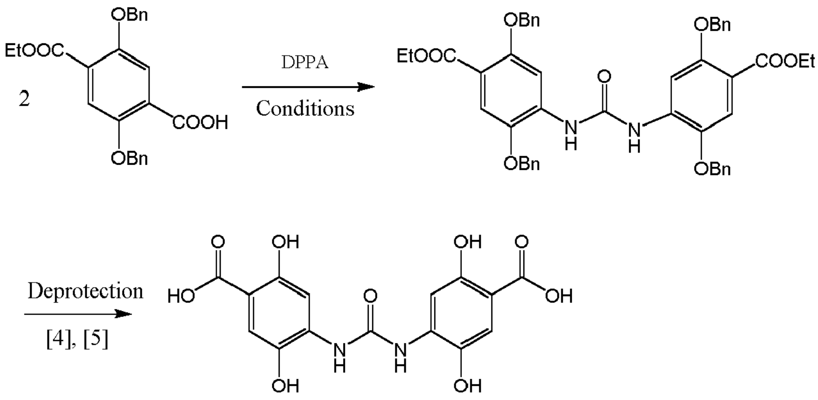


*Formation of urea Linkage*

Procedure from **KI-1**:

**N,N'-Bis(2,5-dibenzyloxy-4-ethoxycarbonylphenyl)urea**. To a cooled solution (ice bath) of 2,5-bis(benzyloxy)-4-(ethoxycarbonyl)benzoic acid (**KI-1**) (2.0 g, 4.9 mmol) and $Et_3N$ (1.6 mL, 11.3 mmol) in THF (25 mL) under inert atmosphere ($N_2$), was slowly added DPPA (1.1 mL, 5.2 mmol). The mixture was slowly warmed up to r.t and stirred at this temperature for 3 h. Then tBuOH (8 mL) was added and the reaction mixture was stirred at r.t for 18 h. The reaction profile showed a majority of urea linkage product instead of the desired Boc-aniline. The mixture was poured into a saturated solution of sodium hydrogencarbonate (250 mL) and stirred for 5 min before being extracted with EtOAc (3 x 50 mL). The combined organic layers were dried over sodium sulfate, filtered, and concentrated under reduced pressure to give a colourless oil. The residue was purified by flash column chromatography (iso-Hexane/EtOAc 1:0 then gradient to 30% EtOAc) to yield the title compound (1.1 g, 56%) as an off-white solid.
UPLC-MS (basic method, 2 min): rt = 1.50 min; m/z = 781.2 $[M+H]^+$, peak area 85%
$^1$H NMR (400 MHz, DMSO-$d_6$) δ 9.49 (s, 2H), 8.18 (s, 2H), 7.58 - 7.45 (m, 8H), 7.43 - 7.35 (m, 8H), 7.35 -7.28 (m, 6H), 5.27 (s, 4H), 5.11 (s, 4H), 4.22 (q, J = 7.1 Hz, 4H), 1.24 (t, J = 7.1 Hz, 6H).

Deprotection procedures: See General procedures [4], [5]

For details see Table 5 (Figure 8)

**Example**

67)**N,N'-Bis(2,5-dihydroxy-4-carboxyphenyl)urea (V-003a)**

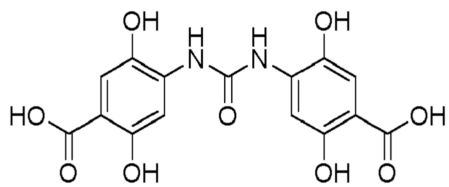

**V-003a**

UPLC-MS (acidic method, 4 min): rt = 0.90 min; m/z = 363.1 $[M-H]^-$, peak area 93%
$^1$H NMR (400 MHz, DMSO-d6) δ 13.36 (s, 2H), 10.86 (s, 2H), 9.64 (s, 2H), 9.48 (s, 2H), 7.77 (s, 2H), 7.17 (s, 2H).

**Diethyl ester**

UPLC-MS (acidic method, 4 min): rt = 1.79 min; m/z = 419.2 $[M-H]^-$, peak area 97%

$^1H$ NMR (400 MHz, DMSO-d6) δ 10.26 (s, 2H), 9.74 (s, 2H), 9.53 (s, 2H), 7.81 (s, 2H), 7.20 (s, 2H), 4.32 (q, J = 7.1 Hz, 4H), 1.33 (t, J = 7.1 Hz, 6H).

**EXAMPLE 2: General Experimental Methods**

NMR spectroscopy

$^1H$ NMR spectra were recorded at 400 MHz on a Bruker Advance III NMR spectrometer. Samples were prepared in deuterated chloroform ($CDCl_3$) or dimethylsulphoxide (DMSO-$d_6$) and the raw data were processed using the **M**nova NMR software. High-resolution mass spectra were recorded with a MaXis ESI qTOF ultrahigh-resolution mass spectrometer

UPLC-MS analysis

UPLC-MS analysis was conducted on a Waters Acquity UPLC system consisting of an Acquity I-Class Sample Manager-FL, Acquity I-Class Binary Solvent Manager and Acquity I-Class UPLC Column Manager. UV detection was achieved using an Acquity I-Class UPLC PDA detector (scanning from 210 - 400 nm), whereas mass detection was achieved using an Acquity QDa detector (mass scanning from 100 - 1250 Da; positive and negative modes simultaneously). A Waters Acquity UPLC BEH C18 column (2.1 × 50 mm, 1.7 mm) was used to achieve the separation of the analytes.

Samples were prepared by dissolving (with or without sonication) into 1 mL of a 1:1 (v/v) mixture of MeCN in $H_2O$. The resulting solutions were filtered through a 0.45 μm syringe filter before being submitted for analysis. All of the solvents (including formic acid and 36% ammonia solution) used were used as the HPLC grade.

Four different analytical methods were used for this work, the details of which are presented below. *Acidic run (2 min):* 0.1% v/v Formic acid in water [Eluent A]; 0.1% v/v Formic acid in MeCN [Eluent B]; Flow rate 0.8mL/min; injection volume 2μL and 1.5 min equilibration time between samples.

Table 14:

| Time (min) | Eluent A (%) | Eluent B (%) |
|---|---|---|
| 0.00 | 95 | 5 |
| 0.25 | 95 | 5 |
| 1.25 | 5 | 95 |
| 1.55 | 5 | 95 |
| 1.65 | 95 | 5 |
| 2.00 | 95 | 5 |

*Acidic run (4 min):* 0.1% v/v formic acid in water [Eluent A]; 0.1% v/v formic acid in MeCN [Eluent B]; Flow rate 0.8mL/min; injection volume 2 μL and 1.5 min equilibration time between samples.

Table 15:

| Time (min) | Eluent A (%) | Eluent B (%) |
|---|---|---|
| 0.00 | 95 | 5 |
| 0.25 | 95 | 5 |
| 2.75 | 5 | 95 |
| 3.25 | 5 | 95 |
| 3.35 | 95 | 5 |
| 4.00 | 95 | 5 |

*Acidic run (6.5 min):* 10 mM ammonium formate + 0.1% v/v formic acid [Eluent A]; 0.1% v/v formic acid in MeCN [Eluent B]; Flow rate 0.6 mL/min; injection volume 2 μL.

Table 16:

| Time (min) | Eluent A (%) | Eluent B (%) |
|---|---|---|
| 0.00 | 95 | 5 |
| 0.50 | 95 | 5 |
| 4.00 | 5 | 95 |
| 4.50 | 5 | 95 |
| 4.52 | 95 | 5 |
| 6.50 | 95 | 5 |

*Basic run (2 min):* 0.1% ammonia in water [Eluent A]; 0.1% ammonia in MeCN [Eluent B]; Flow rate 0.8mL/min; injection volume 2 μL and 1.5 min equilibration time between samples.

Table 17:

| Time (min) | Eluent A (%) | Eluent B (%) |
|---|---|---|
| 0.00 | 95 | 5 |
| 0.25 | 95 | 5 |
| 1.25 | 5 | 95 |
| 1.55 | 5 | 95 |
| 1.65 | 95 | 5 |
| 2.00 | 95 | 5 |

*Basic run (4 min):* 0.1% ammonia in water [Eluent A]; 0.1% ammonia in MeCN [Eluent B]; Flow rate 0.8mL/min; injection volume 2 μL and 1.5 min equilibration time between samples.

Table 18:

| Time (mm) | Eluent A (%) | Eluent B (%) |
|---|---|---|
| 0.00 | 95 | 5 |
| 0.25 | 95 | 5 |
| 2.75 | 5 | 95 |
| 3.25 | 5 | 95 |
| 3.35 | 95 | 5 |
| 4.00 | 95 | 5 |

*Basic run (6.5 min):* 10 mM ammonium bicarbonate + 0.1 % v/v 35% ammonia solution [Eluent A]; 0.1% v/v formic acid in MeCN [Eluent B]; Flow rate 0.6 mL/min; injection volume 2 μL.

Table 19:

| Time (min) | Eluent A (%) | Eluent B (%) |
|---|---|---|
| 0.00 | 95 | 5 |
| 0.50 | 95 | 5 |
| 4.00 | 5 | 95 |
| 4.50 | 5 | 95 |
| 4.52 | 95 | 5 |

(continued)

| Time (min) | Eluent A (%) | Eluent B (%) |
|---|---|---|
| 6.50 | 95 | 5 |

Preparative HPLC purification

Preparative HPLC was performed using Waters autopurification system with XBridge Prep C18 column (19 x 150mm). The Waters system consisted of a Waters 2545 Binary Prep Pump, a Waters 2767 Sample Manager, a Waters SFO Column Organiser, a Waters 2998 DAD, a Waters 515 Make up Pump and an Acquity QDa mass spectrometer

The purification system was controlled by MassLynx software (version 4.2) with Open Access Login module.

Mobile phases consisted of **(A)** 10mM Ammonium Formate (+ 0.1% v/v formic acid) and **(B)** acetonitrile (+ 0.1% v/v formic acid).

Samples were prepared by dissolving in 10% v/v water in DMSO to give a concentration of ~150 mg/mL. A volume of 320 mL of the prepared sample was loaded onto the column and purified using gradient elution at room temperature with a flow rate of 25 mL/min. There is a 1.5 minutes equilibration between injections.

Table 20: Gradient:

| Time (min) | % **(A)** |
|---|---|
| 0.0 | 95 |
| 0.5 | 95 |
| 14.5 | 0 |
| 17.4 | 0 |
| 17.5 | 95 |
| 18.0 | 95 |

**EXAMPLE 3: In Vitro Screening Methods**

**Example 3.1: In vitro HUVEC Cells FGF1, FGF2 and VEGFA inhibition method**

Aim of this in vitro method is to assess compounds effects on FGF-2, FGF-1 or VEGF-A induced sprouting of human umbilical vein endothelial cells (HUVEC) in the spheroid-based cellular angiogenesis assay. Before adding the growth factors to the cells, they were preincubated with the compound. Sunitinib was tested as a control (without preincubation).

Sunitinib control inhibited FGF-2, FGF-1 and VEGF-A induced HUVEC sprouting as expected. IC50 values determined for the tested compounds are expressed in microM [μM] and the values for the inhibition of FGF-1, FGF2 and VEGFA induced growth (sprouting) are listed after each compound in the examples after chemical characterization.

Sprouting test was performed on the compounds dissolved in DMSO to reach 100 fold concentrated stock solutions and dissolved in culture media. Sunitinib as reference compound was provided by ProQinase GmbH. Growth factors were purchased from rhFGF-basic(FGF-2), Peprotech, New Jersey, USA, #100-18C, Lot# 0415571; rhFGF-acidic (FGF-1), Peprotech, New Jersey, USA, #100-17A, Lot# 031207; hVEGF-A165 (ProQinase GmbH, Freiburg, Germany; 05.02.2010) were used for stimulation of HUVEC cells growth.

Test system: Endothelial cells from human umbilical vein (HUVEC) from pooled donors were stored frozen with 70% medium, 20% FCS, 10% DMSO at about 1 x $10^6$ cells/ampoule. HUVEC cells, primary human umbilical vein endothelial cells (PromoCell, Heidelberg, Germany), were used after passage 3 to 4. Morphology of the cells are adherent, cobblestone-like growing as monolayer and were cultured in endothelial cell growth and basal medium (ECGM/ECBM, PromoCell). The subculture was split 1:3; every 3-5 days, seed out at ca. 1 x $10^4$ cells/$cm^2$ and incubated at 37 °C with 5% $CO_2$ with a doubling time 24-48 hours

Dilution of test compounds: 100x concentrated solutions (concerning to the final assay concentrations) of each test compound were prepared with the appropriate solvent (in DMSO or in water). These 100x compound solutions were further diluted in endothelial cell growth and basal medium (ECBM) 1:7.5 to get a 13.33x concentrated solution. 75 μl of the 13,33x solution was subsequently mixed with 25 μl of 40x concentrated stimulus (solved in ECBM), resulting in a 10x concentrated stimulus/compound mixture. This mixture (100 μl) was incubated for 30 min at 37°C and then added

to 900 μl gel, containing the HUVEC spheroids (resulting in the final assay concentration).

Test method: the experiments were pursued in modification of the originally published protocol (Korff and Augustin: J Cell Sci 112: 3249-58, 1999). In brief, spheroids were prepared as described (Korff and Augustin: J Cell Biol 143: 1341-52, 1998) by pipetting 400 HUVEC in a hanging drop on plastic dishes to allow overnight spheroid aggregation. 50 HUVEC spheroids were then seeded in 0.9 ml of a collagen gel and pipetted into individual wells of a 24 well plate to allow polymerization. The test compounds were preincubated with the growth factors (FGF-1, FGF-2 or VEGF-A final concentration 25 ng/mL) in a 10-fold concentrated solution and subsequently, 100 μl of this solution was added on top of the polymerized gel. All compounds were diluted in solvent (DMSO or water) as 100x stocks first (which means 100x stocks of each final assay concentration were prepared in solvent). Subsequently, these stocks were further diluted in medium (resulting in the 13.33x stocks of each final assay concentration) with final concentrations tested: 100, 50, 25, 12.5, 6.25, 3.13 and 1.56 x $10^{-6}$ M). Plates were incubated at 37°C for 24 hours and fixed by adding 4% PFA (Roth, Karlsruhe, Germany).

Quantification of growth factor inhibition: sprouting intensity of HUVEC spheroids treated with the growth factor and the inhibitors were quantitated by an image analysis system determining the cumulative sprout length per spheroid (CSL). Pictures of single spheroids were taken using an inverted microscope and the digital imaging software NIS-Elements BR 3.0 (Nikon). Subsequently, the spheroid pictures were uploaded to the homepage of the company Wimasis for image analysis. The cumulative sprout length of each spheroid was determined using the imaging analysis tool WimSprout. The mean of the cumulative sprout length of 10 randomly selected spheroids was analyzed as an individual data point. For IC50 determination, raw data were converted into percent HUVEC sprouting relative to solvent control (containing the stimulus), which was set to 100% and basal control (without stimulation), which was set to 0%. IC50 values were determined using GraphPad Prism 5 software with constrain of bottom to 0 and top to 100 using a nonlinear regression curve fit with variable hill slope.

Results: The dose response relationship on growth factor induced sprouting of endothelial cells was assessed for all compounds. IC50 values were determined using standard parameters based on the signal of the solvent control as top constraint (100% HUVEC sprouting) and the signal of the basal control as bottom constraint (0%). The respective IC50 values for each compound on FGF1, FGF2 and VEGFA were summarized under each compound below their chemical characterization in paragraph BIODATA.

**Example 3.2: In vitro neutrophil adhesion under static conditions, method**

Human neutrophils obtained from buffy coats of healthy donors were suspended at 3 x $10^6$/ml in adhesion buffer (PBS containing 1 mM CaCl2/MgCl2 and 10% FCS, pH 7.2). Neutrophils were preincubated for 30 min at 37°C with 40 μM and 80 μM of the compounds.

Adhesion assays were performed on 18-well glass slides coated for 18 h at 4°C with human fibrinogen (20 μg/well in endotoxin-free PBS) ; 20 μl of cell suspension were added to each well and stimulated for 1 min at 37°C with 5 μl of fMLP InM final concentration. After washing, adherent cells were fixed in glutaraldehyde 1.5 % in PBS and counted by computer-assisted enumeration. Statistical analysis was performed by calculating mean and standard deviation (SD); significance was calculated by unpaired t-test. All statistical analyses were performed using GraphPad Prism 6 (GraphPad Software).

The respective inhibition of neutrophil adhesion expressed in [%] for each compound were summarized under each compound below their chemical characterization in paragraph BIODATA.

**Example 3.3: In vitro testing on the inhibition of reactive oxygen species (ROS) production in human neutrophils**

NADPH oxidase, located in the plasma membrane and in the membranes of specific granules, produces superoxide anions from which other ROS, such as hydrogen peroxide, singlet oxygen, hypochlorite and active hydroxyl radicals. ROS are released into the surrounding medium or into a membrane-enclosed subcellular organelle. One quick and sensitive method for measuring the generation of these metabolites is chemiluminescence (CL). Isoluminol amplified CL technique, is very sensitive and widely used to study respiratory burst (mainly extracellular production of ROS) induced in neutrophils.

Human neutrophils were isolated from buffy coats which obtained from healthy donors. The isolated neutrophils (3 X $10^6$/ml) were suspended in Hanks' Balanced Salt Solution (HBSS) with Calcium and Magnesium. Neutrophils were pre-incubated with the compounds at different concentrations (0.1, 0.3, 1, 3 and 10 μM) for 30 min at 37°C. The black 96-well cell culture plates were coated with human fibrinogen (0.25 mg/ml in PBS), and incubated overnight at 4°C or 2 hr at 37°C. Then the plates were washed with endotoxin-free PBS. The neutrophils were incubated (priming) with TNF-α (20 ng/ml) for 10 minutes at 37°C. Thereupon the 75 μl of cell suspension and 75 μl (Isoluminol + HRP solution) were added to each well. The chemiluminescence was recorded (every 25 sec for 250 sec) after fMLP (1 μM) activation using a Multilabel Reader victor3 (Perkin Elmer, USA). Background values, defined as the mean chemiluminescent values of

isoluminol diluted in HBSS, were subtracted from all readings (CPS, count per second). The assays were done in duplicate or triplicate.

The results obtained from isoluminol amplified CL assay revealed that compounds strongly reduced ROS level in a concentration-dependent manner in fMLP + TNF-α stimulated PMN

The PMN ROS inhibition at 0.3 μM expressed in [%] and IC50 [μM] were summarized under each compound below their chemical characterization in paragraph BIODATA.

**Example 3.4: In vitro human neutrophil and human monocyte inflammatory cytokine production, method**

Neutrophils and monocytes were isolated from buffy coats of healthy donors, under endotoxin-free conditions. Briefly, buffy coats were stratified on Ficoll-Paque PLUS gradient, and then centrifuged at 400 x g for 30 min at room temperature. Neutrophils were then collected and subjected to dextran sedimentation followed by hypotonic lysis to remove erythrocytes. Monocytes were instead isolated from PBMCs, after centrifugation over Percoll gradients. Human neutrophils and monocytes were suspended at 5x106/ml and 2.5x106/ml, respectively, in RPMI 1640 medium supplemented with 10% low endotoxin FBS (<0.5 EU/ml; from BioWhittaker-Lonza, Basel, Switzerland) and then plated in 24-well tissue culture plates at 37° C, 5% CO2 atmosphere, in the presence or absence of different concentrations (20-40 μM) of the compounds. After 1h of treatment with drugs, monocytes were stimulated with 0.1 μg/ml, whereas neutrophils with 1 μg/ml ultrapure LPS from E. coli 0111:B4 strain (InvivoGen). After 6h of LPS stimulation, neutrophils and monocytes were collected and spun at 300 x g for 5min. Cell-free supernatants were immediately frozen at -80 °C.

Cytokine concentrations in cell-free supernatants were measured by commercially available ELISA kits, specific for human: CXCL8, IL-6, TNF-α (Mabtech, Sweden). Detection limits of these ELISAs were: 4 pg/ml for CXCL8, 10 pg/ml for IL-6 and 4 pg/ml for TNF-α.

Statistical analysis was performed by calculating mean and standard deviation (SD); significance was calculated by unpaired t-test. All statistical analyses were performed using GraphPad Prism 6 (GraphPad Software).

The cytokine values were expressed in ng/ml for each compound and summarized for each compound below their chemical characterization in paragraph BIODATA.

**Example 3.5: In vitro inhibition by compounds of the VEGF-165 induced phosphorylation of VEGF-Receptor on primary human umbilical vein endothelial cells (HUVEC), method**

Cell culture: Primary human umbilical vein endothelial cells (HUVECs) were routinely cultured in 0.1% gelatin pre-coated flasks or dishes, up to passage 6. The effect of compounds alone at different concentrations (0, 1.25, 2.5, 5, 10 and 20 μM) on cellular viability was assessed by CCK-8 kit using a Tecan Microplate Reader (Genios). To measure the effect of compounds on VEGF-induced cell viability, HUVECs ($1 \times 10^4$ cells/well) were treated with VEGF (25 ng/ml) pre-mixed with various concentrations of compounds (0, 1.25, 2.5, 5, 10 and 20 μM) in culture medium without serum and growth factors (starvation medium) for 24 h and 48 h. Compounds in this concentration range did not affect cell viability.

Rabbit primary antibodies for VEGFR-2, p-Tyr1175 were acquired from Cell Signaling Technology (Leiden, The Netherlands). Phospho-VEGFR-2 (Tyr1175) Sandwich ELISA Kit was from Cell Signaling Technology.

HUVECs were pre-incubated with compounds at 0, 0.16, 0.31, 0.63, 1.25, 2.5, 5, 10 and 20 μM for 60 min with three subsequent washing steps with warm medium before stimulation with VEGF165 (25 ng/mL) for 2 min. Western blot analysis was performed using anti-phospho-VEGFR-2 antibody and total VEGFR2 was used as a loading control after membrane stripping.

Inhibition of VEGFR2-phosphorylation results are expressed as IC50 in microM [μM] versus VEGF-treated HUVECs control without inhibitory compound.

The respective IC50 expressed in [μM] values for each compound are summarized under each compound below their chemical characterization in paragraph BIODATA.

**Example 3.6: In vitro cytokine release inhibition by compounds following LPS induction on human whole blood**

Aim of this in vitro method is to assess compounds effects on release of a panel of cytokines from whole blood following lipopolysaccharide (LPS) induced inflammatory reaction. Whole blood was incubated with compounds for 1h prior to LPS induction. Cytokine levels in blood were measured using a multiplex FACS-based panel.

IC50 values determined for the tested compounds expressed in microM [μM] and the values for the inhibition of cytokines were listed for each compound.

Effect on cytokines release after LPS induction was performed on the compounds dissolved in DMSO or $H_2O$ to reach 10-fold concentrated stock solutions and dissolved in culture media.

Test system: Whole blood was removed from a single donor (into lithium heparin coated tubes ; BD Vacutainer ; 367886) and diluted 1:5 with RPMI 1640 (Thermo Fisher, 61870036). Within 30 minutes of sampling, blood was incubated

with compounds for 1h prior to LPS stimulation (O111:B4 - Sigma L4391;10ng/ml final concentration). Stimulation continued under standard cell culture conditions (37 °C with 5% $CO_2$) overnight (18h), after which plates were centrifuged and supernatant removed and frozen at -80°C until used for analyses.

Dilution of test compounds: 10x concentrated solutions (concerning to the final assay concentrations) of each test compound were prepared with the appropriate solvent (in DMSO or in water). These 10x compound solutions (10 μl) were then added to 90 μl pre-diluted whole blood (whole blood: RPMI1640, 1:5) resulting in the final assay concentration.

Test method: Diluted whole blood (80 μl) was pre-incubated with compounds (10 μl) to give final assay concentrations of 100, 30, 10, 3, 1, 0.3 and 0.1 x $10^{-6}$M. Blood was incubated at 37 °C with 5% $CO_2$ for 1h. LPS was diluted in cell culture media to a concentration of 110ng/ml; 10 μl was added to the whole blood to give a final concentration of 10ng/ml. Plates were incubated at 37°C for 18h. Plates were centrifuged at 3,000 RPM for 5 minutes at 4°C and supernatant removed and frozen at - 80°C until FACS analysis was carried out.

Cytokine release was measured using a custom multiplex system (ELISA Genie) according to manufacturers instructions. Prepared samples were run on an Attune NxT flow cytometer (Thermo Fisher).

Quantification of cytokine release inhibition: known concentration standards were run as part of the cytokine panel to allow quantification of each cytokine concentration in each sample. This was carried out using FCAP Array v3.0 software. For IC50 determination, raw data were converted into percent concentration relative to solvent control, which was set to 100%. IC50 values were determined using GraphPad Prism 8 software with constrain of bottom to 0 and top to 100 using a nonlinear regression curve fit with variable hill slope.

Results: The dose response relationship on cytokine release following LPS induction of inflammatory response in whole blood was assessed for all compounds. IC50 values were determined for each compound for a panel of cytokines (GM-CSF, IFN-gamma, IL-1beta, IL-2, IL-4, IL-5, IL-6, IL-8, IL-9, IL-10, IL-12p40, IL-13, IL-17A, IL-17F, IL-18, IL-21, IL-22, IL-33, TGF-beta, TNF-alpha, TNF-beta).

The respective cytokines IC50s expressed in [μM] values for each compound were summarized under each compound below their chemical characterization in paragraph BIODATA.

## EXAMPLE 4: Compound Formulations

### Example 4.1: Oral formulations of compound Ic-007a

Five formulations were developed and reviewed for suitability. These were both suspension and solutions whose details are given in the following Table.

Table 21: Formulation details for the suspension and solutions of compound Ic-007a.

| | **Suspension A** | **Solution D** | **Solution A** | **Solution B** | **Solution C** |
|---|---|---|---|---|---|
| **Component** | Amount (%) | | | | |
| API | 1% w/v (10 mg/mL) | 0.1% w/v (1 mg/mL) | 1% w/v (10 mg/mL) | 1% w/v (10 mg/mL) | 1% w/v (10 mg/mL) |
| DMSO | 5% v/v | 5% v/v | 5% v/v | 5% v/v | 5% v/v |
| PEG 400 | 10% v/v | 10% v/v | 10% v/v | 10% v/v | 10% v/v |
| Solutol HS15 | 10% w/v | 10% w/v | 10% w/v | 10% w/v | 10% w/v |
| Sodium Hydroxide 1M | n/a | n/a | To pH 6 | To pH 8 | To pH 9 |
| Water | to 100% | to 100% | to 100% | to 100% | to 100% |

Manufacture: Suspension A and Solution D:

Method example for 100 mL (scale as appropriate for final volume required). Solvent stock was prepared by measuring 5 mL DMSO into a clean vessel. Correct quantity of compound Ic-007a was weighed out and gradually added to the DMSO solution with mixing on a shaker, vortex or sonicator.

The Solutol was melted in a water bath at 60 °C in a clean dry vessel. As solutol solidified at room temperature, the molten Solutol was kept in a water bath at 60 °C during formulation preparation. 10 g (10% w/v) of molten Solutol was added into a warmed suitable marked (to final volume 100 mL) container. 10 mL PEG 400 was added to the Solutol in the warmed 100 mL container. The DMSO drug stock solution was added into the Solutol and PEG400 mixture and

the contents were instantly mixed, and 35 mL of deionised water was added and mixed in a 40 °C bath with intermittent vortexing to dissolve the large particles. The formulation was cooled to room temperature and made up to final 100 mL volume with deionised water.

Manufacture of Solutions A, B & C:

Method example for 100 mL (scale as appropriate for final volume required). Solvent stock was prepared by measuring 5 mL DMSO into a clean vessel. 1 g of compound Ic-007a was weighed out and gradually added to the DMSO solution with mixing on a shaker, vortex or sonicate if required).

The Solutol was melted in a water bath at 60°C in a clean dry vessel. As Solutol solidifies at room temperature, the molten Solutol was kept in a water bath at 60 °C during formulation preparation. 10 g (10% w/v) of molten Solutol was weighed into a warmed suitable marked (to final volume 100 mL) container. 10 mL PEG 400 was added to the Solutol in the warmed 100 mL container. The DMSO drug stock solution was added into the marked container of Solutol and PEG 400 mixture and instantly mixed to the contents. 35 mL of deionised water was added and mixed to the contents and placed in a 40°C bath with intermittent vortexing to dissolve large particles. The formulation was cooled down to room temperature and further adjusted the pH of the formulation using 1M sodium hydroxide (NaOH) solution to pH 6, 7, 8 and 9. Initial formulations where made on a 1 mL scale and had a starting pH of 4.3. A volume of 3 μL NaOH 1M was added to achieve pH 6 (solution A), 22 μL for pH 8 (Solution B) and 43 μL for pH 9 (Solution C). These formulations were dosed immediately after manufacture.

The 10 mg/mL suspension formulation and the 1 mg/mL solution formulation were found to be physically stable over two weeks at ambient conditions.

**Example 4.2: Oral formulations of compounds Ic-007a and Ia-001a-Tz**

Formulation Development: The solubility of compounds Ic-007a and Ia-001a-Tz was assessed in different GRAS (generally recognized as safe) excipients.

The following Table shows the excipients that were screened.

Table 22: Excipients were screened for oral formulation development of compounds Ic-007a and Ia-001a-Tz

| Excipient | Category | Temperature (°C) |
|---|---|---|
| Tween 80 | Surfactant | 25 |
| Tween 20 | Surfactant | 25 |
| Labrafil M1944 CS | Co-surfactant | 25 |
| Capryol 90 | Co-surfactant | 25 |
| Lauroglycol 90 | Co-surfactant | 25 |
| Transcutol | Solvent | 25 |
| Capmul MCM C8 | Oil | 50 |
| Maisine CC | Oil | 50 |
| Peppermint oil | Oil | 50 |
| Solutol HS15 | Surfactant | 50 |
| Cremophor RH40 | Surfactant | 50 |
| Vitamin E TPGS | Surfactant | 50 |
| Gelucire 44/14 | Surfactant | 50 |

The solubility of compound Ic-007a was < 2.5 mg/mL after 24 hours in all excipients tested and heating of samples to 50 °C showed no improvement. All samples appeared to be yellow cloudy suspensions whereas Ic-007a in Cremophor RH40 and Solutol HS15 appeared to be amber in colour with particles.

Observations for compound Ia-001a-Tz are given in the following Table 23.

Table 23: Excipient screening data obtained for compound Ia-001a-Tz

| Excipient | Category | Temperature (°C) | Estimated solubility (mg/mL) | Comments |
|---|---|---|---|---|
| Capmul MCM C8 | Oil | 50 | < 2.5 | Light yellow, cloudy suspension |
| Maisine CC | Oil | 50 | < 2.5 | Yellow, cloudy suspension with large particles |
| Peppermint oil | Oil | 25 | < 2.5 | Light yellow suspension with very fine particles |
| Solutol HS15 | Surfactant | 50 | ≥ 50 | Yellow solution, dissolved fast (1-2 h per aliquot) |
| Cremophor RH40 | Surfactant | 50 | ≥ 45 | Yellow solution, dissolved slowly (4-5 h per aliquot) |
| Vitamin E TPGS | Surfactant | 50 | ≥ 5 | Yellow solution, dissolved slowly (22-24 h per aliquot) |
| Gelucire 44/14 | Surfactant | 50 | ≥ 10 | Yellow solution, dissolved fast (1-2 h per aliquot) |
| Tween 80 | Surfactant | 25 | ≥ 10 | Yellow solution, dissolved slowly (4-5 h per aliquot) |
| Tween 20 | Surfactant | 25 | ≥ 5 | Yellow solution, dissolved slowly (4-5 h per aliquot) |
| Labrafil M1944 CS | Co-surfactant | 25 | < 2.5 | Light yellow, cloudy suspension with particles |
| Capryol 90 | Co-surfactant | 25 | ≥ 5 | Yellow solution, dissolved when heated at 50 °C |
| Lauroglycol 90 | Co-surfactant | 25 | ≥ 2.5 | Yellow solution, dissolved when heated at 50 °C |
| Transcutol | Solvent | 25 | ≥ 75 | Yellow solution, dissolved very fast (5- 10 min) |
| PEG400 | Solvent | 25 | ≥ 45 | Yellow solution, dissolved fast (1-2 h per aliquot) |
| Propylene glycol | Solvent | 25 | ≥ 5 | Yellow solution, dissolved slowly (4-5 h per aliquot) |

Compound Ic-007a showed low solubility in oils and surfactants (low and high HLB) tested, therefore lipid-based formulations are not possible to develop. As Compound Ic-007a has a pKa of 3.3, basic counter ions such as meglumine, lysine, arginine (pKa 8-9) are investigated for *in situ* salt formation to improve the solubility of the compound. This will be assessed in water initially with further vehicle development to prevent precipitation in simulated fasted and fed state fluids. Alternatively, the current benchmark formulation will also be optimised using higher level of co-solvents and alternate surfactants.

Compound Ia-001a-Tz showed good solubility in co-solvents and surfactants with high HLB. Low solubility was observed in oils and surfactants with low HLB values. This suggested that lipid-based formulations are not possible to develop. Solution formulation trials will be performed using a cosolvent approach with/without pH adjustment approach.

Stock solutions will be prepared in mixture of Transcutol and PEG400 which will then be diluted in aqueous phases comprising surfactants/polymers. In case precipitation is observed, pH adjustment of the aqueous phase will be investigated.

**Example 4.3: Intravenous (IV) formulations**

Compounds Ia-001a, Ia-001a-Tz, Ia-001a-Tz/1-004a, Ic-007a, Ib-010a, IVc-059a, IIIc-061a were prepared as

intravenous formulations.

The formulation details given in the following Table 24 were used for IV administration in the different preclinical in vivo models. Solutions of each compound were manufactured by adjusting the pH with sodium hydroxide solution until a solution was obtained.

Table 24: IV formulation composition

| Component | Amount (%) |
|---|---|
| API | 1 % w/v |
| DMSO | 5 % v/v |
| PEG 400 | 10 % v/v |
| Solutol HS15 | 10 % w/v |
| Sodium Hydroxide 1M | q.s. |
| Saline solution 0.9% | to 100 % |

Method of manufacture:

Method example for 10 mL (scale as appropriate for final volume required). Solvent stock was prepared by measuring 0.5 mL DMSO into a clean vessel. 100 mg of each compound was weighed out and gradually added to the DMSO solution with mixing on a shaker, vortex or sonicate if required).

Solutol was melted in a water bath at 60°C in a clean dry vessel. As Solutol solidifies at room temperature, the molten Solutol was kept in a water bath at 60 °C during formulation preparation. 1 g (10% w/v) of molten Solutol was weighed into a warmed suitable marked (to final volume 10 mL) container. 1 mL PEG 400 was added to the Solutol in the warmed 10 mL container. The DMSO drug stock solution was added into the marked container of Solutol and PEG 400 mixture and instantly mixed to the contents. An 0.9% saline solution* was added to achieve 85% of target volume and mixed the contents in a 40 °C bath with intermittent vortexing to dissolve large particles. The formulation was cooled down to room temperature, made up to final target volume (10 mL) with 0.9% saline solution* and pH of the formulation adjusted using 1M sodium hydroxide (NaOH) solution to pH 7.4. The formulations were filtered using a 0.2 μm filter into a sterile vial inside a Class II biological hood. Formulations were used for dosing in animals.

**[0347]** * For some compounds, 0.9% saline solution was substituted for pH 7.4 PBS solution

**Example 4.4: Ocular formulations**

Several compounds according to the present invention were submitted for formulation development into an ocular format. The compounds, their final concentration and form are given in the following Table 25.

Table 25: Ocular formulations

| Compounds | Concentration (% w/v) | Suspension/solution |
|---|---|---|
| IVc-059a | 5 mg/mL (0.5) | Solution |
| Ia-001a-Tz/1-004a | 5 mg/mL (0.5) | Solution |
| Ia-001a-Tz | 10 mg/mL (1.0) | Solution |
| IIIc-061a | 10 mg/mL (1.0) | Solution |
| Ia-001a | 10 mg/mL (1.0) | Solution |
| Ic-007a | 5 mg/mL (0.5) | Suspension |
| Ib-010a | 10 mg/mL (1.0) | Suspension |

Composition: The formulation composition is detailed in the following Table 26. The concentration of the APIs and their resulting format varied.

Table 26: Ocular formulation composition.

| Component | Amount (%) |
|---|---|
| API | See Tables above |
| Kolliphor EL | 5 % w/v |
| Povidone | 0.5 % w/v |
| HPMC | 0.5 % w/v |
| Polysorbate 80 | 0.1 % v/v |
| pH 7.4 PBS buffer (adjustment of pH with NaOH 1M) | To volume |

Method of manufacture:

For those compounds that created solutions, the following method of manufacture was followed:
A total of 50 mg of Kolliphor EL was weight out into a clean dry vessel. The correct amount of compound was weighed out and gradually added to the Kolliphor EL whilst mixing on a magnetic stirrer. Heating to 30 °C helped to mix the drug into the Kolliphor preparation. In a separate container, an aqueous solution of HPMC at 0.5% w/v, povidone at 0.5% w/v and polysorbate 80 at 0.1% in pH 7.4 Phosphate-buffered saline (PBS) buffer was prepared. A volume of 600 μL of the aqueous solution was added to the Kolliphor EL and drug whilst mixing and was vortexed immediately. The pH was adjusted to 7.4 using 1 M NaOH in the formulation while mixing. Volume was adjusted to target volume using the aqueous solution. The formulation was filtered using a 0.2 μm filter into a sterile vial inside a Class II biological hood. Formulations are ready for dosing.

For the compounds that created suspensions, the following method of manufacture was followed: An amount of 50 mg of Kolliphor EL was weighed into a clean dry vessel. The correct amount of the different compounds was weighed and gradually added to the Kolliphor EL whilst mixing on a magnetic stirrer. Heating to 30 °C helped to mix the drug into the Kolliphor. In a separate container, an aqueous solution of HPMC at 0.5% w/v, povidone at 0.5% w/v and polysorbate 80 at 0.1% in pH 7.4 Phosphate-buffered saline (PBS) buffer was prepared. The aqueous solution was filtered using a 0.22 μm filter. A volume of 600 μL of the aqueous solution was added to the Kolliphor and drug whilst mixing, and then vortex immediately. The pH was adjusted to 7.4 using filtered 1 M NaOH with continuous mixing the formulation. Volume was adjusted to target volume using the aqueous solution. The formulations were sonicated prior to dosing to allow flocculates to break up and disperse.

Ocular formulation of compounds Ic-007a, IIc-007a

Sitagliptin (as sitagliptin phosphate monohydrate) was added to formulations containing both APIs and the details are given in the following Table 27.

Table 27: Composition of each ocular formulation.

| | **1** | **2** | **3** | **4** |
|---|---|---|---|---|
| **Components** | Amount (%) | | | |
| Ic-007a | 0.5 %w/v (5 mg/mL) | 0.5 %w/v (5 mg/mL) | - | - |
| IIc-007a | - | - | 0.5 %w/v (5 mg/mL) | 0.5 %w/v (5 mg/mL) |
| Sitagliptin# | - | 0.5 %w/v (5 mg/mL) | - | 0.5 %w/v (5 mg/mL) |
| Kolliphor EL | 5 % w/v | 5 % w/v | 5 % w/v | 5 % w/v |
| Povidone | 0.5 % w/v | 0.5 % w/v | 0.5 % w/v | 0.5 % w/v |
| HPMC | 0.5 % w/v | 0.5 % w/v | 0.5 % w/v | 0.5 % w/v |
| Polysorbate 80 | 0.1 % v/v | 0.1 % v/v | 0.1 % v/v | 0.1 % v/v |

(continued)

| | 1 | 2 | 3 | 4 |
|---|---|---|---|---|
| **Components** | Amount (%) | | | |
| pH 7.4 PBS buffer (adjustment of pH with NaOH 0.5M) | To volume | To volume | To volume | To volume |
| #6.7 mg Sitagliptin phosphate monohydrate is equivalent to 5.0 mg sitagliptin | | | | |

Method of manufacture:

A total of 50 mg of Kolliphor EL was weight out into a clean dry vessel. The correct amount of compound was weighed out and gradually added to the Kolliphor EL whilst mixing on a magnetic stirrer. Heating to 30 °C helped to mix the drug into the Kolliphor preparation. In a separate container, an aqueous solution of HPMC at 0.5% w/v, povidone at 0.5% w/v and polysorbate 80 at 0.1% in pH 7.4 Phosphate-buffered saline (PBS) buffer was prepared. A volume of 600 μL of the aqueous solution was added to the Kolliphor EL and drug whilst mixing and was vortexed immediately. The pH was adjusted to 7.4 using 1 M NaOH in the formulation while mixing. Volume was adjusted to target volume using the aqueous solution. The formulation was filtered using a 0.2 μm filter into a sterile vial inside a Class II biological hood.

Solution of compound IIc-007a: A total of 50 mg of Kolliphor EL was weight out into a clean dry vessel. The correct amount of compound (target concentration 5 mg/mL) was weighed out and gradually added to the Kolliphor EL whilst mixing on a magnetic stirrer. Heating to 30 °C helped to mix the drug into the Kolliphor preparation. In a separate container, an aqueous solution of HPMC at 0.5% w/v, povidone at 0.5% w/v and polysorbate 80 at 0.1% in pH 7.4 Phosphate-buffered saline (PBS) buffer was prepared. If required add an appropriate quantity of sitagliptin phosphate monohydrate to allow for the final composition of the formulation to be 5 mg/mL sitagliptin. A volume of 600 μL of the aqueous solution was added to the Kolliphor EL and drug whilst mixing and was vortexed immediately. The pH was adjusted to 7.4 using 0.5 M NaOH in the formulation while mixing. Volume was adjusted to target volume using the aqueous solution. The formulation was filtered using a 0.2 μm filter into a sterile vial inside a Class II biological hood. Formulations are ready for dosing. Prior to use, vortex compound IIc-007a solutions for 2 minutes and use within 10 minutes.

*appearance of gel/colloidal structure may be observed but is re-dispersed after vortexing.

Suspension of compound Ic-007a: A total of 50 mg of Kolliphor EL was weight out into a clean dry vessel. The correct amount of compound (target concentration 5 mg/mL) was weighed out and gradually added to the Kolliphor EL whilst mixing on a magnetic stirrer. Heating to 30 °C helped to mix the drug into the Kolliphor preparation. In a separate container, an aqueous solution of HPMC at 0.5% w/v, povidone at 0.5% w/v and polysorbate 80 at 0.1% in pH 7.4 Phosphate-buffered saline (PBS) buffer was prepared.

If required add an appropriate quantity of sitagliptin phosphate monohydrate to allow for the final composition of the formulation to be 5 mg/mL sitagliptin. The aqueous solution was filtered using a 0.22 μm filter. A volume of 600 μL of the aqueous solution was added to the Kolliphor EL and drug whilst mixing and was vortexed immediately. The pH was adjusted to 7.4 using 0.5 M NaOH in the formulation while mixing. Volume was adjusted to target volume using the aqueous solution.

Prior to use, suspension of compound Ic-007a was sonicated to break the flocculate/aggregates using the following protocol: a small petri dish/beaker was filled with water inside a sonicator. The vial was put inside the beaker/petri dish and sonicated for 10 minutes at 40°C. After 10 minutes the vial was removed and vortexed for 2 minutes and sonicated again for 10 minutes. The process was repeated for total of 50 minutes.

Stability: Two samples (one for each compound) of the formulation given in Table 8 were manufactured and stored at 5°C and 25°C for 7 days. These samples also contained sitagliptin. 0.1M NaOH was used to adjust the pH rather than 0.5M for this trial. The volume of NaOH required to adjust the pH to 7.4 meant the percentage composition was changed from that given in the following Table.

Table 28: Formulation composition for stability purposes.

| **Formulation Composition** | **Concentration** |
|---|---|
| 1/ 2 | 0.5% (5mg/g) |
| Cremophor EL | 5% |

(continued)

| Formulation Composition | | Concentration |
|---|---|---|
| Aqueous phase (PBS, pH 7.4) | Sitagliptin | 0.36% (3.6 mg/g) |
| | Povidone (PVP) | 0.36% |
| | Tween 80 | 0.07% |
| | Hydroxypropyl methyl cellulose (HPMC) 40-60 cP | 0.36% |
| 0.1 M sodium hydroxide | | ~ 23% |

Assays of each API plus sitagliptin were performed along with pH measurements. In addition, for compound Ic-007a in suspension, the amount of compound Ic-007a dissolved was also quantified. The following Table provides data on suspension of compoundIc-007a:

Table 29: Stability of compound Ic-007a suspension of the formulation as described in the above table.

| Time point | Storage condition | Sampling | Assay of 1 suspension (%) at 254 nm | % RSD | Peak purity of 1 (%) at 254 nm | % of 1 dissolved | Assay of Sitagliptin (%) at 268 nm | Peak purity of Sitagliptin (%) at 268 nm | pH |
|---|---|---|---|---|---|---|---|---|---|
| t-0 | - | Top | 123.57±6.61 | 5.16 | 94.7 | 73.94 | 100.04±1.33 | 100 | 7.23 |
| | | Bottom | 114.87±1.93 | | 94.3 | | | 100 | |
| t=24 h | 5 °C | Top | 110.67±0.54 | 3.21 | 93.1 | 48.4 | 95.77±3.15 | 100 | 7.13 |
| | | Bottom | 105.44±2.89 | | 93.4 | | | 100 | |
| | 25 °C | Top | 102.89±0.45 | 3.58 | 93.9 | 46.39 | 98.82±3.07 | 100 | 7.05 |
| | | Bottom | 97.00±1.90 | | 93.4 | | | 100 | |
| t=7 days | 5 °C | Top | 91.26±0.76 | 1.40 | 90.2 | 19.34 | 88.71±1.71 | 100 | 7.02 |
| | | Bottom | 89.39±0.37 | | 89.9 | | | 100 | |
| | 25 °C | Top | 98.28±0.69 | 3.28 | 91.5 | 27.65 | 93.64±1.01 | 100 | 7.04 |
| | | Bottom | 93.49±2.49 | | 91.0 | | | 100 | |

The following Table provides data on suspension of compound IIc-007a. Where the table refers to peak purity, this is the area percent with respect to any related peaks.

Table 30: Stability of compound IIc-007a solution of the formulation as described in the above formulation Table

| Time point | Storage condition | Assay of 2 solution (%) at 254 nm | Peak purity of 2 (%) at 254 nm | Assay of Sitagliptin (%) at 268 nm | Peak purity of Sitagliptin (%) at 268 nm | pH |
|---|---|---|---|---|---|---|
| t-0 | - | 100.62±2.00 | 96.70 | 97.68±1.60 | 100 | 7.43 |
| t=24 h | 5 °C | 100.41±1.86 | 95.09 | 97.01±1.45 | 100 | 7.38 |
| | 25 °C | 100.63±1.06 | 95.11 | 96.44±0.93 | 100 | 7.36 |
| t=7 days | 5 °C | 92.40±0.71 | 93.2 | 84.92±0.64 | 100 | 7.27 |
| | 25 °C | 86.72±0.76 | 90.5 | 74.14±0.23 | 100 | 7.24 |

**Example 4.5: Formulations of compound Ia-001a-Tz**

Protocol for the preparation of 10 mg/g solution of Ia-001a-Tz

Table 31: Details of two formulations for oral administration.

| Formulation | API concentration |
|---|---|
| 40% PEG400, 10% Transcutol, 10% Solutol HS15 and 40% aqueous solution containing 0.5% Kollidon VA64 | 10 mg/g |
| 40% PEG400, 10% Transcutol, 10% Solutol HS15 and 40% aqueous solution containing 0.5% HPMC 606 | 10 mg/g |

Table 32: Excipients used

| Excipients | Supplier |
|---|---|
| Type 1 water | Millipore Milli-Q |
| PEG400 | Sigma Aldrich |
| Transuctol | Gattefosse |
| Solutol HS 15 | Sigma Aldrich |
| HPMC 606 | ShinEtsu |
| Kollidon VA64 | BASF |

Material required: Magnetic stirrer, Magnetic stirring bar, 100 mL volumetric flask, 10 mL glass vial, 4 mL glass vial, Pipette

Preparation of vehicles

Preparation of Kollidon VA64 solution (0.5 % w/v) - 100 mL scale

- In a 100 mL volumetric flask accurately weigh 0.5 g of Kollidon VA64.
- Add approximately 80 mL type 1 water into the volumetric flask and stir/sonicate until a clear solution is obtained.
- Once dissolved, adjust the volume to 100 mL using type 1 water.
- Stir or mix the volumetric flask to obtain a homogenous solution.

Preparation of HPMC 606 solution (0.5 % w/v) - 100 mL scale

- In a 100 mL volumetric flask accurately weigh 0.5 g of HPMC 606.

- Add approximately 80 mL type 1 water into the volumetric flask and stir/sonicate until a clear solution is obtained.
- Once dissolved, adjust the volume to 100 mL using type 1 water.
- Stir or mix the volumetric flask to obtain a homogenous solution.

Preparation of PEG400 and Transcutol vehicle (4:1)

- In a 10 mL vial, 4 mL of PEG400 is added.
- To this, 1 mL of Transcutol is added.
- The solution is stirred to obtain a homogeneous solution

Preparation of Ia-001aTz formulation

Preparation of stock solution at 20 mg/g.

- In a 4 mL vial, 20 mg of API was accurately weighed.
- To this, ca. 980 mg of mixture of PEG400: Transcutol (4:1) (prepared above) is added
- The vial is stirred at room temperature (20 - 25 °C) using a magnetic stirrer for ca. 30 minutes.
- A bright yellow solution is obtained.

Preparation of Formulation at 10 mg/g.

- In a 4 mL vial, 500 mg of the stock solution prepared above is added.
- To this, 100 mg of Solutol HS15 (pre-heated at 50 °C) is added.
- The vial is stirred for 5-10 minutes and then 400 mg of aqueous solution (prepared in section 2.1 for either) containing polymer is added.
- The vial is stirred for 5 minutes to obtain a homogenous solution. A yellow solution is obtained
- The solution is physically stable for at least 24 h upon storage at room temperature.

**EXAMPLE 5: Therapeutic activity of the compounds**

**Example 5.1: Treatment and/or prevention of acute pancreatis**

The efficacy of the compounds according to the present invention were assessed on an acute pancreatitis murine model. Tissue samples were obtained to allow post in-life analysis of pancreatitis parameters. Plasma samples were also obtained to assess animal exposure levels after administration of the compounds.

Animals: A total of 70 female Balb/c mice aged 8-10 weeks weighing approximately 20-25g were used for the study (Charles River). After 7 days acclimatization, they were allocated into the different groups. Mice were housed in IVC cages (up to 5 mice per cage) with individual mice identified by tail mark. Cages, bedding and water were sanitized before use. Animals were provided with Corn-o-cobs enrichment bedding to provide environment enrichment and nesting material. All animals had free access to a standard certified commercial diet and water. The animal holding room were maintained as follows - room temperature at 20-24°C, humidity at 30-70% and a 12h light/dark cycle used. Although animals used in this study are immuno-competent, preparation of dosing solutions and dosing/weighing of animals were carried out in a sterile biosafety cabinet.

Test Substance and Formulation: in a first experiment, compounds Ia-001a, Ia-001aTz, Ic-001aTz/004a, Ic-007a, Ib-010a, IIIc-061a, IVb-059a were weighed and solubilized in DMSO. Final formulation was in 5% DMSO, 10% Solutol, 10% PEG400, 75% 0.9% Saline and pH adjusted to 7 with 0.5M NaOH solution.

In a second experiment, compounds Ic-001aTz2, IIc-007a, IIIa-001aTz, IIIc-061a, IIIc-061a-E3, IVb-059a were weighed and solubilized in DMSO. Final formulation is in 5% DMSO, 10% Solutol, 10% PEG400, 75% 0.9% Saline and pH adjusted to 7 with 0.5M NaOH solution. For per os (PO) dosing IVb-059a is formulated in water. Drug solutions were formulated on the day of dosing.

The purposes of this study was to assess efficacy compounds in an acute pancreatitis model (14 days), generate tissue samples to allow post in-life analysis of pancreatitis parameters and generate four plasma samples to assess animal exposure levels after first and last injection obtained from blood samples taken 1h and 24h after first and last injection.

Study Design: On the first study day animals were randomly be assigned to treatment groups, ensuring an equal spread of bodyweight. Mice (except negative control group) were treated with test compounds were given 5 minutes

prior to caerulein dosing, followed by injection with caerulein at 2μg/kg via intraperitoneal route (IP) daily during the study.

Table 33: First experiment

| Group | No. Animals | Agent | Dose level; Route |
|---|---|---|---|
| 1 | 3 | Negative control (non-induced) | Vehicle (tbc); IV |
| 2 | 3 | Negative control (Induced) | Vehicle (tbc); IV |
| 3 | 8 | Pirfenidone | 40mg/kg; IV |
| 4 | 8 | Ia-001a | 15mg/kg; IV |
| 5 | 8 | Ia-001aTz | 15mg/kg; IV |
| 6 | 8 | Ic-001aTz/004a | 15mg/kg; IV |
| 7 | 8 | Ic-007a | 15mg/kg; IV |
| 8 | 8 | Ib-010a | 150mg/kg; PO |
| 9 | 8 | IIIc-061a | 15mg/kg; IV |
| 10 | 8 | IVb-059a | 150mg/kg; PO |

Table 33 : Second experiment

| Group | No. Animals | Compounds | Dose level; Route |
|---|---|---|---|
| 1 | 3 | Negative control (non-induced) | Vehicle (tbc); IV |
| 2 | 3 | Negative control (Induced) | Vehicle (tbc); IV |
| 3 | 8 | Pirfenidone | 40mg/kg; IV |
| 4 | 8 | Ic-001aTz2 | 15mg/kg; IV |
| 5 | 8 | IIc-007a | 15mg/kg; IV |
| 6 | 8 | IIIa-001aTz | 15mg/kg; IV |
| 7 | 8 | IIIc-061a | 15mg/kg; IV |
| 8 | 8 | IIIc-061a-E3 | 150mg/kg; PO |
| 9 | 8 | IVb-059a | 15mg/kg; IV |
| 10 | 8 | IVb-059a | 150mg/kg; PO |

Treatment of test compounds were given 5 minutes prior to caerulein dosing.

The study schedule is detailed below in Table 34:

| Time (days) | T0 | T1 | T2 | T3 | T4 | T5 | T6 | T7 | T8 | T9 | T10 | T11 | T12 | T13 | T14 |
|---|---|---|---|---|---|---|---|---|---|---|---|---|---|---|---|
| Compound I.V. | Dose | Dose | Dose | Dose | Dose | Dose | Dose | Dose | Dose | Dose | Dose | Dose | Dose | Dose | Sacrifice |
| Caerulein I.P. | 1st | 2nd | 3rd | 4th | 5th | 6th | 7th | 8th | 9th | 10th | 11th | 12th | 13th | 14th | |
| Blood sampling for PK | T0+1h T0+24h | | | | | | | | | | | | | T12+24h T13+1h | |
| Organ analysis | | | | | | | | | | | | | | | Pancreas |

Serial observations

Bodyweight: the bodyweight of all mice on the study were measured and recorded daily; this information was used to calculate precise dosing for each animal. The Sponsor were informed if any animal loses more than 10% bodyweight and a dosing holiday may be considered as needed.

General signs and symptoms: mice were observed daily and any signs of distress or changes to general condition e.g. starred fur, lack of movement, difficulty breathing were noted.

Sampling and post in-life analyses: at times indicated above a whole blood sample (60μl) were taken from the lateral tail vein into tubes coated with K2-EDTA. Plasma samples were prepared and stored frozen at -20°C. Plasma samples were sent to client bioanalysis provider Eurofins (FR) for quantification of compound.

Terminal Sampling: Prior to termination animals were weighed. Terminal study animals were culled via CO2 inhalation. A terminal blood sample was taken via cardiac puncture and plasma prepared.

Lipase and Amylase activity were measured via ELISA. Remaining plasma was stored for future cytokine analyses. Pancreas tissue was resected and weighed and a section (50μg) snap frozen and analysed for quantification of compound in pancreas sample. A section (50μg) was processed for measurement of MPO activity, IL-33 and TGF-B levels via ELISA. The remainder was fixed in formalin and embedded in paraffin wax. H&E staining was carried out and section assessed using Schmidts standard scoring system, examining: edema, inflammatory infiltration, parenchymal necrosis, haemorrhage. Massons' Trichrome staining was carried out and area covered by fibrotic tissue quantified digitally using QuPath software. Terminal serum samples was used to assess blood chemistry using an IdeXX CHEM15 and LYTE4 clip (4 animals per treatment group).

Acute Pancreatitis Results after 15 days IV treatment

Histopathology assessment of pancreatic injury based on 4 criteria: Oedema, Inflammatory infiltration, Parenchymal necrosis, Haemorrhage and scored as described below.

Table 35:

| **Item** | **Score Levels** | | | |
|---|---|---|---|---|
| | 0 | 1 | 2 | 3 |
| Interstitial oedema | None | Interlobular | Lobule involved | Isolated island like acinar cells |
| Leukocyte infiltration | None | < 20% | 20% - 50% | > 50% |
| Acinar cell necrosis | None | < 5% | 5% - 20% | > 20% |
| Haemorrhage | None | 1-2 points | 3-5 points | > 5 points |

Table 36: Results score levels for animal groups

| | Scoring summary | | | |
|---|---|---|---|---|
| | Interstitial oedema | Leukocyte infiltration | Acinar cell necrosis | Haemorrhage |
| Score | 1 | 2 | 3 | **4** |
| Negative control (non-induced) | 0-0-0 | 0-0-0 | 0-0-0 | 0-0-0 |
| Negative control (caerulein Induced) | 2-3-3 | 3-3-3 | 2-2-3 | 2-2-2 |
| Ia-001a (caerulein Induced) | 1-1-1-1-1-2-2 | 3-3-3-3-3-3-3 | 0-1-1-1-1-1-1 | 1-1-1-1-1-1-2 |
| Ia-001aTz (caerulein Induced) | 1-1-1-1-2-2-2-2 | 2-3-3-3-3-3-3-3 | 0-0-0-0-1-1-1-1 | 0-1-1-1-1-1-1-1 |
| Ic-001aTz/004a (caerulein Induced) | 1-1-1-1-2-2-2-3 | 1-3-3-3-3-3-3-3 | 1-1-1-1-2-2-2-2 | 1-1-1-1-1-1-1-1 |
| Ic-007a (caerulein Induced) | 1-1-1-1-1-1-1-1 | 3-3-3-3-3-3-3-3 | 1-1-1-1-1-1-1-1 | 1-1-1-1-1-1-1-1 |

(continued)

| | Scoring summary | | | |
|---|---|---|---|---|
| | Interstitial oedema | Leukocyte infiltration | Acinar cell necrosis | Haemorrhage |
| Ib-010a | 1-1-1-1-2-2-2-2 | 3-3-3-3-3-3-3-3 | 1-1-1-1-1-1-1-1 | 0-0-1-1-1-1-1-1 |
| (caerulein Induced) | | | | |
| IIIc-061a (caerulein Induced) | 1-1-1-1-1-1-1-1 | 1-1-1-1-1-1-1-1 | 0-0-0-0-1-1-1-1 | 1-1-1-1-1-1-1-1 |
| IVb-059a (caerulein Induced) | 1-1-1-1-1-1-1-1 | 1-1-1-1-1-1-1-1 | 0-0-1-1-1-1-2-2 | 1-1-1-1-1-1-1-1 |
| Pirfenidone (caerulein Induced) | 1-1-1-1-1-1-1-1 | 2-2-2-2-2-2-2-2 | 0-0-1-1-1-1-1-1 | 0-0-0-0-1-1-1-1 |

Schmidt Score

Schmidt score is the evaluation of the efficacy of the compounds based on the four histopathology criteria as described in the table below. Order of compounds with increasing Schmidt score (highest score = 10 is caerulein induced + vehicle treatment and lowest score = 0 is non-induced animals). Graph of Schmidt score for the compounds in Figure 2A were all products show improved Schmidts score compared to caerulein control animals.

Table 37: Schmidt Score and four parameters table

| Test compound | Interstitial oedema | Leukocyte infiltration | Acinar cell necrosis | Haemorrhage | Schmidt Score (H&E) | |
|---|---|---|---|---|---|---|
| Non-induced + vehicle | 0 | 0 | 0 | 0 | **0** | Intact apical boarder<br>Healthy acini with abundance of secretory granules<br>No signs of necrosis<br>No signs of oedema<br>No signs of haemorrhage |
| Induced + IIIc-061a | 1.0 | 1.0 | 0.5 | 1.0 | **3.5** | Mild oedema observed<br>Mild haemorrhage observed<br>Mild acinar cell necrosis observed<br>Mild leukocyte infiltration observed<br>Normal islet morphology observed<br>Unlike other treatment groups, areas had relatively normal overall pathology however mild oedema was observed |
| Induced<br><br>+ IVb-059a | 1.0 | 1.0 | 1.0 | 1.0 | **4.0** | Mild oedema less observed<br>Mild haemorrhage less observed<br>Mild acinar cell necrosis observed<br>Mild leukocyte infiltration observed<br>Some pancreatic islet hyperplasia observed |

(continued)

| Test compound | Interstitial oedema | Leukocyte infiltration | Acinar cell necrosis | Haemorrhage | Schmidt Score (H&E) | |
|---|---|---|---|---|---|---|
| | | | | | | Disruption to the basophilic region |
| Induced<br><br>+ Pirfenidone | 1.0 | 2.0 | 0.8 | 0.4 | **4.1** | Mild oedema observed<br>Mild haemorrhage observed<br>Mild acinar cell necrosis observed<br>Leukocyte infiltration observed<br>Normal islet morphology observed in areas |
| Induced<br><br>+ Ib-001aTz | 1.5 | 2.9 | 0.6 | 0.9 | **5.9** | Oedema observed throughout pancreas<br>Mild haemorrhage observed<br>Mild acinar cell necrosis observed<br>Leukocyte infiltration throughout<br>Abnormal islet morphology observed (trace)<br>Disruption to the basophilic region |
| Induced<br>+ Ic-007a | 1.0 | 3.0 | 1.0 | 1.0 | **6.0** | Mild oedema observed<br>Mild haemorrhage observed<br>Mild acinar cell necrosis observed<br>Leukocyte infiltration observed<br>Disruption to the basophilic region |
| Induced<br><br>+ Ib-010a | 1.5 | 3.0 | 1.0 | 0.8 | **6.3** | Oedema observed throughout pancreas<br>and within acini cells<br>Mild haemorrhage observed<br>Mild acinar cell necrosis observed<br>Leukocyte infiltration observed<br>Disruption to the basophilic region |
| Induced<br><br>+ Ia-001a | 1.3 | 3.0 | 0.9 | 1.1 | **6.3** | Oedema observed throughout pancreas<br>(black arrow)<br>Haemorrhage observed<br>Mild acinar cell less necrosis observed (Blue square)<br>Leukocyte infiltration throughout<br>Abnormal islet morphology observed<br>Disruption to the basophilic region |

(continued)

| Test compound | Interstitial oedema | Leukocyte infiltration | Acinar cell necrosis | Haemorrhage | Schmidt Score (H&E) | |
|---|---|---|---|---|---|---|
| Induced<br><br>+ Ic-001aTz/004a | 1.6 | 2.8 | 1.5 | 1.0 | **6.9** | Oedema observed throughout pancreas<br>Mild haemorrhage observed<br>Acinar cell necrosis observed<br>Leukocyte infiltration throughout<br>Disruption to the basophilic region |
| Induced<br><br>+ vehicle | 2.7 | 3.0 | 2.3 | 2.0 | **10.0** | Oedema observed throughout pancreas<br>Haemorrhage observed, particularly<br>within the pancreas head<br>Acinar cell necrosis observed<br>Leukocyte infiltration throughout<br>Disruption to the basophilic region |

As showed in the Tables... below, all compounds Ia-001a, Ia-001aTz, Ic-001aTz/004a, Ic-007a, Ib-010a, IIIc-061a, IVb-059a showed a significant decrease in pancreatic tissue levels IL33 compared to the "induced" group. TGF-B levels were decreased significantly for Ic-007a, Ib-010a, IIIc-061a, IVb-059a. Global Schmidt scores especially improved for all compounds but especially for compounds IIIc-061a, IVb-059a.

Tables 38 and 39 below show terminal Pancreas concentrations of MPO in pancreatic tissue sample, IL33 tisse concentrations (Figure 2B)and TGF-B concentrations (Figure 2C).

Table 38:

| Test Compounds | Mean terminal BW (% of initial) | Mean terminal pancreas weight (g) | Pancreas MPO activity | Pancreas IL33 level | P value versus induced | Pancreas TGF-B level | P value versus induced |
|---|---|---|---|---|---|---|---|
| Vehicle | 93.8 | 0.148 | 2.81 | 76 ± 19 | --- | 184 ± 107 | --- |
| Ia-001a | 90.3 | 0.083 | 3.76 | 330 ± 162 | 0.017 | 592 ± 358 | 0.984 |
| Ia-001aTz | 89.1 | 0.084 | 2.68 | 154 ± 49 | 0.000 | 507 ± 289 | 0.663 |
| Ic-001aTz/004a | 93.5 | 0.104 | 2.15 | 180 ± 54 | 0.000 | 369 ± 195 | 0.113 |
| Ic-007a | 91.2 | 0.109 | 1.65 | 147 ± 47 | 0.000 | 188 ± 77 | 0.000 |
| Ib-010a | 91 | 0.091 | 1.83 | 157 ± 70 | 0.000 | 219 ± 140 | 0.00035 |
| IIIc-061a | 91.4 | 0.086 | 2.13 | 177 ± 80 | 0.000 | 295 ± 146 | 0.015 |
| IVb-059a | 93.3 | 0.09 | 2.88 | 86 ± 23 | 0.000 | 231 ± 199 | 0.020 |
| Pirfenidone | 97.6 | 0.136 | 1.26 | 78 ± 23 | 0.000 | 289 ± 170 | 0.024 |
| Induced | 93.8 | 0.148 | 2.81 | 628 ± 57 | 0.000 | 592 ± 358 | 0.015 |

Table 39:

| Test Compounds | Serum amylase level | Serum lipase level | Serum IL33 level | Serum TGF-B level | Saline/PBS for formulation | Schmidt Score (H&E) |
|---|---|---|---|---|---|---|
| Vehicle | 43.69 | 11.66 | 37.22 | 10.88 | - | 1.0 |
| Ia-001a | 34.07 | 6.72 | 39.52 | 8.69 | Saline | 6.3 |
| Ia-001aTz | 35.35 | 8.57 | 37.02 | 8.69 | Saline | 5.9 |
| Ic-001aTz/ 004a | 36.51 | 8.21 | 35.43 | 8.99 | PBS | 6.9 |
| Ic-007a | 34.86 | 9.28 | 34.93 | 6.17 | PBS | 6.0 |
| Ib-010a | 37.46 | 9.06 | 35.36 | 6.87 | PBS | 6.3 |
| IIIc-061a | 38.38 | 9.79 | 33.72 | 4.65 | Saline | 3.5 |
| IVb-059a | 38.56 | 10.21 | 46.54 | 5.69 | Saline | 4.0 |
| Pirfenidone | 37.71 | 7.65 | 39.16 | 5.5 | - | 4.1 |
| Induced | 43.69 | 11.66 | 37.22 | 10.88 | | 10.0 |

**EXAMPLE 5.2: Treatment and/or prevention of Pancreatic and Kidney cancer:**

The objective of this study was to evaluate preclinically the in vivo therapeutic efficacy study of compounds in the treatment of subcutaneously murine pancreatic cancer syngenic model (Pan02) in female C57BL/6 mice and kidney cancer syngenic model (Renca) in female BALB/c mice.

Animals: C57BL/6 mouse females (For Pan02 model); BALB/c mouse females (For Renca model), 6-8 weeks, >17 g, up to 5 mice per cage

Pan02 model treatment and groups: compounds Ia-001a-Tz, Ic-007a, Ib-010a, IVc-059a, IIc-007a as daily for 5 days/week for two weeks via IV bollus injection at a dose of 15 mg/kg.

Renca model treatment and groups: compounds Ia-001a-Tz, Ic-007a, Ib-010a, IVc-059a, IIc-007a as daily for 7 days/week for three weeks via IV bollus injection at a dose of 15 mg/kg

Cell Culture: the Renca tumor cells were maintained in vitro with DMEM medium supplemented with 10% fetal bovine serum at 37°C in an atmosphere of 5% CO2 in air. The cells in exponential growth phase were harvested and quantitated by cell counter before tumor inoculation.

Cell Culture: the Pan02 tumor cells were maintained in vitro with RPMI 1640 medium supplemented with 10% fetal bovine serum at 37°C in an atmosphere of 5% CO2 in air. The cells in exponential growth phase were harvested and quantitated by cell counter before tumor inoculation.

Tumor Inoculation for Renca model: each mouse was inoculated subcutaneously in the right rear flank region with Renca tumor cells (1 x $10^6$) in 0.1 mL of PBS for tumor development.

Tumor Inoculation for Pan02 model: each mouse was inoculated subcutaneously in the right front flank region with Pan02 tumor cells (3 x $10^6$) in 0.1 mL of PBS for tumor development.

Randomization: The randomization was started when the mean tumor size reaches approximately 100 (80 - 110) mm3. For both models, 48 mice were enrolled in the study. All animals were randomly allocated to 6 study groups. Randomization were performed based on "Matched distribution" method using the multi-task method (StudyDirectorTM software, version 3.1.399.19). The date of randomization was denoted as day 0.

Observation and Data Collection: After tumor cells inoculation, the animals were checked daily for morbidity and mortality. During routine monitoring, the animals were checked for any effects of tumor growth and treatments on behavior such as mobility, food and water consumption, body weight gain/loss (body weights were measured twice per week after randomization or based on sponsor's request after randomization), eye/hair matting and any other abnormalities. Mortality and observed clinical signs were recorded for individual animals in detail.

Tumor volumes were measured twice per week after randomization in two dimensions using a caliper, and the volume were expressed in mm3 using the formula: "V = (L x W x W)/2, where V is tumor volume, L is tumor length (the longest tumor dimension) and W is tumor width (the longest tumor dimension perpendicular to L). (Tumor weight were measured at the end of study based on sponsor's request). Dosing as well as tumor and body weight measurements were conducted in a Laminar Flow Cabinet.

Study Endpoints: Tumor growth inhibition (TGI): TGI% is an indication of antitumor activity, and expressed as: TGI (%) = 100*(1-T/C) T and C are the mean tumor volume (or weight) of the treated and control groups, respectively,

on a given day.

Statistical analysis of the difference in mean tumor volume among the groups were conducted using one of the methods below:

Use the data collected on the last dosing/ observation day for every single group in despite of diverse individual termination date.

Use the data collected on the day when mean Tumor Volume of vehicle group reaches the humane endpoints so that TGI can be derived for all/most mice enrolled in the study.

Use the data collected on the day when any of the treated or control groups is terminated even if the remaining groups are treated as scheduled.

Difference in AUC (ΔAUC) = Statistical analysis performed with a linear mixed effect regression model.

Study Termination: The efficacy study was performed for 3 weeks.

Statistical Analysis: To compare tumor volumes of different groups at a pre-specified day, Bartlett's test is firstly used to check the assumption of homogeneity of variance across all groups. When the p-value of Bartlett's test is ≥0.05, One-way ANOVA were used to test overall equality of means across all groups. If the p-value of the One-way ANOVA is <0.05, post hoc testing by running Tukey's HSD (honest significant difference) tests for all pairwise comparisons, and Dunnett's tests for comparing each treatment group with the vehicle group were performed. When the p-value of Bartlett's test is

<0.05, Kruskal-Wallis test were used to test overall equality of medians among all groups. If the p-value the Kruskal-Wallis test is <0.05, post hoc testing by running Conover's non-parametric test for all pairwise comparisons or for comparing each treatment group with the vehicle group were performed, both with single-step p-value adjustment. All statistical analyses were done in R-a language and environment for statistical computing and graphics (version 3.3.1). All tests are two-sided unless otherwise specified, and p-values of <0.05 are regarded as statistically significant.

Results

Table 40: Antitumor activity of test articles with data collected on day 13

| **Group** | **Treatment** | **Tumor volume ± SEM ($mm^3$)** | **TGI (%)** |
|---|---|---|---|
| Control | Vehicle, 5 μL/g, I.V., QD*5 for 2 weeks | 187.91 ± 11.19(8) | - |
| Compound IVc-059a | IVc-059a, 15 mg/kg, 5 μL/g, I.V., QD*5 for 2 weeks | 148.49 ± 7.05(8) | 20.98% |

Table 41: Statistical analysis of tumor volume with data collected on day 13

| Test | p.values | Significance.level |
|---|---|---|
| Test of homogenity of variance and normality | | |
| Bartlett's test | 2.76E-05 | *** |
| Test of overall equality among groups | | |
| Kruskal-Wallis test | 0.00965 | ** |
| Test of equality between individual groups (Conover's non-parametric many-to-one comparison test) | | |
| Group Control versus Compound IVc-059a | 0.0226 | * |

Result summary

In this study, the *in vivo* therapeutic efficacies of test compound IVb-059a in the treatment of subcutaneous murine pancreatic cancer syngenic model (Pan02) in female C57BL/6 mice was evaluated. Compound IVb-059a, administrated at 15 mg/kg I.V. significantly suppressed tumor growth, with TGI value of 20.98% (P<0.05).

Table 42: Mean % inhibition of tumor volume Pan02 model

| Day | 0 | 3 | 6 | 9 | 13 |
|---|---|---|---|---|---|
| IVc-059a | -0.11% | 19.86% | 18.64% | 18.49% | 20.98% |

$$\text{Mean \% Inhibition} = (\text{mean(Control NaCl)} - \text{mean(Cpd Treated)})/\text{mean(CControl NaCl)} * 100\%$$

Table 43: Mean % inhibition of tumor volume Renca model

| Day | 0 | 3 | 6 | 9 | 13 | 16 |
|---|---|---|---|---|---|---|
| IVc-059a | 0.05% | -0.90% | 5.02% | 14.83% | 23.07% | 19.42% |

**EXAMPLE** 5.3: **Treatment and/or prevention of Diabetic Retinopathy (DR)**

Animal model: The db/db mouse model was used and compared with db/+ mice as a non-diabetic control. The db/db mouse carries a mutation in the leptin receptor gene and is a model for obesity-induced type 2 diabetes. Bogdanov et al. PLoS One, 2014, 9, e97302 reported that db/db mice reproduce the features of the neurodegenerative process that occurs in the human diabetic eye. In addition, this model develops early microvascular abnormalities (vascular leakage) induced by diabetes (Hernández et al. Diabetes 2016, 65, 172-187; Hernandez et al. Diabetologia 2017, 60, 2285-2298). Therefore, it seems an appropriate model for testing drugs addressed to treat early stages of diabetic retinopathy (DR).

The effect of eye-drops containing Ia-007a on vascular leakage induced by diabetes was tested in the db/db mouse model. The mice, db/db (BKS.Cg-Dock7m +/+ Leprdb/J) mice and non-diabetic (db/+) mice aged 8 weeks were purchased from Charles River Laboratories (Calco, Italy).

Animals had free access to ad libitum food (ENVIGO Global Diet Complete Feed for Rodents, Mucedola, Milan, Italy) and filtered water. Mice were maintained under tight environmental conditions of temperature (20°C) and humidity (60%). Moreover, they had cycles of 12 h/12 h light/darkness.

Interventional Study: When the db/db mice were aged 10 weeks, Ia-007a eye-drops or vehicle eye drops were randomly administered directly onto the superior corneal surface of each eye using a syringe. One drop (5μL) of Ia-007a (5 mg/mL) to each eye or vehicle (5μL of 0.9% sodium chloride) to each eye were administered twice daily for 14 days. On day 15, the animals' eyes were instilled with a drop of Ia-007a or vehicle approximately one hour prior to necropsy. All the experiments were performed in accordance with the tenets of the European Community (86/609/CEE).

The permeability of retinal vasculature was examined ex vivo by assessing albumin leakage from the blood vessels into the retina using Evans Blue albumin method. For this purpose, four animals per group were intraperitoneally injected with a solution of Evans Blue (E2129 SIGMA, Sant Louis, Missouri, USA) (5 mg/mL dissolved in PBS pH 7.4). After injection, the animals turned blue, confirming dye uptake and distribution. After 2h, the mice were euthanized by cervical dislocation and the eyes were enucleated. The retinas of each animal were isolated, weighed and rapidly protected from light. Flat-mounted slides were obtained, and cover slipped with a drop of the mounting medium Prolong Gold antifade reagent (Invitrogen, Thermo Fisher Scientific, Oregon, USA). Digital images from different random fields of all retinas were acquired using a confocal laser scanning microscope (FV1000; Olympus, Hamburg, Germany) at ×60 using the 561-nm laser line, and each image was recorded with identical beam intensity at a size of 1024 pixels × 1024 pixels. For quantitative analysis of the albumin-bound Evans Blue, the number of extravasations per field of 0.44 $mm^2$ was counted. This analysis was performed by investigators unaware of treatment received by the mice.

Results

Blood glucose concentration and body weight at the end of treatment were similar in db/db mice treated with Ia-007a eye drops than in db/db mice treated with vehicle.

We identified extravascular location of Evans Blue in the diabetic mice retina (Figure 1, arrows). Topical treatment with Ia-007a was able to significantly reduce the number of extravasations per field, thus preventing the vascular leakage due to the disruption of the blood retinal barrier.

**EXAMPLE 5.4: Treatment and/or prevention of peritonitis**

Efficacy of the compounds of the present invention are assessed on thioglycolate-induced peritonitis murine

models as described by Cook AD et al. (J Immunol. 2003;171(9):4816-4823) and Tsai JM et al. (Blood Adv. 2019;3(18):2713-2721. doi:10.1182/bloodadvances.2018024026).

10- to 16-week-old C57BL/6J mice are injected intraperitoneally (i.p.) with 1 ml of sterile thioglycolate broth 4% (wt/vol) or PBS as control. After 3 h, mice are anesthetized by i.p. injection with a solution of PBS containing 5 mg/ml ketamine and 1 mg/ml xylazine and peritoneal cavities are washed with 5 ml ice-cold sterile Ca2+/Mg2+-free HBSS IX containing EDTA 2 mM. Peritoneal exudate cells (PECs) are centrifuged at 1200 rpm for 5 min at 4°C and red cells lysed. PECs are washed, resuspended in PBS 10% FBS, incubated with anti-CD16/CD32 and mIgG FcR blockers and then stained with fluorescently conjugated antibodies to characterize migrated leukocytes (anti-CD45, anti-CD11b, anti-Ly6G, anti-CD8a, anti-CD4, anti-CD3). Cell viability are measured by 7-AAD exclusion. Specimens are acquired by flow cytometry using MACSQuant Analyzer (Miltenyi Biotec) and analysed using FlowJo software.

Animals: 5-6 C57BL/6J mice per group for each compound.

Treatment with cpds: 50mg/kg by i.p. injection 1h before peritonitis induction.

Readout method: flow cytometry to characterise migrated leukocytes using anti-CD45, anti-CD11b, anti-Ly6G, anti-CD8a, anti-CD4, anti-CD3.

Results and statistical analysis: Results are analysed using FlowJo software. Data are shown as mean $\pm$ SEM. Two-tailed Student's t test using a significance level of 0.05 is used for the statistical analysis.

**EXAMPLE 5.5: Treatment and/or prevention of diabetic cardiomyopathy**

Efficacy of the compounds of the present invention will be tested on diabetic cardiomyopathy murine models described by Li C et al. (Cardiovasc Diabetol. 2019;18(1):15. Published 2019 Feb 2. doi:10.1186/s12933-019-0816-2).

Animal procedure and drug treatment: Thirty 8-week old KK-Ay mice (genetic type 2 diabetes model) and C57BL/6J mice are housed in cages (4-6 per cage) with free access to the drink/feed boxes. Mice are housed in a room kept at 24 °C with 12:12 h light/dark cycle.

The type 2 diabetes model mice are fed a high-fat diet. Blood glucose is measured daily. The mice with a blood glucose concentration greater than 15 mM (200 mg/dL) for 2 consecutive weeks are used for the following experiments. Subsequently, animals are randomized into groups (15 per group) and raised for 8 weeks. The groups include the control groups: (1) healthy C57BL/6J mice with no treatment; (2) type 2 diabetic KK-Ay mice with no treatment; and (3) type 2 diabetic KK-Ay mice treated with the compounds of the present invention.

Echocardiographic evaluation: Echocardiographic measurement is performed before the experimental intervention and at the end of the study period. We used M-mode echocardiography equipped with 17.5 MHz liner array transducer system (Vevo 2100™ High Resolution Imaging System; Visual Sonics). Heart rate (HR) and the following structural variables are evaluated: left ventricular internal dimension in diastole (LVIDD), left ventricular internal dimension in systole (LVIDS), interventricular septal thickness in systole (IVSs) and in diastole (IVSd), and LV posterior wall thickness in systole (LVPWs) and diastole (LVPWd). LV mass was calculated using the formula $[(LVIDd + LVPWd + IVSd)^3 - (LVIDd)^3 \times 1.04 \times 0.8 + 0.6]$. LV function is assessed by the following parameters including fractional shortening (FS), ejection fraction (EF), and E/A ratio. All measurements are conducted by a single investigator who was blinded to the experimental groups.

Myocardial hydroxyproline concentration: The myocardial hydroxyproline concentration of the left ventricle is measured to estimate the myocardial collagen content. The measurements are performed by a spectrophotometer using commercial kit (BioVision, Mountain View, CA, USA) according to the manufacturer's protocols.

Detection of serum lipids, glucose, insulin, and HbAlc levels: At the end of the study, mice are anaesthetized by inhalation of 3% isopentane in air. The fasting blood specimens are collected into commercial tubes containing lithium heparin as an anticoagulant via the sublingual vein from each animal and centrifuged for 6 min at 3000 rpm/min. The plasma is kept in a plain tube and stored at - 20 °C until analysis. Total cholesterol (TC), triglyceride (TG), low-density lipoprotein cholesterol (LDL-C), and high-density lipoprotein cholesterol (HDL-C) plasma concentrations are determined by spectrophotometric methods. Blood glucose levels are measured using a glucometer (ACCU-CHEK, Roche, USA). After an overnight fast, fasting insulin and proinsulin levels are determined using mouse insulin and proinsulin enzyme-linked immunosorbent assay (ELISA) kits, respectively (Nanjing Jiancheng Biotech, China). Blood is also retained for measurement of HbAlc by a chromatographic-spectrophotometric-ion exchange kit (Biosystems, Spain).

Assessment of oxidative stress in heart tissue: Animals are euthanized, and hearts are removed and rinsed retrogradely with a Krebs-Ringer solution (115 mM NaCl, 5 mM KCl, 1.2 mM $KH_2PO_4$, 25 mM $NaHCO_3$, 1.2 mM $MgSO_4$, 1.25 mM $CaCl_2$ and 11 mM glucose) at the end of the study. The temperature of the perfusing solution is maintained at 37 °C. The hearts are then weighed, and cardiac tissues are homogenized on ice in chilled phosphate-buffered saline (PBS) at pH 7.4, containing 1 mM EDTA. The homogenates are centrifuged in cold saline for 10 min at 7000 rpm/min. The protein concentration of the supernatant is determined by the bovine serum albumin kit as a standard. The supernatants are used for analysis of the lipid hydroperoxide level and glutathione peroxidase (GSH-Px), superoxide dismutase (SOD), and malondialdehyde (MDA) levels. The measurements are performed by a spectrophotometer using commercial

kits (Solarbio, China) according to the manufacturer's protocols. The lipid hydroperoxide level is expressed as nmol/mg protein. The GSH-Px, SOD, and MDA levels are expressed as μmol/mg protein, nmol/mg protein, and mmol/mg protein, respectively. The expression level of NOX4 is measured by Western blotting. Mouse monoclonal anti-Nox4 (1:1000, Abcam) and horseradish peroxidase-conjugated secondary antibody (CWBIO) are used. β-Actin (1:1000, Abcam) is used as a reference.

Histological and immunohistochemical analysis: The heart tissues are fixed in 4% paraformaldehyde in 0.1 M phosphate buffer for 48 h, dehydrated and embedded in paraffin, sectioned at 4-μm thickness, and mounted on glass slides. Masson's trichrome staining is used to assess the extent of fibrosis in cardiac muscle.

For antigen retrieval, the deparaffinized slides are kept in a solution of 10 mM sodium citrate (pH 6.0) for 10 min at 100 °C. The sections are then incubated in 3% hydrogen peroxide for blocking endogenous peroxidase activity and incubated with primary antibody (mouse monoclonal antibody to TGF-β1, collagen I and collagen III; Abcam) for 1.5 h, followed by corresponding secondary antibody for 2.5 h at room temperature. Subsequently, the sections are washed in PBS three times and incubated in 0.02% diamino benzidine solution for 2-8 min. After counterstaining with haematoxylin, the slides are washed briefly, mounted with resinene, and observed in the light microscope.

Analysis of the Nrf2/ARE and TGF-β/SMAD pathway by western blotting: The Western blotting protocol is described in our previous study. Briefly, myocardial tissue is lysed in ice-cold RIPA buffer (150 of mM sodium chloride, 0.1% sodium dodecyl sulphate (SDS), 0.5% sodium deoxycholate, 1.0% NP-40, PMSF 1 mM, and 50 mM of Tris, pH 8.0) for total protein extraction. The total protein concentration is quantified by a BCA Protein Assay Kit (Medchem Express, USA). Equal amounts of protein are separated by 12% SDS-polyacrylamide gel electrophoresis (PAGE). Then, the protein is transferred from the gel to a polyvinylidene fluoride membrane. After blocking with 5% skim milk, the membrane is incubated overnight in primary antibody (Nrf2, HO-1, TGF-β1, p-Smad2, Smad2, p-Smad3, Smad3, Smad7, α-SMA 1:1000, Abcam). Bands are detected with specific horseradish peroxidase-conjugated secondary antibody (CWBIO). β-actin (1:1000, Abcam) is used as a reference of total cell protein.

**EXAMPLE 5.6: Treatment and/or prevention of diabetes and diabetic wound healing**

Diabetes murine models using streptozotocin (STZ-induced diabetes) will be used to assess efficacy of compounds of the present invention for a diabetic wound healing and generate tissue samples to allow post in-life analysis of skin tissue.

Animals: a total 104 Female CD-1 mice aged 5-7 weeks weighing approximately 25 - 30g are implanted for the study. These are purchased from Charles River and acclimated for 7 days upon arrival.

Animal Housing: Mice are housed in IVC cages (up to 5 mice per cage) with individual mice identified by tail mark. Cages, bedding and water are sanitized before use. Animals are provided with Corn-o-cobs enrichment bedding to provide environment enrichment and nesting material. Each cage is clearly labeled with a card indicating the number of animals, sex, strain, DOB, study number and start date and treatment. Cages are changed once a week with food and water replaced when necessary.

The animal holding room is maintained as follows - room temperature at 20-24°C, humidity at 30-70% and a 12h light/dark cycle used.

Aseptic technique and dosing: Although animals to be used in this study are immuno-competent, preparation of dosing solutions and dosing/weighing of animals are carried out in a sterile biosafety cabinet. IV dosing: Compounds are formulated in 5% DMSO: 10% Solutol: 10% PEG400: 75% PBS and pH adjusted to 7 with 0.5M NaOH solution. PO dosing: formulation is in water.

Drug solutions are formulated on the day of dosing, any remaining at the end of dosing are stored at - 20°C.

Study Design: Mice (except negative control group) are injected with STZ (55mg/kg; IP) daily for 5 consecutive days. Mice are starved for 6 hours prior to injection. One week after completion of induction glucose levels are monitored and mice with levels less than 15mml/L (280mg/gL) are removed from the study as they have not developed the ideal severity of diabetes. Animals are anaethetised and a full thickness 6mm wound created using a sterilized biopsy punch. Wounds are imaged (Days 0). Animals are treated and imaged every 2 days out to day 14 (when control, non-diabetic mouse wounds were healed). Wound images are digitally assessed for area of wound and percentage closure versus time plotted graphically.

Table 44:

| Group | No. Animals | Tested compounds | Dose level; Route |
|---|---|---|---|
| 1 | 3 (3) | Negative control (non-induced) | Vehicle; IV |
| 2 | 3 (3) | Negative control (Induced) | Vehicle; IV |
| 3 | 8 (3) | Pirfenidone | 250mg/kg; PO |

(continued)

| Group | No. Animals | Tested compounds | Dose level; Route |
|---|---|---|---|
| 4 | 8 (3) | Ia-001a | 15mg/kg; IV |
| 5 | 8 (3) | Ia-001aTZ | 15mg/kg; IV |
| 6 | 8 (3) | Ic-007a | 15mg/kg; IV |
| 7 | 8 (3) | Ib-010a | 15mg/kg; IV |
| 8 | 8 (3) | IIIc-061a | 15mg/kg; IV |
| 9 | 8 (3) | IVc-059a | 15mg/kg; IV |
| 10 | 8 (3) | IVc-059a | 150mg/kg; PO |

Treatment will continue for 2 weeks (14 days). Animal numbers in brackets are dosed for 7 days, then sampled for mid-study PD analysis.

Table 45: study schedule:

| **Time (Weeks)** | **W1** | **W2** | **W3** | **W4** | **W5** |
|---|---|---|---|---|---|
| STZ | Dose (5 days) | --- | --- | --- | --- |
| Testing | | Blood Glucose | Blood Glucose | Blood Glucose Wound image immediately after image every 2 days | Blood Glucose Wound Image every 2 days |
| Compound | --- | --- | --- | IV Dose, daily<br>PO Dose, daily | IV Dose, daily<br>PO Dose, daily |
| Organ | | | | 3 animals per group sampled after 7 days dosing. Skin tissue sampled. | |

Serial observations

Bodyweight: the bodyweight of all mice on the study are measured and recorded daily; this information was used to calculate precise dosing for each animal.

General signs and symptoms: mice are observed daily and any signs of distress or changes to general condition e.g. starred fur, lack of movement, difficulty breathing were noted.

Mid Study sampling: Following 7 days dosing 3 animals per group are killed and wound tissue removed and divided into 2 sections (A) and (B):

(A) First section is FFPE and stained with the following: H&E, Masson's Trichrome, CD31, TGF-β.

(B) Second section are homogenised and used for the following ELISAs: SOD, GTPx, Catalase, TNF-α, IL-6, TGF-β

Termination: Terminal study animals are culled via $CO_2$ inhalation. Prior to termination animals were weighed. *Early Termination:* Early termination of an animal is performed if a weight loss of ≥20% was measured and for any compromised animal showing critical signs and symptoms or inability to eat/drink.

Results: After 7 days already all compounds with the exception of NDB-1-007a exhibited some degree of accelerating wound healing in the STZ-induced diabetes model (Figure 9, 3 animals per group sampled after 7 days dosing). Skin tissue was sampled.

**EXAMPLE 5.7: Treatment and/or prevention of diabetic foot ulcers**

Compounds of the present inventions are tested on a rat model of diabetic ulcers as described by Zhang Y et al. (J Diabetes Res. 2016, 2016, 5782904. doi:10.1155/2016/5782904).

Wound-healing in skin and other mucosal tissues involves a complex sequence of events including the clotting case, acute and chronic inflammation, reepithelialization, granulation tissue formation, wound contraction, and connective-tissue remodelling. However, several genetic and acquired conditions, such as aging, malnutrition (e.g., vitamin C and protein deficiency), infection, hypoxia, and genetic diseases such as Ehlers-Danlos syndrome, can impair this reparative process. Among these conditions, diabetes mellitus is a most common cause of impaired or nonhealing wounds. As an example, the clinical significance of long-term hyperglycemia is highlighted by alarming data showing that 85% of nonhealing diabetic foot ulcers ultimately require amputation. The "pathway to a chronic wound," as outlined by authors of a recent study, focused on prolonged or chronic inflammation characterized by activation of macrophages (as well as accumulation of neutrophils) that resulted in elevated levels of proinflammatory cytokines, reactive oxygen species, matrix metalloproteinases (MMPs), and other neutral proteinases (e.g., elastase). This, coupled with a deficiency of endogenous proteinase inhibitors, all leads to "excessive matrix degradation, degradation of growth factors, and impaired epithelialization."

In the first experiment, fifteen adult male Sprague-Dawley rats (body weight 300-325 g, Charles River Laboratories International, Inc., Wilmington, MA) are injected through the tail vein with either 10 mM citrated saline buffer pH 4.5 (nondiabetic controls, NDCs) or the same solution containing streptozotocin (STZ; ENZO Life Sciences, Inc., Plymouth Meeting, PA; 70 mg/kg body weight) to induce type I diabetes. The rats were then distributed into the five experimental groups described below (n = 3 rats/group). All rats were given unlimited access to food and water. Within 48 hours, the STZ-injected rats exhibited severely elevated glucose levels in their urine. Three weeks after inducing diabetes, the back skins of all the rats are shaved and a series of six standard wounds per rat, each 6 mm in diameter, are made using a surgical trephine. The following five experimental groups were established (in this initial experiment, treatment in all groups was for seven days; a longer-term study is described below in experiment 3): nondiabetic control (NDC) rats treated by daily topical application of white petrolatum jelly ("vehicle"); diabetic rats (D group) topically treated daily with vehicle alone; diabetic rats treated with the tested compounds.

At the end of this time period, the six circular wounds per rat were clinically assessed by measuring with a caliper the diameter of the wounds in millimeters, blood samples were collected, the rats were sacrificed, and skin samples were dissected for histological/histochemical and biochemical assessment as described below.

On day seven after creating the standardized wounds, all of the animals are anesthetized, blood samples are collected for blood glucose (One Touch Ultra Glucometer; Johnson & Johnson, New Brunswick, NJ) and HbAlc (Bayer A1CNow Selfcheck, Sunnyvale, CA) measurements, and, after the procedures below are completed, the rats are sacrificed by CO2 inhalation.

Photographs are taken for clinical measurements to assess wound closure (18 wounds per experimental group). The percent reduction of the wound surface is calculated by measuring the diameter (in millimeters) of each wound before and after the treatment protocol.

Wound tissues on day 7 are excised from two sites per rat and pooled for biochemical analysis. Each pool of tissue is homogenized, extracted at 4°C with 5 M urea in 50 mM Tris-HCl buffer (pH 7.8) containing 0.2 M NaCl and 5 mM CaCl2 overnight, and then centrifuged for 1 hour at 11,000 $\times$ g, as described by us previously. The supernatants are dialyzed against the Tris-HCl, NaCl, and CaC12 buffer, and the proteinases are partially purified by ammonium sulfate added to 60% saturation. The precipitated proteinases are analyzed by ELISA for collagenases MMP-8 (Sigma-Aldrich Life Sciences Inc., St. Louis, MO) and MMP-13 (TSZ Scientific LLC, Framingham, MA).

Biopsies of each of two wound sites, including surrounding nonwounded tissue, are taken and fixed in 10% neutral buffered formalin for 24 hours and then transferred to 50% ethanol prior to grossing, alcohol dehydration, xylene clearing, paraffin embedding, and sectioning. Five-micron sections are stained with H&E and Masson's Trichrome and the distance between wound margins is measured histomorphometrically using a calibrated ocular micrometer and confirmed image analysis. The last two wounds per rat are dissected, hydrolyzed, and analyzed for hydroxyproline as described below.

At the end of the 14-day and 30-day treatment protocols, the physical measurements and histologic and histochemical assessments are the same as those described above for the 7-day experiment. In addition, for all three time periods, tissue samples from the 6 mm punch biopsies are hydrolyzed twice in 2 N NaOH at 120°C for 1 hour each time. 50 $\mu$L aliquots of the skin tissue hydrolysates are then analyzed for hydroxyproline, an amino acid essentially found only in collagen.

**EXAMPLE 5.8: Treatment and/or prevention of diabetic retinopathy and diabetic nephropathy**

Efficacy of the compounds according to the present invention is assessed on diabetic mouse model using the DBA/2 mouse strain with STZ induction of diabetes and follow diabetic retinopathy including kidney damage. Five weeks after induction of diabetes via low dose STZ injection for 5 consecutive days, the urinary albumin:Cr ratio is 424-fold compared to 36.9 for age-matched controls.

For treatment of diabetic nephrology, DBA/2 mice are induced with STZ (low dose for 5 consecutive days). One week after completion of induction, glucose levels are monitored and mice with levels less than 15mml/L (280

mg/0.1L) are removed from the study as they will not develop diabetes of sufficient severity to results in renal injury. Glucose levels are monitored weekly and 5 weeks after STZ induction biochemical assessment of renal injury are carried out by measuring urine albumin and creatinine levels and confirming they were within the desired range. After successful completion of this stage animals are assigned to treatment groups.

Treatment is performed for four weeks (IV dosing) with weekly assessment of blood glucose and urine albumin/creatinine levels. At the end of the treatment period animals, the following samples are taken:

- Blood for measurement of glucose and BUN
- Blood for measurement of serum creatinine
- Urine for final albumin/creatinine ratio.
- Kidney was resected and FFPE: Picro-Sirius Red, Masson's Trichrome and H&E staining are carried out to identify areas of fibrosis, along with evidence of Mesangial sclerosis, Arteriolar hyalinosis, Glomerular Basal Membrane (GBM) thickening

To assess compound effect on diabetic retinopathy, animals have weekly images taken via Fundus Camera. Immediately prior to sacrifice, animals are injected with FITC-dextran via tail vein. During sampling, the eyes are sampled and fixed in formalin. Retinal Flatmounts are prepared and examined fluorescently looking at compound effects on vascularity and leakiness of vessels (indicted by high background staining). Animal groups (n=8 mice per group):

Table 46:

| **Group** | No. Animals | Compounds |
|---|---|---|
| 1 | 3 | Negative control (non-induced) |
| 2 | 3 | Positive control (Induced) |
| 3 | 8 | Captopril 50mg/kg |
| 4 | 8 | Ia-001a 15 mg/kg I.V. |
| 5 | 8 | Ia-001aTZ 15 mg/kg I.V. |
| 6 | 8 | Ic-007a 15 mg/kg I.V. |
| 7 | 8 | Ib-010a 15 mg/kg I.V. |
| 8 | 8 | IIIc-061a 15 mg/kg I.V. |
| 9 | 8 | IVc-059a 15 mg/kg I.V. |
| 10 | 8 | IVc-059a 150 mg/kg P.O. |

Treatment will continue for 4 weeks (28 days) with weekly measurement of blood glucose and urine albumin/creatinine.

Sacrifice after last compound (agent) dosing, organ and blood sampling.

Table 47: study schedule

| **Time (Weeks)** | **W1** | **W2** | **W3** | **W4** | **W5** | **W6** | **W7** | **W8** | **W9** | **End W9 Sacrifice** |
|---|---|---|---|---|---|---|---|---|---|---|
| STZ | Dose (5 days) | --- | ---- | --- | --- | --- | --- | --- | --- | --- |
| Testing | | Blood Glucose | Blood Glucose | | Blood Glucose Urine ALB/CREA | Blood Glucose Urine ALB/CREA | Blood Glucose Urine ALB/CREA | Blood Glucose Urine ALB/CREA | Blood Glucose Urine ALB/CREA | Blood Glucose Urine ALB/ CREA |
| Compound | --- | --- | --- | --- | --- | IV Dose, daily | IV Dose, daily | IV Dose, daily | IV Dose, daily | --- |
| Organ | | | | | | | | | | Blood (Plasma) Kidneys Eyes |

Serial observations

*Bodyweight:* The bodyweight of all mice on the study are measured and recorded daily; this information will be used to calculate precise dosing for each animal. The Sponsor are informed if any animal loses more than 10% bodyweight and a dosing holiday may be considered as needed.
*General signs and symptoms:* Mice are observed daily and any signs of distress or changes to general condition e.g. starred fur, lack of movement, difficulty breathing will be noted.

Sampling and post in-life analyses

- Immediately prior to terminal sampling 4 animals per group are injected with FITC-dextran for retinal flatmount assessment.
- Terminal Sampling:
    - Terminal blood samples are taken via cardiac puncture and plasma/serum prepared from each animal.
        - Blood glucose measured
        - Serum creatinine measured
        - BUN measured
        - Urine ALB/CREATININE
        - ELISAs for CRP, TNF-α, IL-6 and TGF-β
        - Remaining plasma are stored for cytokine analyses
    - Kidney tissue are resected and weighed
        - One kidney is snap frozen for possible bioanalysis or other analysis.
        - One kidney is fixed in formalin and embedded in paraffin wax.
            - H&E and Masson's Trichrome staining Screening for
                - Mesangial and glomerulo- sclerosis,
                - Arteriolar hyalinosis
                - GBM thickening
    - Eye tissue to be FFPE
        - Other eye (4 per treatment group) are used for Retinal flatmount analysis to measure vascular patterning
        - Area covered by vessels
        - Assessment of vascular leakage

Termination: Terminal study animals are culled via $CO_2$ inhalation. Prior to termination animals are weighed.
Results: compounds Ia-001a, IIIc-061 and IVc-059a where able to reduce significantly the blood glucose level in DBA/2 mouse strain with STZ induction of diabetes as shown in Figure 10 (two weeks shown).

**EXAMPLE 5.9: Treatment and/or prevention of Behcet's Disease**

Efficacy of the compounds according to the present invention will be tested on Behçet's disease murine model as described by ZHENG et al. (Acta Derm Venereol 2015; 95: 952-958).
Behçet's disease is a chronic, recurrent, multisystemic, inflammatory disorder affecting mainly the oral and urogenital mucosa and the uveal tract. Although the etiology and pathogenesis of Behçet's disease were unknown,

numerous etiologies have been proposed, including environmental, infectious, and immunological factors; an autoimmune basis, characterized by circulating immune complexes and complement activation, has gained increasing acceptance. To test and understand immunopathogenesis of Behçet's disease, animal models were developed based on environmental pollutants, bacterial and human heat shock protein derived peptides, and virus injections. Using these animal models separately and/or concurrently allows for a more effective investigation into Behçet's disease.

Animal models have been recently developed to find efficient and safe treatment options. Neutrophil activation is one of the immunopathogenesis aspects of BD. Neutrophils have a pivotal role in innate immune responses. As typical BD lesions such as pustular folliculitis, pathergy reactions, and hypopyon have significant neutrophil infiltrations, neutrophil functions and activation status have been investigated. There were conflicting reports of increased, normal, or decreased basal and fMLP stimulated superoxide productions, phagocytosis, chemotaxis, and neutrophil-endothelial adhesion in BD. In HLA-transgenic mic presumed model for BD, the only abnormality seen is increased superoxide release in response to fMLP. High superoxide responses were also present in HLA-B51+ patients and healthy controls in the same study.

Behcet Disease-like mouse model is developed by inoculating HSV type 1 grown in Vero cells in 4-5-week-old male Institute of Cancer Research (ICR) mice. Briefly, the earlobes of experimental mice are scratched with a needle and they are inoculated twice with the virus, with a 10-day interval. The infected mice are observed for 16 weeks after the final inoculation. HSV-inoculated mice presenting at least 2 Behcet-like symptoms are used as a BD-like mouse model (n=5). Both uninfected ICR mice (n = 2) and HSV-inoculated, but asymptomatic, mice.

## EXAMPLE 5.10: Treatment and/or prevention of Uveitis (UVE)

Efficacy of the compounds of the present invention will be tested on animal models of autoimmune uveitis including Endotoxin-Induced Uveitis (EIU) in the rat as described by Fruchon S et al. (Molecules. 2013;18(8):9305-9316. Published 2013 Aug 5. doi:10.3390/molecules18089305). This model is considered as a clinically relevant model for human anterior uveitis. It consists in the systemic administration of lipopolysaccharide (LPS) which results in an acute inflammatory response in the anterior and posterior segments of the eye with a breakdown of blood-ocular barrier and inflammatory cell infiltration. Clinical signs of EIU reflect the changes observed in human disease. The therapeutic effect of compounds of the present invention will thus be tested in the model of EIU in rats, in comparison with the "gold standard" dexamethasone.

Animals: Only animals with no visible signs of ocular defects are enrolled. Animals are examined during the pre-test period and particular attention is given to the eyes. They are held in observation for one week before experimentation. Animals are housed individually in standard cages and had free access to food and tap water.

Ocular Tolerability Study: The study consisted of three groups of three male Sprague-Dawley rats. On day 0, animals are weighed, anesthetized and administered by a single 5 μL intra-vitreal injection in both eyes. The first group receive the saline vehicle (NaCl 0.9%), and the other groups received different doses of the compounds of the present invention. Each animal is then assessed by clinical observation and eye examinations. Ocular examinations include: funduscopy, slit-lamp examination (SLE) of the cornea using fluorescein dye enabling McDonald-Shadduck scoring. The McDonald-Shadduck Scoring System addresses: conjunctival parameters (congestion, swelling and discharge), aqueous flare (intensity of the Tyndall phenomenon) as presumptive evidence of breakdown of the blood-aqueous barrier; injection of secondary and tertiary vessels in the iris; cloudiness, relative area thereof, neo-vascularization and epithelial integrity (fluorescein labelling) of the cornea; integrity of the lens. After final ocular examination, all of the animals are sacrificed. Eyes are collected at necropsy, fixed in modified Davidson's solution for 12 h, followed by 10% neutral buffered formalin and processed for histology. Hematoxylin-Eosin stained tissue sections are evaluated via light microscopy by a board-certified veterinary pathologist.

Endotoxin-Induced Uveitis (EIU) Rat Model: Thirty-six female albino Lewis rats are randomly divided into six groups of six animals each. EIU was induced by a 100 μL footpad injection of sterile pyrogen-free saline solution containing 200 μg of LPS (lipopolysaccharide from Salmonella typhimurium, Sigma-Aldrich, Saint-Quentin, France). Animals are treated immediately before EIU induction by a 5 μL intra-vitreal injection in both eyes of a saline solution (NaCl 0.9%) containing no active ingredient, or with tested compounds, or 20 μg of dexamethasone. Animals are examined by slit-lamp (SLE) at 24 h, i.e., the clinical peak of the disease in this model. The intensity of clinical ocular inflammation is scored on a scale from 0 to 5 for each eye. Grade 0 indicates no inflammation. Grade 1 indicates the presence of a minimal iris and conjunctival vasodilatation but without the observation of flare or cells in the anterior chamber (AC). Grade 2 indicates the presence of moderate iris and conjunctival vessel dilation but without evident flare or cells in the AC. Grade 3 indicates the presence of intense iris vessel dilation, flare and less than ten cells per slit-lamp field in the AC. Grade 4 indicates the presence of more severe clinical signs than Grade 3, with more than ten cells per slit-lamp field in the AC, with or without the formation of a hypopyon. Grade 5 indicates the presence of intense inflammatory reaction, fibrin formation in the AC and total seclusion of the pupil. At the end of experiment, i.e., 24 h after LPS challenge, rats are anesthetized by intra-peritoneal injection of pentobarbital (30 mg/kg) then killed with a lethal dose of pentobarbital.

Measurement of Cytokine Concentrations in Ocular Fluids: Aqueous humour and vitreous from both eyes of each animal are taken after sacrifice. Pro-inflammatory T helper cytokines TNFα, IL-1β, IL-2, IL-6, IL-17 and IFNγ as well as anti-inflammatory cytokines IL-4 and IL-10 quantities are determined by Multiplex analysis.
Measurement of Cytokine Concentrations in Serum: Sera from the three groups of rats (saline vehicle, compound 10 μg and dexamethasone) are collected at the end of the experiment and stored at -80 °C. They are used for the simultaneous determination of five cytokine (IFNγ, TNFα, IL-2, IL-4 and IL-10) levels with Cytometric Bead Array (rat CBA Flex set, BD Biosciences, San Jose, CA, USA) on a FACS Calibur flow cytometer (BD Biosciences) according to the manufacturer's instructions. The amounts of each of the cytokines are analyzed in relation to standard curves using the FCAP Array software (BD Biosciences).

**EXAMPLE 5.11: Treatment and/or prevention of diabetic sensorimotor polyneuropathy and diabetic neuropathy**

Efficacy of the compounds of the present invention are tested on rat models of diabetic peripheral neuropathy (Kambiz S. et al. (PLoS One. 2015;10(6):e0131144]; PLoS One. 2015;10(5):e0126892. Published 2015 May 18. doi:10.1371/journal.pone.0126892).
Animals: WAG/RijHsd female rats (n = 27, 10 weeks old, weighing 130-150 gram) are purchased from Charles River (l'Arbresle, France). The animals are pair-housed in hooded cages at room temperature on a 12-hour light/dark schedule and were given water and food ad libitum
Induction of diabetes: Diabetes is induced in 21 rats by a single intra-peritoneal injection of STZ (Sigma-Aldrich, St. Louis, MO, USA) at a dose of 65 mg/kg body weight in 0.05 mol/L sodium citrate buffer, pH 4.5, as described previously. The rats are randomly assigned into 3 groups: A, B and C (n = 7 in each group). Following diabetes induction, group A is killed after 4 weeks, group B after 6 weeks and group C after 8 weeks. The control group consisted of 6 rats who receive a single intra-peritoneal injection with an equal volume of vehicle without STZ. Control rats are followed for 8 weeks. Blood glucose is measured from tail vein blood by a glucometer (OneTouch, LifeScan, Milpitas, California, USA). Diabetes is diagnosed in rats, when blood glucose levels are higher than 20 mmol/L during the entire 4 weeks after the induction of diabetes.
The blood flow and oxygenation of the plantar hind paws' skin: A combined laser doppler flowmetry and spectrophotometry system (O2C, LEA Medizintechnik, Giessen, Germany), is used to non-invasively measure blood flow and oxygen saturation of the glabrous plantar hind paws. In both diabetic and control rats the percentage oxygen saturation and amount of skin blood flow are assessed at 4, 6, and 8 weeks.
Rewarming rate after cold exposure: The temperature of the skin is assessed using the built-in infrared digital video camera (320 × 240 pixels) by 1 Hz data acquisition system (ThermaCAM Researcher 2001 HS; FLIR Systems, Berchem, Belgium), and all data are continuously collected by a laptop. The distance between the camera and the hind paw is 13 cm ± 2 cm. The pixel size of the temperature recordings is 0.8 × 0.8 mm. The skin temperature of the entire plantar hind paw is recorded while the animal was fixed after placing the animal on a 14°C plate for 5 seconds. The minimum temperature of the plantar hind paws is exported to text files using ThermaCAM Researcher Pro (version 2001-HS; FLIR Systems, Wilsonville, Oregon, USA). The area of interest is selected by drawing a line surrounding the entire plantar hind paws. The average rewarming rate is demonstrated as the increase in skin temperature per 120 seconds.
Thermal sensitivity: In order to determine the occurrence of thermal hypersensitivity, cold and hot plate tests are performed as described previously. In short, rats are placed in an open-ended chamber with clear walls with a surface temperature of either 5°C (cold plate) or 50°C (hot plate). These experiments are performed on separate days to prevent interference. The time until hind paw withdrawal or licking is observed.
Von Frev test: In the von Frey test, used to determine the mechanical sensitivity threshold for nociception, each rat is placed in a chamber with a mesh metal floor. Then, von Frey hairs, ranging from 2 to 300 grams, are applied 5 times, and was scored positive when a minimum of 3 paw flicks (the animal's reflex withdrawal response) are observed, as described previously. The control group served as the reference group.
Electromyography (EMG): Innervation of motor axons in muscles is evaluated by recording the evoked CMAP peak-peak amplitudes and latencies of the gastrocnemius muscles in the diabetic groups and control animals. CMAP peak-peak amplitudes and latencies are recorded and averaged over a batch of 20 responses. The average amplitudes in each diabetes group are compared to the control group. The MNCV is calculated as the distance of stimulating electrode to recording electrode (mm)/latency (ms).
Tissue preparation: After 4, 6, or 8 weeks, the animals are killed by an overdose of pentobarbital (100mg/kg ip). For each rat, the plantar skin of the hind paw is dissected and directly immersion-fixed in 2% paraformaldehyde-lysin-periodate (PLP) for 24 hours at 4°C. The skin is further processed and embedded in gelatin as described previously. Finally, the embedded skin is sectioned at 40 μm with a freezing microtome and collected in glycerol for long-term storage at -20°C.
The pancreas tissue of the rats is harvested, fixed in 10% neutral buffered formalin solution, and embedded in paraffin. Subsequently, these specimens are stained with hematoxylin and eosin (H&E). Each specimen is evaluated by a bright-field microscope and scanned into digital slides (Nanozoomer 2.0 series, Hamamatzu, Japan).
Immunohistochemistry: Immunohistochemistry of the skin sections is performed as previously described to semi quantify the density of sensory nerve fibers innervating the skin, and to evaluate the presence of CD-31 positive endothelial cells. The skin sections are incubated for 48 hours in a cocktail of 2% BSA containing the diluted primary antibody Protein Gene Product 9.5 (PGP9.5, 1/10.000, anti-rabbit, Enzo Life Sciences, New York, USA), or anti-CD31+ (1/5000, anti-rabbit, Spring Bioscience, California, USA) at 4°C. Subsequently, skin sections are incubated with the appropriate secondary biotinylated antibody labelled with horseradish peroxidase (HRP) (1/200, Biotine, Sigma-Aldrich, St. Louis, MO, USA) for 90 min at RT. The 3, -3' diaminobenzidine (DAB) reaction is then used to reveal the antigenic binding sites of the primary antibodies. Thereafter, the sections are mounted on slides and the CD31+ stained sections stained with 0.05% thionin for 4 minutes, which coloured the keratinocytes blue. Finally, all skin sections are dehydrated using absolute ethanol (< 0.01% methanol), transferred to xylene, and cover slipped with Permount (Fisher Scientific, Hampton, NH).
Each skin section is scanned in 3 layers of 8μm each by Nanozoomer 2.0 series (Nanozoomer 2.0 series, Hamamatzu, Japan). Four proximal and 4 distal skin sections of the plantar hind paw are quantified for epidermal nerve fibers in the center part of the plantar hind paw (80.000 μm2) using a 40x objective in ImageScope software (Aperio ImageScope v11.1.2.760). The average labeled nerve fibers per mm2 and the average epidermal thickness are calculated for each rat. Percentage CD31-positive cells is calculated by Leica Cell-D (Olympus, Imaging software for life science microscopy, USA) in 4 proximal and 4 distal skin sections over the entire upper dermis of the plantar hind paw.

**EXAMPLE 5.12: Treatment and/or prevention of intestinal fibrosis**

Efficacy of the compounds of the present invention is tested on rat or murine models as described by Phαm BT et αl. (Physiol Rep. 2015;3(4):e12323. doi:10.14814/phy2.12323*).*

Preparation of rat and mouse intestinal cores: Adult nonfasted male Wistar rats and C57BL/6 mice are used (Harlan PBC, Zeist, The Netherlands). The rats and mice are housed on a 12 h light/dark cycle in a temperature and humidity-controlled room with food (Harlan chow no 2018, Horst, The Netherlands) and water ad libitum. The animals are allowed to acclimatize for at least seven days before the start of the experiment.
Rats and mice are anesthetized with isoflurane/O2 (Nicholas Piramal, London, UK). Rat jejunum (about 25 cm distal from the stomach and 15 cm in length) and mouse jejunum (about 15 cm distal from the stomach and 10 cm in length) are excised and preserved in ice-cold Krebs-Henseleit buffer (KHB) supplemented with 25 mm d-glucose (Merck, Darmstadt, Germany), 25 mm NaHCO3 (Merck), 10 mm HEPES (MP Biomedicals, Aurora, OH), saturated with carbogen (95% O2/5% CO2) and adjusted to pH 7.4. The jejunum is cleaned by flushing KHB through the lumen and subsequently divided into 2 cm segments. These segments are filled with 3% (w/v) agarose solution in 0.9% NaCl at 37°C and embedded in an agarose core-embedding unit.

Preparation of human intestinal cores: Healthy human jejunum tissue is obtained for research from intestine that was resected from patients who underwent a pancreaticoduodenectomy.
The healthy jejunum is preserved in ice-cold KHB until the embedding procedure. The submucosa, muscularis, and serosa are carefully removed from the mucosa within an hour after collection of the tissue. The mucosa is divided into 0.4 cm × 1 cm sheets. These sheets are embedded in 3% agarose (w/v) solution in 0.9% NaCl at 37°C and inserted in embedding unit.
Preparation of PCIS: PCIS is prepared in ice-cold KHB by the Krumdieck tissue slicer (Alabama Research and Development). The slices with a wet weight of 3-4 mg had an estimated thickness of 300-400 μm. Slices are stored in ice-cold KHB until the start of the experiments.
Incubation of intestinal slices: Slices are incubated in 12-well plates for human PCIS (hPCIS) and rat PCIS (rPCIS) or in 24-well plates for mouse PCIS (mPCIS). hPCIS and rPCIS are incubated individually in 1.3 mL and mPCIS in 0.5 mL of Williams Medium E with L-glutamine (Invitrogen, Paisly, UK) supplemented with 25 mm glucose, 50 μg/mL gentamycin (Invitrogen), and 2.5 μg/mL amphotericin-B (Invitrogen). During incubation (at 37°C and 80% O2/5% CO2) in an incubator (MCO-18M, Sanyo), the plates are horizontally shaken at 90 rpm (amplitude 2 cm). rPCIS are incubated up to 24 h, mPCIS and hPCIS were incubated up to 72 h, with and without human TGF-β1 (Roche Diagnostics, Mannheim, Germany) in the concentration range from 1 to 10 ng/mL. All incubations are performed manifold (using 3-6 slices incubated individually in separate wells) and are repeated with intestine from 3 to 16 different humans, rats, or mice.
Viability and morphology: The viability is assessed by measuring the adenosine triphosphate (ATP) content of the PCIS. Briefly, after incubation, slices are transferred to 1 mL sonication solution (containing 70% ethanol and 2 mm EDTA), snap-frozen in liquid nitrogen and stored at -80°C. To determine the viability, ATP levels are measured in the supernatant of samples sonicated for 45 sec and centrifuged for 2 min at 4°C at 16.000 × g, using the ATP bioluminescence kit (Roche Diagnostics, Mannheim, Germany). ATP values (pmol) are normalized to the total protein content (μg) of the PCIS estimated by Lowry method (BIO-rad RC DC Protein Assay, Bio Rad, Veenendaal, The Netherlands). To assess the morphology, incubated slices are fixed in 4% formalin and embedded in paraffin. Sections of 4 μm are cut and

stained with hematoxylin and eosin (HE). HE sections are scored according to a modified Park score, describing the sequence of development of tissue injury in the intestine after ischemia and reperfusion. The integrity of seven segments of the PCIS are scored on a scale from 0 to 3. Viability of the epithelium, stroma, crypts, and muscle layer are scored separately rating 0 if there is no necrosis, and 3 if massive necrosis is present. The other parts of the intestinal slice are rated as follows: Shape of the epithelium: 0 = cubic epithelium, 3 = more than 2/3 of the cells are flat, flattening of the villi: 0 = normal, 3 = more than 2/3 of the villi are flattened, and the amount of edema: 0 = no edema, 3 = severe edema. A maximum score of 21 indicates severe damage. In human samples, the morphological score of muscularis mucosae is determined in the "muscle layer" section. B.T.P., W.T.v.H. and J.N. performed the blind scoring; the mean of three total scores is calculated.

Gene expression: The expression of the fibrosis genes, namely COL1A1, αSMA, HSP47, CTGF, FN2, TGF-β1, PAI-1, and SYN were determined by either the Taqman or SYBRgreen method. In hPCIS, ELA gene expression was also measured by SYBRgreen method.

Intestinal fibrosis is a serious, yet common, outcome in patients with inflammatory bowel disease (IBD). Despite advances in developing novel treatment modalities to control chronic gut inflammation characteristic of IBD, no effective anti-fibrotic therapies exist to date. As such, a deeper understanding of the molecular mechanisms underlying intestinal fibrosis and the availability of relevant animal models are critical to move this area of investigation forward.

IL-17 and associated mediators: Th17 cells define a subset of T helper cells that mainly produce IL-17A, but also IL-17F, IL-21, and IL-22, and are increasingly recognized as paramount in several chronic inflammatory disorders, including IBD (7). IL-17A has been implicated in fibrosis in multiple organs, including lung (8), liver (9), and heart (10); recent studies also support its role in the intestine, linking IL-17/Th17 immune responses and other associated mediators in the pathogenesis of gut fibrosis.

Other animal models of gut fibrosis are described herein below:

Table 48:

| CATEGORY | MODEL | MODE OF ADMINISTRATION/OUTCOME |
|---|---|---|
| Chemically-Induced | DSS | Administered in drinking water, causing epithelial damage and permeabilization of the colonic mucosa with subsequent acute inflammation; cycling of DSS causes chronic inflammation and ensuing fibrosis |
| | TNBS | Colonic enema administration of TNBS/ethanol damages epithelial barrier and causes a T cell-dependent transmural inflammation; long-term administration results in colonic fibrosis |
| Microbial | PG-PS | Injection directly into the cecal or small bowel wall induces granulomatous enterocolitis with significant fibrosis |
| | Fecal injection | Injection of fecal suspension directly into the bowel wall of colon causes aggressive colitis and transmural fibrosis |
| | Chronic *Salmonella* infection | Oral administration after pre-treatment with streptomycin causes colonic mucosal and transmural inflammation with significant fibrosis |
| | AIEC infection | Gavage of the human CD isolate of AIEC, NRG857, after pre-treatment with streptomycin causes ileo-colonic inflammation and Th1-and Th17-mediated and fibrosis |
| Genetically-Manipulated Mice | TGFβ1-Tg TβRIIΔk-fib-Tg | Enema administration of these adenoviral vectors overexpressing TGFβ into the colon leads to acute and chronic inflammation, ECM deposition and thickening of muscularis layers |
| | MCP-1-Tg | Intramural injection of adenoviral vector carrying MCP-1 in the rectum leads to transmural inflammation, collagen deposition, and fibrosis |
| | IL-10 deficient | Genetic deletion of IL-10 results in transmural inflammatory lesions of the colon, crypt abscesses, and thickening of the bowel wall; evidence of increased susceptibility to developing post-surgical fibrosis in small intestines after ileo-cecal resection |
| Immune-Mediated | T cell transfer | Transfer of donor $CD4^+/CD45RB^{high}$ T cells into immunodeficient recipient mice results in wasting disease, colitis and mild fibrosis |
| Spontaneous | SAMP1/YitFc mouse strain | Develops spontaneous terminal ileitis and subsequent fibrosis |

**EXAMPLE 5.13: Treatment and/or prevention of ovarian fibrosis**

Efficacy of the compounds of the present invention will be tested on in ovarian hyperstimulation syndrome (OHSS) murine model described by Pala Ş et al. (Drug Des Devel Ther. 2015;9:1761-1766. Published 2015 Mar 24. doi:10.2147/DDDT.S75266) in order to examine the effects of the tested compounds on ovarian histopathology, serum VEGF, and endothelin 1 levels in ovarian hyperstimulation syndrome (OHSS) in an experimental setting.

Animals: Female Wistar albino rats, 22 days of age are used and randomly divided into groups. Follicle-stimulating hormone 10 IU was administered subcutaneously in 15 rats on 4 consecutive days, with OHSS induction on day 5 by 30 IU of human chorionic gonadotropin. Group 1 comprised 35-day-old control rats, group 2 comprised 35-day-old OHSS rats, group 3 comprised 27-day-old OHSS rats receiving the tested compounds for 7 days. All rats are then decapitated on day 35. Serum VEGF, endothelin 1, and ovarian follicular reserve are assessed in all rats.

Hematocrit and weight measurements are performed on day 13 and decapitation performed on day 35 under general anesthesia by intraperitoneal administration of ketamine (75 mg/kg) and xylazine (10 mg/kg).

Enzyme-linked immunosorbent assay: Approximately 3 mL of blood sample obtained from each rat decapitated. Sera are separated by centrifugation of blood samples at 2,500 rpm for 4 minutes and kept at -20°C until VEGF and endothelin 1 assays. VEGF are assayed using a mouse VEGF enzyme-linked immunosorbent assay kit (ELM-VEGF-001; RayBio, USA) and endothelin are measured using an endothelin 1 E/Kit (EK-023-01; Phoenix Pharmaceuticals, USA).

Ovarian morphology: After laparotomy, ovaries are removed and cleaned of adhering tissue in culture medium and weighed. Ovarian tissue is fixed with 10% formaldehyde, and then paraffin-embedded tissue samples are cut into 4 μm cross sections for estimation of mean ovarian follicle count. The sections are stained with Masson's trichrome to determine OFR under light microscopy (Olympus BX-50). The 4 μm-thick cross sections are mounted at 50 μm intervals onto microscope slides to prevent counting of the same structure twice, according to a previously described method. 12 Follicles are classified as primordial, primary, secondary, and tertiary. An atretic follicle (AF) is defined as a follicle presenting more than ten pyknotic nuclei; for the smallest follicles, the criteria for atresia are a degenerate oocyte, precocious antrum formation, or both.

Main outcome measures: The main outcome measures are age (days), weight (g), hematocrit (%), weight of ovaries (mg), serum levels of VEGF (pg/mL) and endothelin 1 (ng/mL), and total follicle count with determination of primordial, primary, secondary, and tertiary follicle numbers.

**EXAMPLE 5.14: Treatment and/or prevention of polycystic ovary syndrome (PCOS)**

Efficacy of the compounds of the present invention will be tested on DHEA-induced ovarian hyperfibrosis animal model as described by Wang D et al. (J Ovarian Res. 2018;11(1):6. Published 2018 Jan 10. doi:10.1186/s13048-017-0375-7).

The polycystic ovary syndrome (PCOS) is a common metabolic and endocrine disorder with pathological mechanisms remain unclear. The following study investigates the ovarian hyperfibrosis forming via transforming growth factor-β (TGF-β) signaling pathway in Dehydroepiandrosterone (DHEA)- induced polycystic ovary syndrome (PCOS) rat model.

Animals: Thirty female Sprague-Dawley (SD) rats, 21 days old, weighing ~50 g, will be used fpr this assay. The animals are housed in specific-pathogen-free (SPF) environment with temperature of 22 ± 1 °C, relative humidity of 50 ± 1%, and a light/dark cycle of 12/12 h. Free access to food and water are provided.

Methods: Thirty female Sprague-Dawley rats are randomly divided into Blank group (n = 6), Oil group (n = 6), and Oil + DHEA-induced model group (n = 6 + 12). The model groups are established by subcutaneous injection of DHEA for 35 consecutive days. Rats are additionally divided in vehicle group (n = 6) and treated group (n = 6). The treatment is given once a day and treatment will last for 35 days. Eighteen days post- treatment, vaginal smears are collected from all the rats on daily basis by judging cell types for 14 days, in order to determine their estrous cycles daily. On day 36, all the rats in Blank and Oil-treated groups and six rats of DHEA-induced model groups are euthanized (using intraperitoneal injection of excess 5% chloral hydrate), blood is collected (from the superior vena cava), bilateral ovaries and uteri is dissected.

Ovaries were fixed in 4% paraformaldehyde for 24 h at 4 °C, and then embedded in paraffin. The rest of the tissues were frozen in -80 °C for further western blotting and real time- polymerase chain reaction (RT -PCR) analysis.

The rest 12 rats from the DHEA-induced model groups were randomized in additional two groups: treated group and vehicle treatment group (control group), six rats per group. During this treatment, DHEA was no longer given to rats. The treatment lasted for 2 weeks, after which all the animals are euthanized, and blood is collected, and bilateral ovaries and uteri are dissected following the instructions mentioned above.

Ovarian morphology, fibrin and collagen localization and expression in ovaries are detected using H&E staining, immunohistochemistry and Sirius red staining. The ovarian protein and RNA are examined using Western blot and RT-PCR.

Estrous cycle: On day 18 after DHEA treatment, vaginal smears were collected form all rats. Samples were then treated with toluidine blue for 30 min, and consequently cell morphology and estrous cycles were examined under the optical microscope (Leica Microsystems, Germany).

Serum hormones levels: Blood samples were collected from the superior vena cava. The serum was separated immediately and stored at -20 °C for further hormones determination by enzyme-linked immunosorbent assay (ELISA) (testosterone (T), estradiol (E2), luteinizing hormone (LH), follicle stimulating hormone (FSH)) (rat T, E2, LH and FSH ELISA Kits, USCN, Wuhan, China).

Haematoxylin- eosin (H&E) and Sirius red tissue staining

**EXAMPLE 5.15: Treatment and/or prevention of Primary biliary cholangitis (PBC)**

Efficacy of the compounds of the present invention will be assessed on PBC murine model as described by Hohenester S et al. (Cells. 2020, 9(2), 281. doi: 10.3390/cells9020281).

Cholestatic liver diseases such as primary biliary cholangitis (PBC) or primary sclerosing cholangitis (PSC) are chronic progressive disorders that frequently result in liver cirrhosis, with its subsequent complications. Hydrophobic bile salts are considered to promote liver fibrosis in cholestasis. However, evidence for this widely accepted hypothesis remains scarce.

In established animal models of cholestasis, e.g., by Mdr2 knockout, cholestasis and fibrosis are both secondary to biliary damage.

Therefore, to test the specific contribution of accumulating bile salts to liver fibrosis in cholestatic disease, the unique model of inducible hepatocellular cholestasis in cholate-fed Atp8b1G308V/G308V mice will be used. Glycochenodeoxycholate (GCDCA) is supplemented to humanize the murine bile salt pool, as confirmed by HPLC. Biomarkers of cholestasis and liver fibrosis are quantified. Hepatic stellate cells (HSC) isolated from wild-type mice are stimulated with bile salts. Proliferation, cell accumulation, and collagen deposition of HSC are determined. In cholestatic Atp8b1G308V/G308V mice, increased hepatic expression of $\alpha$SMA and collagenla mRNA and excess hepatic collagen deposition will indicate development of liver fibrosis only upon GCDCA supplementation. In vitro, numbers of myofibroblasts and deposition of collagen are increased after incubation with hydrophobic but not hydrophilic bile salts and associated with EGFR and MEK1/2 activation. Bile salts may have direct pro-fibrotic effects on HSC, putatively involving EGFR and MEK1/2 signalling.

Animal Experiments: Male animals are used for in vivo studies at 8 weeks of age. Animals are kept in a 12 h light-dark cycle and housed in an enriched environment with ad libitum access to diet and water.

Serum Biochemistry and Serum Bile Salt Measurements Serum levels of alkaline phosphatase, bilirubin, and alanine aminotransferase are quantified from fresh serum in a respons® 910 fully automated analyzer (DiaSys, Holzheim, Germany). Total serum bile salt levels are quantified enzymatically using a Diazyme total bile salts kit (Diazyme Laboratories, Poway, CA, USA) according to the manufacturer's instructions.

Liver Histology. Immunohistochemistry, and Hydroxyproline Quantification Paraffin blocks are cut into 4 $\mu$m thick slices and mounted on microscope slides (Superfrost plus, Thermo Scientific/Menzel, Braunschweig, Germany). After step-wise deparaffinization and rehydration, slides are stained with hematoxylin and eosin according to standard procedures. Immunohistochemistry is performed against alpha-SMA, using a monoclonal rabbit anti-alpha smooth muscle actin antibody (Abcam, Cambridge, UK). For collagen quantification, slides are stained for 1 h with Direct Red 80 (Sirius Red, Sigma-Aldrich, Darmstadt, Germany) and destained twice in ethanol and once in xylol. To quantify total DNA as a surrogate of cell number, HSCs were incubated with PicoGreen® (Invitrogen, Carlsbad, CA, USA) and fluorescence signals were detected with a CytoFluor 4000 system (PerSeptive Biosystems, Framingham, MA, USA). Proliferation of HSC is quantified using a BrdU-assay kit (Roche, Penzberg, Germany) according to the manufacturer's instructions. To quantify total cell count, HSCs, seeded in Lab-Tek II Chamber Slides (Nunc, Rochester, NY, USA USA), are mounted on cover slides with Vectashield mounting medium including DAPI (Vector, Burlingame, CA, USA). Slides are scanned with a Pannoramic Midi Slide Scanner (3DHistech, Budapest, Hungary) and nucleus count is performed with ImageJ2 software on the complete slide (0,7cm2).

Collagen Quantification In Vitro Cells are washed with PBS and stained for 1 h with 0.1% Sirius Red in saturated picric acid. Cells are then washed three times with 100% ethanol, the bound dye is dissolved in 50% methanol/sodium hydroxide (50 mmol/L), and absorption is measured at 540 nm.

**EXAMPLE 5.16: Treatment and/or prevention of hepatic fibrosis or cirrhosis (HF)**

Efficacy of the compounds according to the present invention for inhibiting spontaneous, chronic liver inflammation and fibrosis will be assessed on established NOD-Inflammation Fibrosis (N-IF) murine models as described by Fransén Pettersson et al. (PLoS One. 2018; 13(9): e0203228. doi: 10.1371/joumal.pone.0203228).

Animals: N-IF mice spontaneously develop chronic inflammation and liver fibrosis driven by T-cell receptor (TCR) transgenic natural killer T (NKT)-II cells generated in immunodeficient NOD.Rag2-/- mice. Several components of the liver pathology in the N-IF mouse overlap with those of human conditions in which a progressive chronic inflammation precedes the development of fibrosis. Moreover, the fibrosis developing in the N-IF mouse liver has been demonstrated to be associated with an accumulation of α-SMA expressing hepatic stellate cells. These phenotypic similarities make the N-IF mouse model unique compared to other available rodent models for liver fibrosis and provides a novel tool to test the efficacy of anti-fibrotic drug candidates.

Animals will be observed daily for signs of bad health and urine will be collected and protein will be measured in the urine. Liver and kidney weight will be recorded at time of dissection. Liver and kidney will be dissected from each animal fixed, sectioned and stained with Sirius Red and H&E. A piece of each liver was used for hydroxyproline measurements.

Hydroxyproline measurement: The hydroxyproline content of the liver and spleen will be determined using the Hydroxyproline Colorimetric Assay Kit (BioVision, Milpitas, CA, USA).

Histology: Following treatment, the mice will be anesthetized and perfused with PBS via intra-cardiac puncture. Livers and spleens will be weighed, and organ biopsies fixed in 4% neutral-buffered formalin, embedded in paraffin and sectioned.

**EXAMPLE 5.17: Treatment and/or prevention of non-alcoholic steatohepatitis (NASH)**

Efficacy of the compounds according to the present invention will be assessed on NASH murine model as described by Barbara Ulmasov B. et al. (Hepatology Communications, 2018, 3(2*) -*

*https://doi.org/10.1002/hep4.1298)*.

Mice: C57BL/6J 5-week-old male mice are housed in standard facilities under controlled conditions of temperature, humidity, and a 12-hour/12-hour light/dark cycle with free access to water.

Choline Deficient, L-Amino-Acid Defined, High-Fat Mouse Model of NASH: The choline deficient, amino-acid defined, high-fat diet (CDAHFD)22 are obtained from Research Diets (A06071309; New Brunswick, NJ). This diet is formulated as a high-fat, choline-deficient diet that includes 0.1% methionine and 45% Kcal% fat (20% lard, 25% soybean oil). The control diet is a standard rodent chow containing 13.6% of calories from fat. Starting from the age of 6 weeks, 30 mice are placed on the CDAHFD and 30 mice are placed on the standard diet. At the end of 6 weeks, 10 mice from each dietary group are fasted for 5 hours and killed. Blood and liver samples are collected. Livers are divided into sections that were fixed in 10% phosphate-buffered formalin, frozen in liquid nitrogen, or placed in an RNA stabilization solution for future evaluation. The remaining mice are kept on the diets for 4 more weeks for a total of 10 weeks and then fasted, killed, and samples are collected.

Biochemical Analysis: Hepatic triglyceride content was measured using the Triglyceride Colorimetric Assay kit (Cayman Chemicals, Ann Arbor, MI) according to the manufacturer's instructions. Plasma levels of alanine aminotransferase (ALT), aspartate aminotransferase (AST), glucose, insulin, triglyceride, and cholesterol were measured commercially by Advanced Veterinary Laboratory (Saint Louis, MO).

RT-PCR: Real-Time Quantitative Reverse-Transcription Polymerase Chain Reaction are performed to calculate changes in mRNA abundance.

Histopathology: Formalin-fixed liver sections are embedded in paraffin, sectioned at 5 μm, and stained by hematoxylin and eosin (H&E) using a standard protocol for microscopic evaluation. To evaluate liver collagen content, paraffin-embedded liver sections are stained with sirius red/fast green dyes. Sirius red binds to all types of collagens, whereas fast green stains noncollagenous proteins.

Liver sections are pretreated to remove paraffin, and nuclei are stained using Weigert's iron hematoxylin solution. After washing, tissues are stained with 0.1% sirius red (Direct Red 80; Sigma, Saint Louis, MO), 0.1% fast green FCF certified (Sigma) in saturated picric acid for 2 hours. Slides are then washed in water, dehydrated with ethanol and xylene, and finally are mounted in Permaslip (Alban Scientific, Inc., Saint Louis, MO). The degree of collagen accumulation is assessed by morphometric analysis.

Determination of Hepatic Hydroxyproline Content: Hydroxyproline content is determined as a measure of the amount of total collagen present in the liver. Liver tissues are homogenized in distilled water, precipitated with trichloroacetic acid, and hydrolyzed for 48 hours in 12 N HCl at 105°C. Samples are evaporated, and dry pellets are reconstituted in distilled water. Reconstituted samples are centrifuged for 10 minutes at 13,000g, and supernatants are diluted with 12 N HCl to achieve 4 N HCl concentration in the final samples. Liver hydroxyproline content is determined using the Sensitive Tissue Hydroxyproline Assay.

Immunohistochemistry: Formalin-fixed liver tissues are pretreated to remove paraffin by standard methods. Endogenous peroxidase activity is blocked by incubation in 3.3% hydrogen peroxide in methanol for 1 hour at room

temperature.

Apoptotic Cell Detection in Liver Tissues: Apoptotic cells in liver tissues are identified by labelling and detecting DNA strand breaks by the terminal deoxynucleotidyl transferase-mediated deoxyuridine triphosphate nick-end labeling method (TUNEL) using the ApopTag Peroxidase Detection kit (Millipore, Temecula, CA). Stained apoptotic cells with hepatocyte morphology are counted. To detect apoptotic cells with HSC morphology, TUNEL-stained liver tissues are subsequently stained with the antibody to desmin (a marker of HSC), except that secondary antibody peroxidase activity is detected with the ImmP ACT VIP Peroxidase Substrate kit (Vector Laboratories).

Western Blot: Western blotting is performed as described using the following primary antibodies: anti-phosphorylated mothers against decapentaplegic homolog 3 (p-SMAD3), ab52903, 1:1,000 (Abcam); anti-glyceraldehyde 3-phosphate dehydrogenase (anti-GAPDH), sc-25778 (Santa Cruz Biotechnology, Inc., Dallas, TX).

**EXAMPLE 5.18: Treatment and/or prevention of Diabetic Nephropathy or Diabetic Kidney Disease (DN or DKD)**

Efficacy of the compounds according to the present invention will be investigated *in vivo* using type I diabetes mouse model (STZ-induced diabetes).

Animal model: Male 10 weeks old 129/SV mice (Charles River, Germany) are held in individually ventilated cages, receiving a standard diet with free access to tap water. Weight-matched 129/SV mice receive either 125 mg/kg body weight STZ (Sigma-Aldrich) in 50 mM sodium citrate (pH 4.5) or sodium citrate buffer (nondiabetic control group) intraperitoneally on day 1 and 4 for induction of hyperglycemia (glucose > 15 mmol/l). Mice received no insulin during the study.

Diabetic mice are administered placebo (saline) for 12 weeks or the tested compounds. Body weight and glucose levels are measured every week. HbAlc (Olympus AU400) and Kidney function (serum creatinine) are measured at 6 and 12 weeks. Albuminuria is assessed using a mouse albumin ELISA kit.

Sensory nerve conduction velocity (NCV) studies are performed in mice anesthetized with 2% isoflurane at week 6 and 12. Tail sensory NCV is determined by stimulating proximally along the tail at a recorded distance of 3 cm. For the measurement, a neuro-screen from Toennies Inc. is used. After 12 weeks of follow up the mice are euthanized, and bilateral thoracotomy and laparotomy are performed and kidneys are perfused with ice cold saline solution via the left heart ventricle.

Immunohistochemistry: For immunofluorescence, paraformaldehyde-fixed and paraffin-embedded tissue sections (2 μm) are processed. After blocking with 10% rabbit serum, paraffin sections are stained with antibodies against pP38 and F4/80 and with a secondary antibody conjugated to Cy3.

**EXAMPLE 5.19: Treatment and/or prevention of Polycystic Kidney Disease (PKD or PCKD)**

Efficacy of the compounds according to the present invention will be investigated *in vivo* using Pcy mouse model as described by Lee EC et al. (Nat Commun 10, 4148 (2019*).*

Preclinical model: the pcy mouse, a genetically relevant rodent PKD model has a close correlation to human disease for use in novel agent efficacy evaluation. This model develops PKD associated with the gene that causes human disease, providing a translatable rodent model, which closely mirrors human PKD development. It has been thoroughly characterized for disease progression and response to treatment, providing a high level of confidence for drug discovery studies.

Animal model: pcy mouse, CD-1-pcy $^{lusm}$ strain (CrownBio) bearing a gene mutation associated with the same gene that causes human nephronophthisis type 3. Disease symptoms and progression are slowly progressive renal cystic disease with cysts that develop in the collecting tubules and other segments of the nephron become cystic as disease progresses. Male and female mice are similarly affected by the disease. Mice are 5 weeks old and receive either normal diet, normal diet with the vehicle (negative control), respectively the positive control and the test compounds all mixed with the normal diet. Controls: vehicle used for the compounds is used as the negative control in one animal group and tolvaptan a vasopressin receptor-2 (V2) antagonist is used as a positive control in one animal group.

Read out and endpoints: Cyst volume and fibrosis in kidney, including histological verification, kidney weight, serum BUN, concentration of test article in the blood, body weight and food intake will be recorded weekly.

**EXAMPLE 5.20: Treatment and/or prevention of Radiation-induced fibrosis (RIF)**

Efficacy of the compounds according to the present invention will be investigated in vivo using RIF mouse model as described by Ryu SH et al. (Oncotarget. 2016;7(13):15554-15565. doi:10.18632/oncotarget.6952).

Radiation-induced fibrosis (RIF) is one of the most common late complications of radiation therapy. In RIF a mouse model, leg contracture assay will be used to test the in vivo efficacy of compounds.

Radiation-induced fibrosis (RIF) is characterized by excessive accumulation of extracellular matrix in skin and

soft tissue, and the proliferation of fibroblasts is one of the most common late complications of radiation therapy. Also, RIF is an irreversible process to dead fibrous tissue and a dynamic process related to the remodelling of scar tissue by reactivated myofibroblasts.

RIF mouse model: Male BALB/c mice are used for the RIF mouse model. Under anesthesia, the right hind limb of each mouse receives radiation doses of 44 Gy (22 Gy × 2 times for 2 weeks) using a linear accelerator. Specially designed shielding is used to protect the rest of the body, and a 1 cm thick bolus are applied over the skin to ensure an adequate radiation dose on the surface of the hind leg. After irradiation, mice are randomly divided into two groups. Each group will be treated once daily with saline (control group) or compounds (treated group). Mice who received no irradiation or drug are used as a negative control group.

To demonstrate the anti-fibrotic effect of compounds in the skin and soft tissue of irradiated legs, epithelial thickness from the surface of the epidermis to the base of the dermis is measured using H & E staining.

**EXAMPLE 5.21: Treatment and/or prevention of Stargards Disease (SD)**

Efficacy of the compounds according to the present invention will be investigated in vivo using SD mouse model as described by Fang Y. et al. (Faseb Journal, 2020, DOI: 10.1096/fj.201901784RR).

Animals: Mice Pigmented Abca4-/- mice (129S4/SvJae-Abca4tm1Ght) will be purchased from The Jackson Laboratory (Bar Harbor, Maine, USA). Pigmented wild-type (WT) mice (129S2/SvPas) will be purchased from Janvier Labs (Le Genest-Saint-Isle, France). In each group, the male-to-female ratio was 1:1. The mice will be bred and housed in a 12:12-h light (approximately 50 lux in cages)-dark cycle with food and water ad libitum.

Blue-light illumination (BLI): Age-matched pigmented Abca4-/- and WT mice (9 monthold) are intraperitoneally anesthetized with three-component narcosis comprising 0.05 mg/kg of fentanyl, 5 mg/kg of midazolam, and 0.5 mg/kg of medetomidine. The pupils are dilated with a mixture of 0.5% tropicamide and 2.5% phenylephrine hydrochloride. METHOCEL (Omni Vision, Puchheim, Germany) is applied to moisten the cornea. During light illumination, a glass slip is placed on the cornea of the exposed eye. The illuminated eye of each mouse is exposed to blue light (wavelength: 430-nm) at an intensity of 50 mW/cm2 for 15 minutes. The nonilluminated eye as a control is covered carefully to shield from stray light.

A series of tests will be performed including Optical Coherence Tomography (OCT), Light Microscopy and Transmission Electron Microscopy (TEM), quantification of bisretinoids by HPLC, as well as full-field electroretinography ERG before and seven days after BLI.

**EXAMPLE 5.22: Treatment and/or prevention of proliferative vitreoretinopathy (PVR)**

Efficacy of the compounds according to the present invention will be investigated in vivo using PVR mouse model as described by Hou H et al. (Ophthalmic Res 2018;60:195-204. doi: 10.1159/000488492) or by Márkus B. et al. (FEBS Open Bio. 2017;7(8):1166-1177. Published 2017 Jun 29. doi:10.1002/2211-5463.12252).

Animal samples: A mouse model of PVR is used by applying an intravitreal injection of a proteolytic enzyme, dispase. This model is known to induce glial activation as well as both epi- and subretinal membrane formation.

Female 4- to 6-month-old wild-type are anesthetized with pentobarbital (90 mg·kg-1, i.p.), and also receive one drop of 1% procaine hydrochloride (Novocaine, EGIS, Budapest, Hungary) for local anesthesia and one drop of tropicamide (Mydrum, Chauvin Aubeans, Montpellier, France) for iris dilation. Four microliters of dispase (Sigma-Aldrich; 0.4 U·μL-1, dissolved in sterile physiological saline solution) is injected intravitreally into the right eyes under stereomicroscopic control (Ctrl) using an automatic pipette. Ctrl animals receive 4 μL of sterile physiological saline solution. Stratus optical coherence tomography images (OCT; Carl Zeiss Meditec, Dublin, CA, USA) are taken following injections to confirm PVR induction and monitor disease progression. Ctrl and dispase-treated mice are sacrificed at the 14th day following injections when signs of PVR formation were evident: presence of epiretinal membrane and/or retinal detachment on OCT examination.

Sample preparation and purification: The vitreous bodies of mice are isolated after guillotine removal of the cornea together with a scleral galler using a scalpel blade, followed by removal of the lens. After solubilization of the vitreous body with lysis buffer (pH 8.5) containing 7 m urea, 30 mm Tris, 2 m thiourea and 4% CHAPS, the lysates are sonicated in an ice-cold water bath for 5 min and centrifuged at 16 900 g for 10 min at 4 °C. The supernatants are transferred to LoBind Eppendorf tubes and purified by Ready-Prep 2-D CleanUp Kit (Bio-Rad Laboratories, Hercules, CA, USA) according to the manufacturer's protocol. Briefly, following precipitation and centrifugation, the pellets are dried and resuspended in 240 μL rehydration buffer containing 7 m urea, 2 m thiourea, 4% w/v CHAPS, 1% dithiothreitol (DTT), 2% v/v Bio-Lyte (Bio-Rad) and 0.001% bromophenol blue, and used immediately for isoelectric focusing.

2D gel electrophoresis: Three vitreous samples originating from each group were subjected to 2-DE. First immobilized pH gradient (IPG) strips with an IPG (24 cm, pH 4-7; Bio-Rad) were rehydrated with extracted vitreous proteins using passive rehydration at 20 °C overnight. This is followed by isoelectric focusing performed by applying

300 V for 3 h, which is gradually increased to 3500 V in 5 h and then held at 3500 V for 18 h. After isoelectric focusing, the IPG strips are immediately placed at -70 °C until equilibration. The IPG strips are equilibrated for 15 min in equilibration buffer (500 mm Tris/HCl, pH 8.5, 6 m urea, 2% SDS, 20% glycerol) containing 0.6% DTT and for 15 min in equilibration buffer containing 1.2% iodoacetamide. In the second dimension, the strips are laid on top of 12% polyacrylamide gels and covered with agarose. Using a Protean Plus Dodeca Cell (Bio-Rad) the electrophoresis is carried out at 100 mA per gel for 24 h until the bromophenol blue dye reached the bottom of the gel. All 12 gels are run together under the same conditions. Proteins are stained using in-house prepared ruthenium II tris(bathophenanthroline disulfonate) fluorescent dye 25 and gel images are recorded using Pharos FX Plus Molecular Imager (Bio-Rad). Three biological replicates are analyzed in all cases, the samples from the Ctrl and the dispase-treated groups are processed together on the same day.

**EXAMPLE 5.23: Treatment and/or prevention of Wet and Dry Age Related Macular Degeneration (ARMD or AMD)**

Efficacy of the compounds according to the present invention will be investigated in vivo using AMD murine model as described by Matsuda Y et al. (Mol Ther Nucleic Acids. 2019;17:819-828. doi:10.1016/j.omtn.2019.07.018).

Animals: Female C57BL/6J mice were obtained from Charles River Laboratories Japan. Male BN rats are obtained. Male New Zealand White (NZW) rabbits are obtained from Kitayama Labes, Japan. Mice, rats, and rabbits are maintained under special pathogen-free conditions.

EMT Assay of RPE Cells: RPE cells cultured at 37°C are seeded at 1 × 105 cells/well into 96-well microtiter culture plates. After incubation for 24 h, cells are treated with FGF2 (2 ng/mL) and TGF-β2 (3 ng/mL) with and without compounds according to the present invention for 3 days. The mRNA expression levels of the EMT biomarkers α-SMA and collagen type I are evaluated with quantitative real-time PCR.

Matrigel Plug Assay: 0.5 mL Matrigel Matrix GFR (growth factor reduced)-PRF (phenol red free) (BD Biosciences) solution is mixed with 1 μg FGF2 and subcutaneously implanted into the right flank of female C57BL/6J mice (8 weeks, n = 3). The compounds according to the present invention are administered intraperitoneally, and Matrigel plugs are removed and photographed on day 7 post-implantation. Grading of angiogenesis is determined by the hemoglobin concentration in the Matrigel plug, using the cyanmethemoglobin method according to the manufacturer's instructions. The optical density at 540 nm (OD540) values of the samples are measured in a microplate reader, and the hemoglobin concentrations are calculated in accordance with hemoglobin standards (Sigma-Aldrich).

Mouse Laser-Induced CNV Models: Mydrin-P ophthalmic solution is instilled into the eyes of the male C57BL/6J mice to dilate the pupils. Then laser irradiation (wavelength, 532 nm; spot size, 50 μm; irradiation time, 0.1 s; laser output, 120 mW) is conducted on 6 sites of the eye using a slit lamp (SL-130) and a multicolor laser photocoagulator (MC-300), avoiding large retinal capillaries. Immediately after CNV induction by laser irradiation, the tested compounds according to the invention and control are administered via intravitreal injection. Seven days after laser irradiation, a 4% FITC-dextran solution is administered into the tail vein at a volume of 0.5 ml/animal. One to five minutes after administration of FITC-dextran, the animals are euthanized by cervical dislocation. The eyeballs are removed and fixed in 4% paraformaldehyde-phosphate buffer for 12-24 h. Choroidal flat mounts are prepared under a stereoscopic microscope. Photographs of CNV sites are taken using a confocal microscope. Throughout the laser induced CNV animal experiments, successful irradiation is confirmed for each irradiation as air bubble formation in the eye of animal models.

Rat Laser-Induced CNV and Subretinal Fibrosis Models: The same technique will be employed in the rat model using male BN rats. For the CNV model, laser settings are spot size 80 μm and irradiation time of 0.05 s at 120 mW at 8 sites in the retina-choroid. For the subretinal fibrosis CNV model, the laser power used is 240 mW. Immediately after laser irradiation, intravitreal injection is performed with the control and tested compounds according to the present invention. For the subretinal fibrosis CNV studies, (1) saline vehicle and (2) compounds of the present invention (15 μg/eye for a single injection or 2 or 3 injections at 2-week intervals) are used. After laser irradiation, for the CNV studies, the FITC-dextran protocol as above is used 14 days after laser irradiation, with preparation of enucleated eyes for confocal microscopy of flat mount choroid. For the subretinal fibrosis studies, after 6 weeks, animals are sacrificed, and the enucleated eyes are prepared for histopathologic studies to quantify subretinal fibrosis using light microscopy. The eyes are embedded in paraffin blocks and sectioned and stained with Masson trichrome according to routine methods. Fibrosis is graded in a blinded manner by two or three independent investigators according to the following scale: grade 0, none; grade 1, minimal; grade 2, mild; grade 3, moderate; grade 4, severe.

**EXAMPLE 5.24: Treatment and/or prevention of Pterygium (PTE)**

Purpose will be to determine the in vivo effect of the compounds according to the present invention on choroidal angiogenesis and pterygium growth by using a choroidal neovascular murine model (CNV), a directed in vivo angiogenesis assay (DIVAA) and a pterygium murine model.

Pterygium is a benign fibrovascular growth of the ocular surface commonly associated to discomfort and red

eye, and as disease progresses is often related to decreased vision (topographic astigmatism) and ocular motility restriction in severe cases. A wide variety of pro-inflammatory cytokines induced by fibrogenic growth factors, oxidative stress and DNA methylation have been implicated in the pathogenesis of pterygium. Since some of these factors are affected by exposure to ultraviolet (UV) light, current evidence from multiple sources suggests that individuals with high exposure to sunlight are at increased risk of pterygium. Despite the extensive research, there is no clear understanding of the pathogenesis of pterygium, but one of the most important factors contributing to the pathogenesis are the neoplastic changes of limbal stem cells associated to UV light exposure and the possible role of oncogenic virus (Human papillomavirus).

To date, three animal models for pterygium have been described, using injection of human epithelial pterygium cells, exogenous extracellular matrix or UV scattered radiation in rabbit and mice. The results of the rabbit model using UV scattered light are focused on the computational prediction of the size and shape of the tissue growth, with no histological analysis. The mouse model showed histological characteristics of human pterygium, however, manipulation of rabbits for ophthalmological procedures is easier given that the ocular structure and size resembles more to that of human.

Methods: Mice will receive water with or without tested compounds. For the CNV, the neovascular lesion volume is determined in choroid-retinal pigment epithelial (RPE) flat mounts using confocal microscopy seven days after laser induction. For DIVAA, silicone capsules containing 10,000 human pterygium epithelial cells were implanted in the flanks of mice subcutaneously. After eleven days, neovascularization (NV) is quantified using spectrofluorimetry after murine tail-vein injection of fluorescein isothiocyanate (FITC)-labeled dextran. A pterygium epithelial cell model is developed by injecting 10,000 human pterygium epithelial cells in the nasal subconjunctival space in athymic nude mice.

Main outcome measures: Student's t-test was used to evaluate the data for the CNV and DIVAA models and histologic preparations were used to evaluate pterygia lesions.

**EXAMPLE 5.25: Treatment and/or prevention of Central serous chorioretinopathy (CSC)**

Therapeutic efficacy of the compounds according to the present invention will be investigated in vivo using CSC animal model as described by Wei-Da Chio, et al., Life Sci J 2019;16(12):115-126]. ISSN: 1097-8135).

The pathogenesis of central serous chorioretinopathy (CSC) is still not fully understood. The involvement of corticosteroids is undisputed, although their exact role has not been clarified; other parts of the underlying mechanism of CSC have been mainly elucidated by imaging techniques such as fluorescein and indocyanine green angiography. Even though most cases of CSC are self-limiting, severe as well as recurrent courses exist, and for these patients only a limited number of treatment options are available: laser photocoagulation, with a risk of scotoma and choroidal neovascularization, and photodynamic therapy.

Method: The treatment will be performed with males SD rats with CSCR. Right eye of cases were randomly divided into 2 groups (with control and with tested compounds) which underwent a series of examinations including indirect ophthalmoscopy, and OCT scanning every week. All SD rats will be inducted into the CSCR model first. If CSCR disappeared under OCT imaging, the compound is considered as efficient.

**EXAMPLE 5.26: Treatment and/or prevention of Cystic fibrosis (CF) affecting the lungs, but also the pancreas, liver, kidneys, and intestine**

Therapeutic efficacy of the compounds according to the present invention will be investigated in vivo using CF murine model as described by McCarron A et al. (Respir Res. 2018;19(1):54. Published 2018 Apr 2. doi:10.1186/s12931-018-0750-y*)*

In humans, cystic fibrosis (CF) lung disease is characterised by chronic infection, inflammation, airway remodelling, and mucus obstruction. A lack of pulmonary manifestations in CF mouse models has hindered investigations of airway disease pathogenesis, as well as the development and testing of potential therapeutics. However, recently generated CF animal models including rat, ferret and pig models demonstrate a range of well characterised lung disease phenotypes with varying degrees of severity. Different airway phenotypes of currently available CF animal models are available and presents potential applications of each model in airway-related CF research. In particular, mutant alleles with common human CF-causing mutations (e.g. Phe508del or Gly551Asp) introduced into the mouse CFTR sequence have been developed.

**EXAMPLE 5.27: Treatment and/or prevention of Idiopathic Pulmonary Fibrosis (IPF)**

Therapeutic efficacy of the compounds according to the present invention will be investigated in vivo using IPF animal model as described by Chen SS et al. (J Thorac Dis. 2017;9(1):96-105. doi:10.21037/jtd.2017.01.22).

IPF is a chronic progressive interstitial lung disease with severe pulmonary fibrosis. The etiology of IPF remains unclear. Patients usually only survive 2-3 years after being diagnosed with IPF. The incidence and development of IPF

are associated with epithelial cell injury, fibrocyte proliferation, inflammatory reactions, and extracellular matrix deposition. The pathological manifestation of IPF is usual interstitial pneumonia (UIP). Currently, the mortality in patients with IPF is quite high. However, effective treatments for IPF are lacking. The main cause of IPF-associated death is acute exacerbation of IPF (AE-IPF), which accounts for more than 50% of IPF-related deaths. Most patients with AE-IPF are unable to tolerate bronchoscopy or lung biopsy because of their critical condition. Lack of the biopsy of AE-IPF substantially limits in-depth investigations of AE-IPF. Experimental animal models that could mimic AE-IPF would be useful tools to study AE-IPF.

Animal models of bleomycin (BLM)-induced IPF were initially described in 1970. The rat model of IPF, which is developed by an intratracheal perfusion with BLM, has already been commonly used. Theoretically, a second intratracheal perfusion with BLM should induce additional lung injury in the rats that already develop pulmonary fibrosis from the first perfusion. The additional lung injury may resemble the pathological characteristics of AE-IPF.

Sprague Dawley (SD) rats are randomized into different groups: a control group treated with compounds alone, an AE-IPF model group with compounds + BLM + BLM group, an AE-IPF model group (cpd + BLM + BLM group), an IPF model group treated with compounds (Cpd + BLM group), an IPF model group (BLM group), and a normal control group without BLM.

Rats in the BLM + BLM groups undergo a second perfusion with BLM on day 28 after the first perfusion with BLM. Rats in the other two groups receive saline as the second perfusion. Six rats in each group are sacrificed on day 31, day 35, and day 42 after the first perfusion, respectively.

The pathophysiological characteristics of rats in the BLM + BLM group resemble those of patients with AE-IPF and as described (Chen SS, J Thorac Dis 2017;9(1):96-105), a second perfusion with BLM appears to induce acute exacerbation of pulmonary fibrosis and may be used to model AE-IPF in rats.

Effects of compounds formulations in this rat model using AE-IPF induced using single and respectively two intratracheal bleomycin perfusions should improve histopathology findings (H & E-and Masson stained sections analyzed and scored for acute lung injury) as well as survival.

## Claims

**1.** A compound of formula (I):

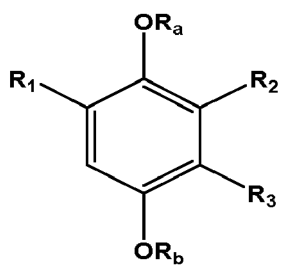

(I)

wherein:

$R_a$, $R_b$ = H, $C_{1-4}$acyl, aryl$C_{1-4}$alkyl;
$R_1$ = $COOR_4$, $(CH_2)_nCOOR_4$, $SO_3H$, $(CH_2)_nSO_3H$, CONH-$R_{10}$;
$R_2$ and $R_3$ are H or $R_5$, with the proviso that when one of $R_2$ and $R_3$ is $R_5$, the other is H;
$R_4$ = H, $C_{1-4}$alkyl, aryl$C_{1-4}$alkyl;
$R_5$ = $R_6$, $R_7$, or $R_8$
$R_6$ = CONH-$R_9$, CONHCO$R_9$, CONH$(CH_2)_n$-$R_9$, CONHCH$(COOR_4)(CH_2)_kR_9$, heteroaryl-$R_9$; wherein k = 0-4;
$R_7$ = benzoheteroaryl optionally substituted with one or more groups selected from: $COOR_4$, $(CH_2)_nCOOR_4$, $SO_3H$, $(CH_2)_nSO_3H$, $OR_4$, azoles [5-membered N-containing heterocycles], fluorine;
$R_8$ = $(CH_2)_mX(CH_2)_pR_9$;
X = O, S, $SO_2$, NH, NAc, $N(CH_2)_qR_9$;
$R_9$ = aryl, heteroaryl, cycloalkyl optionally substituted with one or more groups selected from $COOR_4$, $(CH_2)_nCOOR_4$, $(CH_2)_nSO_3H$, $OR_4$, $SO_3H$, azoles [5-membered N-containing heterocycles], fluorine, C=O, NH-CO-$R_{10}$;
$R_{10}$ = aryl, heteroaryl, optionally substituted with one or more groups selected from $COOR_4$, $(CH_2)_nCOOR_4$,

$(CH_2)_nSO_3H$, $OR_4$, $SO_3H$, azoles [5-membered N-containing heterocycles], fluorine;
n, m, p and q are independently 1-4.

**2.** The compound according to claim 1, wherein:

(A)

$R_a$, $R_b$ = H or $C_{1-4}$acyl;
$R_1$ = $COOR_{4;}$ $R_4$ = H or $C_{1-4}$alkyl;
$R_2$ = H; $R_3$ = $R_{5;}$ and
$R_5$ = $R_6$ = CONH-$R_9$;

or
(B)

$R_a$, $R_b$ = H or $C_{1-4}$acyl;
$R_1$ = $COOR_{4;}$ $R_4$ = H or $C_{1-4}$alkyl;
$R_3$ = H; $R_2$ = $R_{5;}$ and
$R_5$ = $R_6$ = CONH-$R_9$;

or
wherein said compound is selected form the group consisting of:

4-(2-carboxyphenylaminocarbonyl)-2,5-dihydroxybenzoic acid;
4-(2-(1H-tetrazol-5-yl)phenylaminocarbonyl)-2,5-dihydroxybenzoic acid;
2,5-dihydroxy-4-(2-sulfophenylaminocarbonyl)benzoic acid;
4-(3-carboxyphenylaminocarbonyl)-2,5-dihydroxybenzoic acid;
4-(3-(1H-tetrazol-5-yl)phenylaminocarbonyl)-2,5-dihydroxybenzoic acid;
2,5-dihydroxy-4-(3-sulfophenylaminocarbonyl)benzoic acid;
4-(4-carboxyphenylaminocarbonyl)-2,5-dihydroxybenzoic acid;
4-(4-(1H-tetrazol-5-yl)phenylaminocarbonyl)-2,5-dihydroxybenzoic acid;
2,5-dihydroxy-4-(4-sulfophenylaminocarbonyl)benzoic acid;
4-(3-carboxy-4-hydroxyphenylaminocarbonyl)-2,5-dihydroxybenzoic acid;
4-(4-carboxy-3-hydroxyphenylaminocarbonyl)-2,5-dihydroxybenzoic acid;
4-(4-carboxy-2,5-dihydroxyphenylaminocarbonyl)-2,5-dihydroxybenzoic acid;
4-(2-carboxy-4-hydroxyphenylaminocarbonyl)-2,5-dihydroxybenzoic acid;
3,5-bis(2,5-dihydroxy-4-carboxybenzoylamino)benzoic acid;
3-(4-carboxy-2,5-dihydroxybenzamido)phthalic acid;
2-(4-carboxy-2,5-dihydroxybenzamido)terephthalic acid;
2-(4-carboxy-2,5-dihydroxybenzamido)isophthalic acid;
4-(4-carboxy-2,5-dihydroxybenzamido)phthalic acid;
5-(4-carboxy-2,5-dihydroxybenzamido)isophthalic acid;
2-(4-carboxy-2,5-dihydroxybenzamido)nicotinic acid;
2-(4-carboxy-2,5-dihydroxybenzamido)isonicotinic acid;
6-(4-carboxy-2,5-dihydroxybenzamido)nicotinic acid;
6-(4-carboxy-2,5-dihydroxybenzamido)picolinic acid;
2-(4-carboxy-2,5-dihydroxybenzamido)-5-fluoronicotinic acid;
6-(4-carboxy-2,5-dihydroxybenzamido)pyridine-2,5-dicarboxylic acid;
2-(4-carboxy-2,5-dihydroxybenzamido)pyridine-3,5-dicarboxylic acid;
3-(4-carboxy-2,5-dihydroxybenzamido)isonicotinic acid;
5-(4-carboxy-2,5-dihydroxybenzamido)nicotinic acid;
5-(4-carboxy-2,5-dihydroxybenzamido)picolinic acid;
3-(4-carboxy-2,5-dihydroxybenzamido)picolinic acid;
5-(4-carboxy-2,5-dihydroxybenzamido)-2-fluoroisonicotinic acid;
4-(4-carboxy-2,5-dihydroxybenzamido)nicotinic acid;
4-(4-carboxy-2,5-dihydroxybenzamido)picolinic acid;
4-(4-(carboxymethyl)phenylaminocarbonyl)-2,5-dihydroxybenzoic acid;
4-(3-(carboxymethyl)phenylaminocarbonyl)-2,5-dihydroxybenzoic acid;

2,5-dihydroxy-4-(3-(1-hydroxy-1H-pyrazol-4-yl)phenylaminocarbonyl)benzoic acid;
4-(2-(carboxymethyl)phenylaminocarbonyl)-2,5-dihydroxybenzoic acid;
ethyl 4-(2-(ethoxycarbonyl)phenylaminocarbonyl)-2,5-diacetoxybenzoate;
ethyl 4-(2-(ethoxycarbonyl)phenylaminocarbonyl)-2,5-dihydroxybenzoate;
ethyl 4-(2-(1H-tetrazol-5-yl)phenylaminocarbonyl)-2,5-diacetoxybenzoate;
ethyl 4-(2-(1H-tetrazol-5-yl)phenylaminocarbonyl)-2,5-dihydroxybenzoate;
ethyl 2,5-dihydroxy-4-(4-hydroxy-3-(methoxycarbonyl)phenylaminocarbonyl)benzoate;
ethyl 3,5-bis(2,5-diacetoxy-4-ethoxycarbonylbenzoylamino)benzoate;
ethyl 3,5-bis(2,5-dihydroxy-4-ethoxycarbonylbenzoylamino)benzoate;
ethyl 3-(4-(ethoxycarbonyl)-2,5-dihydroxybenzamido)isonicotinate;
Ethyl 4-(4-ethoxycarbonyl-2,5-dihydroxyphenylaminocarbonyl)-2,5-dihydroxybenzoate;
3-(2-carboxyphenylaminocarbonyl)-2,5-dihydroxybenzoic acid;
3-(2-(1H-tetrazol-5-yl)phenylaminocarbonyl)-2,5-dihydroxybenzoic acid;
2,5-dihydroxy-3-(2-sulfophenylaminocarbonyl)benzoic acid;
3-(3-carboxyphenylaminocarbonyl)-2,5-dihydroxybenzoic acid;
3-(3-(1H-tetrazol-5-yl)phenylaminocarbonyl)-2,5-dihydroxybenzoic acid;
2,5-dihydroxy-3-(3-sulfophenylaminocarbonyl)benzoic acid;
3-(4-carboxyphenylaminocarbonyl)-2,5-dihydroxybenzoic acid;
3-(4-(1H-tetrazol-5-yl)phenylaminocarbonyl)-2,5-dihydroxybenzoic acid;
2,5-dihydroxy-3-(4-sulfophenylaminocarbonyl)benzoic acid;
3-(3-carboxy-4-hydroxyphenylaminocarbonyl)-2,5-dihydroxybenzoic acid;
3-(4-carboxy-3-hydroxyphenylaminocarbonyl)-2,5-dihydroxybenzoic acid;
3-(2-carboxy-4-hydroxyphenylaminocarbonyl)-2,5-dihydroxybenzoic acid;
3,5-Bis(2,5-dihydroxy-3-carboxybenzoylamino)benzoic acid;
3-(3-carboxy-2,5-dihydroxybenzamido)phthalic acid;
2-(3-carboxy-2,5-dihydroxybenzamido)terephthalic acid;
2-(3-carboxy-2,5-dihydroxybenzamido)isophthalic acid;
4-(3-carboxy-2,5-dihydroxybenzamido)phthalic acid;
5-(3-carboxy-2,5-dihydroxybenzamido)isophthalic acid;
2-(3-carboxy-2,5-dihydroxybenzamido)nicotinic acid;
2-(3-carboxy-2,5-dihydroxybenzamido)isonicotinic acid;
6-(3-carboxy-2,5-dihydroxybenzamido)nicotinic acid;
6-(3-carboxy-2,5-dihydroxybenzamido)picolinic acid;
2-(3-carboxy-2,5-dihydroxybenzamido)-5-fluoronicotinic acid;
6-(3-carboxy-2,5-dihydroxybenzamido)pyridine-2,5-dicarboxylic acid;
2-(3-carboxy-2,5-dihydroxybenzamido)pyridine-3,5-dicarboxylic acid;
3-(3-carboxy-2,5-dihydroxybenzamido)isonicotinic acid;
5 -(3 -carboxy-2,5-dihydroxybenzamido)nicotinic acid;
5 -(3 -carboxy-2,5-dihydroxybenzamido)picolinic acid;
3 -(3 -carboxy-2,5-dihydroxybenzamido)picolinic acid
5-(3-carboxy-2,5-dihydroxybenzamido)-2-fluoroisonicotinic acid;
4-(3-carboxy-2,5-dihydroxybenzamido)nicotinic acid;
4-(3-carboxy-2,5-dihydroxybenzamido)picolinic acid;
3-(4-(carboxymethyl)phenylaminocarbonyl)-2,5-dihydroxybenzoic acid;
3-(3-(carboxymethyl)phenylaminocarbonyl)-2,5-dihydroxybenzoic acid;
2,5-dihydroxy-3-(3-(1-hydroxy-1H-pyrazol-4-yl)phenylaminocarbonyl)benzoic acid;
3-(2-(carboxymethyl)phenylaminocarbonyl)-2,5-dihydroxybenzoic acid;
ethyl 3-(2-(1H-tetrazol-5-yl)phenylaminocarbonyl)-2,5-dihydroxybenzoate; and
ethyl 3-(2-(ethoxycarbonylmethyl)phenylaminocarbonyl)-2,5-dihydroxybenzoate;

**3.** The compound according to claim 1, wherein:

(A)

$R_a$, $R_b$ = H;
$R_1$ = $SO_3H$;
$R_2$ = H; $R_3$ = $R_5$; and
$R_5$ = $R_6$ = CONH-$R_9$;

or

(B)

$R_a$, $R_b$ = H;

$R_1$ = $SO_3H$;

$R_3$ = H; $R_2$ = $R_5$; and

$R_5$ = $R_6$ = CONH-$R_9$;

or

wherein said compound is selected form the group consisting of:

2-(2,5-dihydroxy-4-sulfobenzamido)benzoic acid;
2,5-dihydroxy-4-(2-sulfophenylaminocarbonyl)benzenesulfonic acid;
3-(2,5-dihydroxy-4-sulfobenzamido)benzoic acid;
2,5-dihydroxy-4-(3-sulfophenylaminocarbonyl)benzenesulfonic acid;
4-(2,5-dihydroxy-4-sulfobenzamido)benzoic acid;
2,5-dihydroxy-4-(4-sulfophenylaminocarbonyl)benzenesulfonic acid;
5-(2,5-dihydroxy-4-sulfobenzamido)-2-hydroxybenzoic acid;
4-(2,5-dihydroxy-4-sulfobenzamido)-2-hydroxybenzoic acid;
2-(2,5-dihydroxy-4-sulfobenzamido)-5-hydroxybenzoic acid;
3,5-bis(2,5-dihydroxy-4-sulfobenzamido)benzoic acid;
3-(2,5-dihydroxy-4-sulfobenzamido)phthalic acid;
2-(2,5-dihydroxy-4-sulfobenzamido)terephthalic acid;
2-(2,5-dihydroxy-4-sulfobenzamido)isophthalic acid;
4-(2,5-dihydroxy-4-sulfobenzamido)phthalic acid;
5-(2,5-dihydroxy-4-sulfobenzamido)isophthalic acid;
2-(2,5-dihydroxy-4-sulfobenzamido)nicotinic acid;
2-(2,5-dihydroxy-4-sulfobenzamido)isonicotinic acid;
6-(2,5-dihydroxy-4-sulfobenzamido)nicotinic acid;
6-(2,5-dihydroxy-4-sulfobenzamido)picolinic acid;
2-(2,5-dihydroxy-4-sulfobenzamido)-5-fluoronicotinic acid;
6-(2,5-dihydroxy-4-sulfobenzamido)pyridine-2,5-dicarboxylic acid;
2-(2,5-dihydroxy-4-sulfobenzamido)pyridine-3,5-dicarboxylic acid;
3-(2,5-dihydroxy-4-sulfobenzamido)isonicotinic acid;
5-(2,5-dihydroxy-4-sulfobenzamido)nicotinic acid;
5-(2,5-dihydroxy-4-sulfobenzamido)picolinic acid;
3-(2,5-dihydroxy-4-sulfobenzamido)picolinic acid;
5-(2,5-dihydroxy-4-sulfobenzamido)-2-fluoroisonicotinic acid;
4-(2,5-dihydroxy-4-sulfobenzamido)nicotinic acid;
4-(2,5-dihydroxy-4-sulfobenzamido)picolinic acid;
(4-(2,5-dihydroxy-4-sulfobenzamido)phenyl)acetic acid;
(3-(2,5-dihydroxy-4-sulfobenzamido)phenyl)acetic acid;
(2-(2,5-dihydroxy-4-sulfobenzamido)phenyl)acetic acid;
2-(2,5-dihydroxy-3-sulfobenzamido)benzoic acid;
2,5-dihydroxy-3-(2-sulfophenylaminocarbonyl)benzenesulfonic acid;
3 -(2,5-dihydroxy-3 -sulfobenzamido)benzoic acid;
2,5-dihydroxy-3-(3-sulfophenylaminocarbonyl)benzenesulfonic acid;
4-(2,5-dihydroxy-3-sulfobenzamido)benzoic acid;
2,5-dihydroxy-3-(4-sulfophenylaminocarbonyl)benzenesulfonic acid;
5-(2,5-dihydroxy-3-sulfobenzamido)-2-hydroxybenzoic acid;
4-(2,5-dihydroxy-3-sulfobenzamido)-2-hydroxybenzoic acid;
2-(2,5-dihydroxy-3-sulfobenzamido)-5-hydroxybenzoic acid;
3-(2,5-dihydroxy-3-sulfobenzamido)-5-(2,5-dihydroxy-4-sulfobenzamido)benzoic acid
3 -(2,5-dihydroxy-3 -sulfobenzamido)phthalic acid;
2-(2,5-dihydroxy-3-sulfobenzamido)terephthalic acid;
2-(2,5-dihydroxy-3-sulfobenzamido)isophthalic acid;
4-(2,5-dihydroxy-3-sulfobenzamido)phthalic acid;
5-(2,5-dihydroxy-3-sulfobenzamido)isophthalic acid;

2-(2,5-dihydroxy-3-sulfobenzamido)nicotinic acid;
2-(2,5-dihydroxy-3-sulfobenzamido)isonicotinic acid;
6-(2,5-dihydroxy-3-sulfobenzamido)nicotinic acid;
6-(2,5-dihydroxy-3-sulfobenzamido)picolinic acid;
2-(2,5-dihydroxy-3-sulfobenzamido)-5-fluoronicotinic acid;
6-(2,5-dihydroxy-3-sulfobenzamido)pyridine-2,5-dicarboxylic acid;
2-(2,5-dihydroxy-3-sulfobenzamido)pyridine-3,5-dicarboxylic acid;
3-(2,5-dihydroxy-3-sulfobenzamido)isonicotinic acid;
5-(2,5-dihydroxy-3-sulfobenzamido)nicotinic acid;
5-(2,5-dihydroxy-3-sulfobenzamido)picolinic acid;
3-(2,5-dihydroxy-3-sulfobenzamido)picolinic acid;
5-(2,5-dihydroxy-3-sulfobenzamido)-2-fluoroisonicotinic acid;
4-(2,5-dihydroxy-3-sulfobenzamido)nicotinic acid;
4-(2,5-dihydroxy-3-sulfobenzamido)picolinic acid;
(4-(2,5-dihydroxy-3-sulfobenzamido)phenyl)acetic acid;
(3-(2,5-dihydroxy-3-sulfobenzamido)phenyl)acetic acid; and
(2-(2,5-dihydroxy-3-sulfobenzamido)phenyl)acetic acid.

**4.** The compound according to claim 1, wherein:

(A)

$R_a$, $R_b$ = H;
$R_1$ = $(CH_2)_nCOOR_4$; $R_4$ = H, $C_{1-4}$alkyl, aryl$C_{1-4}$alkyl;
$R_2$ = H or $R_5$, $R_3$ = $R_{5;}$ and
$R_5$ = $R_6$ = CONH-$R_9$;

Or
(B)

$R_a$, $R_b$ = H;
$R_1$ = $(CH_2)_nCOOR_4$; $R_4$ = H, $C_{1-4}$alkyl, aryl$C_{1-4}$alkyl;
$R_3$ = H; $R_2$ = $R_{5;}$ and
$R_5$ = $R_6$ = CONH-$R_9$;

or wherein said compound is selected form the group consisting of:

2-(4-(carboxymethyl)-2,5-dihydroxybenzamido)benzoic acid;
(2-(1H-tetrazol-5-yl)phenylaminocarbonyl)-2,5-dihydroxyphenyl)acetic acid;
(2,5-dihydroxy-4-(2-sulfophenylaminocarbonyl)phenyl)acetic acid;
3-(4-(carboxymethyl)-2,5-dihydroxybenzamido)benzoic acid;
(2,5-dihydroxy-4-(3-sulfophenylaminocarbonyl)phenyl)acetic acid;
4-(4-(carboxymethyl)-2,5-dihydroxybenzamido)benzoic acid;
2-(2,5-dihydroxy-4-(4-sulfophenylaminocarbonyl)phenyl)acetic acid;
5-(4-(carboxymethyl)-2,5-dihydroxybenzamido)-2-hydroxybenzoic acid;
4-(4-(carboxymethyl)-2,5-dihydroxybenzamido)-2-hydroxybenzoic acid;
2-(4-(carboxymethyl)-2,5-dihydroxybenzamido)-5-hydroxybenzoic acid;
3,5-Bis(2,5-dihydroxy-4-carboxymethylbenzoylamino)benzoic acid
3-(4-(carboxymethyl)-2,5-dihydroxybenzamido)phthalic acid;
2-(4-(carboxymethyl)-2,5-dihydroxybenzamido)terephthalic acid;
2-(4-(carboxymethyl)-2,5-dihydroxybenzamido)isophthalic acid;
4-(4-(carboxymethyl)-2,5-dihydroxybenzamido)phthalic acid;
5-(4-(carboxymethyl)-2,5-dihydroxybenzamido)isophthalic acid;
2-(4-(carboxymethyl)-2,5-dihydroxybenzamido)nicotinic acid;
2-(4-(carboxymethyl)-2,5-dihydroxybenzamido)isonicotinic acid;
6-(4-(carboxymethyl)-2,5-dihydroxybenzamido)nicotinic acid;
6-(4-(carboxymethyl)-2,5-dihydroxybenzamido)picolinic acid;
2-(4-(carboxymethyl)-2,5-dihydroxybenzamido)-5-fluoronicotinic acid;

6-(4-(carboxymethyl)-2,5-dihydroxybenzamido)pyridine-2,5-dicarboxylic acid;
2-(4-(carboxymethyl)-2,5-dihydroxybenzamido)pyridine-3,5-dicarboxylic acid;
3-(4-(carboxymethyl)-2,5-dihydroxybenzamido)isonicotinic acid;
5-(4-(carboxymethyl)-2,5-dihydroxybenzamido)nicotinic acid;
5-(4-(carboxymethyl)-2,5-dihydroxybenzamido)picolinic acid;
3-(4-(carboxymethyl)-2,5-dihydroxybenzamido)picolinic acid;
5-(4-(carboxymethyl)-2,5-dihydroxybenzamido)-2-fluoroisonicotinic acid;
4-(4-(carboxymethyl)-2,5-dihydroxybenzamido)nicotinic acid;
4-(4-(carboxymethyl)-2,5-dihydroxybenzamido)picolinic acid;
(4-(4-(carboxymethyl)-2,5-dihydroxybenzamido)phenyl)acetic acid;
(3-(4-(carboxymethyl)-2,5-dihydroxybenzamido)phenyl)acetic acid;
(2-(4-(carboxymethyl)-2,5-dihydroxybenzamido)phenyl)acetic acid;
methyl 2-(4-(ethoxycarbonylmethyl)-2,5-dihydroxybenzamido)benzoate;
ethyl (4-(2-(1H-tetrazol-5-yl)phenylaminocarbonyl)-2,5-dihydroxyphenyl)acetate;
diethyl 5-(4-(ethoxycarbonylmethyl)-2,5-dihydroxybenzamido)isophthalate;
methyl 3,5-bis(4-(ethoxycarbonylmethyl)-2,5-dihydroxybenzamido)benzoate;
2-(3-(carboxymethyl)-2,5-dihydroxybenzamido)benzoic acid;
(2,5-dihydroxy-3-(2-sulfophenylaminocarbonyl)phenyl)acetic acid;
3-(3-(carboxymethyl)-2,5-dihydroxybenzamido)benzoic acid;
(2,5-dihydroxy-3-(3-sulfophenylaminocarbonyl)phenyl)acetic acid;
4-(3-(carboxymethyl)-2,5-dihydroxybenzamido)benzoic acid;
(2,5-dihydroxy-3-(4-sulfophenylaminocarbonyl)phenyl)acetic acid;
5-(3-(carboxymethyl)-2,5-dihydroxybenzamido)-2-hydroxybenzoic acid;
4-(3-(carboxymethyl)-2,5-dihydroxybenzamido)-2-hydroxybenzoic acid;
2-(3-(carboxymethyl)-2,5-dihydroxybenzamido)-5-hydroxybenzoic acid;
3,5-Bis(2,5-dihydroxy-3-carboxymethylbenzoylamino)benzoic acid
3-(3-(carboxymethyl)-2,5 -dihydroxybenzamido)phthalic acid;
2-(3-(carboxymethyl)-2,5-dihydroxybenzamido)terephthalic acid;
2-(3-(carboxymethyl)-2,5-dihydroxybenzamido)isophthalic acid;
4-(3-(carboxymethyl)-2,5-dihydroxybenzamido)phthalic acid;
5 -(3 -(carboxymethyl)-2,5 -dihydroxybenzamido)isophthalic acid;
2-(3-(carboxymethyl)-2,5-dihydroxybenzamido)nicotinic acid;
2-(3-(carboxymethyl)-2,5-dihydroxybenzamido)isonicotinic acid;
6-(3-(carboxymethyl)-2,5-dihydroxybenzamido)nicotinic acid;
6-(3-(carboxymethyl)-2,5-dihydroxybenzamido)picolinic acid;
2-(3-(carboxymethyl)-2,5-dihydroxybenzamido)-5-fluoronicotinic acid;
6-(3-(carboxymethyl)-2,5-dihydroxybenzamido)pyridine-2,5-dicarboxylic acid;
2-(3-(carboxymethyl)-2,5-dihydroxybenzamido)pyridine-3,5-dicarboxylic acid;
3-(3-(carboxymethyl)-2,5-dihydroxybenzamido)isonicotinic acid;
5 -(3 -(carboxymethyl)-2,5 -dihydroxybenzamido)nicotinic acid;
5 -(3 -(carboxymethyl)-2,5 -dihydroxybenzamido)picolinic acid;
3 -(3 -(carboxymethyl)-2,5 -dihydroxybenzamido)picolinic acid;
5-(3-(carboxymethyl)-2,5-dihydroxybenzamido)-2-fluoroisonicotinic acid;
4-(3-(carboxymethyl)-2,5-dihydroxybenzamido)nicotinic acid;
4-(3-(carboxymethyl)-2,5-dihydroxybenzamido)picolinic acid;
(4-(3-(carboxymethyl)-2,5-dihydroxybenzamido)phenyl)acetic acid;
(3-(3-(carboxymethyl)-2,5-dihydroxybenzamido)phenyl)acetic acid; and
(2 -(3-(carboxymethyl)-2,5-dihydroxybenzamido)phenyl)acetic acid.

**5.** The compound according to claim 1, wherein:

(A)

$R_a$, $R_b$ = H; $C_{1-4}$acyl, aryl$C_{1-4}$alkyl;
$R_1$ = CONH-$R_{10}$;
$R_2$ = H; $R_3$ = $R_5$; and
$R_5$ = $R_6$ = CONH-$R_9$;

Or

(B)

$R_a$, $R_b$ = H; $C_{1-4}$acyl, aryl$C_{1-4}$alkyl;
R1 = CONH-$R_{10}$;
$R_3$ = H; $R_2$ = $R_5$; and
$R_5$ = $R_6$ = CONH-$R_9$.

or

wherein said compound is selected form the group consisting of:

5-(4-(2-(1H-tetrazol-5-yl)phenylaminocarbonyl)-2,5-dihydroxybenzamido)-2-hydroxybenzoic acid;
$N_1$,$N_4$-bis(2-(1H-tetrazol-5-yl)phenyl)-2,5-dihydroxyterephthalamide;
5-(4-(3-carboxy-4-hydroxyphenylaminocarbonyl)-2,5-dihydroxybenzamido)-2-hydroxybenzoic acid;
5-(2,5-dihydroxy-4-(4-hydroxy-3-sulfophenylaminocarbonyl)benzamido)-2-hydroxybenzenesulphonic acid;
4-(4-(4-carboxy-3-hydroxyphenylaminocarbonyl)-2,5-dihydroxybenzamido)-2-hydroxybenzoic acid;
4-(2,5-dihydroxy-4-(3-hydroxy-4-sulfophenylaminocarbonyl)benzamido)-2-hydroxybenzenesulphonic acid;
2-(2,5-dihydroxy-4-(4-hydroxy-2-carboxyphenylaminocarbonyl)benzamido)-5-hydroxybenzoic acid;
2-(2,5-dihydroxy-4-(4-hydroxy-2-sulfophenylaminocarbonyl)benzamido)-5-hydroxybenzene sulphonic acid;
methyl 5-(4-(2-(1H-tetrazol-5-yl)phenylaminocarbonyl)-2,5-dihydroxybenzamido)-2-hydroxybenzoate;
methyl 5-(2,5-diacetoxy-4-(4-acetoxy-3-(methoxycarbonyl)phenylaminocarbonyl)benzamido)-2-acetoxybenzoate;
methyl 5-(2,5-dihydroxy-4-(4-hydroxy-3-(methoxycarbonyl)phenylaminocarbonyl)benzamido)-2-hydroxybenzoate;
methyl 2-(2,5-dihydroxy-4-(4-hydroxy-2-(methoxycarbonyl)phenylaminocarbonyl)benzamido)-5-hydroxybenzoate;
5-(3-(3-carboxy-4-hydroxyphenylaminocarbonyl)-2,5-dihydroxybenzamido)-2-hydroxybenzoic acid;
5-(2,5-dihydroxy-3-(4-hydroxy-3-sulfophenylaminocarbonyl)benzamido)-2-hydroxybenzenesulphonic acid;
4-(3-(3-hydroxy-4-carboxyphenylaminocarbonyl)-2,5-dihydroxybenzamido)-2-hydroxybenzoic acid;
4-(2,5-dihydroxy-3-(3-hydroxy-4-sulfophenylaminocarbonyl)benzamido)-2-hydroxybenzenesulphonic acid;
2-(3-(2-carboxy-4-hydroxyphenylaminocarbonyl)-2,5-dihydroxybenzamido)-5-hydroxylbenzoic acid; and
2-(2,5-dihydroxy-3-(4-hydroxy-2-sulfophenylaminocarbonyl)benzamido)-5-hydroxybenzenesulphonic acid.

**6.** The compound according to claim 1, wherein:

(A)

$R_a$, $R_b$ = H;
R1 = $COOR_4$; $(CH_2)_nCOOR_4$; $R_4$ = H or $C_{1-4}$alkyl;
$R_2$ = H; $R_3$ = $R_5$; and
$R_5$ = $R_6$ = $CONHCOR_9$;

or

(B)

$R_a$, $R_b$ = H;
$R^1$ = $COOR_4$; $(CH_2)$nCOOR4; R4 = H or $C_{1-4}$alkyl;
$R_3$ = H; $R_2$ = $R_5$; and
$R_5$ = $R_6$ = $CONHCOR_9$;

or wherein said compound is selected form the group consisting of:

N-(1,3,4,5 -tetrahydroxycyclohexylcarbonyl) 4-carboxy-2,5 -dihydroxybenzamide;
N-(4-carboxy-2,5-dihydroxybenzoyl)4-carboxy-2,5-dihydroxy-benzamide;
N-(3-carboxy-2,5-dihydroxybenzoyl)4-carboxy-2,5-dihydroxy-benzamide;
N-(3,4-dihydroxybenzoyl) 4-carboxy-2,5-dihydroxybenzamide;
N-(3,5-dihydroxybenzoyl) 4-carboxy-2,5-dihydroxybenzamide;
N-(1,3,4,5-tetrahydroxycyclohexylcarbonyl) 3-carboxy-2,5-dihydroxybenzamide;

N-(4-carboxy-2,5-dihydroxybenzoyl) 3-carboxy-2,5-dihydroxy-benzamide;
N-(3-carboxy-2,5-dihydroxybenzoyl) 3-carboxy-2,5-dihydroxy-benzamide;
N-(3,4-dihydroxybenzoyl) 3-carboxy-2,5-dihydroxybenzamide;
N-(3,5-dihydroxybenzoyl) 3-carboxy-2,5-dihydroxybenzamide;
N-(1,3,4,5-tetrahydroxycyclohexylcarbonyl) 4-carboxymethyl-2,5-dihydroxybenzamide;
N-(4-carboxy-2,5-dihydroxybenzoyl) 4-carboxymethyl-2,5-dihydroxy-benzamide;
N-(3-carboxy-2,5-dihydroxybenzoyl) 4-carboxymethyl-2,5-dihydroxy-benzamide;
N-(3,4-dihydroxybenzoyl) 4-carboxymethyl-2,5-dihydroxybenzamide;
N-(3,5-dihydroxybenzoyl) 4-carboxymethyl-2,5-dihydroxybenzamide;
N-(1,3,4,5-tetrahydroxycyclohexylcarbonyl) 3-carboxymethyl-2,5-dihydroxybenzamide;
N-(4-carboxy-2,5-dihydroxybenzoyl) 3-carboxymethyl-2,5-dihydroxy-benzamide;
N-(3-carboxy-2,5-dihydroxybenzoyl) 3-carboxymethyl-2,5-dihydroxy-benzamide;
N-(3,4-dihydroxybenzoyl) 3-carboxymethyl-2,5-dihydroxybenzamide; and
N-(3,5-dihydroxybenzoyl) 3-carboxymethyl-2,5-dihydroxybenzamide.

**8.** A compound according to claim 1, wherein

(A)

$R_a$, $R_b$ = H;
$R_1$ = $COOR_4$; $(CH_2)_nCOOR_4$; $R_4$ = H or $C_{1-4}$alkyl;
$R_2$ = H; $R_3$ = $R_5$; and
$R_5$ = $R_6$ = $CONH(CH_2)_n$-$R_9$;

Or
(B)

$R_a$, $R_b$ = H;
$R_1$ = $COOR_4$; $(CH_2)_nCOOR_4$; $R_4$ = H or $C_{1-4}$alkyl;
$R_3$ = H; $R_2$ = $R_5$; and
$R_5$ = $R_6$ = $CONH(CH_2)_n$-$R_9$;

or wherein said compound is selected form the group consisting of:

4-(3,4-dihydroxyphenylmethylaminocarbonyl)-2,5-dihydroxybenzoic acid;
4-(2-(3,4-dihydroxyphenyl)ethylaminocarbonyl)-2,5-dihydroxybenzoic acid;
4-(3,5-dihydroxyphenylmethylaminocarbonyl)-2,5-dihydroxybenzoic acid;
4-((4,5-dihydroxy-3-oxocyclohex-1-enyl)methylaminocarbonyl)-2,5-dihydroxybenzoic acid;
2,5-dihydroxy-4-((3,4,5-trihydroxycyclohexyl)methylaminocarbonyl)benzoic acid;
4-(2-carboxyphenylmethylaminocarbonyl)-2,5-dihydroxybenzoic acid;
4-(3-carboxyphenylmethylaminocarbonyl)-2,5-dihydroxybenzoic acid;
4-(4-carboxyphenylmethylaminocarbonyl) 2,5-dihydroxybenzoic acid;
3-(3,4-dihydroxyphenylmethylaminocarbonyl)-2,5-dihydroxybenzoic acid;
3-(2-(3,4-dihydroxyphenyl)ethylaminocarbonyl)-2,5-dihydroxybenzoic acid;
3-(3,5-dihydroxyphenylmethylaminocarbonyl)-2,5-dihydroxybenzoic acid;
3-((4,5-dihydroxy-3-oxocyclohex-1-enyl)methylaminocarbonyl)-2,5-dihydroxybenzoic acid;
2,5-dihydroxy-3-((3,4,5-trihydroxycyclohexyl)methylaminocarbonyl)benzoic acid;
3-(2-carboxyphenylmethylaminocarbonyl)-2,5-dihydroxybenzoic acid;
3-(3-carboxyphenylmethylaminocarbonyl)-2,5-dihydroxybenzoic acid;
3-(4-carboxyphenylmethylaminocarbonyl)-2,5-dihydroxybenzoic acid;
(4-(3,4-dihydroxyphenylmethylaminocarbonyl)-2,5-dihydroxyphenyl)acetic acid;
(4-(3,5-dihydroxyphenylmethylaminocarbonyl)-2,5-dihydroxyphenyl)acetic acid;
(4-((4,5-dihydroxy-3-oxocyclohex-1-enyl)methylaminocarbonyl)-2,5-dihydroxyphenyl)acetic acid;
(2,5-dihydroxy-4-((3,4,5-trihydroxycyclohexyl)methylaminocarbonyl)phenyl)acetic acid;
(4-(2-carboxyphenylmethylaminocarbonyl)-2,5-dihydroxyphenyl)acetic acid;
(4-(3-carboxyphenylmethylaminocarbonyl)-2,5-dihydroxyphenyl)acetic acid;
(4-(4-carboxyphenylmethylaminocarbonyl)-2,5-dihydroxyphenyl)acetic acid;
(3-(3,4-dihydroxyphenylmethylaminocarbonyl)-2,5-dihydroxyphenyl)acetic acid;

(3-(3,5-dihydroxyphenylmethylaminocarbonyl)-2,5-dihydroxyphenyl)acetic acid;
(3-((4,5-dihydroxy-3-oxocyclohex-1-enyl)methylaminocarbonyl)-2,5-dihydroxyphenyl)acetic acid;
(2,5-dihydroxy-3-((3,4,5-trihydroxycyclohexyl)methylcarbonylamino)phenyl)acetic acid;
(3-(2-carboxyphenylmethylaminocarbonyl)-2,5-dihydroxyphenyl)acetic acid;
(3-(3-carboxyphenylmethylaminocarbonyl)-2,5-dihydroxyphenyl)acetic acid; and
(3-(4-carboxyphenylmethylaminocarbonyl)-2,5-dihydroxyphenyl)acetic acid.

**9.** A compound according to claim 1, wherein

$R_a$, $R_b$ = H;
$R_1$ = $SO_3H$;
$R_2$ = H; $R_3$ = $R_5$; and
$R_5$ = $R_6$ = CONH$(CH_2)_n$-$R_9$;

or wherein said compound is selected form the group consisting of:

2-((2,5-dihydroxy-4-sulfobenzamido)methyl)benzoic acid;
3-((2,5-dihydroxy-4-sulfobenzamido)methyl)benzoic acid; and
4-((2,5-dihydroxy-4-sulfobenzamido)methyl)benzoic acid.

**10.** The compound according to claim 1, wherein:

(A)

$R_a$, $R_b$ = H;
$R_1$ = $COOR_4$; $(CH_2)_nCOOR_4$; $R_4$ = H or $C_{1-4}$alkyl;
$R_2$ = H; $R_3$ = $R_5$; and
$R_5$ = $R_6$ = CONHCH$(COOR_4)(CH_2)_kR_9$;

or
(B)

$R_a$, $R_b$ = H;
$R_1$ = $COOR_4$; $(CH_2)_nCOOR_4$; $R_4$ = H or $C_{1-4}$alkyl;
$R_3$ = H; $R_2$ = $R_5$; and
$R_5$ = $R_6$ = CONHCH$(COOR_4)(CH_2)_kR_9$;

or wherein said compound is selected form the group consisting of:

4-(1-carboxy-2-(3,4-dihydroxyphenyl)ethylaminocarbonyl)-2,5-dihydroxybenzoic acid;
4-(carboxy(3,4-dihydroxyphenyl)methylaminocarbonyl)-2,5-dihydroxybenzoic acid;
3-(1-carboxy-2-(3,4-dihydroxyphenyl)ethylaminocarbonyl)-2,5-dihydroxybenzoic acid
3-(carboxy(3,4-dihydroxyphenyl)methylaminocarbonyl)-2,5-dihydroxybenzoic acid;
(4-(carboxy(3,4-dihydroxyphenyl)methylaminocarbonyl)-2,5-dihydroxyphenyl)acetic acid; and
(3-(carboxy(3,4-dihydroxyphenyl)methylaminocarbonyl)-2,5-dihydroxyphenyl)acetic acid.

**11.** The compound according to claim 1, wherein

(A)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl;, aryl$C_{1-4}$alkyl;
$R_2$ = H; $R_3$ = $R_5$; and
$R_5$ = $R_6$ = heteroaryl-$R_9$;

or
(B)

$R_a$, $R_b$ = H;
$R_1$ = $COOR_4$; $(CH_2)_nCOOR_4$; $SO_3H$, $(CH_2)_nSO_3H$, CONH-$R_{10}$;
$R_4$ = H, $C_{1-4}$alkyl;, aryl$C_{1-4}$alkyl;
$R_3$ = H; $R_2$ = $R_5$; and
$R_5$ = $R_6$ = heteroaryl-$R_9$;

or wherein said compound is selected form the group consisting of:

4,6-Bis(4-carboxy-2,5-dihydroxyphenyl)-1,3,5-triazin-2-one;
4-(4-carboxy-2,5-dihydroxyphenyl)-6-(3-carboxy-2,5-dihydroxyphenyl)-1,3,5-triazin-2-one;
4-(4-carboxy-2,5-dihydroxyphenyl)-6-(3,4-dihydroxyphenyl)-1,3,5-triazin-2-one;
4-(4-carboxy-2,5-dihydroxyphenyl)-6-(3,5-dihydroxyphenyl)-1,3,5-triazin-2-one;
4,6-Bis(4-carboxy-2,5-dihydroxyphenyl)-1,3,5-triazine;
2-(4-Carboxy-2,5-dihydroxyphenyl)-4-(3-carboxy-2,5-dihydroxyphenyl)-1,3,5-triazine;
2-(4-Carboxy-2,5-dihydroxyphenyl)-4-(3,4-dihydroxyphenyl)-1,3,5-triazine;
2-(4-Carboxy-2,5-dihydroxyphenyl)-4-(3,5-dihydroxyphenyl)-1,3,5-triazine;
4,6-Bis(3-carboxy-2,5-dihydroxyphenyl)-1,3,5-triazin-2-one;
4-(3-Carboxy-2,5-dihydroxyphenyl)-6-(4-carboxy-2,5-dihydroxyphenyl)-1,3,5-triazin-2-one;
4-(3-Carboxy-2,5-dihydroxyphenyl)-6-(3,4-dihydroxyphenyl)-1,3,5-triazin-2-one;
4-(3-Carboxy-2,5-dihydroxyphenyl)-6-(3,5-dihydroxyphenyl)-1,3,5-triazin-2-one;
4,6-Bis(3-carboxy-2,5-dihydroxyphenyl)-1,3,5-triazine;
2-(3-Carboxy-2,5-dihydroxyphenyl)-4-(4-carboxy-2,5-dihydroxyphenyl)-1,3,5-triazine;
2-(3-Carboxy-2,5-dihydroxyphenyl)-4-(3,4-dihydroxyphenyl)-1,3,5-triazine;
2-(3-Carboxy-2,5-dihydroxyphenyl)-4-(3,5-dihydroxyphenyl)-1,3,5-triazine;
2-(3 -Carboxymethyl-2,5-dihydroxyphenyl)-4-(3,4-dihydroxyphenyl)-1,3,5-triazine;
2-(3-Carboxymethyl-2,5-dihydroxyphenyl)-4-(3,5-dihydroxyphenyl)-1,3,5-triazine;
4-(4-Carboxymethyl-2,5-dihydroxyphenyl)-6-(3,4-dihydroxyphenyl)-1,3,5-triazin-2-one;
4-(4-Carboxymethyl-2,5-dihydroxyphenyl)-6-(3,5-dihydroxyphenyl)-1,3,5-triazin-2-one;
2-(4-Carboxy-2,5-dihydroxyphenyl)-4-(4-carboxymethyl-2,5-dihydroxyphenyl)-1,3,5-triazine;
2-(3-Carboxy-2,5-dihydroxyphenyl)-4-(4-carboxymethyl-2,5-dihydroxyphenyl)-1,3,5-triazine;
2-(4-Carboxymethyl-2,5-dihydroxyphenyl)-4-(3,4-dihydroxyphenyl)-1,3,5-triazine;
2-(4-Carboxymethyl-2,5-dihydroxyphenyl)-4-(3,5-dihydroxyphenyl)-1,3,5-triazine;
4-(4-Carboxy-2,5-dihydroxyphenyl)-6-(3-carboxymethyl-2,5-dihydroxyphenyl)-1,3,5-triazin-2-one;
4-(3-Carboxy-2,5-dihydroxyphenyl)-6-(3-carboxymethyl-2,5-dihydroxyphenyl)-1,3,5-triazin-2-one;
4-(3-Carboxymethyl-2,5-dihydroxyphenyl)-6-(3,4-dihydroxyphenyl)-1,3,5-triazin-2-one;
4-(3-Carboxymethyl-2,5-dihydroxyphenyl)-6-(3,5-dihydroxyphenyl)-1,3,5-triazin-2-one;
2-(4-Carboxy-2,5-dihydroxyphenyl)-4-(3-carboxymethyl-2,5-dihydroxyphenyl)-1,3,5-triazine;
2-(3-Carboxy-2,5-dihydroxyphenyl)-4-(3-carboxymethyl-2,5-dihydroxyphenyl)-1,3,5-triazine;
2-(3-Carboxymethyl-2,5-dihydroxyphenyl)-4-(3,4-dihydroxyphenyl)-1,3,5-triazine; and
2-(3-Carboxymethyl-2,5-dihydroxyphenyl)-4-(3,5-dihydroxyphenyl)-1,3,5-triazine.

**12.** The compound according to claim 1, wherein:

(A)

$R_a$, $R_b$ = H;
$R_1$ = $COOR_4$, $(CH_2)_nCOOR_4$, $SO_3H$, $(CH_2)_nSO_3H$, CONH-$R_{10}$;
$R_4$ = H, $C_{1-4}$alkyl;, aryl$C_{1-4}$alkyl;
$R_2$ = H; $R_3$ = $R_5$; and
$R_5$ = $R_7$ = benzoheteroaryl optionally substituted with one or more groups selected from: $COOR_4$, $(CH_2)_nCOOR_4$, $SO_3H$, $(CH_2)_nSO_3H$, $OR_4$, azoles [5-membered N-containing heterocycles], fluorine;

or
(B)

$R_a$, $R_b$ = H;
$R_1$ = $COOR_4$, $(CH_2)_nCOOR_4$, $SO_3H$, $(CH_2)_nSO_3H$, CONH-$R_{10}$;
$R_4$ = H, $C_{1-4}$alkyl;, aryl$C_{1-4}$alkyl;

$R_3$ = H; $R_2$ = $R_5$; and
$R_5$= $R_7$ = benzoheteroaryl optionally substituted with one or more groups selected from: COOR4, (CH2)nCOOR4, $SO_3H$, (CH2)nSO3H, OR4, azoles [5-membered N-containing heterocycles], fluorine;

or wherein said compound is selected form the group consisting of:

2-(4-Carboxy-2,5-dihydroxyphenyl)-1H-benzo[d]imidazole-4-carboxylic acid;
Methyl 2-(4-ethoxycarbonyl-2,5-dihydroxyphenyl)-1H-benzo [d] imidazole-4-carboxylate;
2-(2,5-Dihydroxy-4-sulfophenyl)-1H-benzo[d]imidazole-4-carboxylic acid;
2-(4-Carboxy-2,5-dihydroxyphenyl)-1H-benzo[d]imidazole-5-carboxylic acid;
2-(2,5-Dihydroxy-4-sulfophenyl)-1H-benzo[d]imidazole-5-carboxylic acid;
2-(3-Carboxy-2,5-dihydroxyphenyl)-1H-benzo[d]imidazole-4-carboxylic acid;
2-(2,5-Dihydroxy-3-sulfophenyl)-1H-benzo[d]imidazole-4-carboxylic acid;
2-(3-Carboxy-2,5-dihydroxyphenyl)-1H-benzo[d]imidazole-5-carboxylic acid;
2-(2,5-Dihydroxy-3-sulfophenyl)-1H-benzo[d]imidazole-5-carboxylic acid;
2-(4-(Carboxymethyl)-2,5-dihydroxyphenyl)-1H-benzo[d]imidazole-4-carboxylic acid;
2-(4-(Carboxymethyl)-2,5-dihydroxyphenyl)-1H-benzo[d]imidazole-5-carboxylic acid;
2-(3-(Carboxymethyl)-2,5-dihydroxyphenyl)-1H-benzo[d]imidazole-4-carboxylic acid; and
2-(3-(Carboxymethyl)-2,5-dihydroxyphenyl)-1H-benzo[d]imidazole-5-carboxylic acid.

**13.** The compound according to claim 1, wherein:

(A)

$R_a$, $R_b$ = H, $C_{1-4}$ acyl, aryl$C_{1-4}$alkyl;
$R_1$ = $COOR_4$, $(CH_2)_nCOOR_4$, $SO_3H$, $(CH_2)_nSO_3H$, CONH-$R_{10}$;
$R_4$ = H, $C_{1-4}$alkyl;, aryl$C_{1-4}$alkyl;
$R_2$ = H; $R_3$ = $R_5$; and
$R_5$ = $R_8$ = $(CH_2)_mX(CH_2)pR_9$;
wherein X = O, S, $SO_2$, NH, NAc, $N(CH_2)_qR_9$;

or
(B)

$R_a$, $R_b$ = H, C1-4acyl, arylC1-4alkyl;
R1 = $COOF_4$, (CH2)nCOOR4, $SO_3H$, $(CH_2)_nSO_3H$, CONH-$R_{10}$;
$R_4$ = H, $C_{1-4}$alkyl;, aryl$C_{1-4}$alkyl;
$R_3$ = H; $R_2$ = $R_5$; and
$R_5$ = $R_8$ = $(CH_2)_mX(CH_2)_pR_9$;
wherein X = O, S, $SO_2$, NH, NAc, $N(CH_2)_qR_9$;

or wherein said compound is selected form the group consisting of:

Bis(4-carboxy-2,5-dihydroxyphenylmethyl)amine;
4-((2,5-Dihydroxy-4-sulfophenylmethylamino)methyl)-2,5-dihydroxybenzoic acid;
4-((2,5-Dihydroxy-3-sulfophenylmethylamino)methyl)-2,5-dihydroxybenzoic acid;
Bis(2,5-dihydroxy-4-sulfophenylmethyl)amine;
N,N-Bis(4-carboxy-2,5-dihydroxyphenylmethyl)acetamide;
N-(2,5-dihydroxy-4-sulfophenylmethyl) N-(4-carboxy-2,5-dihydroxyphenylmethyl)acetamide;
N-(2,5-dihydroxy-3-sulfophenylmethyl) N-(4-carboxy-2,5-dihydroxyphenylmethyl)acetamide;
N,N-Bis(2,5-dihydroxy-4-sulfophenylmethyl)acetamide;
4-((2,5-Dihydroxy-4-carboxyphenyl)methylthiomethyl)-2,5-dihydroxybenzoic acid;
4-((2,5-Dihydroxy-4-sulfophenyl)methylthiomethyl)-2,5-dihydroxybenzoic acid;
4-((2,5-Dihydroxy-3-sulfophenyl)methylthiomethyl)-2,5-dihydroxybenzoic acid;
4-((2,5-Dihydroxy-4-sulfophenyl)methylthiomethyl)-2,5-dihydroxybenzenesulfonic acid;
4-((2,5-Dihydroxy-4-carboxyphenyl)methylsulfonylmethyl)-2,5-dihydroxybenzoic acid;
4-((2,5-Dihydroxy-4-sulfophenyl)methylsulfonylmethyl)-2,5-dihydroxybenzoic acid;
4-((2,5-Dihydroxy-3-sulfophenyl)methylsulfonylmethyl)-2,5-dihydroxybenzoic acid;

4-((2,5-Dihydroxy-4-sulfophenyl)methylsulfonylmethyl)-2,5-dihydroxybenzenesulfonic acid;
4-((2,5-Dihydroxy-4-carboxyphenyl)methoxymethyl)-2,5-dihydroxybenzoic acid;
4-((2,5-dihydroxy-4-sulfophenyl)methoxymethyl)-2,5-dihydroxybenzoic acid;
4-((2,5-dihydroxy-3-sulfophenyl)methoxymethyl)-2,5-dihydroxybenzoic acid;
4-((2,5-Dihydroxy-4-sulfophenyl)methoxymethyl)-2,5-dihydroxybenzenesulfonic acid;
Tris (4-carboxy-2,5-dihydroxyphenylmethyl)amine;
Tris (4-ethoxycarbonyl-2,5-dihydroxyphenylmethyl)amine;
Bis(3-carboxy-2,5-dihydroxyphenylmethyl)amine;
3-((2,5-Dihydroxy-3-sulfophenylmethylamino)methyl)-2,5-dihydroxybenzoic acid;
3-((2,5-Dihydroxy-4-sulfophenylmethylamino)methyl)-2,5-dihydroxybenzoic acid;
Bis(2,5-dihydroxy-3-sulfophenylmethyl)amine;
N,N-Bis(3 -carboxy-2,5-dihydroxyphenylmethyl)acetamide;
N-(2,5-dihydroxy-3-sulfophenylmethyl) N-(3-carboxy-2,5-dihydroxyphenylmethyl)acetamide;
N-(2,5-dihydroxy-4-sulfophenylmethyl) N-(3-carboxy-2,5-dihydroxyphenylmethyl)acetamide;
N,N-Bis(2,5-dihydroxy-3-sulfophenylmethyl)acetamide;
3-((2,5-Dihydroxy-3-carboxyphenyl)methylthiomethyl)-2,5-dihydroxybenzoic acid;
3-((2,5-Dihydroxy-3-sulfophenyl)methylthiomethyl)-2,5-dihydroxybenzoic acid;
3 -((2,5 -Dihydroxy-4-sulfophenyl)methylthiomethyl)-2,5 -dihydroxybenzoic acid;
3-((2,5-Dihydroxy-3-sulfophenyl)methylthiomethyl)-2,5-dihydroxybenzenesulfonic acid;
3-((2,5-Dihydroxy-3-carboxyphenyl)methylsulfonylmethyl)-2,5-dihydroxybenzoic acid;
3-((2,5-Dihydroxy-3-sulfophenyl)methylsulfonylmethyl)-2,5-dihydroxybenzoic acid;
3-((2,5-Dihydroxy-4-sulfophenyl)methylsulfonylmethyl)-2,5-dihydroxybenzoic acid;
3-((2,5-Dihydroxy-3-sulfophenyl)methylsulfonylmethyl)-2,5-dihydroxybenzenesulfonic acid;
3-((2,5-Dihydroxy-3-carboxyphenyl)methoxymethyl)-2,5-dihydroxybenzoic acid;
3-((2,5-dihydroxy-3-sulfophenyl)methoxymethyl)-2,5-dihydroxybenzoic acid;
3-((2,5-dihydroxy-4-sulfophenyl)methoxymethyl)-2,5-dihydroxybenzoic acid;
3-((2,5-Dihydroxy-3-sulfophenyl)methoxymethyl)-2,5-dihydroxybenzenesulfonic acid;
Methyl 3-((2,5-diacetoxy-3-methoxycarbonylphenyl)methylsulfonylmethyl)-2,5-diacetoxybenzoate;
Methyl 3-((2,5-dihydroxy-3-methoxycarbonylphenyl)methylsulfonylmethyl)-2,5-hydroxybenzoate;
Tris (3-carboxy-2,5-dihydroxyphenylmethyl)amine; and
Tris (3-ethoxycarbonyl-2,5-dihydroxyphenylmethyl)amine;

**14.** A pharmaceutical composition comprising a therapeutically effective amount of the compound according to any one of claims 1-13 or a pharmaceutically acceptable salt, or a prodrug, or stereoisomer thereof, and a pharmaceutically acceptable excipient.

**15.** A compound according to any one of the claims 1-13 for use in a method of treating and/or preventing autoimmune, immunological, rheumatology, vascular disorders, ophthalmologic disorders, fibrotic disorders, metabolic and gastrointestinal disorders, neuroinflammatory and neurodegenerative diseases, neoplasms and cancer associated disorders, hormone related diseases and immunological disorders resulting from viral and bacterial infectious diseases and complications thereof.

Figure 1

A)

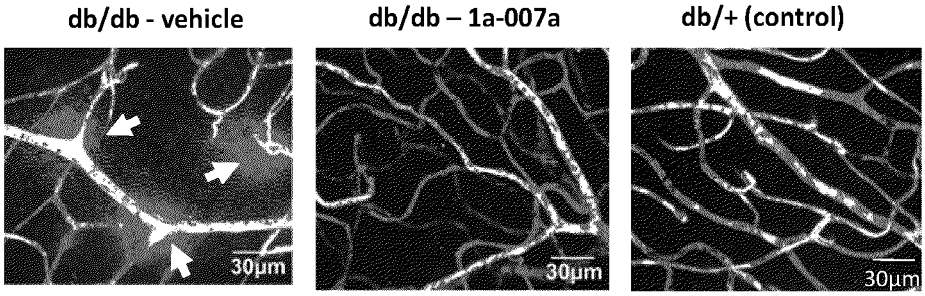
db/db - vehicle
db/db – 1a-007a
db/+ (control)
30µm
30µm
30µm

B)

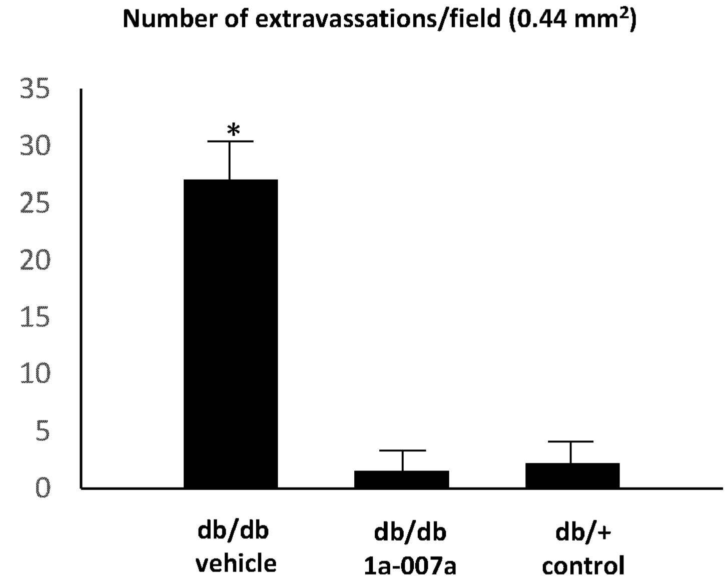
Number of extravassations/field (0.44 mm²)
35
30
25
20
15
10
5
0
*
db/db
vehicle
db/db
1a-007a
db/+
control

Figure 2

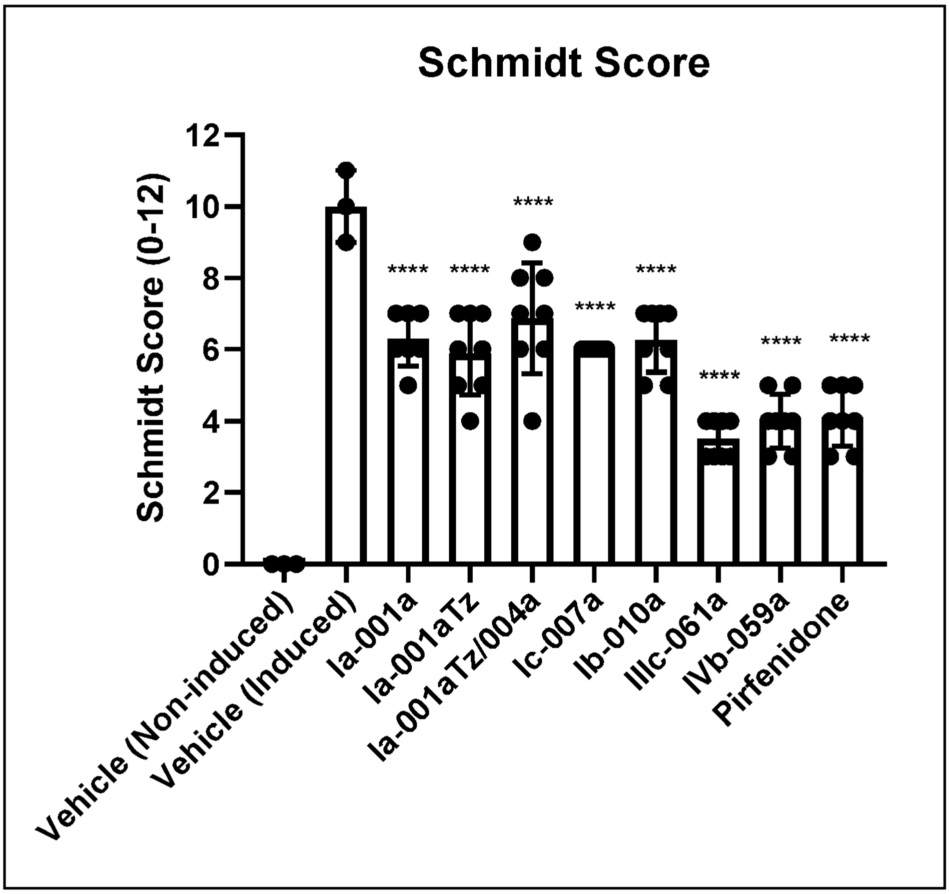
Schmidt Score
Schmidt Score (0-12)
0
2
4
6
8
10
12
****
Vehicle (Non-induced)
Vehicle (Induced)
Ia-001a
Ia-001aTz
Ia-001aTz/004a
Ic-007a
Ib-010a
IIIc-061a
IVb-059a
Pirfenidone

Figure 3 A

| Cytokines / Diseases | IL-1b | IL-2 | IL-3 | IL-4 | IL-5 | IL-6 | IL-7 | IL-8 | IL-9 | IL-10 | IL-12p70 | IL-13 | IL-14 | IL-15 | IL-17A | IL-17F | IL-18 | IL-21 | IL-22 | IL-23 | IL-33 | IFNγ | IFNb | TNFα | TGFbeta | TNFbeta | MCP-1 | MMPs |
|---|---|---|---|---|---|---|---|---|---|---|---|---|---|---|---|---|---|---|---|---|---|---|---|---|---|---|---|---|
| ALL/AML | | | x | | | | | x | | | | | | | | | | | | | | | | | | | | |
| Allergic Asthma | x | | x | x | x | x | | | x | | | x | | x | x | | x | | | | | | x | x | | | | |
| Allergic Rhinitis | | | | x | | | | | | x | | | | | | | x | | | | | | | x | x | | | |
| Alzheimer | x | | | | | | | | | | | | | | | | x | | | | | | | | x | | | |
| Ankylosing spondylitis | | | | | | | | | | | | | | | x | | | | | | | | | x | | | | x |
| Atherosclerosis | | | | | | | | | | | | | | | | | | | | | | | | | | | | |
| Atopic Dermatitis | | | | | x | | x | | | x | | | | | x | | x | | | | | x | | x | | | | |
| Bacterial infections | x | | | | | | | | | | | | | | | | | | | | | | | | | | | |
| Behcet disease | | | | | | | | | | | | | | | | | | x | | | | | | | | | | |
| Cancer | x | | x | | | | | | | x | x | | | | | | | x | x | | | x | | x | | | | |
| Cardiovascular pathologies | | | | | | | | | | | | | | | | | | | | | | | | | x | | | |
| CLL | | | | x | | | | | | | | | | | | | | | | | | | | | | | | |
| COPD | x | | | | x | | | x | | | | | | | | | | | | | | | | | | | | |
| Crohn's disease | | | | | | | | | | | | | | | | x | x | | | x | x | | | x | | | | |
| Cystic fibrosis | | | | | | | | x | | | | | | | | | | | | | | | | | | | | |
| Diabetes | | | | x | | | x | | | x | | | | x | | | x | | | | | x | | | | | | |
| Eosinophilic GI | | | | | x | | | | | | | | | | | | | | | | | | | | | | | |
| Experimental Autoimmune Encephalomyelitis | | | | | | | | | | | | | | | | | | | | | | x | | | | | | |
| Fibrosis | | | | | | x | | | | | | x | | | | | | | | | | | | | x | | | |
| Food Allergy | | | | | | | | | x | | | | | | | | | | | | | | | | | | | |
| GHVD | | | | | | | | | | | | | | | | | | | | | | | | | | | | |
| Giant cell arthritis | | | | | | x | | | | | | | | | | | | | | | | | | x | | | | |
| Gout | x | | | | | x | | x | | | | | | | | | | | | | | | | | | | x | |
| Grave's disease | x | | | | | | | | | | | | | | | | | | | | | | | | | | | |

Figure 3 B

| Diseases \ Cytokines | IL-1b | IL-2 | IL-3 | IL-4 | IL-5 | IL-6 | IL-7 | IL-8 | IL-9 | IL-10 | IL-12p70 | IL-13 | IL-14 | IL-15 | IL-17A | IL-17F | IL-18 | IL-21 | IL-22 | IL-23 | IL-33 | IFNγ | IFNb | TNFα | TGFbeta | TNFbeta | MCP-1 | MMPs |
|---|---|---|---|---|---|---|---|---|---|---|---|---|---|---|---|---|---|---|---|---|---|---|---|---|---|---|---|---|
| Helminth infections | | | | | | | | | x | | | | | | | | | | | | | | | | | | | |
| Inflammatory Bowel Disease | x | | | | | | x | | | x | x | | | x | x | x | | | x | | | | | x | | | | |
| JID juvenile idiopathic arthritis<br>AID, autoinflammatory disease including Still's disease | x | | | | | x | | | | | | | | | | | | | | | | | | x | | | | |
| Lymphoma | | | | | | | | | | | | | x | | | | | | | | | | | | | | | |
| Multiple sclerosis | x | | | | | | x | | | | | | | x | | | x | | | | | | x | | | | | |
| Myocardial infection | x | | | | | | | | | | | | | | | | | | | | | | | | | | | |
| Osteoarthritis | x | | | | | | | x | | | | | | | | | | | | | | | | | | | | x |
| Pancreatitis | | | | | | x | | x | | | | | | | x | | | | | | | | | | x | | | |
| Parasites infections | | | | x | | | | | | | | | | | | | | | | | x | | | | | | | |
| Psoriasis | x | | | | | x | | x | | | x | | | x | x | | x | | x | x | | | | x | | | | |
| Psoriatic arthritis | | | | | | | | | | | | | | | | | | | | | | | | x | | | | |
| Rheumatoid arthritis | x | | | | | x | x | x | | x | | | | x | x | | x | x | | | x | x | x | x | | | | |
| Sjogren syndrom | | | | | | | | | | | | | x | | | | | | | | | | | | | | | |
| Systematic Lupus Ery. | | | | | | x | | | | x | | | x | x | | | | x | | | | | x | x | | | | |
| Systemic Sclerosis | | | | | | | | | | | | | | | | | | | | | | | | x | | | | |
| T cell mediated autoimmune diseases | | x | | | | | | | | | | | | | | | | | | | | | | | | | | |
| Ulcerative colitis | | | | | | | | | x | | | | | | | | | | | x | | | | x | | | | |
| Vascular diseases | | | | | | | | | | | | | | | | | | | | | x | | | x | | | | |
| Viral infections | x | | | | | | | | | | | | | | | | | | | | | | x | | | | | |

Figure 3 C

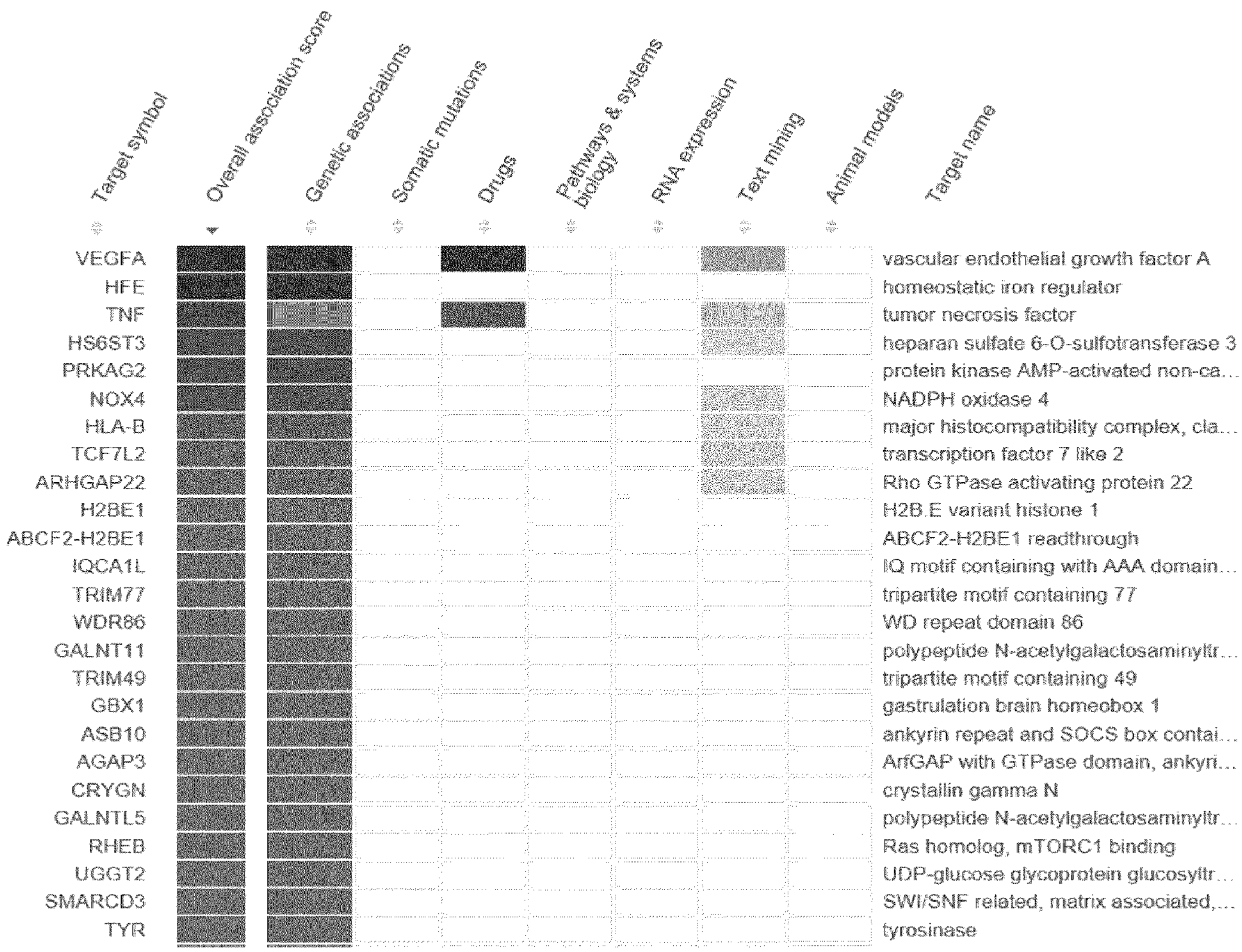
Target symbol
Overall association score
Genetic associations
Somatic mutations
Drugs
Pathways & systems biology
RNA expression
Text mining
Animal models
Target name
VEGFA
HFE
TNF
HS6ST3
PRKAG2
NOX4
HLA-B
TCF7L2
ARHGAP22
H2BE1
ABCF2-H2BE1
IQCA1L
TRIM77
WDR86
GALNT11
TRIM49
GBX1
ASB10
AGAP3
CRYGN
GALNTL5
RHEB
UGGT2
SMARCD3
TYR
vascular endothelial growth factor A
homeostatic iron regulator
tumor necrosis factor
heparan sulfate 6-O-sulfotransferase 3
protein kinase AMP-activated non-ca...
NADPH oxidase 4
major histocompatibility complex, cla...
transcription factor 7 like 2
Rho GTPase activating protein 22
H2B.E variant histone 1
ABCF2-H2BE1 readthrough
IQ motif containing with AAA domain...
tripartite motif containing 77
WD repeat domain 86
polypeptide N-acetylgalactosaminyltr...
tripartite motif containing 49
gastrulation brain homeobox 1
ankyrin repeat and SOCS box contai...
ArfGAP with GTPase domain, ankyri...
crystallin gamma N
polypeptide N-acetylgalactosaminyltr...
Ras homolog, mTORC1 binding
UDP-glucose glycoprotein glucosyltr...
SWI/SNF related, matrix associated,...
tyrosinase

Figure 3 D

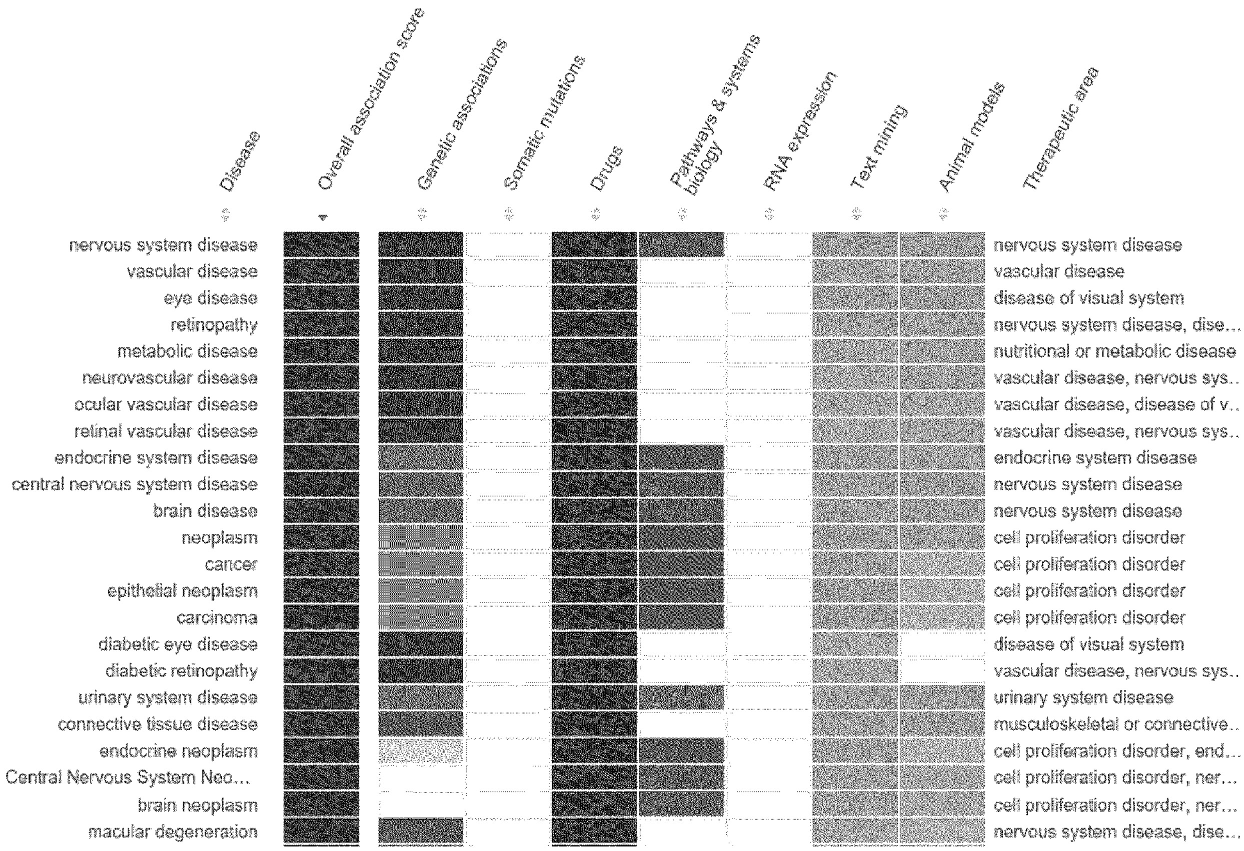
Disease
Overall association score
Genetic associations
Somatic mutations
Drugs
Pathways & systems biology
RNA expression
Text mining
Animal models
Therapeutic area
nervous system disease
vascular disease
eye disease
retinopathy
metabolic disease
neurovascular disease
ocular vascular disease
retinal vascular disease
endocrine system disease
central nervous system disease
brain disease
neoplasm
cancer
epithelial neoplasm
carcinoma
diabetic eye disease
diabetic retinopathy
urinary system disease
connective tissue disease
endocrine neoplasm
Central Nervous System Neo...
brain neoplasm
macular degeneration
nervous system disease
vascular disease
disease of visual system
nervous system disease, dise...
nutritional or metabolic disease
vascular disease, nervous sys..
vascular disease, disease of v..
vascular disease, nervous sys..
endocrine system disease
nervous system disease
nervous system disease
cell proliferation disorder
cell proliferation disorder
cell proliferation disorder
cell proliferation disorder
disease of visual system
vascular disease, nervous sys..
urinary system disease
musculoskeletal or connective..
cell proliferation disorder, end...
cell proliferation disorder, ner...
cell proliferation disorder, ner...
nervous system disease, dise...

Figure 4A

Table 1: Products from **KI-I** and **KI-6**

| ArCOOH | R-NH$_2$ | Coupling conditions | Yield | Deprotection Conditions-A | Yield | Deprotection Conditions-B | Yield | Purification procedure | Final Product (amount, yield[1]) | Comments |
|---|---|---|---|---|---|---|---|---|---|---|
| **KI-1**<br>(12.95 g) | 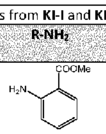 | TCFH (1.2 eq)<br>NMI (5 eq)<br>MeCN | 86% | LiOH (4 eq.)<br>THF-$H_2O$<br>Time = 36 h,<br>T° = 23 °C | 89% | $H_2$, Pd/C<br>DCM/EtOH<br>Time = 48 h,<br>T° = 23 °C | 84% | Trituration with MeCN | **Ia-001a**<br>(13.4 g, 36%) | |
| **KI-1**<br>(53 g) | 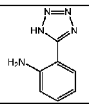 | TCFH (1.2 eq)<br>NMI (5 eq)<br>MeCN | 86% | LiOH (4 eq.)<br>THF-$H_2O$<br>Time = 26 h,<br>T° = 23 °C | Quant. | $H_2$, Pd/C<br>THF/EtOH<br>Time = 18 h,<br>T° = 23 °C | 82% | Trituration with MeCN and $Et_2O$ | **Ia-001aTz**<br>(30 g, 40%) | |
| **KI-1**<br>(1 g) | 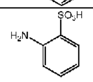 | 1) $SOCl_2$ (27 eq), DMF (drops)<br>2) DIPEA (2.2 eq), DMF | 43% | LiOH (5 eq.)<br>THF-$H_2O$<br>Time = 18 h,<br>T° = 23 °C | 86% | $H_2$, Pd/C<br>EtOAc<br>Time = 18 h,<br>T° = 23 °C | 80% | Normal work up | **Ia-001c**<br>(95 mg, 17%) | |
| **KI-1**<br>(203 mg) | 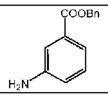 | 1) $SOCl_2$ (49 eq)<br>DCM, 50°C, 4h<br>2) DIPEA (1.1 eq)<br>DCM | 62% | $H_2$, Pd/C<br>DCM/EtOH<br>Time = 1.5h,<br>T° = 23 °C | 57% | NaOH aq (22 eq)<br>2h, then<br>HCl 1N<br>T° = 23 °C | 60% | Filtration after addition of acid and trituration of solid in MeOH | **Ia-002a**<br>(34 mg, 60%) | |
| **KI-1**<br>(1 g) | 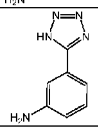 | HATU (1.5 eq)<br>DIPEA (5 eq)<br>DMF | 64% | LiOH (6 eq.)<br>THF-$H_2O$<br>Time = 18 h,<br>T° = 23 °C | 90% | $H_2$, Pd/C<br>EtOAc/EtOH<br>Time = 18 h,<br>T° = 23 °C | 91% | Trituration with MeCN and MTBE | **Ia-002aTz**<br>(285 mg, 30%) | |
| **KI-1**<br>(190 mg)<br>AX050 +<br>LG043 | 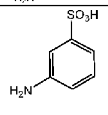 | 1) $SOCl_2$ (50 eq)<br>DCM, 50°C<br>2) NaOH aq. 0.5 M (1.1 eq), $Na_2CO_3$ (0.6 eq), TBAB (0.1 eq), THF, rt, 5 d. | 95% ($Na^-$ salt) | $H_2$, Pd/C<br>$H_2O$/EtOH<br>Time = 2.5h<br>T° = 23 °C | 88% | NaOH aq (18 eq)<br>2h15, then<br>HCl 1N<br>T° = 23 °C | 50% | Filtration after addition of acid; lyophilisation of filtrate affords product | **Ia-002c** | |
| **KI-1Bn**<br>(76 mg) | 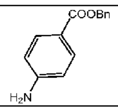 | 1) $SOCl_2$ (50 eq)<br>DCM, 50°C, 3h<br>2) DIPEA (1.1 eq)<br>DCM | 82% | $H_2$, $Pd(OH)_2$<br>DCM/EtOH<br>Time = 4 h,<br>T° = 23 °C | 95% | | | Final product has low solubility in EtOH<br>Purif. Chromatogr. AcOEt/AcOH 95:5 | **Ia-003a**<br>(11 mg, 95%) | Single deprotection step as both precursors are benzyl esters<br>NMR ok |

Figure 4B

| | | | | | | | | | | |
|---|---|---|---|---|---|---|---|---|---|---|
| **KI-1**<br>(1 g) | 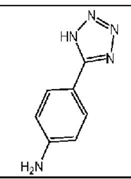 | HATU (1.5 eq)<br>DIPEA (5 eq)<br>DMF | 96% | LiOH (6 eq.)<br>THF-$H_2O$<br>Time = 21 h,<br>T° = 23 °C | 37% | $H_2$, Pd/C<br>EtOAc/EtOH<br>Time = 18 h,<br>T° = 23 °C | 37% | Trituration with MeCN and MTBE | **Ia-003aTz**<br>(0.022 g, 7%) | |
| **KI-1**<br>(118 mg)<br>(LG 038) | 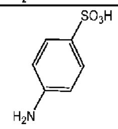 | 1) $SOCl_2$ (58 eq)<br>DCM, 50°C<br>2) NaOH aq. 0.5 M (1.1 eq), $Na_2CO_3$ (0.5 eq), TBAB (0.12 eq), THF, rt. | 69% | $H_2$, Pd/C<br>$H_2O$/EtOH<br>Time = 5.5h<br>T° = 23 °C | 88% | NaOH aq. 0.9 M, 2h, then HCl 37% (3.5 mL) | 82% | Evaporation of solvents and elimination of salts by selective solubilisation in EtOH | **Ia-003c**<br>(45 mg, 82%) | |
| **KI-1**<br>(1 g) | 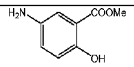 | 1) $SOCl_2$ (27 eq), DMF (drops)<br>2) DIPEA (1.1 eq), DCM, DMF | 24% | LiOH (6 eq.)<br>THF/DMSO-$H_2O$<br>Time = 5 days,<br>T° = 75 °C | 86% | $H_2$, Pd/C<br>DMF/EtOH<br>Time = 24 h,<br>T° = 23 °C | 76% | Trituration with MeCN and $Et_2O$ | **Ia-004a**<br>(0.145 g, 9%) | |
| **KI-1**<br>(1 g) | 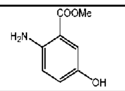 | 1) $SOCl_2$ (27 eq), DMF (drops)<br>2) DIPEA (1.1 eq), DCM, DMF | 33% | LiOH (6 eq.)<br>THF-$H_2O$<br>Time = 18 h,<br>T° = 23 °C | 94% | $H_2$, Pd/C<br>EtOAc<br>Time = 2 h,<br>T° = 23 °C | 84% | Normal work up | **Ia-006a**<br>(0.155 g, 15%) | |
| **KI-1**<br>(1 g) | 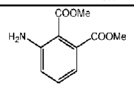 | 1) $SOCl_2$ (27 eq), DMF (drops)<br>2) DIPEA (1.1 eq), DCM. | 75% | LiOH (7 eq.)<br>THF-$H_2O$<br>Time = 18 h,<br>T° = 23 °C | 95% | $H_2$, Pd/C<br>EtOAc/EtOH<br>Time = 4 h,<br>T° = 23 °C | 66% | Normal work up | **Ia-011a**<br>(0.46 g, 27%) | |
| **KI-1**<br>(1 g) | 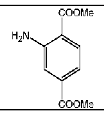 | 1) $SOCl_2$ (27 eq), DMF (drops)<br>2) DIPEA (1 eq), DCM | 57% | LiOH (6 eq.)<br>THF-$H_2O$<br>Time = 20 h,<br>T° = 23 °C | Quant | $H_2$, Pd/C<br>DCM/EtOH<br>Time = 1 h,<br>T° = 23 °C | 88% | Normal work up | **Ia-012a**<br>(0.414 g, 28%) | |
| **KI-1**<br>(1.125 g) | 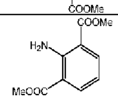 | 1) $SOCl_2$ (27 eq), DMF (drops)<br>2) DIPEA (1.1 eq), DCM | 75% | LiOH (30 eq.)<br>THF-$H_2O$<br>Time = 36 h,<br>T° = 23 °C | Quant | $H_2$, Pd/C<br>DCM/EtOH<br>Time = 18 h,<br>T° = 23 °C | 98% | Normal work up | **Ia-013a**<br>(0.644 g, 42%) | |
| **KI-1**<br>(2.11 g) | 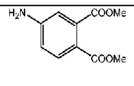 | 1) $SOCl_2$ (27 eq), DMF (drops)<br>2) DIPEA (1.1 eq), DCM | 41% | LiOH (6 eq.)<br>THF-$H_2O$<br>Time = 18 h,<br>T° = 23 °C | Quant | $H_2$, Pd/C<br>DCM/EtOH<br>Time = 18 h,<br>T° = 23 °C | 94% | Normal work up | **Ia-014a**<br>(0.156 g, 22%) | |

Figure 4C

| | | | | | | | | | | |
|---|---|---|---|---|---|---|---|---|---|---|
| KI-1 (1.12 g) | 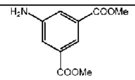 | 1) $SOCl_2$ (50 eq) DCM, 50°C, 4h 2) DIPEA (1.2 eq) DCM – 12h, rt col. chromatogr. PE/AcOEt | | $H_2$, Pd/C DCM/EtOH Time = 18 h, T° = 23 °C | 98% | NaOH aq (65 eq) 2h, rt, then HCl 37% at 0°C, Filtration, drying, wash with aq. HCl, EtOH | 84% | Trituration in $H_2O$, drying | **Ia-015a** (380 mg, 84%) | Product partially soluble in EtOH, avoid EtOH for washings |
| **KI-1** (0.5 g) | 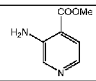 | TCFH (1.2 eq) NMI (5 eq) MeCN | 58% | LiOH (8 eq.) THF-$H_2O$ Time = 42 h, T° = 23 °C | Quant | $H_2$, Pd/C THF/EtOH Time = 18 h, T° = 23 °C | 41% | Trituration with MeCN | **Ia-023a** (0.054 g, 13%) | |
| **KI-1Bn** (84% + 16% diester) | 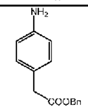 | 1) $SOCl_2$ (25 eq) DCM, 50°C, 4h 2) DIPEA (1.2 eq), DCM – 18h, rt col. chromatogr. PE/DCM 1:1 → DCM | 86% | $H_2$, Pd/C DCM/EtOH Time = 2 h, T° = 23 °C | 99% | | | Trituration with THF | **Ia-032a** (107 mg, 99%) | Single deprotection step as both precursors are benzyl esters |
| **KI-1** (1 g) | 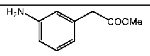 | 1) $SOCl_2$ (27 eq), DMF (drops) 2) DIPEA (1.1 eq), DCM | 73% | $H_2$, Pd/C EtOAc Time = 18 h, T° = 23 °C | 97% | LiOH (8 eq.) THF-$H_2O$ Time = 42 h, T° = 23 °C | 83% | Normal work up | **Ia-033a** (0.147 g, 46%) | |
| **KI-1** (1 g) | 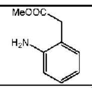 | 1) $SOCl_2$ (27 eq), DMF (drops) 2) DIPEA (1.1 eq), DMF | 39% | LiOH (6 eq.) THF-$H_2O$ Time = 18 h, T° = 23 °C | 85% | $H_2$, Pd/C DCM/EtOH Time = 20 h, T° = 23 °C | 31% | Prep HPLC | **Ia-034a** (0.07 g, 6%) | |
| **KI-1** (1 g) | 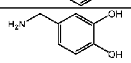 | HOBt (1.5 eq) EDC (1.5 eq) TEA (3.2 eq) DMF | 57% | LiOH (6 eq.) THF-$H_2O$ Time = 18 h, T° = 23 °C | 96% | $H_2$, Pd/C DCM/EtOH Time = 2 h, T° = 23 °C | 92% | Normal work up | **Ia-035a** (0.314 g, 22%) | Benzylation step before hydrolysis: BnBr (2 eq), $K_2CO_3$ (2 eq), DMF. Time = 24 h T° = 23 °C, Y= 79% |
| **KI-1** (1 g) | 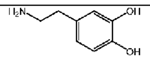 | 1) $SOCl_2$ (26 eq) 2) DIPEA (2.2 eq) DCM | 70% | -- | -- | cHCl (10 mL) AcOH (10 mL) | 31% | Prep HPLC | **Ia-035a-2** (0.081 g, 12%) | |
| **KI-1** (1 g) | 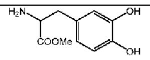 | 1) $SOCl_2$ (28 eq) 2) DIPEA (2.2 eq) DCM | 12% | LiOH (4 eq.) THF-$H_2O$ Time = 18 h, T° = 23 °C | 92% | $H_2$, Pd/C EtOH/EtOAc Time = 20 h, T° = 23 °C | 34% | Prep HPLC | **Ia-035a-3** (0.05 g, 1%) | Benzylation step before hydrolysis: BnBr (4 eq), $K_2CO_3$ (4 eq), DMF. Time = 18 h T° = 23 °C, Y= 99% |
| **KI-1** (0.5 g) | 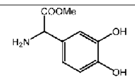 | 1) $SOCl_2$ (25 eq) 2) DIPEA (2.2 eq) DMF | 39% | LiOH (8 eq.) THF-$H_2O$ Time = 18 h, T° = 23 °C | 96% | $H_2$, Pd/C DCM/AcOH Time = 20 h, T° = 23 °C | 24% | Prep HPLC | **Ia-036a** (0.036 g, 2%) | Benzylation step before hydrolysis: BnBr (5 eq), $K_2CO_3$ (8 eq), DMF. Time = 18 h T° = 23 °C, Y= 51% Decomposed after prep purification (80% pure) |

Figure 4D

| | | | | | | | | | | |
|---|---|---|---|---|---|---|---|---|---|---|
| **KI-1**<br>(1 g) | 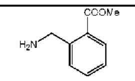 | 1) $SOCl_2$ (27 eq)<br>2) DIPEA (2.2 eq)<br>DCM | 81% | _ | _ | cHCl (15 mL)<br>AcOH (15 mL)<br>Time = 6 h,<br>T° = 80 °C | 44% | Prep HPLC | **Ia-053a**<br>(106 mg, 20%) | |
| **KI-1**<br>(1 g)? | 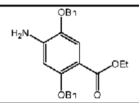 | TCFH (1.2 eq)<br>NMI (5 eq)<br>Aniline (1.1 eq)<br>MeCN | 92% | LiOH (27 eq.)<br>THF-$H_2O$<br>DMSO<br>Time = 120 h,<br>T° = 25 °C | 93% | $H_2$, Pd/C<br>THF/EtOH<br>Time = 18 h,<br>T° = r.t. | 80% | Trituration with MeCN and *n*-pentane | **Ia-056a**<br>(322 mg, 39%) | |
| **KI-1**<br>(35.5 g, 2 eq) | 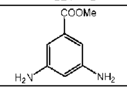 | HATU (3 eq)<br>DiPEA (6 eq)<br>DMF | 87% | LiOH (6 eq.)<br>THF-DMSO-$H_2O$<br>Time = 48 h,<br>T° = 23 °C | 98% | $H_2$, Pd/C<br>DCM/EtOH<br>Time = 18 h,<br>T° = 23 °C | 98% | Trituration with MeCN | **Ib-010a**<br>(19.3 g, 47%) | |
| **KI-6**<br>(500 mg) | 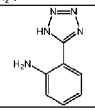 | TCFH (2.4 eq)<br>NMI (6 eq)<br>MeCN | 74% | -- | -- | $H_2$, Pd/C<br>DCM/EtOH<br>Time = 18 h,<br>T° = 23 °C | 41% | Trituration with EtOH and $Et_2O$ | **Ic-001aTz2**<br>(0.191 g, 17%) | |
| **KI-6**<br>(20.3 g) | 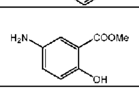 | 1) $SOCl_2$ (40 eq)<br>2) DIPEA (2.2 eq)<br>DCM | 85% | LiOH (6 eq.)<br>DMSO-$H_2O$<br>Time = 18 h,<br>T° = 100 °C | 71% | $H_2$, Pd/C<br>DMF<br>Time = 1 h,<br>T° = 23 °C | quant | Trituration with MeCN | **Ic-007a**<br>(25.7 g, 34%) | |
| **KI-6**<br>(1 g) | 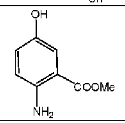 | 1) $SOCl_2$ (23 eq)<br>2) DIPEA (2.2 eq)<br>DCM/DMF | 64% | NaOH (20 eq.)<br>$H_2O$<br>Time = 6 h,<br>T° = 23 °C | 88% | $H_2$, Pd/C<br>DMF<br>Time = 4 h,<br>T° = 23 °C | 24% | Trituration with acetone | **Ic-009a**<br>(41 mg, 8%) | |
| **KI-1**<br>(70 g) | R =<br>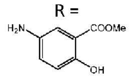<br>R′=<br>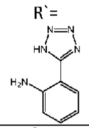 | TCFH (1.2 eq)<br>NMI (5 eq)<br>MeCN | 77% | LiOH (6 eq.)<br>THF-$H_2O$<br>Time = 96 h,<br>T° = 23 °C | 98% | $H_2$, Pd/C<br>THF/EtOH<br>Time = 36 h,<br>T° = 23 °C | 80% | Trituration with $Et_2O$ | **Ic-001aTz/004a**<br>(40.8 g, 34%) | The conditions for first coupling (R′) and hydrolysis have been mentioned in Entry 2 |
| **KI-1**<br>(0.5 g) | 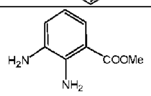 | 1) $SOCl_2$ (28 eq)<br>2) DIPEA (2.2 eq)<br>DMF | 78% | LiOH (7 eq.)<br>THF-$H_2O$<br>Time = 72 h,<br>T° = 23 °C | 60% | $H_2$, Pd/C<br>DCM/EtOH<br>Time = 24 h,<br>T° = 23 °C | 15% | Prep HPLC | **Id-030a**<br>(10 mg, 4%) | Cyclization step:<br>$MeSO_3H$ (3.3 eq),<br>MeOH.<br>Time = 17 h,<br>T° = 70 °C |

Figure 5A

Table 2: Products from **KI-2** and **KI-7**

| ArCOOH | R-NH2 | Coupling conditions | Yield | Deprotection Conditions-A | Yield | Deprotection Conditions-B | Yield | Purification procedure | Final Product (amount, yield) | Comments |
|---|---|---|---|---|---|---|---|---|---|---|
| **KI-2** | 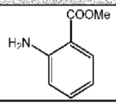 | 1) $SOCl_2$ (32 eq), DMF (drops) 2) DIPEA (1.1 eq), DCM | 72% | – | – | cHCl (10 mL) AcOH (10 mL) Time = 18 h, T° = 80 °C | 64% | Trituration with DCM and MeOH | **IIa-001a** (142 mg, 46%) | |
| **KI-2** | 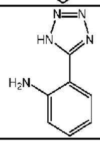 | TCFH (1.2 eq) NMI (5 eq) MeCN | 71% | LiOH (6 eq.) THF-$H_2O$ Time = 48 h, T° = r.t. | 77% | $H_2$, Pd/C THF/EtOH Time = 18 h, T° = r.t. | 53% | Trituration with MeCN, DCM and $Et_2O$ | **IIa-001aTz** (125 mg, 29%) | |
| **KI-2** | 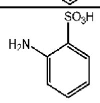 | 1) $SOCl_2$ (70 eq), DMF (drops) 2) DIPEA (2.2 eq), DMF | 70% | LiOH (4 eq.) THF-$H_2O$ Time = 72 h, T° = r.t. | 15% | $H_2$, Pd/C EtOH Time = 18 h, T° = r.t. | 54% | Trituration with MeCN | **IIa-001c** (20 mg, 4%) | |
| **KI-2Ac₂** 675 mg | 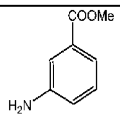 | 1. $SOCl_2$, DCM, reflux, 5h, conc. 2. DCM, amine (1 eq), $IprNEt_2$ (1,1 eq), 12h, rt. Extract. column chromatogr | 15% | EtOH, NaOH 1M, 0ºC then 2h, rt. Acidification, conc. | 55% | Not needed | | | **IIa-002a** | Compound made from the diacetate o 2,5-dihydroxyiso-phtalic acid methyl ester |
| **KI-2Ac₂** 500 mg | 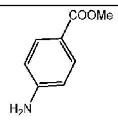 | 1. $SOCl_2$, DCM, reflux, 5h, conc. 2. DCM, amine (1.1 eq), $iprNEt_2$ (1,1 eq 12h rt; extraction, column chromat. | 16% | EtOH, NaOH 1M, 0ºC then 2h, rt. Acidification, conc. | 69% | Not needed | | | **IIa-003a** | Compound made from the diacetate o 2,5-dihydroxyiso-phtalic acid methyl ester |
| **KI-2Ac₂** | 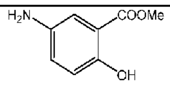 | 1) $SOCl_2$ (30 eq), DMF (drops) 2) DIPEA (1.1 eq), DCM | 75% | – | – | cHCl (10 mL) AcOH (10 mL) Time = 18 h, T° = 80 °C | 61% | Normal work-up | **IIa-004a** (136 mg, 45%) | Compound from KI-2Ac₂ |
| **KI-2** | 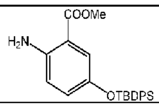 | 1) $SOCl_2$ (26 eq), DMF (drops) 2) Pyridine (1.2 eq), DCM | 72% | LiOH (6 eq.) THF-$H_2O$ Time = 24 h, T° = r.t. | 95% | $H_2$, Pd/C EtOH/EtOAc Time = 24 h, T° = r.t. | 71% | Normal work-up | **IIa-006a** (209 mg, 51%) | The starting aniline was protected with a *tert*-Butyldiphenyl-silyl group |

Figure 5B

| | | | | | | | | | | |
|---|---|---|---|---|---|---|---|---|---|---|
| **KI-2Ac$_2$** | 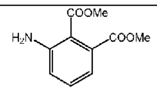 | 1) $SOCl_2$ (32 eq), DMF (drops) 2) DIPEA (2.2 eq), DCM | 70% | _ | _ | NaOH aq (60 eq) 6.5h, then HCl 1N T° = r.t. | 32% | Aqueous work-up then Prep HPLC | **IIa-011a** (130 mg, 22%) | Compound from KI-2Ac$_2$ |
| **KI-2Ac$_2$** | 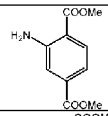 | 1) $SOCl_2$ (40 eq), DMF (drops) 2) DIPEA (1.1 eq), DCM | 33% | _ | _ | cHCl (10 mL) AcOH (10 mL) Time = 18 h, T° = 80 °C | 29% | Prep HPLC | **IIa-012a** (58 mg, 9%) | Compound from KI-2Ac$_2$ |
| **KI-2Ac$_2$** | 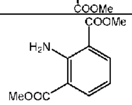 | 1) $SOCl_2$ (32 eq), DMF (drops) 2) DIPEA (1.1 eq), DCM | 34% | LiOH (12 eq.) THF-$H_2O$ Time = 96 h, T° = r.t. | 74% | $H_2$, Pd/C EtOH/EtOAc Time = 24 h, T° = r.t. | 95% | Normal work-up | **IIa-013a** (58 mg, 23%) | The amide coupling was performed from the KI-2Ac$_2$, followec by quantitative methanolysis and subsequent re-protection with benzyl groups. |
| **KI-2** | 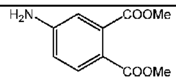 | 1) $SOCl_2$ (61 eq), DMF (drops) 2) DIPEA (1.1 eq), DCM | 59% | LiOH (6 eq.) THF-$H_2O$ Time = 48 h, T° = r.t. | 93% | $H_2$, Pd/C EtOH/EtOAc Time = 18 h, T° = r.t. | 77% | Trituration with MeCN | **IIa-014a** (170 mg, 42%) | |
| **KI-2Ac$_2$** 675 mg | 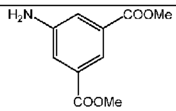 | 1. $SOCl_2$, (25 eq.), DCM, reflux, 5h, conc. 2. DCM, amine (1.0 eq), IprNEt$_2$ (1,1 eq) 12h rt; extraction, chrom. | 12% | EtOH, NaOH 1M, 0°C then 2h, rt. Acidification, conc. | 77% | Not needed | | | **IIa-015a** | Compound made from the diacetate o 2,5-dihydroxyiso-phtalic acid methyl ester |
| **KI-2Ac$_2$** | 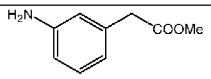 | 1) $SOCl_2$ (32 eq), DMF (drops) 2) DIPEA (1.1 eq), DCM | 55% | _ | _ | cHCl (9 mL) AcOH (9 mL) Time = 24 h, T° = 100 °C | 34% | Prep HPLC | **IIa-033a** (91 mg, 34%) | |
| **KI-2** | 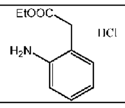 | 1) $SOCl_2$ (25 eq), DMF (drops) 2) DIPEA (2.2 eq), DMF | 35% | LiOH (8 eq.) THF-$H_2O$ Time = 6 h, T° = r.t. | 97% | $H_2$, Pd/C DCM/EtOAc Time = 24 h, T° = r.t. | 26% | Trituration with MeCN | **IIa-034a** (37 mg, 8%) | |

Figure 5C

| | | | | | | | | | | |
|---|---|---|---|---|---|---|---|---|---|---|
| **KI-2** | 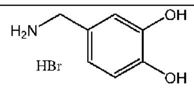 | 1) $SOCl_2$ (25 eq), DMF (drops) 2) DIPEA (2.2 eq), DMF 3)cHCl (5 mL) | 97% | LiOH (4 eq.) THF-$H_2O$ Time = 24 h, T° = r.t. | 56% | $H_2$, Pd/C DCM/EtOH Time = 24 h, T° = r.t. | 59% | Prep HPLC | **IIa-035a** (90 mg, 13%) | The amide coupling was performed from the KI-2Ac$_2$, followec by de-acetylation with cHCl in the work-up then subsequent protection with 4 benzyl protecting groups (43% yield) prior to LiOH hydrolysis. |
| **KI-2Ac$_2$** | 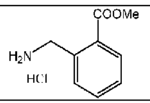 | 1) $SOCl_2$ (40 eq), DMF (drops) 2) DIPEA (2.2 eq), DMF | 78% | _ | _ | cHCl (19 mL) AcOH (19 mL) Time = 17 h, T° = 80 °C | 24% | Prep HPLC | **IIa-053a** (104 mg, 24%) | From KI-2Ac$_2$ |
| **KI-2Ac$_2$** | 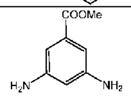 | 1) $SOCl_2$ (40 eq), DMF (drops) 2) DIPEA (1.1 eq), Aniline (0.5 eq.), DCM | 22% | LiOH (8 eq.) THF-$H_2O$ Time = 24 h, T° = r.t | 34% | $H_2$, Pd/C THF/EtOH Time = 24 h, T° = r.t. | 17% | Prep HPLC | **IIb-010a** (8 mg, 5%) | The amide coupling was performed from the KI-2Ac$_2$, followec by methanolysis of the protecting acetyl (98% yield) and subsequent protection with 4 benzyl protecting groups (40% yield) prior to LiOH hydrolysis. |
| **KI-7** | 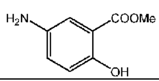 | TCFH (2.4 eq) NMI (10 eq) Aniline (2.4 eq) MeCN | Quant. | LiOH (6 eq.) THF-$H_2O$ Time = 24 h, T° = 80 °C | 70% | $H_2$, Pd/C THF Time = 24 h, T° = r.t. | 88% | Trituration with MeCN and $Et_2O$ | **IIc-007a** (5.7 g, 61%) | |
| **KI-7** | 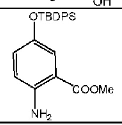 | 1) $SOCl_2$ (50 eq), DMF (drops) 2) Pyridine (2.2 eq), Aniline (2.2 eq.), DCM | 76% | LiOH (6 eq.) THF-$H_2O$ Time = 24 h, T° = r.t. | 95% | $H_2$, Pd/C EtOAc/EtOH Time = 24 h, T° = r.t. | 3% | Purified twice by Prep | **IIc-009a** (11 mg, %) | The starting aniline was protected with a *tert*-Butyldiphenylsilyl (TBDPS) group. |

Figure 6

Table 3: Products from **KI-8** and **KI-9**

| ArCOOH | R-NH$_2$ | Coupling conditions | Yield | Deprotection Conditions-A | Yield | Deprotection Conditions-B | Yield | Purification procedure | Final Product (amount, yield) | Comments |
|---|---|---|---|---|---|---|---|---|---|---|
| **KI-8** | 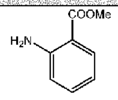 | 1) $(COCl)_2$ (6 eq), DMF (drops), DCM<br>2) DIPEA (1.1 eq), Aniline (1.1 eq), DCM | 75% | LiOH (6 eq.)<br>THF-$H_2O$<br>Time = 24 h,<br>T° = r.t. | 80% | $H_2$, Pd/C<br>DCM/EtOAc<br>Time = 24 h,<br>T° = r.t. | 51% | Trituration with MeCN | **IIIa-001a** | |
| **KI-8** | 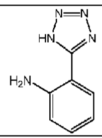 | TCFH (1.2 eq)<br>NMI (5 eq)<br>Aniline (1.2 eq)<br>MeCN | 75% | LiOH (6 eq.)<br>THF-$H_2O$<br>Time = 18 h,<br>T° = 70 °C | 93% | $H_2$, Pd/C<br>DCM/EtOH<br>Time = 18 h,<br>T° = r.t. | 43% | Trituration with MeCN | **IIIa-001aTz**<br>(27 mg, 29%) | |
| **KI-8** | 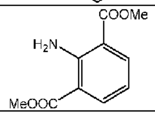 | TCFH (1.2 eq)<br>NMI (5 eq)<br>Aniline (1.2 eq)<br>MeCN | 29% | LiOH (16 eq.)<br>THF-$H_2O$<br>Time = 48 h,<br>T° = r.t. | Quant. | $H_2$, Pd/C<br>DCM/EtOH<br>Time = 18 h,<br>T° = r.t. | Quant. | Trituration with $Et_2O$ | **IIIa-013a**<br>(50 mg, 29%) | |
| **KI-8** | 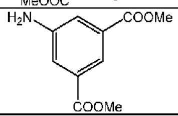 | TCFH (1.2 eq)<br>NMI (5 eq)<br>Aniline (1.2 eq)<br>MeCN | 71% | LiOH (8 eq.)<br>THF-$H_2O$<br>Time = 18 h,<br>T° = r.t. | 90% | $H_2$, Pd/C<br>DCM/EtOH<br>Time = 18 h,<br>T° = r.t. | 26% | Prep HPLC | **IIIa-015a**<br>(26 mg, 16%) | |
| **KI-8** | 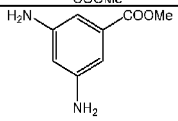 | TCFH (1.2 eq)<br>NMI (3 eq)<br>Aniline (0.5 eq)<br>MeCN | 75% | LiOH (6 eq.)<br>THF-$H_2O$,<br>DMSO<br>Time = 72 h,<br>T° = 55 °C | 85% | $H_2$, Pd/C<br>THF/EtOH<br>Time = 18 h,<br>T° = r.t. | 57% | The residue was dissolved into water, filtered and the water was removed in a freeze-drier | **IIIb-010a**<br>(755 mg, 57%) | |

Figure 7

Table 4: Products from **KI-8** and **KI-9**

| | SUBSTRATE | Deprotection Conditions-A | Yield | Deprotection Conditions-B | Yield | Purification procedure | Final Product (amount, yield) | Comments |
|---|---|---|---|---|---|---|---|---|
| Deprotection Procedures | | | | | | | | |
| | 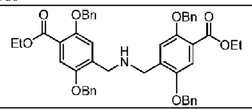 | LiOH (6 eq.)<br>THF-$H_2O$<br>Time = 72 h,<br>T° = 70 °C | Quant. | $H_2$, Pd/C<br>DCM/EtOH<br>Time = 18 h,<br>T° = r.t. | 50% | Trituration with MeCN | **IIIc-056a**<br>(44 mg, 50%) | The product may possibly be the HCl salt (HCl/Dioxane tes didn't revealed protons shifts) |
| | 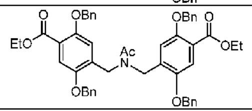 | LiOH (15 eq.)<br>THF-$H_2O$<br>Time = 96 h,<br>T° = 40 °C | Quant. | $H_2$, Pd/C<br>DCM/EtOH<br>Time = 48 h,<br>T° = r.t. | 67% | Trituration with $Et_2O$ | **IIIc-057a** | |
| | 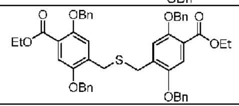 | Same conditions | | Same conditions | | | **IIIc-058a** | |
| | 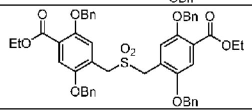 | Same conditions | | Same conditions | | | **IIIc-059a** | |
| | 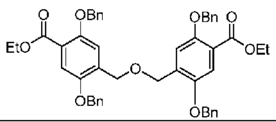 | LiOH (6 eq.)<br>THF-$H_2O$<br>Time = 24 h,<br>T° = 70 °C | Quant. | $H_2$, Pd/C<br>DCM<br>Time = 18 h,<br>T° = r.t. | 55% | Prep HPLC | **IIIc-060a**<br>(39 mg, 55%) | |
| | 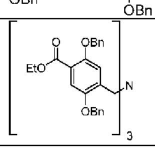 | LiOH (18 eq.)<br>THF-$H_2O$<br>Time = 96 h,<br>T° = 80 °C | 87% | $H_2$, Pd/C<br>THF/EtOH<br>Time = 18 h,<br>T° = r.t. | 73% | Trituration with water/acetone | **IIIc-061a**<br>(9.1 g, | Large scale synthesis |

Figure 8

Table 5:

| ArCOOH | R-NH2 | Coupling conditions | Yield | Deprotection Conditions-A | Yield | Deprotection Conditions-B | Yield | Purification procedure | Final Product (amount, yield) | Comments |
|---|---|---|---|---|---|---|---|---|---|---|
| 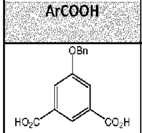 | 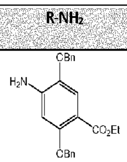 **Aniline A** | TCFH (4.2 eq)<br>NMI (9 eq)<br>MeCN | 58% | LiOH (8 eq.)<br>DMSO-$H_2O$<br>Time = 18 h,<br>T° = 100 °C | 42% | $H_2$, Pd/C<br>THF/EtOH<br>Time = 18 h,<br>T° = 23 °C | 88% | Trituration with MeCN and $Et_2O$ | **V-001a**<br>(110 mg, 21%) | |
| 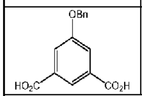 | 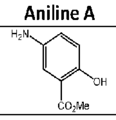 | TCFH (2.2 eq)<br>NMI (6 eq)<br>MeCN | 76% | LiOH (10 eq.)<br>THF-$H_2O$<br>Time = 48 h,<br>T° = r.t. | 77% | $H_2$, Pd/C<br>THF/EtOH<br>Time = 48 h,<br>T° = r.t. | 90% | Trituration with $Et_2O$ | **V-002a**<br>(513 mg, 52%) | |
| Deprotection Procedures | | | | | | | | | | |
| | 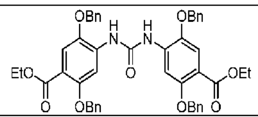 | | | LiOH (20 eq.)<br>THF-$H_2O$<br>Time = 168 h,<br>T° = from r.t to 80 °C | 76% | $H_2$, Pd/C<br>THF/EtOH<br>Time = 72 h,<br>T° = r.t. | 67% | Trituration with $Et_2O$ | **V-003a**<br>(48 mg, 50%) | |

Figure 9

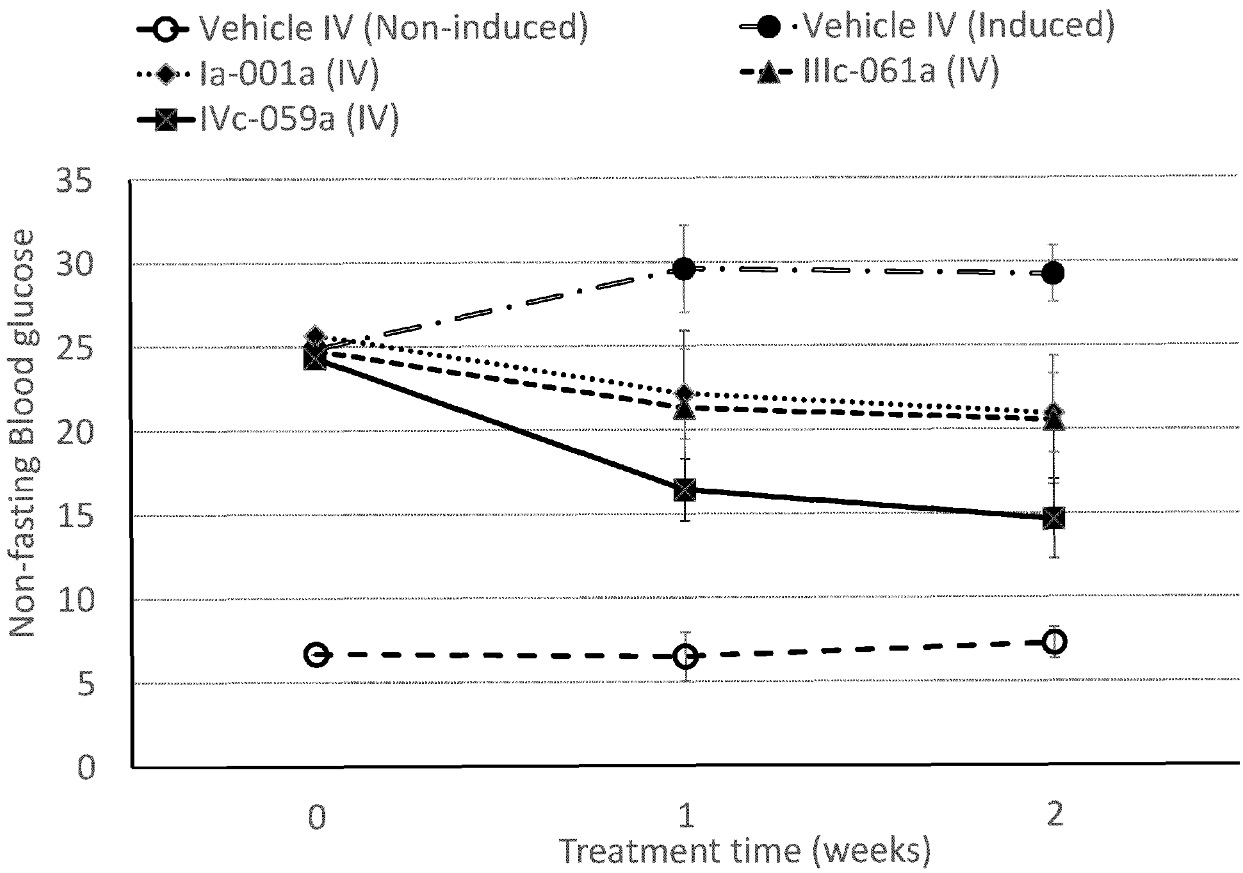
Vehicle IV (Non-induced)
Vehicle IV (Induced)
Ia-001a (IV)
IIIc-061a (IV)
IVc-059a (IV)
35
30
25
20
15
10
5
0
Non-fasting Blood glucose
0
1
2
Treatment time (weeks)

Figure 10

## Day 7 of treatment

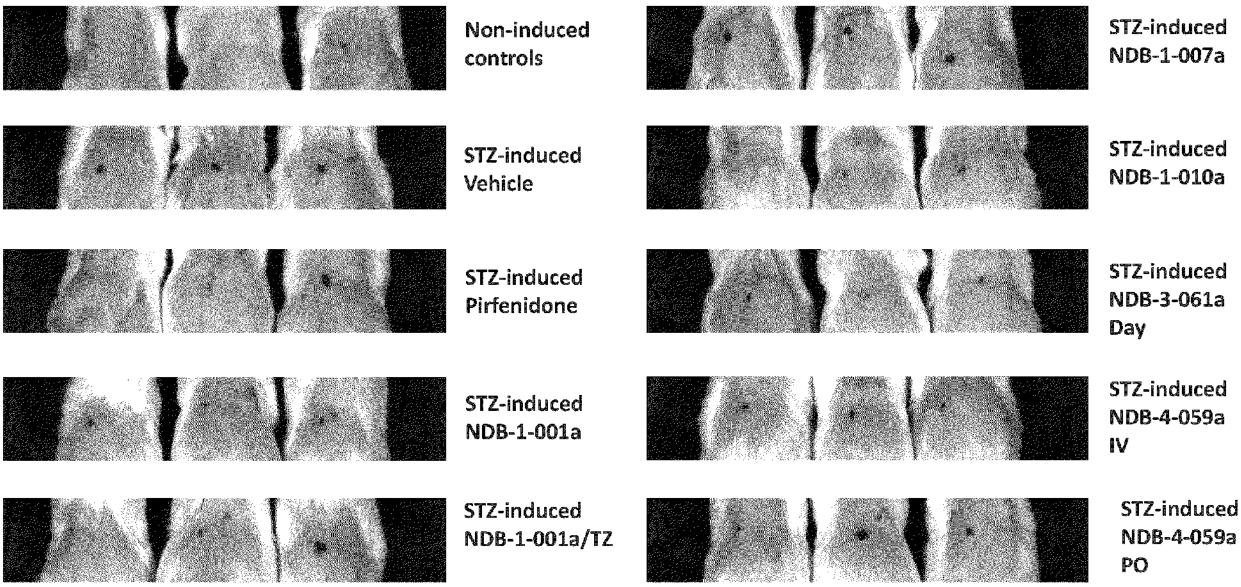

Europäisches Patentamt
European Patent Office
Office européen des brevets

# EUROPEAN SEARCH REPORT

Application Number
EP 20 16 2558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/139632 A1 (ALMSTEAD NEIL G [US] ET AL) 12 June 2008 (2008-06-12) * table page 24, 4th compound; claims 4, 7, 8, 13, 17; example 5 * | 1,11,14, 15 | INV. C07C235/64 C07C66/00 C07C229/38 C07C233/51 C07C275/42 C07C309/51 C07C317/46 C07C323/56 C07D213/79 C07D235/18 C07D257/04 A61K31/196 A61P3/00 A61P35/00 A61P37/00 |
| X | US 2013/172333 A1 (JAIN NEERA [US] ET AL) 4 July 2013 (2013-07-04) * paragraphs [0028], [0114]; claim 1; table 1; compound 99 * | 1,11,14, 15 | |
| X | STEFANI V ET AL: "Synthesis of proton-transfer fluorescent dyes: 2,5-bis(2'-benzazolyl)hydroquinones and related compounds", DYES AND PIGMENTS, vol. 20, no. 2, 1992, pages 97-107, XP000324199, ISSN: 0143-7208, DOI: 10.1016/0143-7208(92)85003-5 * scheme 1, compounds 3, 4, Me-4 * | 1,12 | |
| X | US 2016/269696 A1 (CLABAU FRÉDÉRIC [FR] ET AL) 15 September 2016 (2016-09-15) * examples 2, 4 * | 1,12 | TECHNICAL FIELDS SEARCHED (IPC) C07C C07D |
| X | US 4 208 328 A (LAVALLEE FRANCOIS A [US] ET AL) 17 June 1980 (1980-06-17) * column 1, lines 40-51; claim 1; example 2 * | 1,12 | |
| | -/-- | | |

The present search report has been drawn up for all claims

1

| Place of search | Date of completion of the search | Examiner |
|---|---|---|
| Munich | 5 August 2020 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 2558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ITAMI KENICHIRO ET AL: "Chiral Benzoquinones as a New Class of Ligands for Asymmetric Catalysis: Synthesis and Application to the Palladium(II)-Catalyzed 1,4-Dialkoxylation of 1,3-Dienes", THE JOURNAL OF ORGANIC CHEMISTRY, vol. 63, no. 19, 1998, pages 6466-6471, XP055719911, ISSN: 0022-3263, DOI: 10.1021/jo980561x<br>* table 1; compound 6f *<br>----- | 1,5 | |
| | | | TECHNICAL FIELDS SEARCHED (IPC) |

The present search report has been drawn up for all claims

1

| Place of search | Date of completion of the search | Examiner |
|---|---|---|
| Munich | 5 August 2020 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 16 2558

This annex lists the patent family members relating to the patent documents cited in the above-mentioned European search report. The members are as contained in the European Patent Office EDP file on
The European Patent Office is in no way liable for these particulars which are merely given for the purpose of information.

05-08-2020

| Patent document cited in search report | Publication date | Patent family member(s) | Publication date |
|---|---|---|---|
| US 2008139632 A1 | 12-06-2008 | EP 2068871 A1 | 17-06-2009 |
| | | US 2008139632 A1 | 12-06-2008 |
| | | US 2012087896 A1 | 12-04-2012 |
| | | WO 2008130370 A1 | 30-10-2008 |
| US 2013172333 A1 | 04-07-2013 | US 2013172333 A1 | 04-07-2013 |
| | | US 2015150851 A1 | 04-06-2015 |
| | | WO 2011146801 A1 | 24-11-2011 |
| US 2016269696 A1 | 15-09-2016 | BR 112016007029 A2 | 01-08-2017 |
| | | CA 2925180 A1 | 30-04-2015 |
| | | CN 105658427 A | 08-06-2016 |
| | | EA 201690829 A1 | 31-08-2016 |
| | | EP 3060395 A1 | 31-08-2016 |
| | | FR 3012070 A1 | 24-04-2015 |
| | | JP 6600305 B2 | 30-10-2019 |
| | | JP 2017503737 A | 02-02-2017 |
| | | KR 20160074578 A | 28-06-2016 |
| | | US 2016269696 A1 | 15-09-2016 |
| | | WO 2015059386 A1 | 30-04-2015 |
| US 4208328 A | 17-06-1980 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6147112 A
- US 3509207 A
- WO 2018160618 A1
- JP S5126839 A
- US 3973038 A


### Non-patent literature cited in the description


- **RICHTER et al.** *Synthesis,* 1976, vol. 1976 (3), 192-194
- **AKDIS M. et al.** *J Allergy Clin Immunol,* 2016, vol. 138, 984-1010
- **DENISE CARVALHO et al.** *Nucleic Acids Research,* 08 January 2019, vol. 47 (D1), D1056-D1065, https://doi.org/10.1093/nar/gky 1133
- **SAAL C.** Pharmaceutical salts: A summary on doses of salt formers from the Orange Book. *European Journal of Pharmaceutical Sciences,* 2013, vol. 49, 614-623
- Remington's Pharmaceutical Sciences. Mack Publishing Company, 1985, 1418
- The use of interspecies scaling in toxicokinetics. **MORDENTI, J. ; CHAPPELL, W. et al.** Toxicokinetics and New Drug Development. Pergamon Press, 1989, 42-96
- Remington's Pharmaceutical Sciences. Mack Publishing Company, 1990
- Handbook of Pharmaceutical Excipients. American Pharmacists Association, 2006
- Ullmann's Encyclopedia of Industrial Chemistry. Marcel Dekker, 1989
- **ANSEL et al.** Pharmaceutical Dosage Forms and Drug Delivery Systems. WILLIAMS & WILKINS, 1994
- **DENISE CARVALHO et al.** *Nucleic Acids Research,* 08 January 2019, vol. 47 (D1), D1056-D1065, https://www.targetvalidation.org
- **RODRIGUEZ et al.** *Synth. Comm.,* 1998, vol. 28, 2259-69
- **ZHONG et al.** *Chem Eur. J.,* 2019, vol. 25, 8177-8169
- **NUDENBERG et al.** *J. Org. Chem.,* 1943, vol. 8, 500-508
- **KORFF ; AUGUSTIN.** *J Cell Sci,* 1999, vol. 112, 3249-58
- **KORFF ; AUGUSTIN.** *J Cell Biol,* 1998, vol. 143, 1341-52
- **BOGDANOV et al.** *PLoS One,* 2014, vol. 9, e97302
- **HERNÁNDEZ et al.** *Diabetes,* 2016, vol. 65, 172-187
- **HERNANDEZ et al.** *Diabetologia,* 2017, vol. 60, 2285-2298
- **COOK AD et al.** *J Immunol.,* 2003, vol. 171 (9), 4816-4823
- **TSAI JM et al.** *Blood Adv.,* 2019, vol. 3 (18), 2713-2721
- **LI C et al.** *Cardiovasc Diabetol. 2019,* 02 February 2019, vol. 18 (1), 15
- **ZHANG Y et al.** *J Diabetes Res.,* 2016, vol. 2016, 5782904
- **ZHENG et al.** *Acta Derm Venereol,* 2015, vol. 95, 952-958
- **FRUCHON S et al.** *Molecules,* 05 August 2013, vol. 18 (8), 9305-9316
- **KAMBIZ S. et al.** *PLoS One,* 2015, vol. 10 (6), e0131144
- *PLoS One,* 18 May 2015, vol. 10 (5), e0126892
- **PHαM BT.** *Physiol Rep.,* 2015, vol. 3 (4), e12323
- **WANG D et al.** *J Ovarian Res. 2018,* 10 January 2018, vol. 11 (1), 6
- **HOHENESTER S et al.** *Cells,* 2020, vol. 9 (2), 281
- **FRANSÉN PETTERSSON et al.** *PLoS One,* 2018, vol. 13 (9), e0203228
- **BARBARA ULMASOV B. et al.** *et al. (Hepatology Communications,* 2018, vol. 3 (2, https://doi.org/10.1002/hep4.1298
- **LEE EC et al.** *Nat Commun,* 2019, vol. 10, 4148
- **RYU SH et al.** *Oncotarget,* 2016, vol. 7 (13), 15554-15565
- **FANG Y. et al.** *Faseb Journal,* 2020
- **HOU H et al.** *Ophthalmic Res,* 2018, vol. 60, 195-204
- **MÁRKUS B. et al.** *FEBS Open Bio. 2017,* 29 June 2017, vol. 7 (8), 1166-1177
- **MATSUDA Y et al.** *Mol Ther Nucleic Acids,* 2019, vol. 17, 819-828

- **WEI-DA CHIO et al.** *Life Sci J,* 2019, vol. 16 (12), ISSN 1097-8135, 115-126
- **MCCARRON A et al.** *Respir Res. 2018,* 02 April 2018, vol. 19 (1), 54
- **CHEN SS et al.** *J Thorac Dis.,* 2017, vol. 9 (1), 96-105
- **CHEN SS.** *J Thorac Dis,* 2017, vol. 9 (1), 96-105